# EUROPEAN PATENT APPLICATION

(11) **EP 2 277 900 A1**
(43) Date of publication of application: **26.01.2011**
(21) Application number: 09734697.7
(22) Date of filing: 23.04.2009
(51) Int. Cl.: C07K 14/47, A61K 38/00, A61P 3/06, A61P 3/10, A61P 5/24, A61P 15/08, A61P 25/28, A61P 35/00, A61P 35/04, A61P 43/00, C07K 7/06

(54) **METASTIN DERIVATIVE AND USE THEREOF**

(30) Priority: 24.04.2008 JP 2008114480
(71) Applicant: Takeda Pharmaceutical Company Limited, Osaka 541-0045 (JP)
(72) Inventor: ASAMI, Taiji, Tsukuba-shi Ibaraki 300-4293 (JP); NISHIZAWA, Naoki, Tsukuba-shi Ibaraki 300-4293 (JP)
(74) Representative: Held, Stephan
(86) International application number: PCT/JP2009/058102
(87) International publication number: WO 2009/131191

(57) **Abstract**

The invention provides a stable metastin derivative having excellent biological activities (a cancer metastasis-inhibiting activity, a cancer proliferation-inhibiting activity, a gonadotropin secretion-promoting activity, a sex hormone secretion-promoting activity, etc.). By replacing the constituent amino acids of metastin with specific amino acids in the metastin derivative of the present invention, the blood stability and solubility of the metastin derivative can be more improved, the gel tendency of the metastin derivative can be reduced, the pharmacokinetics of the metastin derivative can be improved, and the metastin derivative can exhibit an excellent cancer metastasis-inhibiting activity and cancer proliferation-inhibiting activity. The metastin derivative can also exhibit a gonadotropin secretion-inhibiting activity, a sex hormone secretion-inhibiting activity, etc.

## Description

### TECHNICAL FIELD

The present invention relates to metastin derivatives and use thereof.

### BACKGROUND ART

Human-derived metastin (also known as KiSS-1 peptide) (Patent Document 1) and mouse or rat-derived metastin (Patent Document 2) are known. Sustained release preparations containing metastin are also known (Patent Document 3).

Metastin has an effect of suppressing cancer metastasis and is thus effective for preventing or treating cancers (for example, lung cancer, gastric cancer, liver cancer, pancreatic cancer, colorectal cancer, rectal cancer, colonic cancer, prostate cancer, ovarian cancer, cervical cancer, breast cancer, renal cancer, bladder cancer, brain tumor, etc.); metastin also has an effect of controlling the pancreatic function and is effective for preventing or treating pancreatic diseases (e.g., acute or chronic pancreatitis, pancreatic cancer, etc.); and metastin further has an effect of controlling the placental function and is effective for preventing or treating choriocarcinoma, hydatid mole, invasive mole, miscarriage, fetal hypoplasia, abnormal glucose metabolism, abnormal lipid metabolism or abnormal delivery (Patent Documents 1 to 3).
Patent Document 1: WO 00/24890
Patent Document 2: WO 01/75104
Patent Document 3: WO 02/85399

### DISCLOSURE OF INVENTION

An object of the present invention is to provide stable metastin derivatives having excellent biological activities (a cancer metastasis suppressing activity, a cancer growth suppressing activity, a gonadotropic hormone secretion stimulating activity, a sex hormone secretion stimulating activity, a gonadotropic hormone secretion suppressing activity, a sex hormone secretion suppressing activity, etc.).

The present inventors have made extensive studies to solve the foregoing problems and as a result, have found that by substituting the constituent amino acids of metastin with specific amino acids, unexpectedly the blood stability, solubility, etc. are more improved, the gel tendency is reduced, the pharmacokinetics are also improved, and such metastin derivatives show an excellent cancer metastasis suppressing activity or a cancer growth suppressing activity. The present inventors have further found that these metastin derivatives unexpectedly exhibit an effect of suppressing the gonadotropic hormone secretion, an effect of suppressing the sex hormone secretion, etc., which are totally different from the effects known so far. Based on these findings, the present inventors have continued further investigations and come to accomplish the present invention.

More specifically, the present invention provides the following features, and so on.
[1] A metastin derivative represented by formula:
   XX0-XX2-XX3-XX4-XX5-XX6-AzaGly-XX8-XX9-XX10-NH₂
   wherein:
   XX0 represents hydrogen, acetyl (Ac), mesyl (Ms), 4-fluorobenzoyl (4F-Benzoyl), decanoyl (Decanoyl), benzylureido (Bn-ureido), ureido, amidino (Amidino), dimethylaminoethylureido (Dimethylaminoethylureido) or isobutylureido (iso-Butylureido);
   XX2 represents Tyr, D-Tyr, D-Ala, D-Leu, D-Phe, D-Lys, D-Trp or a chemical bond;
   XX3 represents:
      i) an amino acid selected from Ala, Arg, Asn, Asp, Cys, Gln, Glu, Gly, His, Ile, Leu, Lys, Met, Phe, Ser, Thr, Trp, Tyr and Val, wherein the α-amino group may optionally be methylated;
      ii) a cyclic amino acid selected from Pro, Aze(2), Aze(3), Pic(2), Pic(3), Hyp, Thz, Abz(2), Abz(3), Pzc(2), Pro(4NH₂), Hyp(Bzl), cisHyp, Pro(4F) and lzc;
      iii) an amino acid selected from D-Pya(4), DL-Ala(Pip), Om, Aib and Tyr(PO₃H₂); or,
      iv) a chemical bond;
   XX4 represents Asn, 2-amino-3-ureidopropionic acid, Nβ-formyl-β-diaminopropionic acid, Nβ-acetyl-β-diaminopropionic acid, Nω-pentylasparagine, Nω-cyclopropylasparagine, Nω-benzylasparagine, 2,4-diaminobutanoic acid, 2,3-diaminopropionic acid, His, Gln, Gly, Arg, Cit, Nva, D-Asn or a chemical bond;
   XX5 represents Ser, Thr, Val, NMeSer, Gly, Ala, Hyp, D-Ala, D-Thr, D-Pro or a chemical bond;
   XX6 represents Phe, Tyr, Trp, Tyr(Me), Thi, Nal(2), Cha, Pya(4), threo-Ser(3Phenyl), erythro-Ser(3Phenyl) or an optionally substituted phenylalanine;
   AzaGly represents azaglycine;
   XX8 represents Leu, Nva, Val or Ala(cPr);
   XX9 represents an optionally substituted arginine, an optionally substituted lysine or an optionally substituted ornithine; and,
   XX10 represents 2-naphthylalanine, 2-thienylalanine, tyrosine, an optionally substituted phenylalanine or an optionally substituted tryptophan;
   or a salt thereof.
[1a] The metastin derivative or a salt thereof according to [1] above, which is represented by the formula:
   XX0-XX2-XX3 -XX4-XX5-XX6-AzaGly-XX8-XX9-XX10-NH₂
   wherein:
   XX0 represents hydrogen, acetyl, mesyl, 4-fluorobenzoyl, decanoyl, benzylureido, ureido, amidino, dimethylaminoethylureido or isobutylureido;
   XX2 represents D-Tyr;
   XX3 represents Glu or Hyp in which the α-amino group may optionally be methylated;
   XX4 represents Asn or 2-amino-3-urcidopropionic acid;
   XX5 represents Thr;
   XX6 represents Phe or Cha;
   AzaGly represents azaglycine;
   XX8 represents Leu or Ala(cPr);
   XX9 represents an optionally substituted arginine; and,
   XX10 represents an optionally substituted tryptophan.
[2] The metastin derivative or a salt thereof according to [1] above, wherein:
   XX0 represents hydrogen, acetyl, mesyl, 4-fluorobenzoyl, decanoyl, benzylureido, ureido, amidino, dimethylaminoethylureido or isobutylureido;
   XX2 represents D-Tyr;
   XX3 represents Glu, Hyp or Pro(4F);
   XX4 represents Asn or 2-amino-3-ureidopropionic acid;
   XX5 represents Thr;
   XX6 represents Phe or Cha;
   AzaGly represents azaglycine;
   XX8 represents Leu or Ala(cPr);
   XX9 represents an arginine optionally substituted with methyl; and,
   XX10 represents tryptophan.
[3] Ms-D-Tyr-Hyp-Asn-Thr-Phe-AzaGly-Leu-Arg(Me)-Trp-NH₂ (Compound No. 963),
   4-fluorobenzoyl-D-Tyr-Hyp-Asn-Thr-Phe-AzaGly-Leu-Arg(Me)-Trp-NH₂ (Compound No. 964),
   decanoyl-D-Tyr-Hyp-Asn-Thr-Phe-AzaGly-Leu-Arg(Me)-Trp-NH₂ (Compound No. 965),
   benzylureido-D-Tyr-Hyp-Asn-Thr-Phe-AzaGly-Leu-Arg(Me)-Trp-NH₂ (Compound No. 966),
   ureido-D-Tyr-Hyp-Asn-Thr-Phe-AzaGly-Leu-Arg(Me)-Trp-NH₂ (Compound No. 967),
   Ac-D-Tyr-Hyp-Asn-Thr-Cha-AzaGly-Leu-Arg(Me)-Trp-NH₂ (Compound No. 969),
   Ac-D-Tyr-Pro(4F)-Asn-Thr-Cha-AzaGly-Leu-Arg(Me)-Trp-NH₂ (Compound No. 970),
   amidino-D-Tyr-Hyp-Asn-Thr-Phe-AzaGly-Leu-Arg(Me)-Trp-NH₂ (Compound No. 971),
   dimethylaminoethylureido-D-Tyr-Hyp-Asn-Thr-Phe-AzaGly-Leu-Arg(Me)-Trp-N H₂ (Compound No. 972),
   iso-butylureido-D-Tyr-Hyp-Asn-Thr-Phe-AzaGly-Leu-Arg(Me)-Trp-NH₂ (Compound No. 973) or
   Hyp-Asn-Thr-Phe-AzaGly-Leu-Arg(Me)-Trp-NH₂ (Compound No. 977), or a salt thereof.
[4] A metastin derivative represented by formula:
   XX0-XX2-XX3-XX4-XX5-T-XX9-XX10-NH₂
   wherein:
   XX0 represents formyl, a C₁₋₂₀ alkanoyl, mesyl, an optionally substituted ureido (e.g., benzylureido, ureido, dimethylaminoethylureido, isobutylureido, etc.), amidino, cyclopropanecarbonyl, 6-(acetyl-D-arginylamino)caproyl, 6-((R)-2,3-diaminopropionylamino)caproyl, 6-(D-norleucylamino)caproyl, 4-(D-arginylamino)butyryl, 3-(4-hydroxyphenyl)propionyl, glycyl, tyrosyl, acetylglycyl, acetylleucyl, D-tyrosyl, acetyl-D-tyrosyl, pyroglutamyl, 3-(pyridin-3-yl)propionyl, adipoyl, glycoloyl or 6-aminocaproyl;
   XX2 represents Tyr, D-Tyr, D-Ala, D-Leu, D-Phe, D-Lys, D-Trp or a chemical bond;
   XX3 represents D-Asp, D-Dap, D-Ser, D-Gln, D-His, D-NMeAla, D-NMePhe, Aze(2), Pic(2), Pic(3), Hyp, Thz, NMeAla, Gly, Aib, Abz(2), Abz(3), Sar, Leu, Lys, Glu, β-alanine, Pzc(2), Om, His(3Me), Tyr(PO₃H₂), Pro(4NH₂), Hyp(Bzl), Trp, Pro, 4-pyridylalanine, Tic, D-Trp, D-Ala, D-Leu, D-Phe, D-Lys, D-Glu, D-2-pyridylalanine, D-3-pyridylalanine, D-4-pyridylalanine, Aad, Pro(4F) or a chemical bond;
   XX4 represents Asn, 2-amino-3-ureidopropionic acid, Nβ-formyldiaminopropionic acid, Nβ-acctyldiaminopropionic acid, Nω-pentylasparagine, Nω-cyclopropylasparagine, Nω-benzylasparagine, 2,4-diaminobutanoic acid, His, Gln, Cit or D-Asn;
   XX5 represents Ser, Thr, Val, NMeSer, Gly, Ala, Hyp, D-Ala, Dap or D-Thr;
   T represents formula II: 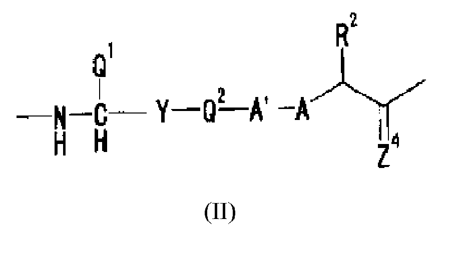
   Z⁴ represents hydrogen atom, O or S;
   R² represents (1) hydrogen atom or (2) a cyclic or linear C₁₋₁₀ alkyl group, (3) a C₁₋₁₀ alkyl group consisting of a cyclic alkyl group and a linear alkyl group, or (4) a C₁₋₈ alkyl group which may optionally be substituted with a group selected from the group consisting of an optionally substituted carbamoyl group, an optionally substituted hydroxy group and an optionally substituted aromatic cyclic group;
   Q¹ represents a C₁₋₄ alkyl group, which may optionally be substituted with a substituent selected from the group consisting of (1) an optionally substituted C₆₋₁₂ aromatic hydrocarbon group, (2) an optionally substituted 5- to 14-membered aromatic heterocyclic group consisting of 1 to 7 carbon atoms and a hetero atom selected from the group consisting of nitrogen atom, oxygen atom and sulfur atom, (3) an optionally substituted C₈₋₁₄ aromatic condensed cyclic group, (4) an optionally substituted 5- to 14-membered aromatic condensed heterocyclic group consisting of 3 to 11 carbon atoms and a hetero atom selected from the group consisting of nitrogen atom, oxygen atom and sulfur atom, (5) an optionally substituted non-aromatic cyclic hydrocarbon group having not greater than 7 carbon atoms and (6) an optionally substituted non-aromatic heterocyclic group having not greater than 7 carbon atoms;
   A represents:
      (1) a nitrogen atom substituted with hydrogen atom or a C₁₋₃ alkyl group;
      (2) a carbon atom substituted with hydrogen atom or a C₁₋₃ alkyl group;
      (3) O; or,
      (4) S;
   A' represents:
      (1) a carbon atom which may optionally be substituted with hydrogen atom, O, S, a halogen atom, an optionally halogenated C₁₋₃ alkyl group, carbamoyl or hydroxy;
      (2) a nitrogen atom which may optionally be substituted with hydrogen atom or an optionally halogenated C₁₋₃ alkyl group;
      (3) O; or,
      (4) S;
   Q² represents:
      (1) CH₂, CO, CS or C=CH₂, which may optionally be substituted with a C₀₋₄ alkyl group optionally substituted with a substituent selected from the group consisting of carbamoyl, hydroxy, a C₁₋₃ alkoxy group, a halogen atom and amino;
      (2) NH which may optionally be substituted with a C₁₋₄ alkyl group optionally substituted with a substituent selected from the group consisting of carbamoyl and hydroxy; or,
      (3) O;
   Y represents:
      (1) a group represented by formula: -CONH-, -CSNH-, -CH₂NH-, -NHCO-, -CH₂O-, -COCH₂-, -CH₂S-, -CSCH₂-, -CH₂SO-, -CH₂SO₂-, -COO-, -CSO-, -CH₂CH₂- or -CH=CH-, which may optionally be substituted with a substituent selected from the group consisting of a C₁₋₆ alkyl group, hydroxy and a halogen atom;
      (2) an optionally substituted C₆₋₇ aromatic hydrocarbon group;
      (3) an optionally substituted 4- to 7-membered aromatic heterocyclic group (preferably 5- to 7-membered aromatic heterocyclic group) consisting of 1 to 5 carbon atoms and a hetero atom selected from the group consisting of nitrogen atom, oxygen atom and sulfur atom;
      (4) an optionally substituted non-aromatic cyclic hydrocarbon group having not greater than 5 carbon atoms;
      (5) an optionally substituted non-aromatic heterocyclic group having not greater than 5 carbon atoms;
   provided that when Y is (2), (3), (4) or (5), Q² may be a chemical bond; the bonds between Y-Q², Q²-A' and A'-A each independently represents a single bond or a double bond;
   XX9 represents Arg, Om, Arg(Me) or Arg(asymMe₂); and,
   XX10 represents Phe, Trp, 2-naphthylalanine, 2-thienylalanine, tyrosine or 4-fluorophenylalanine;
   or a salt thereof.
[4a] The metastin derivative or a salt thereof, according to [4] above, wherein:
   XX0 represents formyl, a C₁₋₂₀ alkanoyl or glycoloyl;
   XX2 represents D-Tyr or a chemical bond;
   XX3 represents Azc(2), Hyp, Gly, Aib, Leu, Lys, Glu, His(3Mc), Tyr(PO₃H₂), Pro(4F) or Hyp(Bzl);
   XX4 represents Asn or 2-amino-3-ureidopropionic acid;
   XX5 represents Ser or Thr;
   Z⁴ represents O;
   R² represents a linear C₁₋₁₀ alkyl group, or a C₁₋₁₀ alkyl group consisting of a cyclic alkyl group and a linear alkyl group;
   Q¹ represents a C₁₋₄ alkyl group, which may optionally be substituted with a substituent selected from the group consisting of (1) an optionally substituted C₆₋₁₂ aromatic hydrocarbon group, (2) an optionally substituted 5- to 14-membered aromatic heterocyclic group consisting of 1 to 7 carbon atoms and a hetero atom selected from the group consisting of nitrogen atom, oxygen atom and sulfur atom, and (5) an optionally substituted non-aromatic cyclic hydrocarbon group having not greater than 7 carbon atoms;
   A represents:
      (1) a nitrogen atom substituted with hydrogen atom;
      (2) a carbon atom substituted with hydrogen atom; or,
      (4) S;
         A' represents (1) a carbon atom substituted with hydrogen atom or O;
         Q² represents (1) CH₂ or C=CH₂, which may optionally be substituted with 1 or 2C₀₋₄ alkyl groups optionally substituted with a substituent selected from the group consisting of carbamoyl, hydroxy, a C₁₋₃ alkoxy group and amino;
         Y represents (1) a group represented by formula: CONH-, -CH₂O-, -CH₂S-, -COCH₂-, -CH₂CH₂-, CSNH-, -NHCO- or -CH=CH-, which may optionally be substituted with a substituent selected from the group consisting of a G₁₋₆ alkyl group, hydroxy and a halogen atom;
         XX9 represents Arg or Arg(Me); and,
         XX10 represents Phe or Trp.
[5] A metastin derivative represented by formula:
   XX0-XX2-XX3-XX4-XX5-T-XX9-XX10-NH₂
   wherein:
   XX0 represents C₁₋₂₀ (preferably C₁₋₁₂, more preferably C₁₋₆) alkanoyl;
   XX2 represents D-Tyr;
   XX3 represents Hyp, Glu or Pro(4F);
   XX4 represents Asn or 2-amino-3-ureidopropionic acid;
   XX5 represents Thr;
   T represents formula II: 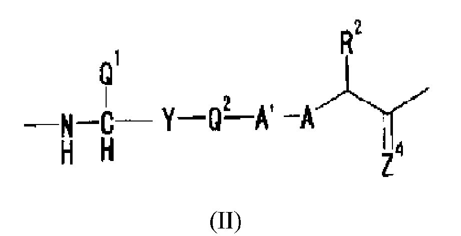
   Z⁴ represents O;
   R² represents (1) a cyclic or linear C₁₋₁₀ alkyl group, or (2) a C₁₋₁₀ alkyl group consisting of a cyclic alkyl group and a linear alkyl group;
   Q¹ represents a C₁₋₄ alkyl group, which may optionally be substituted with a substituent selected from the group consisting of (1) an optionally substituted C₆₋₁₂ aromatic hydrocarbon group and (2) an optionally substituted non-aromatic cyclic hydrocarbon group having not greater than 7 carbon atoms;
   A represents:
      (1) a nitrogen atom substituted with hydrogen atom or a C₁₋₃ alkyl group;
      (2) a carbon atom substituted with hydrogen atom or a C₁₋₃ alkyl group;
      (3) O; or,
      (4) S;
   A' represents:
      (1) a carbon atom which may optionally be substituted with hydrogen atom, O, S, a halogen atom, an optionally halogenated C₁₋₃ alkyl group, carbamoyl or hydroxy;
   Q² represents:
      (1) CH₂, CO, CS or C=CH₂, which may optionally be substituted with a C₀₋₄ alkyl group optionally substituted with a substituent selected from the group consisting of carbamoyl, hydroxy, a C₁₋₃ alkoxy group, a halogen atom and amino;
   Y represents:
      (1) a group represented by formula: -CONH-, -CSNH-, -CH₂NH- or -NHCO-, which may optionally be substituted with a substituent selected from the group consisting of a C₁₋₆ alkyl group, hydroxy and a halogen atom;
   provided that the bonds between Y-Q², Q²-A' and A'-A each independently represents a single bond or a double bond;
   XX9 represents Arg or Arg(Me); and,
   XX10 represents Trp;
   or a salt thereof.
[6] The metastin derivative or a salt thereof according to [5] above, wherein:
   XX0 represents a C₁₋₆ alkanoyl (preferably acetyl);
   XX2 represents D-Tyr;
   XX3 represents Hyp, Glu or Pro(4F);
   XX4 represents Asn or 2-amino-3-ureidopropionic acid;
   XX5 represents Thr;
   T represents formula II: 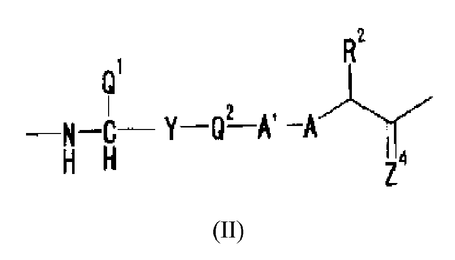
   Z⁴ represents O;
   R² represents propyl, isopropyl, isobutyl or cyclopropylmethyl;
   Q¹ represents benzyl or cyclohexylmethyl;
   A represents, -NH-, -CH- or S;
   A' represents, -CH-, -CH₂- or -CO-;
   Q² represents CH₂, CH(CH₃), CH(CH₂OH), C(CH₃)₂, C=CH₂, CH(CH₂CH₃), CH(CH₂NH₂), CH(CH₂OCH₃) or CH(CH(CH₃)₂);
   Y represents a group represented by formula: -CONH-, -CSNH-, -NHCO- or -CH₂NH-;
   provided that the bonds between Y-Q², Q²-A' and A'-A each independently represents a single bond or a double bond;
   XX9 represents Arg or Arg(Me); and,
   XX10 represents Trp.
[7] A metastin derivative, which is selected from:
   Ac-D-Tyr-Hyp-Asn-Thr-D-Cha-Gly-ψ[(E)-CH=CH]-Leu-Arg(Me)-Trp-NH₂,
   Ac-D-Tyr-Hyp-Asn-Thr-Cha-Gly-ψ[(E)-CH=CH]-D-Leu-Arg(Me)-Trp-NH₂,
   Ac-D-Tyr-Hyp-Asn-Thr-Cha-Gly-ψ[(E)-CMe=CH]-Leu-Arg(Me)-Trp-NH₂,
   Ac-D-Tyr-Hyp-Asn-Thr-Cha-Gly-ψ[(E)-CF=CH]-Leu-Arg(Me)-Trp-NH₂,
   Ac-D-Tyr-Hyp-Asn-Thr-Phe-ψ(CSNH)-Gly-Leu-Arg(Me)-Trp-NH₂ (Compound No.1002),
   Ac-D-Tyr-Hyp-Asn-Thr-Phe-ψ(NHCO)-Gly-Leu-Arg(Me)-Trp-NH₂ (Compound No. 1003),
   Ac-D-Tyr-Hyp-Asn-Thr-Phe-ψ(CH₂NH)-AzaGly-Leu-Arg(Me)-Trp-NH₂,
   Ac-D-Tyr-Hyp-Asn-Thr-Phe-Gly-ψ(CSNH)-Leu-Arg(Me)-Trp-NH₂,
   Ac-D-Tyr-Hyp-Asn-Thr-Cha-ψ(CSNH)-Gly-Leu-Arg(Me)-Trp-NH₂ (Compound No. 1004) and
   Ac-D-Tyr-Hyp-Asn-Thr-Cha-ψ(CSNH)-Gly-Ala(cPr)-Arg(Me)-Trp-NH₂ (Compound No. 1005); or a salt thereof.
[8] A prodrug of the metastin derivative or a salt thereof according to [1] to [7] above.
[9] A pharmaceutical comprising the metastin derivative or a salt thereof according to [1] through [7] above, or a prodrug thereof.
[10] The pharmaceutical according to [9] above, which is a cancer metastasis-inhibiting agent or a cancer proliferation-inhibiting agent.
[11] The pharmaceutical according to [9] above, which is an agent for preventing or treating cancer.
[12] The pharmaceutical according to [9] above, which is an agent for controlling placental function.
[13] The pharmaceutical according to [9] above, which is an agent for preventing or treating choriocarcinoma, hydatid mole, invasive mole, miscarriage, fetal hypoplasia, abnormal glucose metabolism, abnormal lipid metabolism or induction of delivery.
[14] The pharmaceutical according to [9] above, which is an agent for improving gonadal function.
[15] The pharmaceutical according to [9] above, which is an agent for preventing or treating hormone-dependent cancer, infertility, endometriosis, early puberty or myoma of the uterus.
[16] The pharmaceutical according to [9] above, which is an agent for inducing or stimulating ovulation.
[17] The pharmaceutical according to [9] above, which is an agent for promoting gonadotropic hormone secretion or sex hormone secretion.
[18] The pharmaceutical according to [9] above, which is an agent for preventing or treating Alzheimer's disease, moderate cognitive impairment or autism.
[19] The pharmaceutical (or agent) according to [15] above, which is a down-regulating agent for gonadotropic hormone or sex hormone.
[20] The pharmaceutical (agent) according to [15] above, which is a down-regulating agent for human OT7T175 (metastin receptor) protein consisting of the amino acid sequence represented by SEQ ID NO: 9.
[20a] The pharmaceutical (agent) according to [19] or [20] above, which is an agent for preventing or treating hormone-dependent cancer.
[21] A method for preventing or treating cancer, which comprises administering to a mammal an effective dose of the metastin derivative or a salt thereof according to [1] through [7] above, or the prodrug according to [8] above.
[22] Use of the metastin derivative or a salt thereof according to [1] through [7] above or the prodrug according to [8] above to manufacture an agent for preventing or treating cancer.
   The present invention further provides the following features, and so on.
[23] A method of suppressing cancer metastasis or suppressing cancer proliferation, which comprises administering to a mammal an effective dose of the metastin derivative or a salt thereof according to any one of [1] through [7] above, or a prodrug thereof.
[24] A method for controlling placental function, which comprises administering to a mammal an effective dose of the metastin derivative or a salt thereof according to any one of [1] through [7] above, or a prodrug thereof.
[25] A method for preventing or treating choriocarcinoma, hydatid mole, invasive mole, miscarriage, fetal hypoplasia, abnormal glucose metabolism, abnormal lipid metabolism or induction of delivery, which comprises administering to a mammal an effective dose of the metastin derivative or a salt thereof according to any one of [1] through [7] above, or a prodrug thereof.
[26] A method for improving gonadal function, which comprises administering to a mammal an effective dose of the metastin derivative or a salt thereof according to any one of [1] through [7] above, or a prodrug thereof.
[27] A method for preventing or treating hormone-dependent cancer, infertility, endometriosis, early puberty or myoma of the uterus, which comprises administering to a mammal an effective dose of the metastin derivative or a salt thereof according to any one of [1] through [7] above, or a prodrug thereof.
[28] A method for inducing or stimulating ovulation, which comprises administering to a mammal an effective dose of the metastin derivative or a salt thereof according to any one of [1] through [7] above, or a prodrug thereof.
[29] A method for promoting gonadotropic hormone secretion or promoting sex hormone secretion, which comprises administering to a mammal an effective dose of the metastin derivative or a salt thereof according to any one of [1] through [7] above, or a prodrug thereof.
[30] A method for preventing or treating Alzheimer's disease, moderate cognitive impairment or autism, which comprises administering to a mammal an effective dose of the metastin derivative or a salt thereof according to any one of [1] through [7] above, or a prodrug thereof.
[31] A method for down-regulating gonadotropic hormone or sex hormone, which comprises administering to a mammal an effective dose of the metastin derivative or a salt thereof according to any one of [1] through [7] above, or a prodrug thereof.
[32] A method for down-regulating human OT7T175 (metastin receptor) protein consisting of the amino acid sequence represented by SEQ ID NO: 9, which comprises administering to a mammal an effective dose of the metastin derivative or a salt thereof according to any one of [1] through [7] above, or a prodrug thereof.
[33] A method for preventing or treating hormone-dependent cancer, which comprises administering to a mammal an effective dose of the metastin derivative or a salt thereof according to any one of [1] through [7] above, or a prodrug thereof.
[34] Use of the metastin derivative or a salt thereof according to any one of [1] through [7] above, or a prodrug thereof to manufacture an agent for suppressing cancer metastasis or an agent for suppressing cancer proliferation.
[35] The metastin derivative or a salt thereof according to any one of [1] through [7] above, or a prodrug thereof for use in suppressing cancer metastasis or suppressing cancer proliferation.
[36] The metastin derivative or a salt thereof according to any one of [1] through [7] above, or a prodrug thereof for use in preventing or treating cancer.
[37] Use of the metastin derivative or a salt thereof according to any one of [1] through [7] above, or a prodrug thereof to manufacture an agent for controlling placental function.
[38] The metastin derivative or a salt thereof according to any one of [1] through [7] above, or a prodrug thereof for use in controlling placental function.
[39] Use of the metastin derivative or a salt thereof according to any one of [1] through [7] above, or a prodrug thereof to manufacture an agent for preventing or treating choriocarcinoma, hydatid mole, invasive mole, miscarriage, fetal hypoplasia, abnormal glucose metabolism, abnormal lipid metabolism or induction of delivery.
[40a] The metastin derivative or a salt thereof according to any one of [1] through [7] above, or a prodrug thereof for use in preventing or treating choriocarcinoma, hydatid mole, invasive mole, miscarriage, fetal hypoplasia, abnormal glucose metabolism, abnormal lipid metabolism or induction of delivery.
[41] Use of the metastin derivative or a salt thereof according to any one of [1] through [7] above, or a prodrug thereof to manufacture an agent for improving gonadal function.
[42] The metastin derivative or a salt thereof according to any one of [1] through [7] above, or a prodrug thereof for use in improving gonadal function.
[43] Use of the metastin derivative or a salt thereof according to any one of [1] through [7] above, or a prodrug thereof to manufacture an agent for preventing or treating hormone-dependent cancer, infertility, endometriosis, early puberty or myoma of the uterus.
[44] The metastin derivative or a salt thereof according to any one of [1] through [7] above, or a prodrug thereof for use in preventing or treating hormone-dependent cancer, infertility, endometriosis, early puberty or myoma of the uterus.
[45] Use of the metastin derivative or a salt thereof according to any one of [1] through [7] above, or a prodrug thereof to manufacture an agent for inducing or stimulating ovulation.
[46a] The metastin derivative or a salt thereof according to any one of [1] through [7] above, or a prodrug thereof for use in inducing or stimulating ovulation.
[47] Use of the metastin derivative or a salt thereof according to any one of [1] through [7] above, or a prodrug thereof to manufacture an agent for promoting gonadotropic hormone secretion or sex hormone secretion.
[48] The metastin derivative or a salt thereof according to any one of [1] through [7] above, or a prodrug thereof for use in promoting gonadotropic hormone secretion or promoting sex hormone secretion.
[49] Use of the metastin derivative or a salt thereof according to any one of [1] through [7] above, or a prodrug thereof to manufacture an agent for preventing or treating Alzheimer's disease, moderate cognitive impairment or autism.
[50] Use of the metastin derivative or a salt thereof according to any one of [1] through [7] above, or a prodrug thereof for use in preventing or treating Alzheimer's disease, moderate cognitive impairment or autism.
[51] Use of the metastin derivative or a salt thereof according to any one of [1] through [7] above, or a prodrug thereof, to manufacture a down-regulating agent for gonadotropic hormone or sex hormone.
[52a] Use of the metastin derivative or a salt thereof according to any one of [1] through [7] above, or a prodrug thereof, for use in down-regulating of gonadotropic hormone or sex hormone.
[53] Use of the metastin derivative or a salt thereof according to any one of [1] through [7] above, or a prodrug thereof, to manufacture a down-regulating agent for human OT7T175 (metastin receptor) protein consisting of the amino acid sequence represented by SEQ ID NO: 9.
[54a] The metastin derivative or a salt thereof according to any one of [1] through [7] above, or a prodrug thereof, for use in down-regulating human OT7T175 (metastin receptor) protein consisting of the amino acid sequence represented by SEQ ID NO: 9.
[55] Use of the metastin derivative or a salt thereof according to any one of [1] through [7] above, or a prodrug thereof, to manufacture an agent for preventing or treating hormone-dependent cancer.
[56] The metastin derivative or a salt thereof according to any one of [1] through [7] above, or a prodrug thereof, for use in preventing or treating hormone-dependent cancer.
   The present invention further provides the following features and so on.
[57] (1) A pancreatic function regulating agent, (2) an agent for preventing or treating acute or chronic pancreatitis or pancreatic cancer, (3) a hyperglycemic agent, a pancreatic glucagon increasing agent or an agent for promoting urine formation, (4) an agent for preventing or treating obesity, hyperlipemia, type II diabetes mellitus, hypoglycemia, hypertension, diabetic neuropathy, diabetic nephropathy, diabetic retinopathy, edema, urinary disturbances, insulin resistance, unstable diabetes mellitus, fatty atrophy, insulin allergy, insulinoma, arteriosclerosis, thrombotic disorders or lipotoxicity, (5) an agent for suppressing gonadotropic hormone secretion or an agent for suppressing sex hormone secretion, or (6) an agent for inhibiting ovarian follicular maturation, a menstrual cycle-suspending agent or a contraceptive, comprising the metastin derivative or a salt thereof according to any one of [1] through [7] above, or a prodrug thereof.
[58] (1) A method for regulating pancreatic function, (2) a method for preventing or treating acute or chronic pancreatitis or pancreatic cancer, (3) a method for increasing blood glucose levels, a method for increasing pancreatic glucagon levels or a method for promoting urine formation, (4) a method for preventing or treating obesity, hyperlipemia, type II diabetes mellitus, hypoglycemia, hypertension, diabetic neuropathy, diabetic nephropathy, diabetic retinopathy, edema, urinary disturbances, insulin resistance, unstable diabetes mellitus, fatty atrophy, insulin allergy, insulinoma, arteriosclerosis, thrombotic disorders or lipotoxicity, (5) a method for suppressing gonadotropic hormone secretion or a method for suppressing sex hormone secretion, or (6) a method for inhibiting ovarian follicular maturation, a method for suspending menstrual cycle or a method for contraception, which comprises administering to a mammal an effective dose of the metastin derivative or a salt thereof according to any one of [1] through [7] above, or a prodrug thereof.
[59] Use of the metastin derivative or a salt thereof according to any one of [1] through [7] above, or a prodrug thereof to manufacture (1) a pancreatic function regurating agent, (2) an agent for preventing or treating acute or chronic pancreatitis or pancreatic cancer, (3) a hyperglycemic agent, a pancreatic glucagon increasing agent or an agent for promoting urine formation, (4) an agent for preventing or treating obesity, hyperlipemia, type II diabetes mellitus, hypoglycemia, hypertension, diabetic neuropathy, diabetic nephropathy, diabetic retinopathy, edema, urinary disturbances, insulin resistance, unstable diabetes mellitus, fatty atrophy, insulin allergy, insulinoma, arteriosclerosis, thrombotic disorders or lipotoxicity, (5) an agent for suppressing gonadotropic hormone secretion or an agent for suppressing sex hormone secretion, or (6) an agent for inhibiting ovarian follicular maturation, a menstrual cycle-suspending agent or a contraceptive.

### EFFECT OF THE INVENTION

In addition to the excellent effects of suppressing cancer metastasis and cancer proliferation, the metastin derivatives of the present invention or their salts or prodrugs have excellent blood stability, solubility, etc., and arc useful as agents for preventing or treating cancers (for example, lung cancer, gastric cancer, liver cancer, pancreatic cancer, colorectal cancer, rectal cancer, colonic cancer, prostate cancer, ovarian cancer, cervical cancer, breast cancer, etc.). The metastin derivatives of the present invention or their salts or prodrugs have an effect of controlling the pancreatic function and are useful as agents for preventing or treating pancreatic diseases (e.g., acute or chronic pancreatitis, pancreatic cancer, etc.). The metastin derivatives of the present invention or their salts or prodrugs have an effect of controlling the placental function and are useful as agents for preventing or treating choriocarcinoma, hydatid mole, invasive mole, miscarriage, fetal hypoplasia, abnormal glucose metabolism, abnormal lipid metabolism or induction of delivery.

Also, the metastin derivatives of the present invention or their salts or prodrugs have effects of increasing blood glucose level, promoting pancreatic glucagon secretion and promoting urine formation, and are useful as agents for preventing or treating obesity, hyperlipemia, type 11 diabetes mellitus, hypoglycemia, hypertension, diabetic neuropathy, diabetic nephropathy, diabetic retinopathy, edema, urinary disturbances, insulin resistance, unstable diabetes mellitus, fatty atrophy, insulin allergy, insulinoma, arteriosclerosis, thrombotic disorders or lipotoxicity.

Furthermore, the metastin derivatives of the present invention or their salts or prodrugs have excellent activities of promoting gonadotropic hormone secretion, promoting sex hormone secretion, inducing or stimulating ovulation, and are useful as low toxic and stable agents, e.g., for improving gonadal function, preventing or treating hormone-dependent cancer (e.g., prostate cancer, breast cancer, etc.), infertility, endometriosis, early puberty, myoma of the uterus, etc., agents for inducing or stimulating ovulation, agents for promoting gonadotropic hormone secretion, contraceptives, agents for promoting sex hormone secretion, or the like.

Furthermore, the metastin derivatives of the present invention or their salts or prodrugs are useful as agents for preventing or treating Alzheimer's disease, moderate cognitive impairment, autism, etc.

The metastin derivatives of the present invention or their salts or prodrugs are useful as agents for suppressing gonadotropic hormone secretion or sex hormone secretion; down-regulating agents for gonadotropic hormone or sex hormone; down-regulating agents for human OT7T175 (metastin receptor) protein consisting of the amino acid sequence represented by SEQ ID NO: 9; agents for preventing or treating hormone-dependent cancers (e.g., prostate cancer, breast cancer, etc.; particularly, hormone-sensitive prostate cancer, hormone-sensitive prostate cancer, etc.); agents for preventing or treating endometriosis; agents for inhibiting ovarian follicular maturation; menstrual cycle-suspending agents; agents for treating myoma of the uterus; agents for treating early puberty; contraceptives, etc.

In addition, the metastin derivatives of the present invention or their salts or prodrugs are useful as immunopotentiators (prophylactic agents for infection after bone-marrow transplant, immunopotentiators for use in cancer); immuostimulants (thymus regeneration, thymic regrowth, enhanced T cell growth); prophylactic/therapeutic agents for bulbospinal muscular atrophy; agents for protecting ovary; prophylactic/therapeutic agents for benign prostate hypertrophy (BPH); prophylactic/therapeutic agents for gender identity disorders; agents for in vitro fertilization (IVF); etc. These derivatives, salts or prodrugs are also useful as prophylactic/therapeutic agents for infertility, hypogonadism, oligospermia, azoospermia, aspermia, asthenospermia, necrospermia, etc. Furthermore, these derivatives, salts or prodrugs are useful for hormone-dependent diseases (e.g., sex hormone-dependent cancers such as prostate cancer, uterine cancer, breast cancer, hypophyseal tumor, etc.), prostate gland enlargement, endometriosis, uterine fibroid, early puberty, dysmenorrhea, amenorrhea, premenstrual syndrome, multilocular ovary syndrome, postoperative relapse of the above-mentioned cancers, metastasis of the above-mentioned cancers, hypopituitarism, dwarfism (the case where the secretion of growth hormone was compromised with hyposecretion of pituitary hormone), menopausal disorders, indefinite complaint, sex hormone-dependent disorders such as calcium phosphor bone metabolic disorders. It is also applicable for contraception (or infertility when rebound effects after drug cessation are used), and so on.

Moreover, metastin per se or metastin-encoding DNA, etc. are also useful as agents for suppressing gonadotropic hormone secretion or for suppressing sex hormone secretion; down-regulating agents for gonadotropic hormone or sex hormone; down-regulating agents for human OT7T175 (metastin receptor) protein consisting of the amino acid sequence represented by SEQ ID NO: 9; agents for preventing or treating hormone-dependent cancers (e.g., prostate cancer, breast cancer, etc.; particularly, hormone-sensitive prostate cancer, hormone-sensitive breast cancer, etc.); agents for preventing or treating endometriosis; agents for inhibiting ovarian follicular maturation; menstrual cycle-suspending agents; agents for treating myoma of the uterus; agents for treating early puberty; contraceptives, etc.

### EMBODIMENTS FOR CARRYING OUT THE INVENTION

As used herein, Tyr-Asn-Trp-Asn-Ser-Phe-Gly-Leu-Arg-Phe-NH₂ (SEQ ID NO: 16) is referred to as Metastin 10, i.e., MS 10.

Herein, the positions ofN-terminal Tyr and C-terminal Phe in MS10 are counted as the 1- and 10-positions, respectively.

Tyr-Asn-Trp-Asn-Ser-Phe-Gly-Leu-Arg-Phe-NH2
1 2 3 4 5 6 7 8 9 10

For example, [Hph10]MS10 means a peptide wherein Phe at the C terminus (10-position) of MS10 is substituted with Hph.

For example, des(1)-MS10 means a peptide wherein Tyr at the N terminus (1-position) of MS10 is deleted.

For example, des(1-3)-Fmoc-MS10 means a peptide wherein Tyr-Asn-Trp at the N terminus (1 to 3-positions) of MS10 is deleted and the amino group of Asn at the 4-position is modified with Fmoc.

For example, des(1)-Ac-[D-Tyr2,D-Trp3,Thr5,AzaGly7,D-Arg9,Trp10]MS10 means a peptide wherein the amino end of MS 10 is modified with Ac, Tyr at the N terminus (1-position) is deleted, Asn at the 2-position is substituted with D-Tyr, Trp at the 3-position is substituted with D-Trp, Ser at the 5-position is substituted with Thr, Gly at the 7-position is substituted with AzaGly, Arg at the 9-position is substituted with D-Arg and Phe at the 10-position is substituted with Trp, i.e., Ac-D-Tyr-D-Trp-Asn-Thr-Phe-AzaGly-Leu-D-Arg-Trp-NH₂.

In the formulae described above, XX0 represents a modifying group at the amino end; and XX2, XX3, XX4, XX5, XX6, XX8, XX9 and XX10 each represents 2-, 3-, 4-, 5-, 6-, 8-, 9- and 10-positions of MS10 described above, respectively.

Each chemical bond "-" between XX0, XX2, XX3, XX4, XX5, XX6, AzaGly, XX8, XX9 and XX10 and NH₂ in the formula:
"XX0-XX2-XX3-XX4-XX5-XX6-AzaGly-XX8-XX9-XX10-NH₂" has the following meanings.

The chemical bond "-" in the formula "XX0-XX2" means a bond between the group represented by XX0 and the amino group (α-amino group) contained in XX2. More specifically, the formula "XX0-XX2" indicates that the hydrogen atom in the amino group (NH₂) contained in XX2 is substituted with the group represented by XX0.

However, when XX0 is benzylureido, ureido, dimethylaminoethylureido or isobutylureido, the chemical bond means to form an ureido together with the amino group contained in XX2. When the chemical bond is shown with the exception of the amino group contained in XX2, benzylureido, ureido, dimethylaminoethylureido and isobutylureido are described as benzylcarbamoyl, carbamoyl, dimethylaminoethylcarbamoyl and isobutylcarbamoyl, respectively.

The chemical bond "-" in the formula "XX2-XX3" means that the carboxyl group (α-carboxyl group) contained in XX2 is bound to the amino group (α-amino group) in XX3 through an amide bond. The chemical bonds "-" in the formulae "XX3-XX4," "XX4-XX5," "XX5-XX6," "XX8-XX9" and "XX9-XX10" also have the same meanings as described above.

The chemical bond "-" in the formula "XX6-AzaGly" means that the carboxyl group (α-carboxyl group) contained in XX6 is bound to the amino group (α-amino group) in AzaGly [azaglycine] through an amide bond.

The chemical bond "-" in the formula "AzaGly-XX8" means that the carboxyl group contained in AzaGly is bound to the amino group (α-amino group) in XX8 through an amide bond.

The chemical bond "-" in the formula "XX10-NH₂" represents the bond between the carboxyl group (α-carboxyl group) contained in XX10 and -NH₂. More specifically, the formula "XX10-NH₂" indicates that -OH in the carboxyl group (-COOH) contained in XX10 is substituted with -NH₂.

Where XX2, XX3, XX4 or/and XX5 represent the chemical bonds "-," these chemical bonds "-" also have the same meanings as described above.

In the formula above:
XX0-XX2-XX3-XX4-XX5-XX6-AzaGly-XX8-XX9-XX10-NH₂, XX0 represents hydrogen, acetyl (Ac), mesyl (Ms), 4-fluorobenzoyl (4-FluoroBenzoyl), decanoyl (Decanoyl), benzylureido (Benzylureido), ureido, amidino (Amidino), dimcthylaminocthylurcido (Dimcthylaminocthylurcido), isobutylurcido (iso-Butylureido), etc., preferably, acetyl (Ac), mesyl (Ms), benzylureido (Benzylureido), ureido, dimethylaminoethylureido (Dimethylaminoethylureido) and isobutylureido (iso-Butylureido).

In the formula described above, XX2 represents Tyr, D-Tyr, D-Ala, D-Leu, D-Phe, D-Lys, D-Trp or the chemical bond; preferably represents D-Tyr, Tyr or the chemical bond; more preferably, represents D-Tyr or the chemical bond; and most preferably represents D-Tyr.

In the formula described above, XX3 represents (i) an amino acid wherein the α-amino group may optionally be methylated (an amino acid selected from the group consisting of Ala [alanine], Arg [arginine], Asn [asparagine], Asp [aspartic acid], Cys [cysteine], Gln [glutamine], Glu [glutamic acid], Gly [glycine], His [histidine], Ile [isoleucine], Leu [leucine], Lys [lysine], Met [methionine], Phe [phenylalanine], Ser [serine], Thr [threonine], Trp [tryptophan], Tyr [tyrosine] and Val [valine]), (ii) a cyclic amino acid (a cyclic amino acid selected from Pro [proline], Aze(2), Aze(3), Pic(2), Pic(3), Hyp, Thz, Abz(2), Abz(3), Pzc(2), Pro(4NH₂), Hyp(Bzl), cisHyp, Pro(4F) and lzc), (iii) an amino acid selected from D-Dap, D-Pya(4), DL-Ala(Pip), Om, Aib and Tyr(PO₃H₂), or (iv) the chemical bond.

Herein, Aze(2) is [azetidine-2-carboxylic acid], Aze(3) is [azetidine-3-carboxylic acid], Pic(2) is [pipecolinic acid], Pic(3) is [3-piperidinecarboxylic acid], D-Dap is [D-2,3-diaminopropionic acid], D-Pya(4) is [4-pyridyl-D-alanine], Hyp is [trans-4-hydroxyproline], Thz is [thioproline], Aib is [α-aminoisobutanoic acid], Abz(2) is [2-aminobenzoic acid], Abz(3) is [3-aminobenzoic acid], lzc is [imidazolidine-2-carboxylic acid], DL-Ala(Pip) is [DL-(4-piperidin-1-yl)alanine], Pzc(2) is [piperazine-2-carboxylic acid], Om is [ornithine], Tyr(PO₃H₂) is [O-phosphotyrosine], Pro(4NH₂) is [4-aminoproline], Hyp(Bzl) is [trans-4-benzyloxyproline], cisHyp is [cis-4-hydroxyproline] and Pro(4F) is [trans-4-fluoroproline]. As used herein, the amino acid may be either an L-amino acid or a D-amino acid, unless otherwise indicated. Alanine may be α-alanine or β-alanine.

Preferably, XX3 is D-Asp, D-Dap[D-2,3-diaminopropionic acid], D-Ser, D-Gln, D-His, D-Trp, D-Tyr, D-Pya(4), D-NMeAla [D-Nα-methylalanine], D-NMePhe [D-Nα-methylphenylalanine], Aze(2), Aze(3) [azetidine-3-carboxylic acid], Pic(2), Pic(3), Hyp, Thz, NMeAla, Gly, Aib, Abz(2), Abz(3), Sar, Izc, Leu, Lys, Glu, Thr, Trp, Ser, Ala, NMeAla, β-alanine, DL-Ala(Pip), Pzc(2), Om, His(3Me) [3-methylhistidine], Tyr(PO₃H₂), Pro(4NH₂), Hyp(Bzl), cisHyp, Pro(4F) or the chemical bond; more preferably, represents D-Asp, D-Dap, D-Ser, D-Gln, D-His, D-Trp, D-Tyr, D-Pya(4), D-NMeAla, D-NMePhe, Aze(2), Aze(3), Pic(2), Pic(3), Hyp, Thz, Gly, Aib, Abz(2), Sar, Izc, Leu, Lys, Glu, Thr, Trp, Ser, Ala, NMeAla, β-alanine, DL-Ala(Pip), Pzc(2), Orn, His(3Me), Tyr(PO₃H₂), Pro(4NH₂), Hyp(Bzl), cisHyp, Pro(4F) or the chemical bond; particularly preferably, represents D-Gln, D-His, Aze(2), Pic(2), Hyp, Thz, Gly, Aib, D-NMeAla, Leu, Lys, Glu, Om, His(3Me), Tyr(PO₃H₂), Pro(4NH₂), D-NMePhe, Hyp(Bzl), cisHyp or Pro(4F), much more preferably, represents Aze(2), Hyp, Gly, Aib, Leu, Lys, Glu, His(3Me), Tyr(PO₃H₂), Hyp(Bzl), cisHyp or Pro(4F), and most preferably, represents Hyp, Glu, Hyp(Bzl) or Pro(4F). As XX3, D-Asp, D-Dap, D-Ser, D-Gln, D-His, D-NMeAla, D-NMePhe, Aze(2), Pic(2), Pic(3), Hyp, Thz, NMeAla, Gly, Aib, Abz(2), Abz(3), Sar, Leu, Lys, Glu, β-alanine, Pzc(2), Om, His(3Me), Tyr(PO₃H₂), Pro(4NH₂) or Hyp(Bzl) is also preferred.

In the formula described above, XX4 represents Asn, 2-amino-3-ureidopropionic acid, Nβ-formyl-β-diaminopropionic acid, Nβ-acetyl-β-diaminopropionic acid, Nω-pentylasparagine, Nω-cyclopropylasparagine, Nω-benzylasparagine, 2,4-diaminobutanoic acid, 2,3-diaminopropionic acid, His, Gln, Gly, Arg, Cit, Nva, D-Asn or the chemical bond, preferably represents Asn, 2-amino-3-ureidopropionic acid, Nω-pentylasparagine, Nω-cyclopropylasparagine, Nω-benzylasparagine, 2,4-diaminobutanoic acid, 2,3-diaminopropionic acid, His, Gln, Gly, Arg, Cit, Nva, D-Asn or the chemical bond, and more preferably, represents Asn or 2-amino-3-ureidopropionic acid. Preferred XX4 is also Asn, 2-amino-3-ureidopropionic acid, Nβ-formyl-β-diaminopropionic acid, Nβ-acctyl-β-diaminopropionic acid, Nω-pentylasparagine, Nω-cyclopropylasparagine, Nω-benzylasparagine, 2,4-diaminobutanoic acid, His, Gln, Cit or D-Asn; or Asn, 2-amino-3-ureidopropionic acid, Nβ-formyldiaminopropionic acid, Nβ-acetyldiaminopropionic acid, Nω-pentylasparagine, Nω-cyclopropylasparagine, Nω-benzylasparagine or 2,4-diaminobutanoic acid.

In the formula described above, XX5 represents Ser, Thr, Val, NMeSer, Gly, Ala, Hyp, D-Ala, D-Thr, D-Pro or the chemical bond; preferably represents Thr, NMeSer, Gly, Ala, Hyp, D-Ala, D-Thr, D-Pro or the chemical bond; more preferably, represents Scr, Thr or Ala, and most preferably represents Thr. As XX5, Ser, Thr, Val, NMeSer, Gly, Ala, Hyp, D-Ala or D-Thr; or, Ser, Thr or Val is also preferred.

In the formula described above, XX6 represents Phe, Tyr, Trp, Tyr(Me), Thi, Nal(2), Cha, Pya(4), threo-Ser(3Phenyl), erythro-Ser(3Phenyl) or an optionally substituted phenylalanine. Herein, the substituents used in the optionally substituted phenylalanine include those (Substituent Group A) selected from, e.g., oxo, a halogen atom (e.g., fluorine, chlorine, bromine, iodine, etc.), a C₁₋₃ alkylenedioxy (e.g., methylenedioxy, ethylenedioxy, etc.), nitro, cyano, an optionally substituted C₁₋₆ alkyl, an optionally substituted C₂₋₆ alkenyl, an optionally substituted C₂₋₆ alkynyl, an optionally substituted C₃₋₈ cycloalkyl, an optionally substituted C₆₋₁₄ aryl, an optionally substituted C₇₋₁₆ aralkyl, an optionally substituted C₁₋₆ alkoxy, hydroxy, an optionally substituted C₆₋₁₄ aryloxy, an optionally substituted C₇₋₁₆ aralkyloxy, mercapto, an optionally substituted C₁₋₆ alkylthio, an optionally substituted C₆₋₁₄ arylthio, an optionally substituted C₇₋₁₆ aralkylthio, an optionally substituted amino [amino, an optionally substituted mono- or di-C₁₋₆ alkyl-amino (e.g., methylamino, dimethylamino, ethylamino, diethylamino, propylamino, isopropylamino, etc.), an optionally substituted mono- or di-C₂₋₆ alkenyl-amino (e.g., vinylamino, propenylamino, isopropenylamino), an optionally substituted C₂₋₆ alkynyl-amino (e.g., 2-butyn-1-yl-amino, 4-pentyn-1-yl-amino, 5-hexyn-1-yl-amino), an optionally substituted mono- or di-C₃₋₈ cycloalkyl-amino (e.g., cyclopropylamino, cyclohexylamino), an optionally substituted C₆₋₁₄ aryl-amino (e.g., phenylamino, diphenylamino, naphthylamino), an optionally substituted C₁₋₆ alkoxy-amino (e.g., methoxyamino, ethoxyamino, propoxyamino, isopropoxyamino), formylamino, an optionally substituted G₁₋₆ alkyl-carbonylamino (e.g., acetylamino, propionylamino, pivaloylamino, etc.), an optionally substituted C₃₋₈ cycloalkyl-carbonylamino (e.g., cyclopropylcarbonylamino, cyclopentylcarbonylamino, cyclohexylcarbonylamino, etc.), an optionally substituted C₆₋₁₄ aryl-carbonylamino (e.g., benzoylamino, naphthoylamino, etc.), an optionally substituted C₁₋₆ alkoxy-carbonylamino (e.g., methoxycarbonylamino, ethoxycarbonylamino, propoxycarbonylamino, butoxycarbonylamino, etc.), an optionally substituted C₁₋₆ alkylsulfonylamino (e.g., methylsulfonylamino, ethylsulfonylamino, etc.), an optionally substituted C₆₋₁₄ arylsulfonylamino (e.g., phenylsulfonylamino, 2-naphthylsulfonylamino, 1-naphthylsulfonylamino, etc.)], formyl, carboxy, an optionally substituted C₁₋₆ alkyl-carbonyl (e.g., acetyl, propionyl, pivaloyl, etc.), an optionally substituted C₃₋₈ cycloalkyl-carbonyl (e.g., cyclopropylcarbonyl, cyclopentylcarbonyl, cyclohexylcarbonyl, 1-methylcyclohexylcarbonyl, etc.), an optionally substituted C₆₋₁₄ aryl-carbonyl (e.g., benzoyl, 1-naphthoyl, 2-naphthoyl, etc.), an optionally substituted C₇₋₁₆ aralkyl-carbonyl (e.g., phenylacetyl, 3-phenylpropionyl, etc.), an optionally substituted 5- to 7-membered heterocyclic carbonyl containing 1 to 4 hetero atoms of 1 or 2 species selected from nitrogen, sulfur and oxygen atoms in addition to carbon atoms (e.g., nicotinoyl, isonicotinoyl, thenoyl, furoyl, morpholinocarbonyl, thiomorpholinocarbonyl, piperazin-1-ylcarbonyl, pyrolidin-1-ylcarbonyl, etc.), an optionally esterified carboxyl, an optionally substituted carbamoyl group, an optionally substituted C₁₋₆ alkylsulfonyl (e.g., methylsulfonyl, ethylsulfonyl, etc.), an optionally substituted C₁₋₆ alkylsulfinyl (e.g., methylsulfinyl, ethylsulfinyl, etc.), an optionally substituted C₆₋₁₄ arylsulfonyl (e.g., phenylsulfonyl, 1-naphthylsulfonyl, 2-naphthylsulfonyl, etc.), an optionally substituted C₆₋₁₄ arylsulfinyl (e.g., phenylsulfinyl, 1-naphthylsulfinyl, 2-naphthylsulfinyl, etc.), an optionally substituted C₁₋₆ alkyl-carbonyloxy (e.g., acetoxy, propionyloxy, etc.), an optionally substituted C₆₋₁₄ aryl-carbonyloxy (e.g., benzoyloxy, naphthylcarbonyloxy, etc.), an optionally substituted C₁₋₆ alkoxy-carbonyloxy (e.g., methoxycarbonyloxy, ethoxycarbonyloxy, propoxycarbonyloxy, butoxycarbonyloxy, etc.), an optionally substituted mono-C₁₋₆ alkylcarbamoyloxy (e.g., methylcarbamoyloxy, ethylcarbamoyloxy, etc.), an optionally substituted di-C₁₋₆ alkylcarbamoyloxy (e.g., dimethylcarbamoyloxy, diethylcarbamoyloxy, etc.), an optionally substituted mono- or di-C₆₋₁₄ arylcarbamoyloxy (e.g., phenylcarbamoyloxy, naphthylcarbamoyloxy, etc.), an optionally substituted heterocyclic group, sulfo, sulfamoyl, sulfinamoyl, sulfenamoyl, a group of 2 or more (e.g., 2 or 3) of these substituents combined, and the like. The number of the substituents is not particularly limited but these groups may have 1 to 5, preferably 1 to 3 substituents in substitutable positions, and when there are two or more substituents, each substituent may be the same or different.

The "optionally esterified carboxyl group" in the Substituent Group A includes, for example, an optionally substituted C₁₋₆ alkoxy-carbonyl (e g., methoxycarbonyl, ethoxycarbonyl, propoxycarbonyl, tert-butoxycarbonyl, etc.), an optionally substituted C₆₋₁₄ aryloxycarbonyl (e.g., phenoxycarbonyl, etc.), an optionally substituted C₇₋₁₆ aralkyloxy-carbonyl (e.g., bcnzyloxycarbonyl, phenethyloxycarbonyl, etc.), and the like.

The "C₁₋₆ alkyl" used in the "optionally substituted C₁₋₆ alkyl" in the Substituent Group A includes, for example, methyl, ethyl, propyl, isopropyl, butyl, isobutyl, sec-butyl, tert-butyl, pentyl, isopentyl, neopentyl, hexyl, etc.

The "C₂₋₆ alkenyl" used in the "optionally substituted C₂₋₆ alkenyl" in the Substituent Group A includes, for example, vinyl, propenyl, isopropenyl, 2-buten-1-yl, 4-penten-1-yl, 5-hexen-1-yl, etc.

The "C₂₋₆ alkynyl" used in the "optionally substituted C₂₋₆ alkynyl" in the Substituent Group A includes, for example, 2-butyn-1-yl, 4-pentyn-1-yl, 5-hexyn-1-yl, etc.

The "C₃₋₈ cycloalkyl" used in the "optionally substituted C₃₋₈ cycloalkyl" in the Substituent Group A includes, for example, cyclopropyl, cyclobutyl, cyclopentyl, cyclohexyl, etc.

The "C₆₋₁₄ aryl" used in the "optionally substituted C₆₋₁₄ aryl" in the Substituent Group A includes, for example, phenyl, 1-naphthyl, 2-naphthyl, 2-biphenylyl, 3-biphenylyl, 4-biphenylyl, 2-anthryl, etc.

The "C₇₋₁₆ aralkyl" used in the "optionally substituted C₇₋₁₆ aralkyl" in the Substituent Group A includes, for example, benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, 2-biphenylylmethyl, 3-biphenylylmethyl, 4-biphenylylmethyl, etc.

The "C₁₋₆ alkoxy" used in the "optionally substituted C₁₋₆ alkoxy" in the Substituent Group A includes, for example, methoxy, ethoxy, propoxy, isopropoxy, butoxy, isobutoxy, sec-butoxy, pentyloxy, hexyloxy, etc.

The "C₆₋₁₄ aryloxy" used in the "optionally substituted C₆₋₁₄ aryloxy" in the Substituent Group A includes, for example, phenyloxy, 1-naphthyloxy, 2-naphthyloxy, etc.

The "C₇₋₁₆ aralkyloxy" used in the "optionally substituted C₇₋₁₆ aralkyloxy" in the Substituent Group A includes, for example, benzyloxy, phenethyloxy, etc.

The "C₁₋₆ alkylthio" used in the "optionally substituted C₁₋₆ alkylthio" in the Substituent Group A includes, for example, methylthio, ethylthio, propylthio, isopropylthio, butylthio, sec-butylthio, tert-butylthio, etc.

The "C₆₋₁₄ arylthio" used in the "optionally substituted C₆₋₁₄ arylthio" in the Substituent Group A includes, for example, phcnylthio, 1-naphthylthio, 2-naphthylthio, etc.

The "C₇₋₁₆ aralkylthio" used in the "optionally substituted C₇₋₁₆ aralkylthio" in the Substituent Group A includes, for example, benzylthio, phenethylthio, etc.

The substituents for these "C₁₋₆ alkoxy-carbonyl," "C₁₋₆ alkyl group," "C₂₋₆ alkenyl," "C₂₋₆ alkynyl," "C₁₋₆ alkoxy," "C₁₋₆ alkylthio," "C₁₋₆ alkyl-amino," "C₂₋₆ alkenyl-amino," "C₂₋₆ alkynyl-amino," C₁₋₆ alkoxy-amino," "C₁₋₆ alkyl-carbonyl," "C₁₋₆ alkylsulfonyl," "C₁₋₆ alkylsulfinyl," "C₁₋₆ alkyl-carbonylamino," "C₁₋₆ alkoxy-carbonylamino," "C₁₋₆ alkylsulfonylamino," "C₁₋₆ alkyl-carbonyloxy," "C₁₋₆ alkoxy-carbonyloxy," "mono-C₁₋₆ alkylcarbamoyloxy" and "di-C₁₋₆ alkylcarbamoyloxy" include, for example, 1 to 5 substituents selected from, e.g., a halogen atom (e.g., fluorine atom, chlorine atom, bromine atom, iodine atom), carboxy, hydroxy, amino, a mono- or di-C₁₋₆ alkylamino, a mono- or di-C₆₋₁₄ arylamino, a C₃₋₈ cycloalkyl, a C₁₋₆ alkoxy, a C₁₋₆ alkoxy-carbonyl, a C₁₋₆ alkylthio, a C₁₋₆ alkylsulfinyl, a C₁₋₆ alkylsulfonyl, the optionally esterified carboxyl described above, carbamoyl, thiocarbamoyl, a mono-C₁₋₆ alkylcarbamoyl (e.g., methylcarbamoyl, ethylcarbamoyl, etc.), a di-C₁₋₆ alkylcarbamoyl (e g., dimethylcarbamoyl, diethylcarbamoyl, ethylmethylcarbamoyl, etc.), a mono- or di-C₆₋₁₄ arylcarbamoyl (e.g., phenylcarbamoyl, 1-naphthylcarbamoyl, 2-naphthylcarbamoyl, etc.), a mono- or di-5- to 7-membered heterocyclic carbamoyl containing 1 to 4 hetero atoms of 1 or 2 species selected from nitrogen, sulfur and oxygen atoms in addition to carbon atoms (e.g., 2-pyridylcarbamoyl, 3-pyridylcarbamoyl, 4-pyridyl carbamoyl, 2-thienylcarbamoyl, 3-thienylcarbamoyl, etc.), and the like.

The substituents for the "C₆₋₁₄ aryloxy-carbonyl," "C₇₋₁₆ aralkyloxy-carbonyl," "C₃₋₈ cycloalkyl," "C₆₋₁₄ aryl," "C₇₋₁₆ aralkyl," "C₆₋₁₄ aryloxy," "C₇₋₁₆ aralkyloxy," "C₆₋₁₄ arylthio," "C₇₋₁₆ aralkylthio," "C₃₋₈ cycloalkyl-amino," "C₆₋₁₄ aryl-amino," "C₃₋₈ cycloalkyl-carbonyl," "C₆₋₁₄ aryl-carbonyl," "C₇₋₁₆ aralkyl-carbonyl," "5- to 7-membered heterocyclic carbonyl containing 1 to 4 hetero atoms of 1 or 2 species selected from nitrogen, sulfur and oxygen atoms in addition to carbon atoms," "C₆₋₁₄ arylsulfonyl," "C₆₋₁₄ arylsulfinyl," "C₃₋₈ cycloalkyl-carbonylamino," "C₆₋₁₄ aryl-carbonylamino," "C₆₋₁₄ arylsulfonylamino," "C₆₋₁₄ aryl-carbonyloxy" and "mono- or di-C₆₋₁₄ arylcarbamoyloxy" in the Substituent Group A include, for example, 1 to 5 substituents selected from, for example, a halogen atom, hydroxy, carboxy, nitro, cyano, the optionally substituted C₁₋₆ alkyl described above, the optionally substituted C₂₋₆ alkenyl described above, the optionally substituted C₂₋₆ alkynyl described above, the optionally substituted C₃₋₈ cycloalkyl described above, the optionally substituted C₁₋₆ alkoxy described above, the optionally substituted C₁₋₆ alkylthio described above, the optionally substituted C₁₋₆ alkylsulfinyl described above, the optionally substituted C₁₋₆ alkylsulfonyl described above, the optionally esterified carboxyl described above, carbamoyl, thiocarbamoyl, a mono-C₁₋₆ alkylcarbamoyl, a di-C₁₋₆ alkylcarbamoyl, a mono- or di-C₆₋₁₄ arylcarbamoyl, a mono- or di-5- to 7-membered heterocyclic carbamoyl containing 1 to 4 hetero atoms of 1 or 2 species selected from nitrogen, sulfur and oxygen atoms in addition to carbon atoms, and the like.

The "optionally substituted heterocyclic group" in the Substituent Group A includes, for example, a 5- to 14-membered (monocyclic, bicyclic or tricyclic) heterocyclic group containing 1 to 4 hetero atoms of 1 or 2 species selected from nitrogen, sulfur and oxygen atoms in addition to carbon atoms, which may optionally be substituted with a halogen atom, hydroxy, carboxy, nitro, cyano, the optionally substituted C₁₋₆ alkyl described above, the optionally substituted C₂₋₆ alkenyl described above, the optionally substituted C₂₋₆ alkynyl described above, the optionally substituted C₃₋₈ cycloalkyl described above, the optionally substituted C₆₋₁₄ aryl described above, the optionally substituted C₁₋₆ alkoxy described above, the optionally substituted C₁₋₆ alkylthio described above, the optionally substituted C₆₋₁₄ arylthio described above, the optionally substituted C₇₋₁₆ aralkylthio described above, the optionally substituted C₁₋₆ alkylsulfinyl described above, the optionally substituted C₆₋₁₄ arylsulfinyl described above, the optionally substituted C₁₋₆ alkylsulfonyl described above, the optionally substituted C₆₋₁₄ arylsulfonyl described above, the optionally esterified carboxyl described above, carbamoyl, thiocarbamoyl, a mono-C₁₋₆ alkylcarbamoyl, a di-lower alkylcarbamoyl, a mono- or di-C₆₋₁₄ arylcarbamoyl, a mono- or di-5- or 7-membered heterocyclic carbamoyl containing 1 to 4 hetero atoms of 1 or 2 species selected from nitrogen, sulfur and oxygen atoms in addition to carbon atoms, or the like; preferably (i) a 5- to 14-membered (preferably, 5- to 10-membered) aromatic heterocyclic group, (ii) a 5- to 10-membered non-aromatic heterocyclic group or (iii) a monovalent group formed by removing one optional hydrogen atom from 7- to 10-membered bridged-hetero ring, and the like, arc employed; *inter alia,* preferably used is a 5-mcmbcrcd aromatic heterocyclic group. Specifically, the heterocyclic group used includes an aromatic heterocyclic group such as thienyl (e.g., 2-thienyl, 3-thienyl), furyl (e.g., 2-furyl, 3-furyl), pyridyl (e.g., 2-pyridyl, 3-pyridyl, 4-pyridyl), thiazolyl (e.g., 2-thiazolyl, 4-thiazolyl, 5-thiazolyl), oxazolyl (e.g., 2-oxazolyl, 4-oxazolyl), quinolyl (e.g., 2-quinolyl, 3-quinolyl, 4-quinolyl, 5-quinolyl, 8-quinolyl), isoquinolyl (e.g., 1-isoquinolyl, 3-isoquinolyl, 4-isoquinolyl, 5-isoquinolyl), pyrazinyl, pyrimidinyl (e.g., 2-pyrimidinyl, 4-pyrimidinyl), pyrrolyl (e.g., 1-pyrrolyl, 2-pyrrolyl, 3-pyrrolyl), imidazolyl (e.g., 1-imidazolyl, 2-imidazolyl, 4-imidazolyl), pyrazolyl (e.g., 1-pyrazolyl, 3-pyrazolyl, 4-pyrazolyl), pyridazinyl (e.g., 3-pyridazinyl, 4-pyridazinyl), isothiazolyl (e.g., 3-isothiazolyl), isoxazolyl (e.g., 3-isoxazolyl), indolyl (e.g., 1-indolyl, 2-indolyl, 3-indolyl), 2-benzothiazolyl, benzo[b]thienyl, (e.g., 2-benzo[b]thienyl, 3-benzo[b]thienyl), benzo[b]furanyl (e.g., 2-benzo[b]furanyl, 3-benzo[b]furanyl), etc., a non-aromatic heterocyclic group such as pyrrolidinyl (e.g., 1-pyrrolidinyl, 2-pyrrolidinyl, 3-pyrrolidinyl), oxazolidinyl (e.g., 2-oxazolidinyl), imidazolinyl (e.g., 1-imidazolinyl, 2-imidazolinyl, 4-imidazolinyl), piperidinyl (e.g., 1-piperidinyl, 2-piperidinyl, 3 -piperidinyl, 4-piperidinyl), piperazinyl (e.g., 1-piperazinyl, 2-piperazinyl), morpholino, thiomorpholino, etc.

The "optionally substituted carbamoyl group" in the Substituent Group A includes a carbamoyl group, which may optionally be substituted with the optionally substituted C₁₋₆ alkyl, optionally substituted C₂₋₆ alkenyl, optionally substituted C₂₋₆ alkynyl, optionally substituted C₃₋₈ cycloalkyl, optionally substituted C₆₋₁₄ aryl, optionally substituted heterocyclic group described above, etc., and specific examples are carbamoyl, thiocarbamoyl, a mono-C₁₋₆ alkylcarbamoyl (e.g., methylcarbamoyl, ethylcarbamoyl, etc.), a di-C₁₋₆ alkylcarbamoyl (e.g., dimethylcarbamoyl, diethylcarbamoyl, ethylmethylcarbamoyl, etc.), a C₁₋₆ alkyl (C₁₋₆ alkoxy)carbamoyl (e.g., methyl (methoxy)carbamoyl, ethyl(methoxy)carbamoyl), a mono- or di-C₆₋₁₄ arylcarbamoyl (e.g., phenylcarbamoyl, 1-naphthylcarbamoyl, 2-naphthylcarbamoyl, etc.), a mono- or di-5- to 7-membered heterocyclic carbamoyl containing 1 to 4 hetero atoms of 1 or 2 species selected from nitrogen, sulfur and oxygen atoms in addition to carbon atoms (e.g., 2-pyridylcarbamoyl, 3-pyridylcarbamoyl, 4-pyridylcarbamoyl, 2-thienylcarbamoyl, 3-thienylcarbamoyl, etc.), a 5- to 7-membered cyclic carbamoyl (e.g., 1-pyrrolidinylcarbonyl, 1-piperidinylcarbonyl, hcxamcthylcnciminocarbonyl), and the like.

The "optionally substituted amino" in the Substituent Group A includes an amino, which may optionally be substituted with 1 or 2 groups selected from the optionally substituted C₁₋₆ alkyl described above, the optionally substituted C₂₋₆ alkenyl described above, the optionally substituted C₂₋₆ alkynyl described above, the optionally substituted C₃₋₈ cycloalkyl described above, the optionally substituted C₆₋₁₄ aryl described above, the optionally substituted C₁₋₆ alkoxy described above, formyl, the optionally substituted C₁₋₆ alkyl-carbonyl described above, the optionally substituted C₃₋₈ cycloalkyl-carbonyl described above, the optionally substituted C₆₋₁₄ aryl-carbonyl described above, the optionally substituted C₁₋₆ alkoxy-carbonyl described above, the optionally substituted C₁₋₆ alkylsulfonyl described above, the optionally substituted C₆₋₁₄ arylsulfonyl), and the like.

More preferably, the substituents are a halogen atom, hydroxy, a C₁₋₆ alkoxy, an optionally halogenated C₁₋₆ alkyl, an optionally halogenated C₁₋₆ alkoxy, amino, nitro, cyano, etc.

XX6 preferably represents Phe, Tyr, Trp, Tyr(Me) [O-methyltyrosine], Thi [2-thienylalanine], Nal(2) [2-naphthylalanine], Cha [cyclohexylalanine], Pya(4) [4-pyridylalanine], Phe(2F) [2-fluorophenylalanine], Phe(3F) [3-fluorophenylalanine], Phe(4F) [4-fluorophenylalanine], Phe(4Cl) [4-chlorophenylalanine], αMePhe [α-methylphenylalanine], Phe(2Me), Phe(3Me), Phe(4Me), threo-Ser(3Phenyl), erythro-Ser(3Phenyl) or D-Phe; more preferably, represents Phe, Cha, Phe(2F), Phe(3F), Phe(4F), Phe(4Cl), αMePhe, Phe(2Me), Phe(3Me), Phe(4Me), threo-Ser(3Phenyl), erythro-Ser(3Phenyl) or D-Phe; much more preferably, represents Phe, Cha, Phe(2F), Phe(3F), Phe(4F), Phe(4Cl), αMePhe, Phe(2Me), Phe(3Me), Phe(4Me), threo-Ser(3Phenyl), erythro-Ser(3Phenyl) or D-Phe; particularly preferably represents Phe, Cha, Phe(2F), Phe(3F), Phe(4F), Phe(4Cl), Phe(2Me), Phe(3Me), Phe(4Me), threo-Ser(3Phenyl) or erythro-Ser(3Phenyl); and most preferably represents Phe, Cha, Phe(3F) or Phe(4F). As XX6, Phe, Tyr, Trp, Tyr(Me), Thi, Nal(2), Cha, Pya(4), Phe(2F), Phe(3F), Phe(4F), Phe(4Cl) or D-Phe; or Phe, Tyr, Trp, Tyr(Me), Thi, Nal(2), Cha, Pya(4), Phe(2F), Phe(3F), Phe(4F) or Phe(4Cl) is also preferred.

In the formula described above, AzaGly represents azaglycine.

In the formula described above, XX8 represents Leu, Nva [norvaline], Val or Ala(cPr) [cyclopropylalaninc], and preferably represents Leu or Ala(cPr). As XX8, Leu, Nva or Val is also preferred.

In the formula described above, XX9 represents an optionally substituted arginine, an optionally substituted lysine or an optionally substituted ornithine. Herein, substituents for the optionally substituted arginine, the optionally substituted lysine or the optionally substituted ornithine are 1 or substitutable numbers of a C₁₋₆ alkyl (e.g., methyl, ethyl, propyl, isopropyl, butyl, etc.), a C₁₋₆ acyl (e g., acetyl, propionyl, etc.). XX9 preferably represents Arg, Om [ornithine], Arg(Me) [Nω-methylarginine], D-Arg or Arg (asymMe₂) [asymmetric-Nω,ω-dimethylarginine], more preferably, represents Arg, Arg(Me) or D-Arg, and much more preferably, represents Arg or Arg(Me). As XX9, Arg, Orn, Arg (Me) or Arg (asymMe₂) is also preferred.

In the formula described above, XX 10 represents 2-naphthylalanine, 2-thienylalanine, tyrosine, an optionally substituted phenylalanine, or an optionally substituted tryptophan. Herein, the substituents used in the optionally substituted phenylalanine and the optionally substituted tryptophan include, for example, oxo, a halogen atom (e.g., fluorine, chlorine, bromine, iodine, etc.), a C₁₋₃ alkylenedioxy (e.g., methylenedioxy, ethylenedioxy, etc.), nitro, cyano, an optionally substituted C₁₋₆ alkyl, an optionally substituted C₂₋₆ alkenyl, an optionally substituted C₂₋₆ alkynyl, an optionally substituted C₃₋₈ cycloalkyl, an optionally substituted C₆₋₁₄ aryl, an optionally substituted C₇₋₁₆ aralkyl, an optionally substituted C₁₋₆ alkoxy, hydroxy, an optionally substituted C₆₋₁₄ aryloxy, an optionally substituted C₇₋₁₆ aralkyloxy, mercapto, an optionally substituted C₁₋₆ alkylthio, an optionally substituted C₆₋₁₄ arylthio, an optionally substituted C₇₋₁₆ aralkylthio, an optionally substituted amino [amino, an optionally substituted mono- or di-C₁₋₆ alkyl-amino (e.g., methylamino, dimethylamino, ethylamino, diethylamino, propylamine, isopropylamino, etc.), an optionally substituted mono- or di-C₂₋₆ alkenyl-amino (e.g., vinylamino, propenylamino, isopropenylamino), an optionally substituted C₂₋₆ alkynyl-amino (e.g., 2-butyn-1-yl-amino, 4-pentyn-1-yl-amino, 5-hexyn-1-yl-amino), an optionally substituted mono- or di-C₃₋₈ cycloalkyl-amino (e.g., cyclopropylamino, cyclohexylamino), an optionally substituted C₆₋₁₄ aryl-amino (e.g., phenylamino, diphenylamino, naphthylamino), an optionally substituted C₁₋₆ alkoxy-amino (e.g., methoxyamino, ethoxyamino, propoxyamino, isopropoxyamino), formylamino, an optionally substituted C₁₋₆ alkyl-carbonylamino (e.g., acetylamino, propionylamino, pivaloylamino, etc.), an optionally substituted C₃₋₈ cycloalkyl-carbonylamino (e.g., cyclopropylcarbonylamino, cyclopentylcarbonylamino, cyclohexylcarbonylamino, etc.), an optionally substituted C₆₋₁₄ aryl-carbonylamino (e.g., benzoylamino, naphthoylamino, etc.), an optionally substituted C₁₋₆ alkoxy-carbonylamino (e.g., methoxycarbonylamino, ethoxycarbonylamino, propoxycarbonylamino, butoxycarbonylamino, etc.), an optionally substituted C₁₋₆ alkylsulfonylamino (e.g., methylsulfonylamino, ethylsulfonylamino, etc.), an optionally substituted C₆₋₁₄ arylsulfonylamino (e.g., phenylsulfonylamino, 2-naphthylsulfonylamino, 1-naphthylsulfonylamino, etc.)], formyl, carboxy, an optionally substituted C₁₋₆ alkyl-carbonyl (e.g., acetyl, propionyl, pivaloyl, etc.), an optionally substituted C₃₋₈ cycloalkyl-carbonyl (e.g., cyclopropylcarbonyl, cyclopentylcarbonyl, cyclohexylcarbonyl, 1-methylcyclohexyl-carbonyl, etc.), an optionally substituted C₆₋₁₄ aryl-carbonyl (e.g., benzoyl, 1-naphthoyl, 2-naphthoyl, etc.), an optionally substituted C₇₋₁₆ aralkyl-carbonyl (e.g., phenylacetyl, 3-phenylpropionyl, etc.), an optionally substituted 5- to 7-membered heterocyclic carbonyl containing 1 to 4 hetero atoms of 1 or 2 species selected from nitrogen, sulfur and oxygen atoms in addition to carbon atoms (e.g., nicotinoyl, isonicotinoyl, thenoyl, furoyl, morpholinocarbonyl, thiomorpholinocarbonyl, piperazin-1-ylcarbonyl, pyrolidin-1-ylcarbonyl, etc.), an optionally esterified carboxyl, an optionally substituted carbamoyl group, an optionally substituted C₁₋₆ alkylsulfonyl (e.g., methylsulfonyl, ethylsulfonyl, etc.), an optionally substituted C₁₋₆ alkylsulfinyl (e.g., methylsulfinyl, ethylsulfinyl, etc.), an optionally substituted C₆₋₁₄ arylsulfonyl (e.g., phenylsulfonyl, 1-naphthylsulfonyl, 2-naphthylsulfonyl, etc.), an optionally substituted C₆₋₁₄ arylsulfinyl (e.g., phenylsulfinyl, 1-naphthylsulfinyl, 2-naphthylsulfinyl, etc.), an optionally substituted C₁₋₆ alkyl-carbonyloxy (e.g., acetoxy, propionyloxy, etc.), an optionally substituted C₆₋₁₄ aryl-carbonyloxy (e.g., benzoyloxy, naphthylcarbonyloxy, etc.), an optionally substituted C₁₋₆ alkoxy-carbonyloxy (e.g., methoxycarbonyloxy, ethoxycarbonyloxy, propoxycarbonyloxy, butoxycarbonyloxy, etc.), an optionally substituted mono-C₁₋₆ alkylcarbamoyloxy (e.g., methylcarbamoyloxy, ethylcarbamoyloxy, etc.), an optionally substituted di-C₁₋₆ alkylcarbamoyloxy (e.g., dimethylcarbamoyloxy, diethylcarbamoyloxy, etc.), an optionally substituted mono- or di-C₆₋₁₄ arylcarbamoyloxy (e.g., phenylcarbamoyloxy, naphthylcarbamoyloxy, etc ), an optionally substituted heterocyclic group, sulfo, sulfamoyl, sulfinamoyl, sulfcnamoyl, or a group of 2 or more (e.g., 2 or 3) of these substituents combined, and the like (Substituent Group A). The number of the substituents is not particularly limited but these groups may have 1 to 5, preferably 1 to 3 substituents at substitutable positions, and when there are two or more substituents, each substituent may be the same or different.

The "optionally esterified carboxyl group" in the Substituent Group A includes, for example, an optionally substituted C₁₋₆ alkoxy-carbonyl (e.g., methoxycarbonyl, ethoxycarbonyl, propoxycarbonyl, tert-butoxycarbonyl, etc.), an optionally substituted C₆₋₁₄ aryloxy-carbonyl (e.g., phenoxycarbonyl, etc.), an optionally substituted C₇₋₁₆ aralkyloxy-carbonyl (e.g., benzyloxycarbonyl, phenethyloxycarbonyl, etc.), and the like.

The "C₁₋₆ alkyl" used in the "optionally substituted C₁₋₆ alkyl" in the Substituent Group A includes, for example, methyl, ethyl, propyl, isopropyl, butyl, isobutyl, sec-butyl, tert-butyl, pentyl, isopentyl, neopentyl, hexyl, etc.

The "C₂₋₆ alkenyl" used in the "optionally substituted C₂₋₆ alkenyl" in the Substituent Group A includes, for example, vinyl, propenyl, isopropenyl, 2-buten-1-yl, 4-penten-1-yl, 5-hexen-1-yl, etc.

The "C₂₋₆ alkynyl" used in the "optionally substituted C₂₋₆ alkynyl" in the Substituent Group A includes, for example, 2-butyn-1-yl, 4-pentyn-1-yl, 5-hexyn-1-yl, etc.

The "C₃₋₈ cycloalkyl" used in the "optionally substituted C₃₋₈ cycloalkyl" in the Substituent Group A includes, for example, cyclopropyl, cyclobutyl, cyclopentyl, cyclohexyl, etc.

The "C₆₋₁₄ aryl" used in the optionally substituted C₆₋₁₄ aryl in the Substituent Group A includes, for example, phenyl, 1-naphthyl, 2-naphthyl, 2-biphenylyl, 3-biphenylyl, 4-biphenylyl, 2-anthryl, etc.

The "C₇₋₁₆ aralkyl" used in the "optionally substituted C₇₋₁₆ aralkyl" in the Substituent Group A includes, for example, benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, 2-biphenylylmethyl, 3-biphenylylmethyl, 4-biphenylylmethyl, etc.

The "C₁₋₆ alkoxy" used in the "optionally substituted C₁₋₆ alkoxy" in the Substituent Group A includes, for example, methoxy, ethoxy, propoxy, isopropoxy, butoxy, isobutoxy, sec-butoxy, pentyloxy, hexyloxy, etc.

The "C₆₋₁₄ aryloxy" used in the "optionally substituted C₆₋₁₄ aryloxy" in the Substituent Group A includes, for example, phcnyloxy, 1-naphthyloxy, 2-naphthyloxy, etc.

The "C₇₋₁₆ aralkyloxy" used in the "optionally substituted C₇₋₁₆ aralkyloxy" in the Substituent Group A includes, for example, benzyloxy, phenethyloxy, etc.

The "C₁₋₆ alkylthio" used in the "optionally substituted C₁₋₆ alkylthio" in the Substituent Group A includes, for example, methylthio, ethylthio, propylthio, isopropylthio, butylthio, sec-butylthio, tert-butylthio, etc.

The "C₆₋₁₄ arylthio" used in the "optionally substituted C₆₋₁₄ arylthio" in the Substituent Group A includes, for example, phenylthio, 1-naphthylthio, 2-naphthylthio, etc.

The "C₇₋₁₆ aralkylthio" used in the "optionally substituted C₇₋₁₆ aralkylthio" in the Substituent Group A includes, for example, benzylthio, phenethylthio, etc.

The substituents for these "C₁₋₆ alkoxy-carbonyl," "C₁₋₆ alkyl group," "C₂₋₆ alkenyl," "C₂₋₆ alkynyl," "C₁₋₆ alkoxy," "C₁₋₆ alkylthio," "C₁₋₆ alkyl-amino," "C₂₋₆ alkenyl-amino," "C₂₋₆ alkynyl-amino," "C₁₋₆ alkoxy-amino," "C₁₋₆ alkyl-carbonyl," "C₁₋₆ alkylsulfonyl," "C₁₋₆ alkylsulfinyl," "C₁₋₆ alkyl-carbonylamino," "C₁₋₆ alkoxy-carbonylamino," "C₁₋₆ alkylsulfonylamino," "C₁₋₆ alkyl-carbonyloxy," "C₁₋₆ alkoxy-carbonyloxy," "mono-C₁₋₆ alkylcarbamoyloxy" and "di-C₁₋₆ alkylcarbamoyloxy" include, for example, 1 to 5 substituents selected from a halogen atom (e.g., fluorine atom, chlorine atom, bromine atom, iodine atom), carboxy, hydroxy, amino, a mono- or di-C₁₋₆ alkylamino, a mono- or di-C₆₋₁₄ arylamino, a C₃₋₈ cycloalkyl, a C₁₋₆ alkoxy, a C₁₋₆ alkoxy-carbonyl, a C₁₋₆ alkylthio, a C₁₋₆ alkylsulfinyl, a C₁₋₆ alkylsulfonyl, the optionally esterified carboxyl described above, carbamoyl, thiocarbamoyl, a mono-C₁₋₆ alkylcarbamoyl (e.g., methylcarbamoyl, ethylcarbamoyl, etc.), a di-C₁₋₆ alkylcarbamoyl (e.g., dimethylcarbamoyl, diethylcarbamoyl, ethylmethylcarbamoyl, etc.), a mono- or di-C₆₋₁₄ arylcarbamoyl (e.g., phenylcarbamoyl, 1-naphthylcarbamoyl, 2-naphthylcarbamoyl, etc.), a mono- or di-5- to 7-membered heterocyclic carbamoyl containing 1 to 4 hetero atoms of 1 or 2 species selected from nitrogen, sulfur and oxygen atoms in addition to carbon atoms (e.g., 2-pyridylcarbamoyl, 3-pyridylcarbamoyl, 4-pyridylcarbamoyl, 2-thienylcarbamoyl, 3-thienylcarbamoyl, etc.), and the like.

The substituents for the "C₆₋₁₄ aryloxy-carbonyl, " "C₇₋₁₆ aralkyloxy-carbonyl," "C₃₋₈ cycloalkyl," "C₆₋₁₄ aryl," "C₁₋₆ aralkyl," "C₆₋₁₄ aryloxy," "C₇₋₁₆ aralkyloxy," "C₆₋₁₄ arylthio," "C₇₋₁₆ aralkylthio," "C₃₋₈ cycloalkyl-amino," "C₆₋₁₄ aryl-amino," "C₃₋₈ cycloalkyl-carbonyl," "C₆₋₁₄ aryl-carbonyl," "C₇₋₁₆ aralkyl-carbonyl," "5- to 7-membered heterocyclic carbonyl containing 1 to 4 hetero atoms of 1 or 2 species selected from nitrogen, sulfur and oxygen atoms in addition to carbon atoms," "C₆₋₁₄ arylsulfonyl," "C₆₋₁₄ arylsulfinyl," "C₃₋₈ cycloalkyl-carbonylamino," "C₆₋₁₄ aryl-carbonylamino," "C₆₋₁₄ arylsulfonylamino," "C₆₋₁₄ aryl-carbonyloxy" and "mono- or di-C₆₋₁₄ arylcarbamoyloxy" in the Substituent Group A include, for example, 1 to 5 substituents selected from a halogen atom, hydroxy, carboxy, nitro, cyano, the optionally substituted C₁₋₆ alkyl described above, the optionally substituted C₂₋₆ alkenyl described above, the optionally substituted C₂₋₆ alkynyl described above, the optionally substituted C₃₋₈ cycloalkyl described above, the optionally substituted C₁₋₆ alkoxy described above, the optionally substituted C₁₋₆ alkylthio described above, the optionally substituted C₁₋₆ alkylsulfinyl described above, the optionally substituted C₁₋₆ alkylsulfonyl described above, the optionally esterified carboxyl described above, carbamoyl, thiocarbamoyl, a mono-C₁₋₆ alkylcarbamoyl, a di-C₁₋₆ alkylcarbamoyl, a mono- or di-C₆₋₁₄ arylcarbamoyl, a mono- or di-5- to 7-membered heterocyclic carbamoyl containing 1 to 4 hetero atoms of 1 or 2 species selected from nitrogen, sulfur and oxygen atoms in addition to carbon atoms, and the like.

The "optionally substituted heterocyclic group" in the Substituent Group A includes, for example, a 5- to 14-membered (monocyclic, bicyclic or tricyclic) heterocyclic group containing 1 to 4 hetero atoms of 1 or 2 species selected from nitrogen, sulfur and oxygen atoms in addition to carbon atoms, which may optionally be substituted with a halogen atom, hydroxy, carboxy, nitro, cyano, the optionally substituted C₁₋₆ alkyl described above, the optionally substituted C₂₋₆ alkenyl described above, the optionally substituted C₂₋₆ alkynyl described above, the optionally substituted C₃₋₈ cycloalkyl described above, the optionally substituted C₆₋₁₄ aryl described above, the optionally substituted C₁₋₆ alkoxy described above, the optionally substituted C₁₋₆ alkylthio described above, the optionally substituted C₆₋₁₄ arylthio described above, the optionally substituted C₇₋₁₆ aralkylthio described above, the optionally substituted C₁₋₆ alkylsulfinyl described above, the optionally substituted C₆₋₁₄ arylsulfinyl described above, the optionally substituted C₁₋₆ alkylsulfonyl described above, the optionally substituted C₆₋₁₄ arylsulfonyl described above, the optionally esterified carboxyl described above, carbamoyl, thiocarbamoyl, a mono-C₁₋₆ alkylcarbamoyl, a di-lower alkylcarbamoyl, a mono- or di-C₆₋₁₄ arylcarbamoyl, a mono- or di-5- or 7-membered heterocyclic carbamoyl containing 1 to 4 hetero atoms of 1 or 2 species selected from nitrogen, sulfur and oxygen atoms in addition to carbon atoms, or the like; preferably, (i) a 5- to 14-membered (preferably, 5- to 10-membered) aromatic heterocyclic group, (ii) a 5- to 10-membered non-aromatic heterocyclic group or (iii) a monovalent group formed by removing one optional hydrogen atom from a 7- to 10-membered bridged-hetero ring, and the like are employed; *inter alia,* a 5-membered aromatic heterocyclic group is preferably used. Specifically, the heterocyclic group used includes, for example, an aromatic heterocyclic group such as thienyl (e.g., 2-thienyl, 3-thienyl), furyl (e.g., 2-furyl, 3-furyl), pyridyl (e.g., 2-pyridyl, 3-pyridyl, 4-pyridyl), thiazolyl (e.g., 2-thiazolyl, 4-thiazolyl, 5-thiazolyl), oxazolyl (e.g., 2-oxazolyl, 4-oxazolyl), quinolyl (e.g., 2-quinolyl, 3-quinolyl, 4-quinolyl, 5-quinolyl, 8-quinolyl), isoquinolyl (e.g., 1-isoquinolyl, 3-isoquinolyl, 4-isoquinolyl, 5-isoquinolyl), pyrazinyl, pyrimidinyl (e.g., 2-pyrimidinyl, 4-pyrimidinyl), pyrrolyl (e.g., 1-pyrrolyl, 2-pyrrolyl, 3-pyrrolyl), imidazolyl (e.g., 1-imidazolyl, 2-imidazolyl, 4-imidazolyl), pyrazolyl (e.g., 1-pyrazolyl, 3-pyrazolyl, 4-pyrazolyl), pyridazinyl (e.g., 3-pyridazinyl, 4-pyridazinyl), isothiazolyl (e.g., 3-isothiazolyl), isoxazolyl (e.g., 3-isoxazolyl), indolyl (e.g., 1-indolyl, 2-indolyl, 3-indolyl), 2-benzothiazolyl, benzo[b]thienyl, (e.g., 2-benzo[b]thienyl, 3-benzo[b]thienyl), benzo[b]furanyl (e.g., 2-benzo[b]furanyl, 3-benzo[b]furanyl), etc.; a non-aromatic heterocyclic group such as pyrrolidinyl (e.g., 1-pyrrolidinyl, 2-pyrrolidinyl, 3-pyrrolidinyl), oxazolidinyl (e.g., 2-oxazolidinyl), imidazolinyl (e.g., 1-imidazolinyl, 2-imidazolinyl, 4-imidazolinyl), piperidinyl (e.g., 1-piperidinyl, 2-piperidinyl, 3-piperidinyl, 4-piperidinyl), piperazinyl (e.g., 1-piperazinyl, 2-piperazinyl), morpholino, thiomorpholino, etc.

The "optionally substituted carbamoyl group" used in the Substituent Group A includes a carbamoyl group, which may optionally be substituted with the optionally substituted C₁₋₆ alkyl, optionally substituted C₂₋₆ alkenyl, optionally substituted C₂₋₆ alkynyl, optionally substituted C₃₋₈ cycloalkyl, optionally substituted C₆-₁₄ aryl, optionally substituted heterocyclic group described above, etc., and specific examples are carbamoyl, thiocarbamoyl, a mono-C₁-₆ alkylcarbamoyl (e.g., methylcarbamoyl, ethylcarbamoyl, etc.), di-C₁-₆ alkylcarbamoyl (e.g., dimcthylcarbamoyl, dicthylcarbamoyl, ethylmethylcarbamoyl, etc.), C₁₋₆ alkyl(C₁₋₆ alkoxy)carbamoyl (e.g., methyl(methoxy)carbamoyl, ethyl(methoxy)carbamoyl), a mono- or di-C₆₋₁₄ arylcarbamoyl (e.g., phenylcarbamoyl, 1-naphthylcarbamoyl, 2-naphthylcarbamoyl, etc.), a mono- or di-5- to 7-membered heterocyclic carbamoyl containing 1 to 4 hetero atoms of 1 or 2 species selected from nitrogen, sulfur and oxygen atoms in addition to carbon atoms (e.g., 2-pyridylcarbamoyl, 3-pyridylcarbamoyl, 4-pyridylcarbamoyl, 2-thienylcarbamoyl, 3-thienylcarbamoyl, etc.), a 5- to 7-membered cyclic carbamoyl (e.g., 1-pyrrolidinylcarbonyl, 1-piperidinylcarbonyl, hexamethyleneiminocarbonyl), and the like.

The "optionally substituted amino" in the Substituent Group A includes an amino, which may optionally be substituted with 1 or 2 groups selected from the optionally substituted C₁₋₆ alkyl described above, the optionally substituted C₂₋₆ alkenyl described above, the optionally substituted C₂₋₆ alkynyl described above, the optionally substituted C₃₋₈ cycloalkyl described above, the optionally substituted C₆₋₁₄ aryl described above, the optionally substituted C₁₋₆ alkoxy described above, formyl, the optionally substituted C₁₋₆ alkyl-carbonyl described above, the optionally substituted C₃₋₈ cycloalkyl-carbonyl described above, the optionally substituted C₆₋₁₄ aryl-carbonyl described above, the optionally substituted C₁₋₆ alkoxy-carbonyl described above, the optionally substituted C₁₋₆ alkylsulfonyl described above, an optionally substituted C₆₋₁₄ arylsulfonyl, and the like.

More preferably, the halogen atom, hydroxy, C₁₋₆ alkoxy, optionally halogenated C₁₋₆ alkyl, optionally halogenated C₁₋₆ alkoxy, amino, nitro, cyano, etc. are preferred as the substituents.

XX10 preferably represents Phe, Trp, 2-naphthylalanine, 2-thienylalanine, tyrosine or 4-fluorophenylalanine, more preferably, represents Phe or Trp, and most preferably represents Trp.

In combinations ofthese groups, the metastin derivatives of the following formula are preferred:
XXO-XX2-XX3-XX4-XX5-XX6-AzaGly-XX8-XX9-XX10-NH₂
wherein:
XX0 represents acetyl (Ac), mesyl (Ms), 4-fluorobenzoyl (4FluoroBenzoyl), decanoyl (Decanoyl), benzylureido (Benzylureido), amidino (Amidino), dimcthylaminocthylurcido (Dimcthylaminocthylurcido) or isobutylureido (iso-Butylureido);
XX2 represents D-Tyr or the chemical bond;
XX3 represents Aze(2), Hyp, Gly, Aib, Leu, Lys, Glu, His(3Me), Tyr(PO₃H₂), Hyp(Bzl) or Pro(4F);
XX4 represents Asn or 2-amino-3-ureidopropionic acid;
XX5 represents Ser, Thr or Ala;
XX6 represents Phe, Cha, Phe(2F), Phe(3F), Phe(4F) or Phe(4Cl);
AzaGly represents azaglycine;
XX8 represents Leu or Ala(cPr);
XX9 represents Arg or Arg(Me); and,
XX10 represents Phe or Trp;
or salts thereof.

In combinations of these groups, the metastin derivatives of the following formula are more preferred:
XX0-XX2-XX3-XX4-XX5-XX6-AzaGly-XX8-XX9-XX10-NH₂
wherein:
XX0 represents acetyl, mesyl (Ms), benzylureido (Benzylureido), ureido or isobutylureido (iso-Butylureido);
XX2 represents D-Tyr;
XX3 represents Hyp, Glu, Hyp(Bzl) or Pro(4F);
XX4 represents Asn or 2-amino-3-ureidopropionic acid;
XX5 represents Thr;
XX6 represents Phe, Cha, Phe(3F) or Phe(4F);
AzaGly represents azaglycine;
XX8 represents Leu or Ala(cPr);
XX9 represents Arg or Arg(Me); and,
XX10 represents Trp;
or salts thereof.

In combinations ofthese groups, the metastin derivatives of the following formula are much more preferred:
XX0-XX2-XX3-XX4-XX5-XX6-AzaGly-XX8-XX9-XX10-NH₂
wherein:
XX0 represents hydrogen, acetyl, mesyl, 4-fluorobenzoyl, decanoyl, benzylureido, ureido, amidino, dimethylaminoethylureido or isobutylureido;
XX2 represents D-Tyr;
XX3 represents Glu, Hyp or Pro(4F);
XX4 represents Asn or 2-amino-3-ureidopropionic acid;
XX5 represents Thr;
XX6 represents Phe or Cha;
AzaGly represents azaglycine;
XX8 represents Leu or Ala(cPr);
XX9 represents arginine which may optionally be substituted with methyl; and,
XX10 represents tryptophan;
or salts thereof.

In the metastin derivatives, all compounds wherein the groups shown by the respective symbols described above are optionally combined are preferably used. The compounds shown by the following compound numbers are also preferably used.
Compound No. 963:
   des(1)-Ms-[D-Tyr2,Hyp3,Thr5,AzaGly7,Arg(Me)9,Trp10]MS10
Compound No. 964:
   des(1)-4-Fluorobenzoyl-[D-Tyr2,Hyp3,Thr5,AzaGly7,Arg(Me)9,Trp10]MS10
Compound No. 965:
   des(1)-decanoyl-[D-Tyr2,Hyp3,Thr5,AzaGly7,Arg(Me)9,Trp10]MS10
Compound No. 966:
   des(1)-Benzylureido-[D-Tyr2,Hyp3,Thr5,AzaGly7,Arg(Me)9,Trp10]MS10
Compound No. 967:
   des(1)-ureido-[D-Tyr2,Hyp3,Thr5,AzaGly7,Arg(Me)9,Trp10]MS10
Compound No. 969:
   des(1)-Ac-[D-Tyr2,Hyp3,Thr5,Cha6,AzaGly7,Arg(Me)9,Trp10]MS10
Compound No. 970:
   des(1)-Ac-[D-Tyr2,Pro(4F)3,Thr5,Cha6,AzaGly7,Arg(Me)9,Trp10]MS10
Compound No. 971:
   des(1)-amidino-[D-Tyr2,Hyp3,Thr5,AzaGly7,Arg(Me)9,Trp10]MS10
Compound No. 972:
   des(1)-dimethylaminoethyl-ureido-[D-Tyr2,Hyp3,Thr5,Cha6,AzaGly7,Arg(Me)9,
Trp10]MS10
Compound No. 973:
   des(1)-iso-Butylurcido-[D-Tyr2,Hyp3,Thr5,Cha6,AzaGly7,Arg(Mc)9,Trp10]MS1 0
Compound No. 975:
   des(1)-Ac-[D-Tyr2,Hyp3,Alb4,Thr5,D-Cha6,Gly7ψf((E)CH=CH)Leu8,Arg(Me)9, Trp10]MS10
Compound No. 976:
   des(1)-Ac-[D-Tyr2,Hyp3,Alb4,Thr5,Cha6,Gly7ψ((E)CH=CH)D-Leu8,Arg(Me)9, Trp10]MS10
Compound No. 977: des(1-2)-[Hyp3,Thr5,AzaGly7,Arg(Me)9,Trp10]MS10 Compound No. 986:
   des(1)-Ac-[D-Tyr2,Hyp3,Alb4,Thr5,Cha6,Gly7ψ((E)CMe=CH)Leu8,Arg(Me)9,T rp10]MS10
Compound No. 1002:
   des(1)-Ac-[D-Tyr2,Hyp3,Thr5,Phe6ψ(CSNH)Gly7,Arg(Me)9,Trp10]MS10
Compound No. 1003:
   des(1)-Ac-[D-Tyr2,Hyp3,Thr5,Phe6ψ(NHCO)Gly7,Arg(Me)9,Trp10]MS10
Compound No. 1004:
   des(1)-Ac-[D-Tyr2,Hyp3,Thr5,Cha6ψCSNH)Gly7,Arg(Me)9,Trp10]MS10
Compound No. 1005:
   des(1)-Ac-[D-Tyr2,Hyp3,Thr5,Cha6ψ(CSNH)Gly7,Ala(cPr)8,Arg(Me)9,Trp10] MS10

The metastin derivatives of the present invention which are preferred are the compounds shown by the following formula, or salts thereof:
XX0-XX2-XX3-XX4-XX5-T-XX9-XX10-NH₂
wherein:
XX0 represents formyl, C₁₋₂₀ alkanoyl, mesyl, optionally substituted ureido, amidino, cyclopropanecarbonyl, 6-(acetyl-D-arginylamino)caproyl, 6-((R)-2,3-diaminopropionylamino)caproyl, 6-(D-norleucylamino)caproyl, 4-(D-arginylamino)butyryl, 3-(4-hydroxyphenyl)propionyl, glycyl, tyrosyl, acetylglycyl, acetylleucyl, D-tyrosyl, acetyl-D-tyrosyl, pyroglutamyl, 3-(pyridin-3-yl)propionyl, adipoyl, glycoloyl or 6-aminocaproyl;
XX2 represents Tyr, D-Tyr, D-Ala, D-Leu, D-Phe, D-Lys, D-Trp or a chemical bond;
XX3 represents D-Asp, D-Dap, D-Ser, D-Gln, D-His, D-NMeAla, D-NMePhe, Aze(2), Pic(2), Pic(3), Hyp, Thz, NMeAla, Gly, Aib, Abz(2), Abz(3), Sar, Leu, Lys, Glu, (β-alanine, Pzc(2), Om, His(3Me), Tyr(PO₃H₂), Pro(4NH₂), Hyp(Bzl), Trp, Pro, 4-pyridylalanine, Tic, D-Trp, D-Ala, D-Leu, D-Phe, D-Lys, D-Glu, D-2-pyridylalanine, D-3-pyridylalanine, D-4-pyridylalanine, Aad, Pro(4F) or a chemical bond;
XX4 represents Asn, 2-amino-3-ureidopropionic acid, Nβ-formyldiaminopropionic acid, Nβ-acetyldiaminopropionic acid, Nω-pentylasparagine, Nω-cyclopropylasparagine, Nω-benzylasparagine, 2,4-diaminobutanoic acid, His, Gln, Cit or D-Asn;
XX5 represents Ser, Thr, Val, NMeSer, Gly, Ala, Hyp, D-Ala, Dap or D-Thr (more preferably, Ser, Thr, Val, NMeSer, Gly, Ala, Hyp, D-Ala or D-Thr);
T represents formula II: 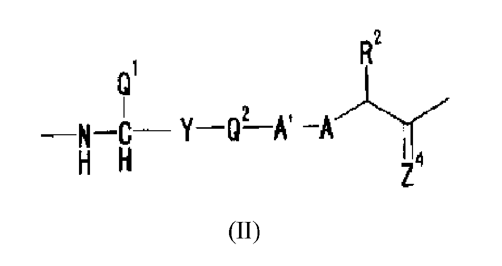
Z⁴ represents, hydrogen atom, O or S;
R² represents (1) hydrogen atom or (2) a cyclic or linear C₁₋₁₀ alkyl group, (3) a C₁₋₁₀ alkyl group consisting of a cyclic alkyl group and a linear alkyl group, or (4) a C₁₋₈ alkyl group which may optionally be substituted with a group selected from the group consisting of an optionally substituted carbamoyl group, an optionally substituted hydroxy group and an optionally substituted aromatic cyclic group;
Q¹ represents a C₁₋₄ alkyl group, which may optionally be substituted with a substituent selected from the group consisting of (1) an optionally substituted C₆₋₁₂ aromatic hydrocarbon group, (2) an optionally substituted 5- to 14-membered aromatic heterocyclic group consisting of 1 to 7 carbon atoms and a hetero atom selected from the group consisting of nitrogen atom, oxygen atom and sulfur atom, (3) an optionally substituted C₈₋₁₄ aromatic condensed cyclic group, (4) an optionally substituted 5- to 14-membered aromatic condensed heterocyclic group consisting of 3 to 11 carbon atoms and a hetero atom selected from the group consisting of nitrogen atom, oxygen atom and sulfur atom, (5) an optionally substituted non-aromatic cyclic hydrocarbon group having not greater than 7 carbon atoms and (6) an optionally substituted non-aromatic heterocyclic group having not greater than 7 carbon atoms;
A represents:
   (1) a nitrogen atom substituted with hydrogen atom or a C₁₋₃ alkyl group;
   (2) a carbon atom substituted with hydrogen atom or a C₁₋₃ alkyl group;
   (3) O; or,
   (4)S;
A' represents:
   (1) a carbon atom which may optionally be substituted with hydrogen atom, O, S, a halogen atom, an optionally halogenated C₁₋₃ alkyl group, carbamoyl or hydroxy;
   (2) a nitrogen atom which may optionally be substituted with hydrogen atom or an optionally halogenated C₁₋₃ alkyl group;
   (3) O; or,
   (4) S;
Q² represents:
   (1) CH₂, CO, CS or C=CH₂, which may optionally be substituted with a C₀₋₄ alkyl group optionally substituted with a substituent selected from the group consisting of carbamoyl, hydroxy, a C₁₋₃ alkoxy group, a halogen atom and amino;
   (2) NH which may optionally be substituted with a C₁₋₄ alkyl group optionally substituted with a substituent selected from the group consisting of carbamoyl and hydroxy; or,
   (3) O;
Y represents:
   (1) a group represented by formula: -CONH-, -CSNH-, -CH₂NH-, -NHCO-, -CH₂O-, -COCH₂-, -CH₂S-, -CSCH₂-, -CH₂SO-, -CH₂SO₂-, -COO-, -CSO-, -CH₂CH₂- or -CH=CH-, which may optionally be substituted with a substituent selected from the group consisting of a C₁₋₆ alkyl group, hydroxy and a halogen atom;
   (2) an optionally substituted C₆₋₇ aromatic hydrocarbon group;
   (3) an optionally substituted 4- to 7-membered aromatic heterocyclic group (preferably, 5- to 7-membered aromatic heterocyclic group) consisting of 1 to 5 carbon atoms and a hetero atom selected from the group consisting of nitrogen atom, oxygen atom and sulfur atom;
   (4) an optionally substituted non-aromatic cyclic hydrocarbon group having not greater than 5 carbon atoms;
   (5) an optionally substituted non-aromatic heterocyclic group having not greater than 5 carbon atoms;
provided that when Y is (2), (3), (4) or (5), Q² may be a chemical bond;
the bonds between Y-Q², Q²-A' and A'-A each independently represents a single bond or a double bond;
XX9 represents Arg, Om, Arg(Me) or Arg(asymMe₂); and,
XX10 represents Phe, Trp, 2-naphthylalanine, 2-thienylalanine, tyrosine or 4-fluorophenylalanine.

As used herein, Tyr-Asn-Trp-Asn-Ser-Phe-Gly-Leu-Arg-Phe-NH₂ (SEQ ID NO: 16) is referred to as Metastin 10, i.e., MS10.

In EXAMPLES later described, the positions of N-terminal Tyr and C-terminal Phe in MS10 are counted as the 1- and 10-positions, respectively.

Tyr-Asn-Trp-Asn-Ser-Phe-Gly-Leu-Arg-Phe-NH2
1 2 3 4 5 6 7 8 9 10

For example, des(1)-Ac-[D-Tyr2,Hyp3,Thr5,Arg(Me)9,Trp10]MS10 means a peptide wherein Tyr at the N terminus (1-position) of MS 10 is deleted, Asn at the 2-position is substituted with D-Tyr, Trp at the 3-position is substituted with Hyp, Ser at the 5-position is substituted with Thr, Arg at the 9-position is substituted with Arg(Me), Phe at the C terminus (10-position) is substituted with Trp and the amino of D-Tyr at the 2-position is modified with Ac.

Each chemical bond "-" between XX0, XX2, XX3, XX4, XX2, T, XX9, XX10 and NH₂ in the formula: "XX1-XX2-XX3-XX4-XX5-T-XX9-XX10-NH₂" has the following meanings.

The chemical bond "-" in the formula "XX0-XX2" means a bond between the group represented by XX0 and the amino group (α-amino group) contained in XX2. More specifically, the formula "XX0-XX2" indicates that the hydrogen atom in the amino group (NH₂) contained in XX2 is substituted with the group represented by XX0.

However, when XX0 is an optionally substituted ureido, the chemical bond means to form an ureido together with the amino group contained in XX2. When the chemical bond is shown with the exception of the amino group contained in XX2, the optionally substituted ureido is described as the optionally substituted carbamoyl.

The chemical bond "-" in the formula "XX2-XX3" means that the carboxyl group (α-carboxyl group) contained in XX2 is bound to the amino group (α-amino group) in XX3 through an amide bond. The chemical bonds "-" in the formulae "XX3-XX4," "XX4-XX5" and "XX9-XX10" also have the same meanings as described above.

The chemical bond "-" in formula "XX5-T" represents a bond between the carboxyl group (α-carboxyl group) in XX5 and the group shown by T. More specifically, the formula "XX5-T" means that -OH in the carboxyl group (-COOH) contained in XX5 is substituted with the group shown by T.

The chemical bond "-" in formula "T-XX9" represents a bond between the group shown by T and the amino group (α-amino group) in XX9. More specifically, the formula "T-XX9" means that the hydrogen atom in the amino group (NH₂) contained in XX9 is substituted with the group shown by T.

The chemical bond "-" in formula "XX10-NH₂" represents a bond between the carboxyl group (α-carboxyl group) in XX10 and -NH₂. More specifically, the formula "XX10-NH₂" means that -OH in the carboxyl group (-COOH) contained in XX10 is substituted with -NH₂.

Where XX2 or/and XX3 represent the chemical bonds "-," these chemical bonds "-" also have the same meanings as described above.

Specific examples of these chemical bonds include the bonds represented by the structural formulae in TABLE 1 later shown.

In the formula: XX0-XX2-XX3-XX4-XX5-T-XX9-XX10-NH₂, XX0 represents formyl, a C₁₋₂₀ alkanoyl (e.g., acetyl, ethylcarbonyl, propylcarbonyl, isopropylcarbonyl, butylcarbonyl, isobutylcarbonyl, sec-butylcarbonyl, tert-butylcarbonyl, pentylcarbonyl, isopentylcarbonyl, neopentylcarbonyl, 1-ethylpropylcarbonyl, hexylcarbonyl, isohexylcarbonyl, 1,1-dimethylbutylcarbonyl, 2,2-dimethylbutylcarbonyl, 3,3-dimethylbutylcarbonyl, 2-ethylbutylcarbonyl, heptylcarbonyl, octylcarbonyl, etc.), mesyl, an optionally substituted ureido group, amidino, cyclopropanecarbonyl, 6-(acetyl-D-arginylamino)caproyl, 6-((R)-2,3-diaminopropionylamino)caproyl, 6-(D-norleucylamino)caproyl, 4-(D-arginylamino)butyryl, 3-(4-hydroxyphenyl)propionyl, glycyl, tyrosyl, acetylglycyl, acetyltyrosyl, D-tyrosyl, acetyl-D-tyrosyl, pyroglutamyl, 3-(pyridin-3-yl)propionyl, adipoyl, glycoloyl or 6-aminocaproyl.

The "optionally substituted ureido group" used includes an optionally substituted ureido group, which may optionally be substituted with the optionally substituted C₁₋₆ alkyl, optionally substituted C₂₋₆ alkenyl, optionally substituted C₂₋₆ alkynyl, optionally substituted C₃₋₈ cycloalkyl, optionally substituted C₆₋₁₄ aryl, optionally substituted heterocyclic group, etc. described above. Specific examples of the optionally substituted ureido group used are ureido, a mono-C₁₋₆ alkylureido (e.g., methylureido, ethylureido, isobutylureido, etc.), a substituted ethylureido (e.g., mono- or di-C₁₋₆ alkylamino-ethylureido such as dimethylaminoethylureido), a di-C₁₋₆ alkylureido (e.g., dimethylureido, diethylureido, ethylmethylureido, etc.), a C₁₋₆ alkyl(C₁₋₆ alkoxy)ureido (e.g., methyl(methoxy)ureido, ethyl(methoxy)ureido), a mono- or di-C₆₋₁₄ arylureido (e.g., phenylureido, 1-naphthylureido, 2-naphthylureido, etc.), a mono- or di-5- or 7-membered heterocyclic ureido containing 1 to 4 hetero atoms of 1 or 2 species selected from nitrogen, sulfur and oxygen atoms in addition to carbon atoms (e.g., 2-pyridylureido, 3-pyridylureido, 4-pyridylureido, 2-thienylureido, 3-thienylureido, etc.), a 5- to 7-membered cyclic ureido (e.g., 1-pyrolidinylcarbonylamino, 1-piperidinylcarbonylamino, hexamethyleneiminocarbonylamino), etc.

XX0 is preferably formyl, C₁₋₂₀ alkanoyl or glycoloyl, more preferably C₁₋₂₀ alkanoyl, much more preferably C₁₋₆ alkanoyl, and most preferably acetyl.

In the formula described above, XX2 represents Tyr, D-Tyr, D-Ala, D-Leu, D-Phe, D-Lys, D-Trp or the chemical bond. XX2 is preferably D-Tyr or the chemical bond, and more preferably D-Tyr.

In the formula described above, XX3 represents D-Asp, D-Dap, D-Ser, D-Gln, D-His, D-NMeAla, D-NMePhe, Aze(2), Pic(2), Pic(3), Hyp, Thz, NMeAla, Gly, Aib, Abz(2), Abz(3), Sar, Leu, Lys, Glu, (β-alanine, Pzc(2), Orn, His(3Me), Tyr(PO₃H₂), Pro(4NH₂), Hyp(Bzl), Trp, Pro, 4-pyridylalanine, Tic, D-Trp, D-Ala, D-Leu, D-Phe, D-Lys, D-Glu, D-2-pyridylalanine, D-3-pyridylalanine, D-4-pyridylalanine, Aad, Pro(4F) or the chemical bond. XX3 is preferably Aze(2), Hyp, Gly, Aib, Leu, Lys, Glu, His(3Me), Tyr(PO₃H₂), Pro(4F) or Hyp(Bzl), and more preferably Hyp, Glu or Pro(4F).

In the formula described above, XX4 represents Asn, 2-amino-3-ureidopropionic acid, Nβ-formyldiaminopropionic acid, Nβ-acetyldiaminopropionic acid, Nωpentylasparagine, Nω-cyclopropylasparagine, Nω-benzylasparagine, 2,4-diaminobutanoic acid, His, Gln, Cit or D-Asn. XX4 is preferably Asn or 2-amino-3-urcidopropionic acid.

In the formula described above, XX5 represents Ser, Thr, Val, NMeSer, Gly, Ala, Hyp, D-Ala, Dap or D-Thr. XX5 is preferably Ser or Thr, and more preferably, Thr.

In the formula described above, T is formula II below.

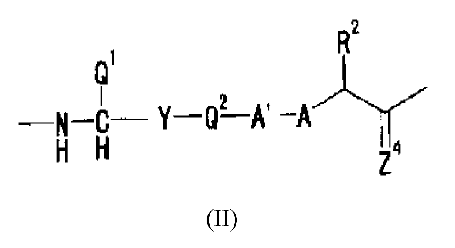

In the formula II shown above, Z⁴ represents hydrogen atom, O or S.

Herein, when Z⁴ represents hydrogen atom, the structure shown by >C=Z⁴ represents the structure >CH₂.

Preferred Z⁴ is O.

R² represents (1) hydrogen atom or (2) a cyclic or linear C₁₋₁₀ alkyl group, (3) a C₁₋₁₀ alkyl group consisting of a cyclic alkyl group and a linear alkyl group, or (4) a C₁₋₈ alkyl group which may optionally be substituted with a group selected from the group consisting of an optionally substituted carbamoyl group, an optionally substituted hydroxy group and an optionally substituted aromatic cyclic group. Among others, (1) hydrogen atom, (2) the cyclic or linear C₁₋₁₀ alkyl group and (3) the C₁₋₁₀ alkyl group consisting of a cyclic alkyl group and a linear alkyl group are preferred, more preferably, the cyclic or linear C₁₋₁₀ alkyl group or the C₁₋₁₀ alkyl group consisting of a cyclic alkyl group and a linear alkyl group.

The cyclic C₁₋₁₀ alkyl group used includes, for example, a C₃₋₈ cycloalkyl group such as cyclopropyl, cyclobutyl, cyclopentyl, cyclohexyl, etc.

The linear C₁₋₁₀ alkyl group includes, for example, methyl, ethyl, propyl, isopropyl, butyl, isobutyl, sec-butyl, tert-butyl, pentyl, isopentyl, neopentyl, hexyl, heptyl, octyl, nonanyl, decanyl, etc.

The C₁₋₁₀ alkyl group consisting of a cyclic alkyl group and a linear alkyl group includes, for example, a C₃₋₇ cycloalkyl-C₁₋₃ alkyl group such as cyclopropylmethyl, cyclobutylmethyl, cyclopentylmethyl, cyclohexylmethyl, etc.

The "C₁₋₈ alkyl group" includes, for example, a linear C₁₋₈ alkyl group such as methyl, ethyl, propyl, isopropyl, butyl, isobutyl, sec-butyl, tert-butyl, pentyl, isopentyl, neopentyl, hcxyl, heptyl, octyl, etc., a cyclic C₁₋₈ alkyl group such as cyclopropyl, cyclobutyl, cyclopentyl, cyclohexyl, etc. Among others, C₁₋₃ alkyl such as methyl, ethyl, etc. are preferred.

The "optionally substituted carbamoyl group" used includes, for example, carbamoyl, a mono-C₁₋₆ alkylcarbamoyl group (e.g., methylcarbamoyl, ethylcarbamoyl, etc.), a di-C₁₋₆ alkylcarbamoyl group (e.g., dimethylcarbamoyl, diethylcarbamoyl, ethylmethylcarbamoyl, etc.), a mono- or di-C₆₋₁₄ arylcarbamoyl group (e.g., phenylcarbamoyl, 1-naphthylcarbamoyl, 2-naphthylcarbamoyl, etc.), a mono- or di-5- to 7-membered heterocyclic carbamoyl group containing 1 to 4 hetero atoms of 1 or 2 species selected from nitrogen, sulfur and oxygen atoms in addition to carbon atoms (e.g., 2-pyridylcarbamoyl, 3-pyridylcarbamoyl, 4-pyridylcarbamoyl, 2-thienylcarbamoyl, 3-thienylcarbamoyl, etc.), or the like.

The "optionally substituted hydroxy group" includes, for example, hydroxy, an optionally substituted C₁₋₆ alkoxy group, an optionally substituted C₆₋₁₄ aryloxy group, an optionally substituted C₇₋₁₆ aralkyloxy group, etc. The "optionally substituted C₁₋₆ alkoxy group," "optionally substituted C₆₋₁₄ aryloxy group" and "optionally substituted C₇₋₁₆ aralkyloxy group" are same as the "optionally substituted C₁₋₆ alkoxy group," "optionally substituted C₆₋₁₄ aryloxy group" and "optionally substituted C₇₋₁₆ aralkyloxy group" in the "Substituent Group A" later described.

The "aromatic cyclic group" used in the "optionally substituted aromatic cyclic group" includes, for example, an aromatic hydrocarbon group, an aromatic heterocyclic group, an aromatic condensed cyclic group, an aromatic condensed heterocyclic group, etc.

The "aromatic hydrocarbon group" used includes, for example, a C₆₋₁₄ aryl group such as phenyl, 2-biphenylyl, 3-biphenylyl, 4-biphenylyl, cyclooctatetraenyl, etc.

The "aromatic heterocyclic group" used includes, for example, a 5- to 14-membered, preferably 5- to 10-membered, more preferably 5- or 6-membered aromatic heterocyclic group containing 1 to 4 hetero atoms of 1 or 2 species selected from nitrogen, sulfur and oxygen atoms in addition to carbon atoms. Specific examples are thienyl (e.g., 2-thienyl, 3-thienyl), furyl (e.g., 2-furyl, 3-furyl), pyridyl (e.g., 2-pyridyl, 3 -pyridyl, 4-pyridyl), thiazolyl (e.g., 2-thiazolyl, 4-thiazolyl, 5-thiazolyl), oxazolyl (e.g., 2-oxazolyl, 4-oxazolyl), pyrazinyl, pyrimidinyl (e.g., 2-pyrimidinyl, 4-pyrimidinyl), pyrrolyl (e.g., 1-pyrrolyl, 2-pyrrolyl, 3-pyrrolyl), imidazolyl (e.g., 1-imidazolyl, 2-imidazolyl, 4-imidazolyl), pyrazolyl (e.g., 1-pyrazolyl, 3-pyrazolyl, 4-pyrazolyl), pyridazinyl (e.g., 3-pyridazinyl, 4-pyridazinyl), isothiazolyl (e.g., 3-isothiazolyl), isoxazolyl (e.g., 3-isooxazolyl), etc.

The "aromatic condensed cyclic group" used includes a C₈₋₁₄ aromatic condensed cyclic group such as naphthyl (e.g., 1-naphthyl, 2-naphthyl), anthryl (e.g., 2-anthryl, 9-anthryl) and the like.

The "aromatic condensed heterocyclic group" used includes, for example, a 5- to 14-memberd (preferably 5- to 10-membered) bicyclic or tricyclic aromatic heterocyclic group containing 1 to 4 hetero atoms of 1 or 2 species selected from nitrogen, sulfur and oxygen atoms in addition to 3 to 11 carbon atoms, or a monovalent group formed by removing one optional hydrogen atom from a 5- to 14-membered (preferably 5- to 10-membered) and 7- to 10-membered aromatic bridged-hetero ring containing 1 to 4 hetero atoms of 1 or 2 species selected from nitrogen, sulfur and oxygen atoms in addition to carbon atoms. Specific examples ofthese groups used are quinolyl (e.g., 2-quinolyl, 3-quinolyl, 4-quinolyl, 5-quinolyl, 8-quinolyl), isoquinolyl (e.g., 1 -isoquinolyl, 3 -isoquinolyl, 4-isoquinolyl, 5-isoquinolyl), indolyl (e.g., 1-indolyl, 2-indolyl, 3-indolyl), 2-benzothiazolyl, benzo[b]thienyl (e.g., 2-benzo[b]thienyl, 3-benzo[b]thienyl), benzo[b]furanyl (e.g., 2-benzo[b]furanyl, 3-benzo[b]furanyl) and the like.

The "substituent" used in the "aromatic cyclic group" includes a substituent selected from the Substituent Group A, which will be later described.

Examples of R² used are methyl, ethyl, propyl, isopropyl, butyl, isobutyl, sec-butyl, tert-butyl, cyclopropylmethyl, cyclobutylmethyl, cyclohexylmethyl, benzyl, hydroxymethyl, 2-carbamoylethyl, tert-pentyl, etc.; *inter alia,* preferred examples are methyl, ethyl, propyl, isopropyl, isobutyl, sec-butyl, tert-butyl, etc., more preferably, propyl, isopropyl, isobutyl, cyclopropylmethyl, etc.

Q¹ represents a C₁₋₄ alkyl group, which may optionally be substituted with a substituent selected from the group consisting of:
(1) an optionally substituted C₆₋₁₂ aromatic hydrocarbon group;
(2) an optionally substituted 5- to 14-membered aromatic heterocyclic group consisting of 1 to 7 carbon atoms and a hetero atom selected from the group consisting of nitrogen atom, oxygen atom and sulfur atom;
(3) an optionally substituted C₈₋₁₄ aromatic condensed cyclic group;
(4) an optionally substituted 5- to 14-membered aromatic condensed heterocyclic group consisting of 3 to 11 carbon atoms and a hetero atom selected from the group consisting of nitrogen atom, oxygen atom and sulfur atom;
(5) an optionally substituted non-aromatic cyclic hydrocarbon group having not greater than 7 carbon atoms; and,
(6) an optionally substituted non-aromatic heterocyclic group having not greater than 7 carbon atoms; (preferably, a substituent selected from the group consisting of (1), (2) and (5) described above, especially a substituent selected from the group consisting of (1) and (5) described above).

The "C₆₋₁₂ aromatic hydrocarbon group" used includes, for example, a monocyclic C₆₋₁₂ aromatic hydrocarbon group such as phenyl, cyclooctatetraenyl, etc.

The "5- to 14-membered aromatic heterocyclic group consisting of 1 to 7 carbon atoms and a hetero atom selected from the group consisting of nitrogen atom, oxygen atom and sulfur atom" used includes, for example, a 5- to 14-membered, preferably 5- to 10-membered, more preferably 5- or 6-membered monocyclic aromatic heterocyclic group containing 1 to 4 hetero atoms of 1 or 2 species selected from nitrogen, sulfur and oxygen atoms in addition to 1 to 7 carbon atoms. Specific examples used are thienyl (e.g., 2-thienyl, 3-thienyl), furyl (e.g., 2-furyl, 3-furyl), pyridyl (e.g., 2-pyridyl, 3-pyridyl, 4-pyridyl), thiazolyl (e.g., 2-thiazolyl, 4-thiazolyl, 5-thiazolyl), oxazolyl (e.g., 2-oxazolyl, 4-oxazolyl), pyrazinyl, pyrimidinyl (e.g., 2-pyrimidinyl, 4-pyrimidinyl), pyrrolyl (e.g., 1-pyrrolyl, 2-pyrrolyl, 3-pyrrolyl), imidazolyl (e.g., 1-imidazolyl, 2-imidazolyl, 4-imidazolyl), pyrazolyl (e.g., 1-pyrazolyl, 3-pyrazolyl, 4-pyrazolyl), pyridazinyl (e.g., 3-pyridazinyl, 4-pyridazinyl), isothiazolyl (e.g., 3-isothiazolyl), isoxazolyl (e.g., 3-isoxazolyl), etc.

The "C₈₋₁₄ aromatic condensed cyclic group" used includes, for example, naphthyl (e.g., 1 -naphthyl, 2-naphthyl), anthryl (e.g., 2-anthryl, 9-anthryl), etc.

The "substituted 5- to 14-membered aromatic condensed heterocyclic group consisting of 3 to 11 carbon atoms and a hetero atom selected from the group consisting of nitrogen atom, oxygen atom and sulfur atom" includes, for example, a 5- to 14-memberd (preferably 5- to 10-membered) bicyclic or tricyclic aromatic heterocyclic group containing 1 to 4 hetero atoms of 1 or 2 species selected from nitrogen, sulfur and oxygen atoms in addition to 3 to 11 carbon atoms, or a monovalent group formed by removing one optional hydrogen atom from a 5- to 14-membered (preferably 5- to 10-membered) and 7- to 10-membered aromatic bridged-hetero ring containing 1 to 4 hetero atoms of 1 or 2 species selected from nitrogen, sulfur and oxygen atoms in addition to carbon atoms. Specific examples of these groups used are quinolyl (e.g., 2-quinolyl, 3-quinolyl, 4-quinolyl, 5-quinolyl, 8-quinolyl), isoquinolyl (e.g., 1-isoquinolyl, 3 -isoquinolyl, 4-isoquinolyl, 5-isoquinolyl), indolyl (e.g., 1-indolyl, 2-indolyl, 3-indolyl), 2-benzothiazolyl, benzo[b]thienyl (e.g., 2-benzo[b]thienyl, 3-benzo[b]thienyl), benzo[b]furanyl (e.g., 2-benzo[b]furanyl, 3-benzo[b]furanyl), etc.

The "non-aromatic cyclic hydrocarbon group having not greater than 7 carbon atoms " used includes, for example, a C₃₋₇ cycloalkyl group such as cyclopropyl, cyclobutyl, cyclopentyl, cyclohexyl, etc.

The "non-aromatic heterocyclic group having not greater than 7 carbon atoms " used includes, for example, a 5- or 10-membered non-aromatic heterocyclic group containing 1 to 4 hetero atoms of 1 or 2 species selected from nitrogen, sulfur and oxygen atoms, in addition to 1 to 7 carbon atoms, such as pyrrolidinyl (e.g., 1-pyrrolidinyl, 2-pyrrolidinyl, 3-pyrrolidinyl), oxazolidinyl (e.g., 2-oxazolidinyl), imidazolinyl (e.g., 1-imidazolinyl, 2-imidazolinyl, 4-imidazolinyl), piperidinyl (e.g., 1-piperidinyl, 2-piperidinyl, 3-piperidinyl, 4-piperidinyl), piperazinyl (e.g., 1-piperazinyl, 2-piperazinyl), morpholino, thiomorpholino, etc.

The substituents used for these "C₆₋₁₂ aromatic hydrocarbon group," "5-to 14-membered aromatic heterocyclic group consisting of 1 to 7 carbon atoms and a hetero atom selected from the group consisting of nitrogen atom, oxygen atom and sulfur atom," "C₈₋₁₄ aromatic condensed cyclic group," "5- to 14-membered aromatic condensed heterocyclic group consisting of 3 to 11 carbon atoms and a hetero atom selected from the group consisting of nitrogen atom, oxygen atom and sulfur atom," "non-aromatic cyclic hydrocarbon group having not greater than 7 carbon atoms " and "non-aromatic heterocyclic group having not greater than 7 carbon atoms " include, for example, substituents (Substituent Group A) selected from oxo, a halogen atom (e.g., fluorine, chlorine, bromine, iodine, etc.), a C₁₋₃ alkylenedioxy (e.g., methylenedioxy, ethylenedioxy, etc.), nitro, cyano, an optionally substituted C₁₋₆ alkyl, an optionally substituted C₂₋₆ alkenyl, an optionally substituted C₂₋₆ alkynyl, an optionally substituted C₃₋₈ cycloalkyl, an optionally substituted C₆₋₁₄ aryl, an optionally substituted C₇₋₁₆ aralkyl, an optionally substituted C₁₋₆ alkoxy, hydroxy, an optionally substituted C₆₋₁₄ aryloxy, an optionally substituted C₇₋₁₆ aralkyloxy, mcrcapto, an optionally substituted C₁₋₆ alkylthio, an optionally substituted C₆₋₁₄ arylthio, an optionally substituted C₇₋₁₆ aralkylthio, an optionally substituted amino [amino, an optionally substituted mono- or di-C₁₋₆ alkyl-amino (e.g., methylamino, dimethylamino, ethylamino, diethylamino, propylamino, isopropylamino, etc.), an optionally substituted mono- or di-C₂₋₆ alkenyl-amino (e.g., vinylamino, propenylamino, isopropenylamino), an optionally substituted C₂₋₆ alkynyl-amino (e.g., 2-butyn-1-yl-amino, 4-pentyn-1-yl-amino, 5-hexyn-1-yl-amino), an optionally substituted mono- or di-C₃₋₈ cycloalkyl-amino (e.g., cyclopropylamino, cyclohexylamino), an optionally substituted C₆₋₁₄ arylamino (e.g., phenylamino, diphenylamino, naphthylamino), an optionally substituted C₁₋₆ alkoxy-amino (e.g., methoxyamino, ethoxyamino, propoxyamino, isopropoxyamino), formylamino, an optionally substituted C₁₋₆ alkyl-carbonylamino (e.g., acetylamino, propionylamino, pivaloylamino, etc.), an optionally substituted C₃₋₈ cycloalkyl-carbonylamino (e.g., cyclopropylcarbonylamino, cyclopentylcarbonylamino, cyclohexylcarbonylamino, etc.), an optionally substituted C₆₋₁₄ aryl-carbonylamino (e.g., benzoylamino, naphthoylamino, etc.), an optionally substituted C₁₋₆ alkoxy-carbonylamino (e.g., methoxycarbonylamino, ethoxycarbonylamino, propoxycarbonylamino, butoxycarbonylamino, etc.), an optionally substituted C₁₋₆ alkylsulfonylamino (e.g., methylsulfonylamino, ethylsulfonylamino, etc.), an optionally substituted C₆₋₁₄ arylsulfonylamino (e.g., phenylsulfonylamino, 2-naphthylsulfonylamino, 1-naphthylsulfonylamino, etc.)], formyl, carboxy, an optionally substituted C₁₋₆ alkyl-carbonyl (e.g., acetyl, propionyl, pivaloyl, etc.), an optionally substituted C₃₋₈ cycloalkyl-carbonyl (e.g., cyclopropylcarbonyl, cyclopentylcarbonyl, cyclohexylcarbonyl, 1-methyl-cyclohexyl-carbonyl, etc.), an optionally substituted C₆₋₁₄ aryl-carbonyl (e.g., benzoyl, 1-naphthoyl, 2-naphthoyl, etc.), an optionally substituted C₇₋₁₆ aralkylcarbonyl (e.g., phenylacetyl, 3-phenylpropionyl, etc.), an optionally substituted 5- to 7-membered heterocyclic carbonyl containing 1 to 4 hetero atoms of 1 or 2 species selected from nitrogen, sulfur and oxygen atoms in addition to carbon atoms (e.g., nicotinoyl, isonicotinoyl, thenoyl, furoyl, morpholinocarbonyl, thiomorpholinocarbonyl, piperazin-1-ylcarbonyl, pyrrolidin-1-ylcarbonyl, etc.), an optionally esterified carboxyl, an optionally substituted carbamoyl group, an optionally substituted C₁₋₆ alkylsulfonyl (e.g., methylsulfonyl, ethylsulfonyl, etc.), an optionally substituted C₁₋₆ alkylsulfinyl (e.g., mcthylsulfinyl, ethylsulfinyl, etc.), an optionally substituted C₆₋₁₄ arylsulfonyl (e.g., phenylsulfonyl, 1-naphthylsulfonyl, 2-naphthylsulfonyl, etc.), an optionally substituted C₆₋₁₄ arylsulfinyl (e.g., phenylsulfinyl, 1-naphthylsulfinyl, 2-naphthylsulfinyl, etc.), an optionally substituted C₁₋₆ alkyl-carbonyloxy (e.g., acetoxy, propionyloxy, etc.), an optionally substituted C₆₋₁₄ aryl-carbonyloxy (e.g., benzoyloxy, naphthylcarbonyloxy, etc.), an optionally substituted C₁₋₆ alkoxy-carbonyloxy (e.g., methoxycarbonyloxy, ethoxycarbonyloxy, propoxycarbonyloxy, butoxycarbonyloxy, etc.), an optionally substituted a mono-C₁₋₆ alkylcarbamoyloxy (e.g., methylcarbamoyloxy, ethylcarbamoyloxy, etc.), an optionally substituted di-C₁₋₆ alkylcarbamoyloxy (e.g., dimethylcarbamoyloxy, diethylcarbamoyloxy, etc.), an optionally substituted mono- or di-C₆₋₁₄ arylcarbamoyloxy (e.g., phenylcarbamoyloxy, naphthylcarbamoyloxy, etc.), an optionally substituted heterocyclic group, sulfo, sulfamoyl, sulfinamoyl, sulfenamoyl, or a group of 2 or more (e.g., 2 or 3) of these substituents combined, and the like. The number of the substituents is not particularly limited but these groups may have 1 to 5, preferably 1 to 3 substituents in their substitutable positions, and when there are two or more substituents, each substituent may be the same or different.

The "optionally esterified carboxyl in the Substituent Group A includes, for example, an optionally substituted C₁₋₆ alkoxy-carbonyl (e.g., methoxycarbonyl, ethoxycarbonyl, propoxycarbonyl, tert-butoxycarbonyl, etc.), an optionally substituted C₆₋₁₄ aryloxy-carbonyl (e.g., phenoxycarbonyl, etc.), an optionally substituted C₇₋₁₆ aralkyloxy-carbonyl (e.g., benzyloxycarbonyl, phenethyloxycarbonyl, etc.), and the like.

The "C₁₋₆ alkyl" in the "optionally substituted C₁₋₆ alkyl" in the Substituent Group A includes, for example, methyl, ethyl, propyl, isopropyl, butyl, isobutyl, sec-butyl, tert-butyl, pentyl, isopentyl, neopentyl, hexyl, etc.

The "C₂₋₆ alkenyl" used in the "optionally substituted C₂₋₆ alkenyl" in the Substituent Group A includes, for example, vinyl, propenyl, isopropenyl, 2-buten-1-yl, 4-penten-1-yl, 5-hexen-1-yl, etc.

The "C₂₋₆ alkynyl" used in the "optionally substituted C₂₋₆ alkynyl" in the Substituent Group A includes, for example, 2-butyn-1-yl, 4-pentyn-1-yl, 5-hexyn-1-yl, etc.

The "C₃₋₈ cycloalkyl" used in the "optionally substituted C₃₋₈ cycloalkyl" in the Substituent Group A includes, for example, cyclopropyl, cyclobutyl, cyclopcntyl, cyclohcxyl, etc.

The "C₆₋₁₄ aryl" used in the "optionally substituted C₆₋₁₄ aryl" in the Substituent Group A includes, for example, phenyl, 1-naphthyl, 2-naphthyl, 2-biphenylyl, 3-biphenylyl, 4-biphenylyl, 2-anthryl, etc.

The "C₇₋₁₆ aralkyl" used in the "optionally substituted C₇₋₁₆ aralkyl" in the Substituent Group A includes, for example, benzyl, phenethyl, diphenylmethyl, 1-naphthylmethyl, 2-naphthylmethyl, 2,2-diphenyl ethyl, 3-phenylpropyl, 4-phenylbutyl, 5-phenylpentyl, 2-biphenylylmethyl, 3-biphenylylmethyl, 4-biphenylylmethyl, etc.

The "C₁₋₆ alkoxy" used in the "optionally substituted C₁₋₆ alkoxy" in the Substituent Group A includes, for example, methoxy, ethoxy, propoxy, isopropoxy, butoxy, isobutoxy, sec-butoxy, pentyloxy, hexyloxy, etc.

The "C₆₋₁₄ aryloxy" used in the "optionally substituted C₆₋₁₄ aryloxy" in the Substituent Group A includes, for example, phenyloxy, 1-naphthyloxy, 2-naphthyloxy, etc.

The "C₇₋₁₆ aralkyloxy," used in the "optionally substituted C₇₋₁₆ aralkyloxy" in the Substituent Group A includes, for example, benzyloxy, phenethyloxy, etc.

The "C₁₋₆ alkylthio" used in the "optionally substituted C₁₋₆ alkylthio" in the Substituent Group A includes, for example, methylthio, ethylthio, propylthio, isopropylthio, butylthio, sec-butylthio, tert-butylthio, etc.

The "C₆₋₁₄ arylthio" used in the "optionally substituted C₆₋₁₄ arylthio" in the Substituent Group A includes, for example, phenylthio, 1-naphthylthio, 2-naphthylthio, etc.

The "C₇₋₁₆ aralkylthio" used in the "optionally substituted C₇₋₁₆ aralkylthio" in the Substituent Group A includes, for example, benzylthio, phenethylthio, etc.

The substituents for these "C₁₋₆ alkoxy-carbonyl," "C₁₋₆ alkyl group," "C₂₋₆ alkenyl," "C₂₋₆ alkynyl," "C₁₋₆ alkoxy," "C₁₋₆ alkylthio," C₁₋₆ alkyl-amino," C₂₋₆ alkenyl-amino," "C₂₋₆ alkynyl-amino," C₁₋₆ alkoxy-amino," "C₁₋₆ alkyl-carbonyl," "C₁₋₆ alkylsulfonyl," "C₁₋₆ alkylsulfinyl," "C₁₋₆ alkyl-carbonylamino," "C₁₋₆ alkoxy-carbonylamino," "C₁₋₆ alkylsulfonylamino," "C₁₋₆ alkyl-carbonyloxy," "C₁₋₆ alkoxy-carbonyloxy," "mono-C₁₋₆ alkylcarbamoyloxy" and "di-C₁₋₆ alkylcarbamoyloxy" include, for example, 1 to 5 substituents selected from a halogen atom (e.g., fluorine atom, chlorine atom, bromine atom, iodine atom), carboxy, hydroxy, amino, a mono- or di-C₁₋₆ alkylamino, a mono- or di-C₆₋₁₄ arylamino, a C₃₋₈ cycloalkyl, a C₁₋₆ alkoxy, a C₁₋₆ alkoxy-carbonyl, a C₁₋₆ alkylthio, a C₁₋₆ alkylsulfinyl, a C₁₋₆ alkylsulfonyl, the optionally esterified carboxyl described above, carbamoyl, thiocarbamoyl, a mono-C₁₋₆ alkylcarbamoyl (e.g., methylcarbamoyl, ethylcarbamoyl, etc.), a di-C₁₋₆ alkylcarbamoyl (e.g., dimethylcarbamoyl, diethylcarbamoyl, ethylmethylcarbamoyl, etc.), a mono- or di-C₆₋₁₄ arylcarbamoyl (e.g., phenylcarbamoyl, 1-naphthylcarbamoyl, 2-naphthylcarbamoyl, etc.), a mono- or di-5- to 7-membered heterocyclic carbamoyl containing 1 to 4 hetero atoms of 1 or 2 species selected from nitrogen, sulfur and oxygen atoms in addition to carbon atoms (e.g., 2-pyridylcarbamoyl, 3-pyridylcarbamoyl, 4-pyridylcarbamoyl, 2-thienylcarbamoyl, 3-thienylcarbamoyl, etc.) and the like.

The substituents in the Substituent Group A for the "C₆₋₁₄ aryloxy-carbonyl," "C₇₋₁₆ aralkyloxy-carbonyl," "C₃₋₈ cycloalkyl," "C₆₋₁₄ aryl," "C₇₋₁₆ aralkyl," "C₆₋₁₄ aryloxy," "C₇₋₁₆ aralkyloxy," "C₆₋₁₄ arylthio," "C₇₋₁₆ aralkylthio," "C₃₋₈ cycloalkyl-amino, "C₆₋₁₄ arylamino," "C₃₋₈ cycloalkylcarbonyl," "C₆₋₁₄ aryl-carbonyl," "C₇₋₁₆ aralkylcarbonyl," "5- to 7-membered heterocyclic carbonyl containing 1 to 4 hetero atoms of 1 or 2 species selected from nitrogen, sulfur and oxygen atoms in addition to carbon atoms," "C₆₋₁₄ arylsulfonyl," "C₆₋₁₄ arylsulfinyl," "C₃₋₈ cycloalkyl-carbonylamino," "C₆₋₁₄ aryl-carbonylamino," "C₆₋₁₄ arylsulfonylamino," "C₆₋₁₄ aryl-carbonyloxy" and "mono- or di-C₆₋₁₄ arylcarbamoyloxy" include, for example, 1 to 5 substituents selected from a halogen atom, hydroxy, carboxy, nitro, cyano, the optionally substituted C₁₋₆ alkyl described above, the optionally substituted C₂₋₆ alkenyl described above, the optionally substituted C₂₋₆ alkynyl described above, the optionally substituted C₃₋₈ cycloalkyl described above, the optionally substituted C₁-₆ alkoxy described above, the optionally substituted C₁-₆ alkylthio described above, the optionally substituted C₁₋₆ alkylsulfinyl described above, the optionally substituted C₁₋₆ alkylsulfonyl described above, the optionally esterified carboxyl described above, carbamoyl, thiocarbamoyl, a mono-C₁₋₆ alkylcarbamoyl, a di-C₁₋₆ alkylcarbamoyl, a mono- or di-C₆₋₁₄ arylcarbamoyl, a mono- or di-5- to 7-membered heterocyclic carbamoyl containing 1 to 4 hetero atoms of 1 or 2 species selected from nitrogen, sulfur and oxygen atoms in addition to carbon atoms, and the like.

The "optionally substituted heterocyclic group" in the Substituent Group A includes, for example, a 5- to 14-membered (monocyclic, bicyclic or tricyclic) heterocyclic group containing 1 to 4 hetero atoms of 1 or 2 species selected from nitrogen, sulfur and oxygen atoms in addition to carbon atoms, which may optionally be substituted with a halogen atom, hydroxy, carboxy, nitro, cyano, the optionally substituted C₁₋₆ alkyl described above, the optionally substituted C₂₋₆ alkenyl described above, the optionally substituted C₂₋₆ alkynyl described above, the optionally substituted C₃₋₈ cycloalkyl described above, the optionally substituted C₆₋₁₄ aryl described above, the optionally substituted C₁₋₆ alkoxy described above, the optionally substituted C₁₋₆ alkylthio described above, the optionally substituted C₆₋₁₄ arylthio described above, the optionally substituted C₇₋₁₆ aralkylthio described above, the optionally substituted C₁₋₆ alkylsulfinyl described above, the optionally substituted C₆₋₁₄ arylsulfinyl described above, the optionally substituted C₁₋₆ alkylsulfonyl described above, the optionally substituted C₆₋₁₄ arylsulfonyl described above, the optionally esterified carboxyl described above, carbamoyl, thiocarbamoyl, a mono-C₁₋₆ alkylcarbamoyl, a di-lower alkylcarbamoyl, a mono- or di-C₆₋₁₄ arylcarbamoyl, a mono- or di-5- to 7-membered heterocyclic carbamoyl containing 1 to 4 hetero atoms of 1 or 2 species selected from nitrogen, sulfur and oxygen atoms in addition to carbon atoms, and the like; preferably, (i) a 5- to 14-membered (preferably, 5- to 10-membered) aromatic heterocyclic group, (ii) a 5- to 10-membered non-aromatic heterocyclic group or (iii) a monovalent group formed by removing one optional hydrogen atom from 7- to 10-membered bridged-hetero ring, and the like are employed; more preferably, a 5-membered aromatic heterocyclic group is used. Specifically, these groups used include an aromatic heterocyclic group such as thienyl (e.g., 2-thienyl, 3-thienyl), furyl (e.g., 2-furyl, 3-furyl), pyridyl (e.g., 2-pyridyl, 3-pyridyl, 4-pyridyl), thiazolyl (e.g., 2-thiazolyl, 4-thiazolyl, 5-thiazolyl), oxazolyl (e.g., 2-oxazolyl, 4-oxazolyl), quinolyl (e.g., 2-quinolyl, 3-quinolyl, 4-quinolyl, 5-quinolyl, 8-quinolyl), isoquinolyl (e.g., 1-isoquinolyl, 3 -isoquinolyl, 4-isoquinolyl, 5-isoquinolyl), pyrazinyl, pyrimidinyl (e.g., 2-pyrimidinyl, 4-pyrimidinyl), pyrrolyl (e.g., 1-pyrrolyl, 2-pyrrolyl, 3-pyrrolyl), imidazolyl (e.g., 1-imidazolyl, 2-imidazolyl, 4-imidazolyl), pyrazolyl (e.g., 1-pyrazolyl, 3-pyrazolyl, 4-pyrazolyl), pyridazinyl (e.g., 3-pyridazinyl, 4-pyridazinyl), isothiazolyl (e.g., 3-isothiazolyl), isoxazolyl (e.g., 3-isoxazolyl), indolyl (e.g., 1-indolyl, 2-indolyl, 3-indolyl), 2-benzothiazolyl, benzo[b]thienyl, (e.g., 2-benzo[b]thienyl, 3-benzo[b]thienyl), benzo[b]furanyl (e.g., 2-bcnzo[b]furanyl, 3-bcnzo[b]furanyl), etc.; a non-aromatic heterocyclic group such as pyrrolidinyl (e.g., 1-pyrrolidinyl, 2-pyrrolidinyl, 3 -pyrrolidinyl), oxazolidinyl (e.g., 2-oxazolidinyl), imidazolinyl (e.g., 1-imidazolinyl, 2-imidazolinyl, 4-imidazolinyl), piperidinyl (e.g., 1-piperidinyl, 2-piperidinyl, 3 -piperidinyl, 4-piperidinyl), piperazinyl (e.g., 1-piperazinyl, 2-piperazinyl), morpholino, thiomorpholino, etc.

The "optionally substituted carbamoyl group" in the Substituent Group A includes a carbamoyl group, which may optionally be substituted with the optionally substituted C₁₋₆ alkyl, optionally substituted C₂₋₆ alkenyl, optionally substituted C₂₋₆ alkynyl, optionally substituted C₃₋₈ cycloalkyl, optionally substituted C₆₋₁₄ aryl, optionally substituted heterocyclic group, etc. described above. Specific examples are carbamoyl, thiocarbamoyl, a mono-C₁₋₆ alkylcarbamoyl (e.g., methylcarbamoyl, ethylcarbamoyl, etc.), a di-C₁₋₆ alkylcarbamoyl (e.g., dimethylcarbamoyl, diethylcarbamoyl, ethylmethylcarbamoyl, etc.), a C₁₋₆ alkyl(C₁₋₆ alkoxy)carbamoyl (e.g., methyl(methoxy)carbamoyl, ethyl(methoxy)carbamoyl), a mono- or di-C₆₋₁₄ arylcarbamoyl (e.g., phenylcarbamoyl, 1-naphthylcarbamoyl, 2-naphthylcarbamoyl, etc.), a mono- or di-5- to 7-membered heterocyclic carbamoyl containing 1 to 4 hetero atoms of 1 or 2 species selected from nitrogen, sulfur and oxygen atoms in addition to carbon atoms (e.g., 2-pyridylcarbamoyl, 3-pyridylcarbamoyl, 4-pyridylcarbamoyl, 2-thienylcarbamoyl, 3-thienylcarbamoyl, etc.), a 5- to 7-membered cyclic carbamoyl (e.g., 1-pyrrolidinylcarbonyl, 1-piperidinylcarbonyl, hexamethyleneiminocarbonyl), and the like.

The "optionally substituted amino" in the Substituent Group A includes an amino, which may optionally be substituted with 1 or 2 groups selected from the optionally substituted C₁₋₆ alkyl described above, the optionally substituted C₂₋₆ alkenyl described above, the optionally substituted C₂₋₆ alkynyl described above, the optionally substituted C₃₋₈ cycloalkyl described above, the optionally substituted C₆₋₁₄ aryl described above, the optionally substituted C₁₋₆ alkoxy described above, formyl, the optionally substituted C₁₋₆ alkyl-carbonyl described above, the optionally substituted C₃₋₈ cycloalkyl-carbonyl described above, the optionally substituted C₆₋₁₄ aryl-carbonyl described above, the optionally substituted C₁₋₆ alkoxy-carbonyl described above, the optionally substituted C₁₋₆ alkylsulfonyl described above, an optionally substituted C₆₋₁₄ arylsulfonyl, and the like.

More preferably, the substituents for the "C₆₋₁₂ aromatic hydrocarbon group," "5- to 14-membered aromatic heterocyclic group consisting of 1 to 7 carbon atoms and a hetero atom selected from the group consisting of nitrogen atom, oxygen atom and sulfur atom," "C₈₋₁₄ aromatic condensed cyclic group," "5- to 14-membered aromatic condensed heterocyclic group consisting of 3 to 11 carbon atoms and a hetero atom selected from the group consisting of nitrogen atom, oxygen atom and sulfur atom," "non-aromatic cyclic hydrocarbon group having not greater than 7 carbon atoms" and "non-aromatic heterocyclic group having not greater than 7 carbon atoms" include a halogen atom, hydroxy, a C₁₋₆ alkoxy, an optionally halogenated C₁₋₆ alkyl, an optionally halogenated C₁₋₆ alkoxy, amino, nitro, cyano, etc.

Q¹ represents:
(1) the "C₁₋₄ alkyl group having an optionally substituted C₆₋₁₂ aromatic hydrocarbon group" such as benzyl, 2-fluorobenzyl, 3-fluorobenzyl, 4-fluorobenzyl, 4-chlorobenzyl, 3,4-difluorobenzyl, 3,4-dichlorobenzyl, pentafluorobenzyl, 4-hydroxybenzyl, 4-methoxybenzyl, 4-trifluoromethylbenzyl, 4-aminobenzyl, 4-nitrobenzyl, 4-cyanobenzyl, phenethyl, etc.;
(2) the "C₁₋₄ alkyl group having an optionally substituted 5- to 14-membered aromatic heterocyclic group consisting of 1 to 7 carbon atoms and a hetero atom selected from the group consisting of nitrogen atom, oxygen atom and sulfur atom" such as 2-pyridylmethyl, 3-pyridylmethyl, 4-pyridylmethyl, 2-thienylmethyl, 3-thienylmethyl, 4-thiazolylmethyl, etc.;
(3) the "C₁₋₄ alkyl group having an optionally substituted C₈₋₁₄ aromatic condensed cyclic group" such as 1-naphthylmethyl, 2-naphthylmethyl, inden-2-ylmethyl, etc.;
(4) the "C₁₋₄ alkyl group having an optionally substituted 5- to 14-membered aromatic condensed heterocyclic group consisting of 3 to 11 carbon atoms and a hetero atom selected from the group consisting of nitrogen atom, oxygen atom and sulfur atom" such as 3-indolemethyl, 1-formylindol-3-ylmethyl, 3-benzo[b]thienylmethyl, 2-quinolylmethyl, etc.;
(5) the "C₁₋₄ alkyl group having an optionally substituted non-aromatic cyclic hydrocarbon group having not greater than 7 carbon atoms " such as cyclohexylmethyl, cyclopentylmethyl, indan-2-ylmethyl, etc.; or,
(6) the "C₁₋₄ alkyl group having an optionally substituted non-aromatic heterocyclic group having not greater than 7 carbon atoms " such as 3-piperidylmethyl, 4-piperidinylmethyl, tetrahydrofurfuryl, tetrahydrofuran-2-yl, tetrahydropyran-3-yl, indolin-3-yl, etc. *Inter alia,* preferred examples are cyclohexylmethyl, benzyl, 4-fluorobenzyl, 4-hydroxybenzyl, 4-methoxybenzyl, pentafluorobenzyl, 2-pyridylmethyl, 4-pyridylmethyl, 1-naphthylmethyl, 2-naphthylmethyl, 3-indolemethyl, 3-piperidylmethyl, 2-thienylmethyl, etc., more preferably, benzyl, 4-fluorobenzyl, 4-pyridylmethyl, cyclohexylmethyl, 3-piperidylmethyl, 2-piperidylmethyl, etc., and most preferably, benzyl and cyclohexylmethyl.

Q² represents (1) CH₂, CO, CS or C=CH₂, which may optionally be substituted with a C₀₋₄ alkyl group optionally substituted with a substituent selected from the group consisting of carbamoyl, hydroxy, a C₁₋₃ alkoxy group, a halogen atom and amino, (2) NH which may optionally be substituted with a C₁₋₄ alkyl group optionally substituted with a substituent selected from the group consisting of carbamoyl and hydroxy, or (3) O. Of these, preferred Q² is (1) CH₂, CO, CS or C=CH₂, which may optionally be substituted with (e.g., 1 or 2) C₁₋₄ alkyl group(s) optionally substituted with a substituent selected from the group consisting of carbamoyl, hydroxy, a C₁₋₃ alkoxy group, a halogen atom and amino, more preferably, (1) CH₂ or C=CH₂, which may optionally be substituted with 1 or 2 C₁₋₄ alkyl group(s) optionally substituted with a substituent selected from the group consisting of carbamoyl, hydroxy, a C₁₋₃ alkoxy group and amino.

The "C₀₋₄ alkyl group" means a bond or a C₁₋₄ alkyl group. More specifically, the "C₀ alkyl group" refers to a bond (e.g., a chemical bond "-"). Accordingly, for example, "CH₂ substituted with the C₀ alkyl group substituted with a substituent selected from the group consisting of carbamoyl, hydroxy, a C₁₋₃ alkoxy group, a halogen atom and amino" represents CH₂ substituted with a substituent selected from the group consisting of carbamoyl, hydroxy, a C₁₋₃ alkoxy group, a halogen atom and amino.

As the "C₀₋₄ alkyl group", a C₁₋₄ alkyl group is preferred.

Examples of the "C₁₋₄ alkyl group" used are methyl, ethyl, propyl, isopropyl, butyl, isobutyl, sec-butyl, tert-butyl, etc.

Examples of the "C₁₋₃ alkoxy group" used are methoxy, ethoxy, propyl, isopropoxy, butoxy, isobutoxy, sec-butoxy, tert-butoxy, etc.

Preferred examples of Q² are CH₂, CH(CH₃), CH(CH₂OH) C(CH₃)₂, C=CH₂, CH(CH₂CH₃), CH(CH₂NH₂), CH(CH₂OCH₃), CH(CH(CH₃)₂), NH, etc., more preferably, CH₂, CH(CH₃), CH(CH₂OH), C(CH₃)₂, C=CH₂, CH(CH₂CH₃), CH(CH₂NH₂), CH(CH₂OCH₃) and CH(CH(CH₃)₂).

Y represents
(1) a group represented by formula: -CONH-, -CSNH-, -CH₂NH-, -NHCO-, -CH₂O- -COCH₂-, -CH₂S-, -CSCH₂-, -CH₂SO-, -CH₂SO₂-, -COO-, -CSO-, -CH₂CH₂- or -CH=CH-, which may optionally be substituted with a substituent selected from the group consisting of a C₁₋₆ alkyl group, hydroxy and a halogen atom;
(2) an optionally substituted C₆₋₇ aromatic hydrocarbon group (e.g., phenyl);
(3) an optionally substituted 4- to 7-membered aromatic heterocyclic group consisting of 1 to 5 carbon atoms and a hetero atom selected from the group consisting of nitrogen atom, oxygen atom and sulfur atom (e.g., 2-pyridyl, 3-pyridyl, 4-pyridyl, 2-thienyl, 3-thienyl, 4-thiazolyl), preferably 5- to 7-membered aromatic heterocyclic group,;
(4) an optionally substituted non-aromatic cyclic hydrocarbon group having not greater than 5 carbon atoms (e.g., cyclobutyl, cyclopentyl); or,
(5) an optionally substituted non-aromatic heterocyclic group having not greater than 5 carbon atoms (e.g., tetrahydrofurfuryl, tetrahydrofuran-2-yl);
provided that when Y is (2), (3), (4) or (5), Q² may be a chemical bond.

The substituents used for these "C₆₋₇ aromatic hydrocarbon group," "4- to 7-membered aromatic heterocyclic group (preferably, 5- to 7-membered aromatic heterocyclic group) consisting of 1 to 5 carbon atoms and a hetero atom selected from the group consisting of nitrogen atom, oxygen atom and sulfur atom," "non-aromatic cyclic hydrocarbon group having not greater than 5 carbon atoms" and "non-aromatic heterocyclic group having not greater than 5 carbon atoms" include, for example, substituents (Substituent Group A) selected from oxo, a halogen atom (e.g., fluorine, chlorine, bromine, iodine, etc.), a C₁₋₃ alkylenedioxy (e.g., methylenedioxy, ethylenedioxy, etc.), nitro, cyano, an optionally substituted C₁₋₆ alkyl, an optionally substituted C₂₋₆ alkenyl, an optionally substituted C₂₋₆ alkynyl, an optionally substituted C₃₋₈ cycloalkyl, an optionally substituted C₆₋₁₄ aryl, an optionally substituted C₇₋₁₆ aralkyl, an optionally substituted C₁₋₆ alkoxy, hydroxy, an optionally substituted C₆₋₁₄ aryloxy, an optionally substituted C₇₋₁₆ aralkyloxy, mercapto, an optionally substituted C₁₋₆ alkylthio, an optionally substituted C₆₋₁₄ arylthio, an optionally substituted C₇₋₁₆ aralkylthio, an optionally substituted amino [amino, an optionally substituted mono- or di-C₁₋₆ alkyl-amino (e.g., methylamino, dimethylamino, ethylamino, diethylamino, propylamino, isopropylamino, etc.), an optionally substituted mono- or di-C₂₋₆ alkenyl-amino (e.g., vinylamino, propenylamino, isopropenylamino), an optionally substituted C₂₋₆ alkynyl-amino (e.g., 2-butyn-1-yl-amino, 4-pentyn-l-yl-amino, 5-hexyn-1-yl-amino), an optionally substituted mono- or di-C₃₋₈ cycloalkyl-amino (e.g., cyclopropylamino, cyclohexylamino), an optionally substituted C₆₋₁₄ arylamino (e.g., phenylamino, diphenylamino, naphthylamino), an optionally substituted C₁₋₆ alkoxy-amino (e.g., methoxyamino, ethoxyamino, propoxyamino, isopropoxyamino), formylamino, an optionally substituted C₁₋₆ alkyl-carbonylamino (e.g., acetylamino, propionylamino, pivaloylamino, etc.), an optionally substituted C₃₋₈ cycloalkyl-carbonylamino (e.g., cyclopropylcarbonylamino, cyclopentylcarbonylamino, cyclohexylcarbonylamino, etc.), an optionally substituted C₆₋₁₄ aryl-carbonylamino (e.g., benzoylamino, naphthoylamino, etc.), an optionally substituted C₁₋₆ alkoxy-carbonylamino (e.g., methoxycarbonylamino, ethoxycarbonylamino, propoxycarbonylamino, butoxycarbonylamino, etc.), an optionally substituted C₁₋₆ alkylsulfonylamino (e.g., methylsulfonylamino, ethylsulfonylamino, etc.), an optionally substituted C₆₋₁₄ arylsulfonylamino (e.g., phenylsulfonylamino, 2-naphthylsulfonylamino, 1-naphthylsulfonylamino, etc.)], formyl, carboxy, an optionally substituted C₁₋₆ alkyl-carbonyl (e.g., acetyl, propionyl, pivaloyl, etc.), an optionally substituted C₃₋₈ cycloalkyl-carbonyl (e.g., cyclopropylcarbonyl, cyclopentylcarbonyl, cyclohexylcarbonyl, 1-methyl-cyclohexyl-carbonyl, etc.), an optionally substituted C₆₋₁₄ aryl-carbonyl (e.g., benzoyl, 1-naphthoyl, 2-naphthoyl, etc.), an optionally substituted C₇₋₁₆ aralkyl-carbonyl (e.g., phenylacetyl, 3-phenylpropionyl, etc.), an optionally substituted 5- to 7-membered heterocyclic carbonyl containing 1 to 4 hetero atoms of 1 or 2 species selected from nitrogen, sulfur and oxygen atoms in addition to carbon atoms (e.g., nicotinoyl, isonicotinoyl, thenoyl, furoyl, morpholinocarbonyl, thiomorpholinocarbonyl, piperazin-1-ylcarbonyl, pyrrolidin-1-ylcarbonyl, etc.), an optionally esterified carboxyl, an optionally substituted carbamoyl group, an optionally substituted C₁₋₆ alkylsulfonyl (e.g., methylsulfonyl, ethylsulfonyl, etc.), an optionally substituted C₁₋₆ alkylsulfinyl (e.g., methylsulfinyl, ethylsulfinyl, etc.), an optionally substituted C₆₋₁₄ arylsulfonyl (e.g., phenylsulfonyl, 1-naphthylsulfonyl, 2-naphthylsulfonyl, etc.), an optionally substituted C₆₋₁₄ arylsulfinyl (e.g., phcnylsulfinyl, 1-naphthylsulfinyl, 2-naphthylsulfinyl, etc.), an optionally substituted C₁₋₆ alkyl-carbonyloxy (e.g., acetoxy, propionyloxy, etc.), an optionally substituted C₆₋₄ aryl-carbonyloxy (e.g., benzoyloxy, naphthylcarbonyloxy, etc.), an optionally substituted C₁₋₆ alkoxy-carbonyloxy (e.g., methoxycarbonyloxy, ethoxycarbonyloxy, propoxycarbonyloxy, butoxycarbonyloxy, etc.), an optionally substituted a mono-C₁₋₆ alkylcarbamoyloxy (e.g., methylcarbamoyloxy, ethylcarbamoyloxy, etc.), an optionally substituted di-C₁₋₆ alkylcarbamoyloxy (e.g., dimethylcarbamoyloxy, diethylcarbamoyloxy, etc.), an optionally substituted a mono- or di-C₆₋₁₄ arylcarbamoyloxy (e.g., phenylcarbamoyloxy, naphthylcarbamoyloxy, etc.), an optionally substituted heterocyclic group, sulfo, sulfamoyl, sulfinamoyl, sulfenamoyl, or a group of 2 or more (e.g., 2 or 3) of these substituents combined, and the like. The number of the substituents is not particularly limited but these groups may have 1 to 5, preferably 1 to 3 substituents in substitutable positions, and when there are two or more substituents, each substituent may be the same or different.

The "optionally esterified carboxyl in the Substituent Group A includes, for example, an optionally substituted C₁₋₆ alkoxy-carbonyl (e.g., methoxycarbonyl, ethoxycarbonyl, propoxycarbonyl, tert-butoxycarbonyl, etc.), an optionally substituted C₆₋₁₄ aryloxy-carbonyl (e.g., phenoxycarbonyl, etc.), an optionally substituted C₇₋₁₆ aralkyloxy-carbonyl (e.g., benzyloxycarbonyl, phenethyloxycarbonyl, etc.), and the like.

The "C₁₋₆ alkyl" used in the "optionally substituted C₁₋₆ alkyl" of the Substituent Group A includes, for example, methyl, ethyl, propyl, isopropyl, butyl, isobutyl, sec-butyl, tert-butyl, pentyl, isopentyl, neopentyl, hexyl, etc.

The "C₂₋₆ alkenyl" in the "optionally substituted C₂₋₆ alkenyl" in the Substituent Group A includes, for example, vinyl, propenyl, isopropenyl, 2-buten-1-yl, 4-penten-1-yl, 5-hexen-1-yl, etc.

The "C₂₋₆ alkynyl" in the "optionally substituted C₂₋₆ alkynyl" of the Substituent Group A includes, for example, 2-butyn-1-yl, 4-pentyn-1-yl, 5-hexyn-1-yl, etc.

The "C₃₋₈ cycloalkyl" in the "optionally substituted C₃₋₈ cycloalkyl" in the Substituent Group A includes, for example, cyclopropyl, cyclobutyl, cyclopentyl, cyclohexyl, etc.

The C₆₋₁₄ aryl in the "optionally substituted C₆₋₁₄ aryl" of the Substituent Group A includes, for example, phenyl, 1-naphthyl, 2-naphthyl, 2-biphcnylyl, 3-biphenylyl, 4-biphenylyl, 2-anthryl, etc.

The "C₇₋₁₆ aralkyl," in the "optionally substituted C₇₋₁₆ aralkyl" of the Substituent Group A includes, for example, benzyl, phenethyl, diphenylmethyl, 1-naphthylmethyl, 2-naphthylmethyl, 2,2-diphenyl ethyl, 3-phenylpropyl, 4-phenylbutyl, 5-phenylpentyl, 2-biphenylylmethyl, 3-biphenylylmethyl, 4-biphenylylmethyl, etc.

The "C₁₋₆ alkoxy" in the "optionally substituted C₁₋₆ alkoxy" of the Substituent Group A includes, for example, methoxy, ethoxy, propoxy, isopropoxy, butoxy, isobutoxy, sec-butoxy, pentyloxy, hexyloxy, etc.

The "C₆₋₁₄ aryloxy" in the "optionally substituted C₆₋₁₄ aryloxy" of the Substituent Group A includes, for example, phenyloxy, 1-naphthyloxy, 2-naphthyloxy, etc.

The "C₇₋₁₆ aralkyloxy" in the "optionally substituted C₇₋₁₆ aralkyloxy" of the Substituent Group A includes, for example, benzyloxy, phenethyloxy, etc.

The "C₁₋₆ alkylthio" in the "optionally substituted C₁₋₆ alkylthio" in the Substituent Group A includes, for example, methylthio, ethylthio, propylthio, isopropylthio, butylthio, sec-butylthio, tert-butylthio, etc.

The "C₆₋₁₄ arylthio" in the "optionally substituted C₆₋₁₄ arylthio" of the Substituent Group A includes, for example, phenylthio, 1-naphthylthio, 2-naphthylthio, etc.

The "C₇₋₁₆ aralkylthio" in the "optionally substituted C₇₋₁₆ aralkylthio" of the Substituent Group A includes, for example, benzylthio, phenethylthio, etc.

The substituents for these "C₁₋₆ alkoxy-carbonyl," "C₁₋₆ alkyl group," "C₂₋₆ alkenyl," "C₂₋₆ alkynyl," "C₁₋₆ alkoxy," "C₁₋₆ alkylthio," C₁₋₆ alkyl-amino," C₂₋₆ alkenyl-amino," "C₂₋₆ alkynyl-amino," C₁₋₆ alkoxy-amino," C₁₋₆ alkyl-carbonyl," "C₁₋₆ alkylsulfonyl," "C₁₋₆ alkylsulfinyl," "C₁₋₆ alkyl-carbonylamino," "C₁₋₆ alkoxy-carbonylamino," "C₁₋₆ alkylsulfonylamino," "C₁₋₆ alkyl-carbonyloxy," "C₁₋₆ alkoxy-carbonyloxy," "mono-C₁₋₆ alkylcarbamoyloxy" and "di-C₁₋₆ alkylcarbamoyloxy" include 1 to 5 substituents selected from, for example, a halogen atom (e.g., fluorine atom, chlorine atom, bromine atom, iodine atom), carboxy, hydroxy, amino, a mono- or di-C₁₋₆ alkylamino, a mono- or di-C₆₋₁₄ arylamino, a C₃₋₈ cycloalkyl, a C₁₋₆ alkoxy, a C₁₋₆ alkoxy-carbonyl, a C₁₋₆ alkylthio, a C₁₋₆ alkylsulfinyl, a C₁₋₆ alkylsulfonyl, the optionally esterified carboxyl described above, carbamoyl, thiocarbamoyl, a mono-C₁₋₆ alkylcarbamoyl (e.g., methylcarbamoyl, cthylcarbamoyl, etc.), a di-C₁₋₆ alkylcarbamoyl (e.g., dimethylcarbamoyl, diethylcarbamoyl, ethylmethylcarbamoyl, etc.), a mono- or di-C₆₋₁₄ arylcarbamoyl (e.g., phenylcarbamoyl, 1-naphthylcarbamoyl, 2-naphthylcarbamoyl, etc.), a mono- or di-5- to 7-membered heterocyclic carbamoyl containing 1 to 4 hetero atoms of 1 or 2 species selected from nitrogen, sulfur and oxygen atoms in addition to carbon atoms (e.g., 2-pyridylcarbamoyl, 3-pyridylcarbamoyl, 4-pyridylcarbamoyl, 2-thienylcarbamoyl, 3-thienylcarbamoyl, etc.) and the like.

The substituents for the "C₆₋₁₄ aryloxy-carbonyl," "C₇₋₁₆ aralkyloxy-carbonyl," "C₃₋₈ cycloalkyl," "C₆₋₁₄ aryl," "C₇₋₁₆ aralkyl," "C₆₋₁₄ aryloxy," "C₇₋₁₆ aralkyloxy," "C₆₋₁₄ arylthio," "C₇₋₁₆ aralkylthio," "C₃₋₈ cycloalkyl-amino," "C₆₋₁₄ aryl-amino," "C₃₋₈ cycloalkyl-carbonyl," "C₆₋₁₄ aryl-carbonyl," "C₇₋₁₆ aralkyl-carbonyl," "5- to 7-membered heterocyclic carbonyl containing 1 to 4 hetero atoms of 1 or 2 species selected from nitrogen, sulfur and oxygen atoms in addition to carbon atoms," "C₆₋₁₄ arylsulfonyl," "C₆₋₁₄ arylsulfinyl," "C₃₋₈ cycloalkyl-carbonylamino," "C₆₋₁₄ aryl-carbonylamino," "C₆₋₁₄ arylsulfonylamino," "C₆₋₁₄ aryl-carbonyloxy" and "mono- or di-C₆₋₁₄ arylcarbamoyloxy" in the Substituent Group A include, for example, 1 to 5 substituents selected from a halogen atom, hydroxy, carboxy, nitro, cyano, the optionally substituted C₁₋₆ alkyl described above, the optionally substituted C₂₋₆ alkenyl described above, the optionally substituted C₂₋₆ alkynyl described above, the optionally substituted C₃₋₈ cycloalkyl described above, the optionally substituted C₁₋₆ alkoxy described above, the optionally substituted C₁₋₆ alkylthio described above, the optionally substituted C₁₋₆ alkylsulfinyl described above, the optionally substituted C₁₋₆ alkylsulfonyl described above, the optionally esterified carboxyl described above, carbamoyl, thiocarbamoyl, a mono-C₁₋₆ alkylcarbamoyl, a di-C₁₋₆ alkylcarbamoyl, a mono- or di-C₆₋₁₄ arylcarbamoyl, a mono- or di-5- to 7-membered heterocyclic carbamoyl containing 1 to 4 hetero atoms of 1 or 2 species selected from nitrogen, sulfur and oxygen atoms in addition to carbon atoms, and the like.

The "optionally substituted heterocyclic group" in the Substituent Group A includes, for example, a 5- to 14-membered (monocyclic, bicyclic or tricyclic) heterocyclic group containing 1 to 4 hetero atoms of 1 or 2 species selected from nitrogen, sulfur and oxygen atoms in addition to carbon atoms, which may optionally be substituted with a halogen atom, hydroxy, carboxy, nitro, cyano, the optionally substituted C₁₋₆ alkyl described above, the optionally substituted C₂₋₆ alkenyl described above, the optionally substituted C₂₋₆ alkynyl described above, the optionally substituted C₃₋₈ cycloalkyl described above, the optionally substituted C₆₋₁₄ aryl described above, the optionally substituted C₁₋₆ alkoxy described above, the optionally substituted C₁₋₆ alkylthio described above, the optionally substituted C₆₋₁₄ arylthio described above, the optionally substituted C₇₋₁₆ aralkylthio described above, the optionally substituted C₁₋₆ alkylsulfinyl described above, the optionally substituted C₆₋₁₄ arylsulfinyl described above, the optionally substituted C₁₋₆ alkylsulfonyl described above, the optionally substituted C₆₋₁₄ arylsulfonyl described above, the optionally esterified carboxyl described above, carbamoyl, thiocarbamoyl, a mono-C₁₋₆ alkylcarbamoyl, a di-lower alkylcarbamoyl, a mono- or di-C₆₋₁₄ arylcarbamoyl, a mono- or di-5- to 7-membered heterocyclic carbamoyl containing 1 to 4 hetero atoms of 1 or 2 species selected from nitrogen, sulfur and oxygen atoms in addition to carbon atoms, and the like; preferably, (i) a 5- to 14-membered (preferably, 5- to 10-membered) aromatic heterocyclic group, (ii) a 5- to 10-membered non-aromatic heterocyclic group or (iii) a monovalent group formed by removing one optional hydrogen atom from 7- to 10-membered bridged-hetero ring, and the like are employed; more preferably, a 5-membered aromatic heterocyclic group is used. Specifically, these groups used include an aromatic heterocyclic group such as thienyl (e.g., 2-thienyl, 3-thienyl), furyl (e.g., 2-furyl, 3-furyl), pyridyl (e.g., 2-pyridyl, 3-pyridyl, 4-pyridyl), thiazolyl (e.g., 2-thiazolyl, 4-thiazolyl, 5-thiazolyl), oxazolyl (e.g., 2-oxazolyl, 4-oxazolyl), quinolyl (e.g., 2-quinolyl, 3-quinolyl, 4-quinolyl, 5-quinolyl, 8-quinolyl), isoquinolyl (e.g., 1-isoquinolyl, 3 -isoquinolyl, 4-isoquinolyl, 5-isoquinolyl), pyrazinyl, pyrimidinyl (e.g., 2-pyrimidinyl, 4-pyrimidinyl), pyrrolyl (e.g., 1-pyrrolyl, 2-pyrrolyl, 3-pyrrolyl), imidazolyl (e.g., 1-imidazolyl, 2-imidazolyl, 4-imidazolyl), pyrazolyl (e.g., 1-pyrazolyl, 3-pyrazolyl, 4-pyrazolyl), pyridazinyl (e.g., 3-pyridazinyl, 4-pyridazinyl), isothiazolyl (e.g., 3-isothiazolyl), isoxazolyl (e.g., 3-isoxazolyl), indolyl (e.g., 1-indolyl, 2-indolyl, 3-indolyl), 2-benzothiazolyl, benzo[b]thienyl, (e.g., 2-benzo[b]thienyl, 3-benzo[b]thienyl), benzo[b]furanyl (e.g., 2-benzo[b]furanyl, 3-benzo[b]furanyl), etc.; a non-aromatic heterocyclic group such as pyrrolidinyl (e.g., 1-pyrrolidinyl, 2-pyrrolidinyl, 3 -pyrrolidinyl), oxazolidinyl (e.g., 2-oxazolidinyl), imidazolinyl (e.g., 1-imidazolinyl, 2-imidazolinyl, 4-imidazolinyl), piperidinyl (e.g., 1-piperidinyl, 2-piperidinyl, 3 -piperidinyl, 4-pipcridinyl), piperazinyl (e.g., 1-piperazinyl, 2-pipcrazinyl), morpholino, thiomorpholino, etc.

The "optionally substituted carbamoyl group" in the Substituent Group A includes a carbamoyl group, which may optionally be substituted with the optionally substituted C₁₋₆ alkyl, optionally substituted C₂₋₆ alkenyl, optionally substituted C₂₋₆ alkynyl, optionally substituted C₃₋₈ cycloalkyl, optionally substituted C₆₋₁₄ aryl, optionally substituted heterocyclic group described above, etc., and specific examples are carbamoyl, thiocarbamoyl, a mono-C₁₋₆ alkylcarbamoyl (e.g., methylcarbamoyl, ethylcarbamoyl, etc.), di-C₁₋₆ alkylcarbamoyl (e.g., dimethylcarbamoyl, diethylcarbamoyl, ethylmethylcarbamoyl, etc.), a C₁₋₆ alkyl (C₁₋₆ alkoxy)carbamoyl (e.g., methyl (methoxy)carbamoyl, ethyl(methoxy)carbamoyl), a mono- or di-C₆₋₁₄ arylcarbamoyl (e.g., phenylcarbamoyl, 1-naphthylcarbamoyl, 2-naphthylcarbamoyl, etc.), a mono- or di-5- to 7-membered heterocyclic carbamoyl containing 1 to 4 hetero atoms of 1 or 2 species selected from nitrogen, sulfur and oxygen atoms in addition to carbon atoms (e.g., 2-pyridylcarbamoyl, 3-pyridylcarbamoyl, 4-pyridylcarbamoyl, 2-thienylcarbamoyl, 3-thienylcarbamoyl, etc.), a 5- to 7-membered cyclic carbamoyl (e.g., 1-pyrrolidinylcarbonyl, 1-piperidinylcarbonyl, hexamethyleneiminocarbonyl), and the like.

The "optionally substituted amino" in the Substituent Group A includes an amino, which may optionally be substituted with 1 or 2 groups selected from the optionally substituted C₁₋₆ alkyl described above, the optionally substituted C₂₋₆ alkenyl described above, the optionally substituted C₂₋₆ alkynyl described above, the optionally substituted C₃₋₈ cycloalkyl described above, the optionally substituted C₆₋₁₄ aryl described above, the optionally substituted C₁₋₆ alkoxy described above described above, formyl, the optionally substituted C₁₋₆ alkyl-carbonyl described above, the optionally substituted C₃₋₈ cycloalkyl-carbonyl described above, the optionally substituted C₆₋₁₄ aryl-carbonyl described above, the optionally substituted C₁₋₆ alkoxy-carbonyl described above, the optionally substituted C₁₋₆ alkylsulfonyl described above, an optionally substituted C₆₋₁₄ arylsulfonyl, and the like.

More preferably, the substituents for these "C₆₋₇ aromatic hydrocarbon group," "4- to 7-membered aromatic heterocyclic group (preferably, 5- to 7-membered aromatic heterocyclic group) consisting of 1 to 5 carbon atoms and a hetero atom selected from the group consisting of nitrogen atom, oxygen atom and sulfur atom," "non-aromatic cyclic hydrocarbon group having not greater than 5 carbon atoms" and "non-aromatic heterocyclic group having not greater than 5 carbon atoms" include, for example, a halogen atom, hydroxy, a C₁₋₆ alkoxy, an optionally halogenated C₁₋₆ alkyl, an optionally halogenated C₁₋₆ alkoxy, amino, nitro, cyano, etc.

The "C₁₋₆ alkyl" includes, for example, methyl, ethyl, propyl, isopropyl, butyl, isobutyl, sec-butyl, tert-butyl, pentyl, isopentyl, neopentyl, hexyl, etc.

Preferred examples of Y are (1) a group represented by formula: -CONH-, -CSNH-, -NHCO-, -CH₂NH-, -CH₂O- -CH₂S-, -COO-, -CSO-, -COCH₂-, -CH₂CH₂- or -CH=CH-, which may optionally be substituted with a substituent selected from the group consisting of a C₁₋₆ alkyl group, hydroxy and a halogen atom (*inter alia,* a group represented by formula: -CONH-, -CSNH-, -NHCO-, -CH₂NH-, -CH₂O- -CH₂S-, -COO-, -CSO-, -COCH₂-, -CH₂CH₂-, -CH(OH)CH₂- or -CH=CH-, particularly, a group represented by formula: -CONH-, -CSNH-, -NHCO-, -CH₂NH-, -CH₂O- -COCH₂-, -CH₂CH₂-, -CH(OH)CH₂- or -CH=CH-, more preferably, a group represented by formula: -CONH-, -CSNH-, -NHCO-, -CH₂NH-, -CH₂O- -CH₂S-, -COCH₂-, -CH=CH-, or -CH₂CH₂-, and much more preferably, a group represented by formula: -CONH-, -CSNH-, -NHCO- or -CH₂NH-).

A represents (1) a nitrogen atom substituted with hydrogen atom or a C₁₋₃ alkyl group, (2) a carbon atom substituted with hydrogen atom or a C₁₋₃ alkyl group, (3) O, or (4) S.

Preferably, A represents (1) a nitrogen atom substituted with hydrogen atom or a C₁₋₃ alkyl group *(inter alia,* a nitrogen atom substituted with hydrogen atom), (2) a carbon atom substituted with hydrogen atom or a C₁₋₃ alkyl group *(inter alia,* a carbon atom substituted with hydrogen atom), or (4) S, and more preferably, represents -NH-, -CH- or S.

The "C₁₋₃ alkyl" used includes methyl, ethyl, propyl or isopropyl.

A' represents:
(1) a carbon atom which may optionally be substituted with hydrogen atom, O, S, a halogen atom, an optionally halogenated C₁₋₃ alkyl group, carbamoyl or hydroxy;
(2) a nitrogen atom which may optionally be substituted with hydrogen atom or an optionally halogenated C₁₋₃ alkyl group;
(3) O; or,
(4) S.

Preferably, A' represents (1) a carbon atom which may optionally be substituted with hydrogen atom, O, S, a halogen atom, an optionally halogenated C₁₋₃ alkyl group, carbamoyl or hydroxy (*inter alia,* a carbon atom substituted with hydrogen atom or O), and more preferably, represents -CH-, -CH₂- or -CO-.

The "C₁₋₃ alkyl" used includes methyl, ethyl, propyl or isopropyl.

The bonds between Y Q², Q²-A' and A'-A each independently represents a single bond or a double bond.

In the formula described above, XX9 represents Arg, Om, Arg(Me) or Arg(asymMe₂). Preferably, XX9 represents Arg or Arg(Me).

In the formula described above, XX10 represents Phe, Trp, 2-naphthylalanine, 2-thienylalanine, tyrosine or 4-fluorophenylalanine. Preferably, XX10 represents Phe or Trp, and more preferably, represents Thr.

In combinations of these groups, the following metastin derivatives or salts thereof are provided.

More specifically, the metastin derivatives or salts thereof which are more preferred in the present invention are the compounds represented by the following formula, or salts thereof:
XX0-XX2-XX3 -XX4-XX5 -T-XX9-XX 10-NH₂
wherein:
XX0 represents formyl, a C₁₋₂₀ alkanoyl or glycoloyl;
XX2 represents D-Tyr or a chemical bond;
XX3 represents Aze(2), Hyp, Gly, Aib, Leu, Lys, Glu, His(3Me), Tyr(PO₃H₂), Pro(4F) or Hyp(Bzl);
XX4 represents Asn or 2-amino-3-ureidopropionic acid;
XX5 represents Ser or Thr;
T represents the group shown by the formula II above;
Z⁴ represents O;
R² represents a linear C₁₋₁₀ alkyl group or a C₁₋₁₀ alkyl group consisting of a cyclic alkyl group and a linear alkyl group;
Q¹ represents a C₁₋₄ alkyl group, which may optionally be substituted with a substituent selected from the group consisting of (1) an optionally substituted C₆₋₁₂ aromatic hydrocarbon group, (2) an optionally substituted 5- to 14-membered aromatic heterocyclic group consisting of 1 to 7 carbon atoms and a hctcro atom selected from the group consisting of nitrogen atom, oxygen atom and sulfur atom, and (5) an optionally substituted non-aromatic cyclic hydrocarbon group having not greater than 7 carbon atoms;
A represents (1) a nitrogen atom substituted with hydrogen atom, (2) a carbon atom substituted with hydrogen atom or (4) S;
A' represents (1) a carbon atom substituted with hydrogen atom or O;
Q² represents (1) CH₂ or C=CH₂, which may optionally be substituted with 1 or 2 C₀₋₄ alkyl groups optionally substituted with a substituent selected from the group consisting of carbamoyl, hydroxy, a C₁₋₃ alkoxy group and amino;
Y represents (1) a group represented by formula: -CONH-, -CH₂O-, -CH₂S-, -COCH₂-, -CH₂CH₂-, -CSNH-, -NHCO- or -CH=CH-, which may optionally be substituted with a substituent selected from the group consisting of a C₁₋₆ alkyl group, hydroxy and a halogen atom;
XX9 represents Arg or Arg(Me); and,
XX10 represents Phe or Trp.

The metastin derivatives or salts thereof which are much more preferred in the present invention are the compounds represented by the following formula, or salts thereof:
XX0-XX2-XX3-XX4-XX5-T-XX9-XX 10-NH2
wherein:
XX0 represents a C₁₋₂₀ (preferably, C₁₋₁₂, more preferably C₁₋₆) alkanoyl;
XX2 represents D-Tyr;
XX3 represents Hyp, Pro(4F) or Glu;
XX4 represents Asn or 2-amino-3-ureidopropionic acid;
XX5 represents Thr;
T represents the group shown by the formula II above;
Z⁴ represents O;
R² represents a cyclic or linear C₁₋₁₀ alkyl group, or a C₁₋₁₀ alkyl group consisting of a cyclic alkyl group and a linear alkyl group;
Q¹ represents a C₁₋₄ alkyl group, which may optionally be substituted with a substituent selected from the group consisting of (1) an optionally substituted C₆₋₁₂ aromatic hydrocarbon group, (2) an optionally substituted 5- to 14-membered aromatic heterocyclic group consisting of 1 to 7 carbon atoms and a hetero atom selected from the group consisting of nitrogen atom, oxygen atom and sulfur atom, and (5) an optionally substituted non-aromatic cyclic hydrocarbon group having not greater than 7 carbon atoms *(inter alia,* a substituent selected from the group consisting of (1) an optionally substituted C₆₋₁₂ aromatic hydrocarbon group and (5) an optionally substituted non-aromatic cyclic hydrocarbon group having not greater than 7 carbon atoms);
A represents (1) a nitrogen atom substituted with hydrogen atom or a C₁₋₃ alkyl group, (2) a carbon atom substituted with hydrogen atom or a C₁₋₃ alkyl group, (3) O or (4) S *(inter alia,* (1) a nitrogen atom substituted with hydrogen atom, (2) a carbon atom substituted with hydrogen atom, or (4) S);
A' represents (1) a carbon atom which may optionally be substituted with hydrogen atom, O, S, a halogen atom, an optionally halogenated C₁₋₃ alkyl group, carbamoyl or hydroxy (*inter alia,* (1) a carbon atom substituted with hydrogen atom or O);
Q² represents (1) CH₂, CO, CS or C=CH₂, which may optionally be substituted with a C₀₋₄ alkyl groups optionally substituted with a substituent selected from the group consisting of carbamoyl, hydroxy, a C₁₋₃ alkoxy group, a halogen atom and amino (*inter alia,* (1) CH₂ or C=CH₂, which may optionally be substituted with 1 or 2 C₁₋₄ alkyl groups optionally substituted with a substituent selected from the group consisting of carbamoyl, hydroxy, a C₁₋₃ alkoxy group and amino);
Y represents (1) a group represented by formula: -CONH-, -CSNH-, -CH₂NH-, -CH₂NH-, -NHCO-, -CH₂O- -CH₂S-, -COCH₂-, -CH=CH- or -CH₂CH₂-, which may optionally be substituted with a substituent selected from the group consisting of a C₁₋₆ alkyl group, hydroxy and a halogen atom (*inter alia,* a group represented by formula: -CONH-, -CSNH-, -CH₂NH- or -NHCO-, which may optionally be substituted with a substituent selected from the group consisting of a C₁₋₆ alkyl group, hydroxy and a halogen atom);
provided that the bonds between Y-Q², Q²-A' and A'-A each independently represents a single bond or a double bond;
XX9 represents Arg or Arg(Me); and,
XX10 represents Trp.

The metastin derivatives or salts thereof which are particularly preferred in the present invention are the compounds represented by the following formula, or salts thereof.
XX0-XX2-XX3 -XX4-XX5-T-XX9-XX 10-NH₂
wherein:
XX0 represents a C₁₋₆ alkanoyl (preferably, acetyl);
XX2 represents D-Tyr;
XX3 represents Hyp, Glu or Pro(4F);
XX4 represents Asn or 2-amino-3-ureidopropionic acid;
XX5 represents Thr;
T represents formula II: 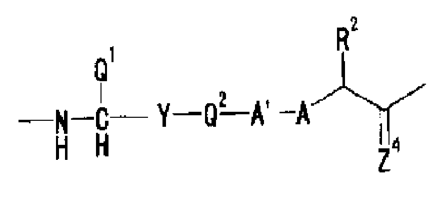
Z⁴ represents O;
R² represents propyl, isopropyl, isobutyl or cyclopropylmethyl;
Q¹ represents benzyl or cyclohexylmethyl;
A represents, -NH-, -CH- or S;
A' represents, -CH-, -CH₂- or -CO-;
Q² represents CH₂, CH(CH₃), CH(CH₂OH) C(CH₃)₂, C=CH₂, CH(CH₂CH₃), CH(CH₂NH₂), CH(CH₂OCH₃) or CH(CH(CH₃)₂);
Y represents a group represented by formula: -CONH-, -CSNH-, -NHCO- or -CH₂NH-;
provided that the bonds between Y-Q², Q²-A' and A'-A each independently represents a single bond or a double bond;
XX9 represents Arg or Arg(Me); and,
XX10 represents Trp.

All of the compounds that are constituted by an optional combination of the groups described above are preferably used as the metastin derivatives of the present invention, and among others, the following compounds are preferred.

The metastin derivatives selected from:
Ac-D-Tyr-Hyp-Asn-Thr-D-Cha-Gly-ψ[(E)-CH=CH]-Leu-Arg(Me)-Trp-NH₂,
Ac-D-Tyr-Hyp-Asn-Thr-Cha-Gly-ψ[(E)-CH=CH]-D-Leu-Arg(Me)-Trp-NH₂,
Ac-D-Tyr-Hyp-Asn-Thr-Cha-Gly-ψ[(E)-CMe=CH]-Leu-Arg(Me)-Trp-NH₂,
Ac-D-Tyr-Hyp-Asn-Thr-Cha-Gly-ψ[(E)-CF=CH]-Leu-Arg(Me)-Trp-NH₂,
Ac-D-Tyr-Hyp-Asn-Thr-Phe-ψ(CSNH)-Gly-Leu-Arg(Me)-Trp-NH₂,
Ac-D-Tyr-Hyp-Asn-Thr-Phe-ψ(NHCO)-Gly-Leu-Arg(Me)-Trp-NH₂,
Ac-D-Tyr-Hyp-Asn-Thr-Phe-ψ(CH₂NH)-AzaGly-Leu-Arg(Me)-Trp-NH₂,
Ac-D-Tyr-Hyp-Asn-Thr-Phe-Gly-ψ(CSNH)-Leu-Arg(Me)-Trp-NH₂,
Ac-D-Tyr-Hyp-Asn-Thr-Cha-ψ(CSNH)-Gly-Leu-Arg(Me)-Trp-NH₂ and
Ac-D-Tyr-Hyp-Asn-Thr-Cha-ψ(CSNH)-Gly-Ala(cPr)-Arg(Me)-Trp-NH₂,
or salts thereof.

The metastin derivatives of the present invention can be prepared by publicly known methods for peptide synthesis. As the methods for peptide synthesis, for example, either solid phase synthesis or liquid phase synthesis may be used. More specifically, the partial peptide or amino acids that can constitute the peptide of the present invention are repeatedly condensed with the remaining part to give the product having a desired sequence. Where the product has protecting groups, these protecting groups are removed to give the desired peptide. Publicly known methods for condensation and removal of the protecting groups are described in (1) to (5) below.
(1) M. Bodanszky & M.A. Ondetti: Peptide Synthesis, Interscience Publishers, New York (1966)
(2) Schroeder & Luebke: The Peptide, Academic Press, New York (1965)
(3) Nobuo Izumiya, et al.: Peptide Gosei-no-Kiso to Jikken (Basics and Experiments of Peptide Synthesis), published by Maruzen Co. (1975)
(4) Haruaki Yajima & Shunpei Sakakibara: Seikagaku Jikken Koza (Biochemical Experiment) 1, Tanpakushitsu no Kagaku (Chemistry of Proteins) IV, 205 (1977)
(5) Haruaki Yajima, ed.: Zoku Iyakuhin no Kaihatsu (A sequel to Development of Pharmaceuticals), Vol. 14, Peptide Synthesis, published by Hirokawa Shoten

After completion of the reaction, the peptide of the present invention may be purified and isolated by a combination of conventional purification methods such as solvent extraction, distillation, column chromatography, liquid chromatography and recrystallization to give the peptide of the present invention. When the peptide obtained by the above methods is in a free form, the peptide can be converted into an appropriate salt by a publicly known method; conversely when the peptide is obtained in a salt form, it can be converted into its free form by publicly known methods.

For condensation of the protected amino acids or peptides, a variety of activation reagents for peptide synthesis may be used, but trisphosphonium salts, tetramethyluronium salts, carbodiimides, etc. are particularly preferred. Examples of trisphosphonium salts include benzotriazol-1-yloxytris(pyrrolidino)phosphonium hexafluorophosphate (PyBOP), bromotris(pyrrolidino) phosphonium hexafluorophosphate (PyBroP) and 7-azabenzotriazol-1-yloxytris(pyrrolidino)phosphonium hexafluorophosphate (PyAOP), examples of tetramethyluronium salts include 2-(1H-benzotriazol-1-yl)-1,1,3,3-hexafluorophosphate (HBTU), 2-(7-azabenzotriazol-1-yl)-1,1,3,3-hexafluorophosphate (HATU), 2-(1H-benzotriazol-1-yl)-1,1,3,3-tetramethyluronium tetrafluoroborate (TBTU), 2-(5-norbornene-2,3-dicarboxyimido)-1,1,3,3-tetramethyluronium tetrafluoroborate (TNTU) and O-(N-succinimidyl)-1,1,3,3-tetramethyluronium tetrafluoroborate (TSTU); examples of carbodiimides include DCC, N,N'-diisopropylcarbodiimide (DIPCDI) and N-ethyl-N'-(3-dimethylaminopropyl) carbodiimide hydrochloride (EDCI.HCl); etc. For condensation using these reagents, the addition of racemization inhibitors (e.g., HONB, HOBt, HOAt, HOOBt, etc.) is preferred. Solvents used in condensation may be appropriately chosen from solvents that are known to be usable for peptide condensation. For example, acid amides such as anhydrous or hydrous N,N-dimethylformamide, N,N-dimethylacetamide, N-methylpyrrolidone, etc., halogenated hydrocarbons such as methylene chloride, chloroform, etc., alcohols such as trifluoroethanol, phenol, etc., sulfoxides such as dimethyl sulfoxide, etc., tertiary amines such as pyridine, etc., ethers such as dioxane, tetrahydrofuran, etc., nitriles such as acetonitrile, propionitrile, etc., esters such as methyl acetate, ethyl acetate, etc., or suitable mixtures thereof, etc. are used. The reaction temperature is appropriately chosen from the range known to be applicable to peptide binding reactions and is normally suitably chosen from the range of about -20°C to 50°C. The activated amino acid derivatives are used generally in 1.5 to 6 times excess. In the case of solid phase synthesis, the condensation is examined using the ninhydrin reaction; when the condensation is insufficient, the condensation can be completed by repeating the condensation reaction without removal of the protecting groups. When the condensation is yet insufficient even after repeating the reaction, the unreacted amino acids are acylated with acetic anhydride or acetylimidazole to cancel any adverse effect on the subsequent reaction.

Examples of the protecting groups used to protect amino groups in the starting amino acids include Z, Boc, tert-pentyloxycarbonyl, isobornyloxycarbonyl, 4-mcthoxybcnzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, trityl, etc. Examples of protecting groups for carboxyl include, in addition to the C₁₋₆ alkyl group, C₃₋₈ cycloalkyl group and C₇₋₁₄ aralkyl group for R described above, allyl, 2-adamantyl, 4-nitrobenzyl, 4-methoxybenzyl, 4-chlorobenzyl, phenacyl, benzyloxycarbonylhydrazide, tert-butoxycarbonylhydrazide, tritylhydrazide, etc.

The hydroxyl group of serine and threonine can be protected, for example, by esterification or etherification. Examples of the group suitable for this esterification include a group derived from organic acids, e.g. a lower (C₂₋₄) alkanoyl group such as acetyl, an aroyl group such as benzoyl, etc. Examples of the group suitable for the etherification include benzyl, tetrahydropyranyl, tert-butyl, trytyl (Trt), etc.

Examples of groups for protecting the phenolic hydroxyl group of tyrosine include Bzl, 2,6-dichlorobenzyl, 2-nitrobenzyl, Br-Z, tert-butyl, etc.

Examples of groups used to protect the imidazole moiety of histidine include Tos, 4-methoxy-2,3,6-trimethylbenzenesulfonyl (Mtr), DNP, Bom, Bum, Boc, Trt, Fmoc, etc.

Examples of protecting groups for the guanidino group in arginine include Tos, Z, 4-methoxy-2,3,6-trimethylbenzenesulfonyl (Mtr), p-methoxybenzenesulfonyl (MBS), 2,2,5,7,8-pentamethylchroman-6-sulfonyl (Pmc), mesitylene-2-sulfonyl (Mts), 2,2,4,6,7-pentamethyldihydrobenzofuran-5-sulfonyl (Pbf), Boc, Z, NO₂ etc.

Examples of protecting groups for side chain amino group of lysine include Z, CI-Z, trifluoroacetyl, Boc, Fmoc, Trt, Mtr, 4,4-dimethyl-2,6-dioxocyclohexylidenethyl (Dde), etc.

Examples of protecting groups for the indolyl of tryptophan include formyl (For), Z, Boc, Mts, Mtr, etc.

Examples of protecting groups for asparagine and glutamine include Trt, xanthyl (Xan), 4,4'-dimethoxybenzhydryl (Mbh), 2,4,6-trimethoxybenzyl (Tmob), etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, 1-hydroxybenzotriazole (HOBt) or 1-hydroxy-7-azabenzotriazole (HOAt)], etc. As the amino acids in which the amino groups in the starting material are activated, the corresponding phosphorous amides are employed.

To eliminate (remove) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid, trifluoroacetic acid, trimethylsilane bromide (TMSBr), trimethylsilyl trifluoromethanesulfonate, tetrafluoroboric acid, tris(trifluoro)boron, boron tribromide or a mixed solution thereof, a base treatment with diisopropylethylamine, triethylamine, piperidine, piperazine, etc., and reduction with sodium in liquid ammonia. The elimination of protecting groups by the acid treatment described above is carried out generally at a temperature of approximately -20°C to 40°C. In the acid treatment, it is efficient to add a cation scavenger such as anisole, phenol, thioanisole, m-cresol, p-cresol, etc., dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is removed by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, removal of the protecting groups and activation of functional groups involved in the reaction may be appropriately chosen from publicly known groups and publicly known means.

In another method for obtaining the amides of the peptide, for example, solid phase synthesis is carried out using a resin for amide synthesis, or the α-carboxyl group of the carboxy terminal amino acid is first protected by amidation; the peptide chain is then extended from the amino group side to a desired length. Thereafter, a peptide in which only the protecting group of the N-terminal α-amino group in the peptide chain has been removed from the peptide and a peptide (or an amino acid) in which only the protecting group of the C-terminal carboxyl group has been eliminated are prepared. The two peptides are condensed in a mixture of the solvents described above. The details of the condensation reaction are the same as described above. After the protected peptide obtained by the condensation is purified, all the protecting groups arc removed by the method described above to give the desired crude peptide. This crude peptide is purified by various known purification means. Lyophilization of the major fraction gives the amide of the desired peptide.

When the metastin derivative of the present invention is present in the form of a configurational isomer, diastereomer, conformer, or the like, each can be isolated by the separating and purifying means described above, if desired. In addition, when the compound of the present invention is racemic, it can be separated into an S isomer and an R isomer by the conventional optical resolving means.

When steric isomers exist for the metastin derivative of the present invention, the present invention includes both of these isomers alone and the isomers present as an admixture thereof.

In addition, the metastin derivative of the present invention may also be hydrate or an-hydrate.

The metastin derivative of the present invention may also be labeled with an isotope (e.g., ³H, ¹⁴C, ³⁵S), etc.

As used herein, the peptides are represented in accordance with the conventional way of describing peptides, that is, the N-terminus (amino terminus) at the left hand and the C-terminus (carboxyl terminus) at the right hand. In the peptides, the C-terminus is usually in the form of an amide (-CONH₂), a carboxyl group (-COOH), a carboxylate (-COO⁻), an alkyl amide (-CONHR) or an ester (-COOR) and the amide (-CONH₂) is particularly preferred. Examples of the ester or alkyl amide as R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl group such as a phenyl-C₁₋₂-alkyl group, e.g., benzyl, phenethyl, benzhydryl, etc., or an α-naphthyl-C₁₋₂-alkyl group such as α-naphthylmethyl, etc.; pivaloyloxymethyl group, which are widely used as an ester for oral use, and the like.

Examples of the salts of the metastin derivative of the present invention include metal salts, ammonium salts, salts with organic bases, salts with inorganic acids, salts with organic acids, salts with basic or acidic amino acids, and the like. Preferred examples of the metal salts include alkali metal salts such as sodium salts, potassium salts, etc.; alkaline earth metal salts such as calcium salts, magnesium salts, barium salts, etc.; aluminum salts; and the like. Preferred examples of the salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc. Preferred examples of the salts with inorganic acids include salts with hydrochloric acid, hydrobromic acid, nitric acid, sulfuric acid, phosphoric acid, etc. Preferred examples of salts with organic acids include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, etc. Preferred examples of salts with basic amino acids include salts with arginine, lysine, ornithine, etc., and preferred examples of salts with acidic amino acids include salts with aspartic, glutamic acid, etc.

In particular, pharmaceutically acceptable salts are preferred. For example, when the compound has an acidic functional group therein, inorganic salts such as alkali metal salts (e.g., sodium salt, potassium salt, etc.), alkaline earth metal salts (e.g., calcium salt, magnesium salt, barium salt, etc.), ammonium salts, etc. are preferable. When the compound has a basic functional group therein, salts with inorganic acids with hydrochloric acid, hydrobromic acid, nitric acid, sulfuric acid, phosphoric acid, etc., and salts with organic acids such as acetic acid, phthalic acid, fumaric acid, oxalic acid, tartaric acid, maleic acid, citric acid, succinic acid, methanesulfonic acid, p-toluenesulfonic acid, etc. are preferred.

The prodrug of the metastin derivative or salts thereof (hereinafter sometimes simply referred to as the metastin derivative of the present invention) refers to such a metastin derivative that is converted into the metastin derivative of the present invention through a reaction using an enzyme, gastric acid, etc., in the living body under physiological conditions. In other words, the prodrug of the present invention is the metastin derivative that undergoes enzymatic oxidation, reduction, hydrolysis, etc. to be converted into the metastin derivative of the present invention, or the metastin derivative that undergoes hydrolysis, etc. by gastric acid, etc. to be converted into the metastin derivative of the present invention.

Examples of the prodrug of the metastin derivative of the present invention include metastin derivatives wherein the amino group of the metastin derivative of the present invention is acylated, alkylated, phosphorylated, etc. (e.g., metastin derivatives wherein the amino group of the metastin derivative of the present invention is cicosanoylatcd, alanylatcd, pcntylaminocarbonylatcd (5-methyl-2-oxo-1,3-dioxolen-4-yl)methoxycarbonylated, tetrahydrofuranylated, pyrrolidylmethylated, pivaloyloxymethylated, tert-butylated, etc); metastin derivatives wherein the hydroxy group of the metastin derivative of the present invention is acylated, alkylated, phosphorylated, borated, etc. (e.g., metastin derivatives wherein the hydroxy group of the metastin derivative of the present invention is acetylated, palmitoylated, propanoylated, pivaloylated, succinylated, fumarylated, alanylated, dimethylaminomethylcarbonylated, etc.); and metastin derivatives wherein the carboxy group of the metastin derivative of the present invention is esterified, amidated, etc. (e.g., metastin derivatives wherein the carboxy group of the metastin derivative of the present invention is converted into the ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, methylamide, etc); and the like. These metastin derivatives can be produced from the metastin derivatives of the present invention by per se known methods.

The prodrugs of the metastins derivative of the present invention may be those that are converted into the metastin derivatives of the present invention under the physiological conditions as described in *"*Iyakuhin no Kaihatsu (Pharmaceutical Research and Development)", Vol. 7 (Bunshisekkei (Drug Design)), pages 163-198, published 1990 by Hirokawa Publishing Co.

The metastin derivatives of the present invention or their salts or prodrugs (hereinafter sometimes simply referred to as the compound of the present invention) possess a cancer metastasis suppressing activity or a cancer growth suppressing activity. Thus, the metastin derivatives are useful for medicaments such as agents for preventing or treating all types of cancer (e.g., lung cancer, gastric cancer, liver cancer, pancreatic cancer, colorectal cancer, rectal cancer, colonic cancer, prostate cancer, ovarian cancer, cervical cancer, breast cancer, etc.), and the like.

The compound of the present invention also possesses the effect of controlling pancreatic function and is thus useful as a medicament such as an agent for preventing or treating various pancreatic diseases (e.g., acute or chronic pancreatitis, pancreatic cancer, etc.).

Furthermore, the compound of the present invention possesses the effect of controlling placental function and is thus useful as a medicament such as an agent for preventing or treating chorio carcinoma, hydatid mole, invasive mole, miscarriage, fetal hypoplasia, abnormal glucose metabolism, abnormal lipid metabolism or induction of delivery, etc.

Moreover, the compound of the present invention possesses the effects of increasing blood glucose level, promoting pancreatic glucagon secretion and promoting urine formation and is thus useful as hyperglycemic agents, pancreatic glucagon secretagogue agents or agents for promoting urine formation, and as a medicament such as agents for preventing or treating obesity, hyperlipemia, type II diabetes mellitus, hypoglycemia, hypertension, diabetic neuropathy, diabetic nephropathy, diabetic retinopathy, edema, urinary disturbances, insulin resistance, unstable diabetes mellitus, fatty atrophy, insulin allergy, insulinoma, arteriosclerosis, thrombotic disorders or lipotoxicity; and the like.

In addition, the compound of the present invention also possesses the effects of promoting gonadotropic hormone (e.g., FSH, LH, etc.) secretion, promoting sex hormone [e.g., androgens (e.g., testosterone, androstenedione, etc.), estrogens (e.g., estradiol, estrone, etc.), progesterones, etc.] secretion, improving gonadal function and inducing or stimulating ovulation, as well as a sexual maturation effect, etc., and hence, can be used as an agent for improving gonadal function, an agent for inducing or stimulating ovulation, an agent for promoting gonadotropic hormone secretion or sex hormone secretion, or an agent for preventing or treating hormone-dependent cancers [e.g., prostate cancer, breast cancer, etc.], infertility [e.g., irregular menstruation, dysmenorrhea, amenorrhea, weight loss-induced amenorrhea, secondary amenorrhea, anovulation, hypoovarianism, hypogonadism, spermatogenetic failure, hypogonadism (e.g., impotence, etc.), genital atrophy, testicular atrophy, testicular function disorder, azoospermia, hypoandrogenemia, etc.], endometriosis, early puberty, myoma of the uterus, etc.

Furthermore, the prodrug of the metastin derivative of the present invention or its salt is useful as an agent for preventing or treating Alzheimer's disease, moderate cognitive impairment, autism, etc.

Moreover, the compound of the present invention is useful as an agent for preventing or treating rheumatic diseases (e.g., rheumatoid arthritis, osteoarthritis, gout, etc.).

Tests can be performed by publicly known procedures to determine that the compound is useful for rheumatic diseases. One specific example of the test procedures is described below.

Female LEW rats of 6 weeks old (weighing 150 to 200 g) are used by 8 rats per group. Bovine type II collagen is dissolved in 0.05 mol/L acetic acid solution, which is adjusted to a concentration of 3 mg/mL. An equivolume of FIA (Freund's incomplete adjuvant) is added to prepare an emulsion. The rats are inoculated with 0.5 mL of the emulsion intracutaneously in the back for sensitization (primary immunization). Seven days after the primary immunization, 0.2 mL of the same emulsion is inoculated intracutaneously at the tail base for booster immunization. On and from the day of booster injection (Day 0), the vehicle solution and the compound solution are subcutaneously administered for 14 days consecutively. On Days 0, 4, 7, 11 and 14, rat rear paw volume is measured using Plethysmometer (UGO BASILE) and the volumes for both rear paws are averaged to give a baseline size of the paws. For assessment of the action on the paw volume, the value obtained by subtracting the pre-value (Day 0) of individual animal from the values on Days 4, 7, 11 and 14 is made the value of individual animal and provided for statistic calculation.

The compound of the present invention is also useful as an agent for preventing or treating autism, an immunostimulator (thymus regeneration, thymic regrowth, enhanced T cell growth), an agent for preventing or treating hypertension, diabetic neuropathy, diabetic nephropathy, diabetic retinopathy, thrombotic disorders or lipotoxicity;
an agent for preventing or treating hyperlipemia, type II diabetes mellitus, hypertension, diabetic neuropathy, diabetic nephropathy or diabetic retinopathy; an antianxiety agent; an antistress agent; an anti-insomnia agent; an antimanic-depressive agent; an agent for preventing or treating hypertension (e.g., essential hypertension, renal hypertension, salt sensitive hypertension, etc.), angina pectoris (e.g., stable angina, unstable angina, etc.), myocardial infarction, cerebrovascular disorders (e.g., asymptomatic cerebrovascular disorder, transient ischemic attack, apoplexy, cerebrovascular dementia, hypertensive encephalopathy, cerebral infarction, etc.), venous insufficiency, obliterative peripheral circulatory disturbances, Raynaud's disease, arteriosclerosis including atherosclerosis (e.g., aneurysm, coronary arteriosclerosis, cerebral arteriosclerosis, peripheral arteriosclerosis, etc.), vascular thickening or occlusion and organ impairments after intervention (e.g., percutaneous coronary intervention, stent placement, coronary thrombolytic therapy, etc.), portal hypertension, respiratory disorders (e.g., asthma, pulmonary hypertension, etc.); an agent for preventing or treating impaired glucose tolerance (IGT); an insulin secretagogue, an inhibitor for transition from IGT to diabetes; and,
an agent for preventing or treating diabetic complications [e.g., neuropathy, nephropathy, retinopathy, cataract, macroangiopathy, osteopenia, hyperosmolar diabetic coma, infectious diseases (e.g., respiratory infection, urinary tract infection, digestive tract infection, skin soft-tissue infection, lower leg infection), diabetic gangrene, xerostomia, hypacusis, cerebrovascular disorder, peripheral circulatory disturbance], osteoporosis, cachexia (e.g., cancerous cachexia, tuberculous cachexia, diabetic cachexia, blood disease cachexia, endocrine disease cachexia, infectious disease cachexia or cachexia due to acquired immunodeficiency syndrome), fatty liver, polycystic ovary syndrome, renal diseases (e.g., diabetic nephropathy, glomerulonephritis, glomerulosclerosis, nephrotic syndrome, hypertensive nephrosclerosis, end stage kidney disease), muscular dystrophy, cardiac infarction, angina pectoris, cerebrovascular disorders (e.g., cerebral infarction, apoplexy), Alzheimer's disease, Parkinson's disease, dementia, insulin resistance syndrome, Syndrome X, metabolic syndrome, hyperinsulinemia, hyperinsulinemia-induced sensory disorders, tumors (e.g., leukemia, skin cancer), irritable bowel syndrome, acute or chronic diarrhea, inflammatory diseases (e.g., spondylitis deformans, arthritis deformans, lumbago, gout, postoperative or posttraumatic inflammation, swelling, neuralgia, pharyngolaryngitis, cystitis, hepatitis (including nonalcoholic steatohepatitis), pneumonia, enteritis, inflammatory bowel disorder (including inflammatory bowel diseases), ulcerative colitis, gastric mucosal injury (including gastric mucosal injury induced by aspirin)), small intestinal mucosal injury, malabsorption, testis function disorder, visceral obesity syndrome, etc.; and,

The compound of the present invention is also useful for reduction of visceral adiposity, inhibition of visceral fat accumulation, improvement of glucose metabolism, improvement of lipid metabolism, suppression of oxidized LDL production, improvement of lipoprotein metabolism, improvement of coronary artery metabolism, prevention or treatment of cardiovascular complications, prevention or treatment of heart failure complications, decrease in blood remnant, prevention or treatment of anovulation, prevention or treatment of hirsutism, or prevention or treatment of hyperandrogenemia; as an agent for improving pancreatic (β cell) function, an agent for regeneration of the pancreas (β cells), an agent for stimulating pancreatic (β cell) regeneration, an appetite regulator, etc.

In addition, the compounds described in WO 2004/063221, the compounds described in WO 2006/001499, the compounds described in WO 2007/072997, or salts thereof are also useful as agents for preventing or treating rheumatic diseases (e.g., rheumatoid arthritis, osteoarthritis, gout, etc.) or the like.

These compounds are also useful as an agent for preventing or treating autism, an immunostimulator (thymus regeneration, thymic regrowth, enhanced T cell growth), an agent for preventing or treating diabetic neuropathy, diabetic nephropathy, diabetic retinopathy, arteriosclerosis, thrombotic disorders or lipotoxicity;
an agent for preventing or treating hyperlipemia, type II diabetes mellitus, hypertension, diabetic neuropathy, diabetic nephropathy or diabetic retinopathy; an antianxiety agent; an antistress agent; an anti-insomnia agent; an antimanic-depressive agent; an agent for preventing or treating hypertension (e.g., essential hypertension, renal hypertension, salt sensitive hypertension, etc.), angina pectoris (e.g., stable angina, unstable angina, etc.), myocardial infarction, cerebrovascular disorders (e.g., asymptomatic cerebrovascular disorder, transient ischemic attack, apoplexy, cerebrovascular dementia, hypertensive encephalopathy, cerebral infarction, etc.), venous insufficiency, obliterative peripheral circulatory disturbances, Raynaud's disease, arteriosclerosis including atherosclerosis (e.g., aneurysm, coronary arteriosclerosis, cerebral arteriosclerosis, peripheral arteriosclerosis, etc.), vascular thickening or occlusion and organ impairments after intervention (e.g., percutaneous coronary intervention, stent placement, coronary thrombolytic therapy, etc.), portal hypertension, respiratory disorders (e.g., asthma, pulmonary hypertension, etc.); an agent for preventing or treating impaired glucose tolerance (IGT); an insulin secretagogue, an inhibitor for transition from IGT to diabetes; and,
an agent for preventing or treating diabetic complications [e.g., neuropathy, nephropathy, retinopathy, cataract, macroangiopathy, osteopenia, hyperosmolar diabetic coma, infectious diseases (e.g., respiratory infection, urinary tract infection, digestive tract infection, skin soft-tissue infection, lower leg infection), diabetic gangrene, xerostomia, hypacusis, cerebrovascular disorder, peripheral circulatory disturbance], osteoporosis, cachexia (e.g., cancerous cachexia, tuberculous cachexia, diabetic cachexia, blood disease cachexia, endocrine disease cachexia, infectious disease cachexia or cachexia due to acquired immunodeficiency syndrome), fatty liver, polycystic ovary syndrome, renal diseases (e.g., diabetic nephropathy, glomerulonephritis, glomerulosclerosis, nephrotic syndrome, hypertensive nephrosclerosis, end stage kidney disease), muscular dystrophy, cardiac infarction, angina pectoris, cerebrovascular disorders (e.g., cerebral infarction, apoplexy), Alzheimer's disease, Parkinson's disease, dementia, insulin resistance syndrome, Syndrome X, metabolic syndrome, hyperinsulinemia, hyperinsulinemia-induced sensory disorders, tumors (e.g., leukemia, skin cancer), irritable bowel syndrome, acute or chronic diarrhea, inflammatory diseases (e.g., spondylitis deformans, arthritis deformans, lumbago, gout, postoperative or posttraumatic inflammation, swelling, neuralgia, pharyngolaryngitis, cystitis, hepatitis (including nonalcoholic steatohepatitis), pneumonia, enteritis, inflammatory bowel disorder (including inflammatory bowel diseases), ulcerative colitis, gastric mucosal injury (including gastric mucosal injury induced by aspirin)), small intestinal mucosal injury, malabsorption, testis function disorder, visceral obesity syndrome, etc.

These compounds are also useful for reduction of visceral adiposity, inhibition of visceral fat accumulation, improvement of glucose metabolism, improvement of lipid metabolism, suppression of oxidized LDL production, improvement of lipoprotein metabolism, improvement of coronary artery metabolism, prevention or treatment of cardiovascular complications, prevention or treatment of heart failure complications, decrease in blood remnant, prevention or treatment of anovulation, prevention or treatment of hirsutism, or prevention or treatment of hyperandrogenemia; as an agent for improving pancreatic (β cell) function, an agent for regeneration of the pancreas (β cells), an agent for stimulating pancreatic (β cell) regeneration, an appetite regulator, etc.

These compounds can also be used in combination with drugs other than the compounds described above.

As drugs (hereinafter sometimes simply referred to as concomitant drugs) that can be used in combination with these compounds, the medicaments described later as drugs that can be used in combination with the compound of the present invention can be used in a similar manner.

The compound of the present invention can be used in combination with medicaments, e.g., chemotherapeutic agents for treating cancer, hormonal therapeutic agents, immunotherapeutic agents, drugs for inhibiting the actions of cell growth factors and their receptors, etc. (hereinafter simply referred to as concomitant drugs). Examples of the "chemotherapeutic agents" are alkylating agents, metabolic antagonists, anticancer antibiotics, plant-derived anticancer agents, etc. Specifically, the drugs described above can be used.

Besides, the compound of the present invention has excellent blood stability, solubility and solution stability, as compared to native metastin such as metastin 54 (1-54) or metastin 10 (45-54).

The metastin derivative of the present invention or its salt or prodrug, the metastin per se, DNA encoding metastin, etc. are useful as an agent for suppressing gonadotropic hormone (e.g., FSH, LH) secretion or sex hormone [e.g., androgen (e.g., testosterone, androstenedione), estrogen (e.g., estradiol, estrone), progesterone] secretion; in particular, it is useful for suppressing gonadotropic hormone secretion or sex hormone secretion through down-regulation of gonadotropic hormone or sex hormone (wherein, the down-regulation of gonadotropic hormone or sex hormone may be pulse loss of LHRH or depletion of LHRH) or down-regulation of human OT7T175 (metastin receptor) protein consisting of the amino acid sequence represented by SEQ ID NO: 9; particularly useful as an agent for preventing or treating hormone-dependent cancers (e.g., prostate cancer, breast cancer, etc.; especially hormone-sensitive prostate cancer, hormone-sensitive breast cancer, etc.); an agent for preventing or treating endometriosis; an agent for inhibiting ovarian follicular maturation; a menstrual cycle-suspending agent; an agent for treating myoma of the uterus; an agent for treating early puberty; or as a contraceptive, etc. Where the metastin derivative of the present invention or its salt or prodrug, metastin per se, or DNA encoding metastin, etc. have normal agonist activity, an effective dose of the metastin derivative sufficient to suppress the secretion of gonadotropic hormone or sex hormone is administered at the site or tissue where the therapeutic effects are to be exerted, so that the metastin derivative is present in a dose more than required (i.e., the metastin derivative is administered in an excess over the normal effective dose, at which the metastin derivative exerts the effects of suppressing cancer metastasis, suppressing cancer growth, etc.; or the gonadotropic hormone secretion agent, the effect of promoting sex hormone secretion, etc.) to exhibit the effects of suppressing gonadotropic hormone secretion or sex hormone secretion. Specific examples include sustained or continuous administration of the normal effective dose (including an administration technique to gradually release the pharmaceutical ingredients by bolus administration); and the like. Further when the metastin derivative of the present invention or its salt or the prodrug thereof, etc. has a sufficient agonist activity more than required (a super-agonist activity), it becomes possible to sustain the activities more than exhibited by the necessary dose at the site or tissue where the therapeutic effect are to be exhibited. It is therefore sufficient even by normal effective dose administration to suppress the secretion of gonadotropic hormone or sex hormone, whereby the effect of suppressing gonadotropic hormone secretion or sex hormone secretion is exhibited.

In other words, an effective dose of the metastin derivative of the present invention or its salt or prodrug, metastin per se, or DNA encoding metastin, etc. sufficient to suppress the secretion of gonadotropic hormone or sex hormone is administered, so that the metastin derivative is present in a dose more than required at the site or tissue where the therapeutic effects are to be exerted, or its activities can be sustained more than required, which enables to exhibit the effects of suppressing gonadotropic hormone secretion or suppressing sex hormone secretion.

The pharmaceutical comprising the compound of the present invention is low toxic. Therefore, the compound of the present invention can be safely administered either directly as it is or as a mixture with pharmacologically acceptable carriers, orally or parenterally (e.g., topically, rectally, intravenously, etc.), in the form of pharmaceutical preparations such as tablets (including dragees and film-coated tablets), powders, granules, capsules (including soft capsules), liquid dosage forms, injections, suppositories, sustained release dosage forms, etc., in accordance with publicly known means generally used in process for producing pharmaceutical preparations.

The compound of the present invention is contained in the pharmaceutical preparation of the present invention in about 0.01 to about 100 wt%, based on the total weight of the preparation.

A dose of the compound of the present invention may vary depending upon subject to be administered, target organ, conditions, route of administration, etc., and in oral administration, the compound is generally administered to the patient with cancer (as 60 kg body weight) in a daily dose of about 0.01 to about 100 mg, preferably about 0.1 to about 50 mg and more preferably about 0.1 to about 20 mg. In parenteral administration, a single dose of the compound may vary depending upon subject to be administered, target organ, conditions, route of administration, etc., and in the form of an injectable dosage form, it is advantageous to administer the compound to the patient with cancer (as 60 kg body weight) generally in a daily dose of about 0.001 to about 30 mg, preferably about 0.01 to about 20 mg, and more preferably about 0.01 to about 10 mg. For other animal species, the corresponding dose as converted per 60 kg weight can be administered.

Pharmacologically acceptable carriers, which may be used to manufacture the pharmaceutical of the present invention, include various organic or inorganic carrier substances conventionally used as materials for pharmaceutical preparations. These substances include, e.g., an excipient, a lubricant, a binder and a disintegrating agent in a solid dosage form, and a solvent, a dissolution aid, a suspending agent, an isotonizing agent, a buffer, a soothing agent, etc. in a liquid dosage form. In addition, conventional additives such as a preservative, an antioxidant, a colorant, a sweetener, an adsorbent, a wetting agent, etc. can be appropriately used in suitable amounts, if necessary.

Examples of excipients include lactose, saccharose, D-mannitol, starch, cornstarch, crystalline cellulose, light anhydrous silicic acid, etc. Examples of lubricants include magnesium stearate, calcium stearate, talc, colloidal silica, etc.

Examples of binders include crystalline cellulose, saccharose, D-mannitol, dextrin, hydroxypropylcellulose, hydroxypropylmethylcellulose, polyvinylpyrrolidone, starch, sucrose, gelatin, methylcellulose, sodium carboxymethylcellulose, etc.

Examples of disintegrating agents include starch, carboxymethylcellulose, carboxymethylcellulose calcium, sodium carboxymethyl starch, L-hydroxypropylcellulose, etc.

Examples of solvents include water for injection, alcohol, propylene glycol, Macrogol, sesame oil, corn oil, olive oil, etc.

Examples of dissolution aids include polyethylene glycol, propylene glycol, D-mannitol, benzyl benzoate, ethanol, trisaminomethane, cholesterol, triethanolamine, sodium carbonate, sodium citrate, etc.

Examples of suspending agents include surfactants such as stearyltriethanolamine, sodium laurylsulfate, lauryl aminopropionate, lecithin, benzalkonium chloride, benzethonium chloride, glycerin monostearate, etc.; hydrophilic polymers such as polyvinyl alcohol, polyvinyl pyrrolidone, sodium carboxymethylcellulose, methylcellulose, hydroxymcthylccllulosc, hydroxyethylcellulose, hydroxypropylcellulose, etc.

Examples of isotonizing agents include glucose, D-sorbitol, sodium chloride, glycerin, D-mannitol, etc.

Examples of buffers include buffering solutions of a phosphate, acetate, carbonate, citrate, etc.

Examples of soothing agents include benzyl alcohol, etc.

Examples of preservatives include p-hydroxybenzoates, chlorobutanol, benzyl alcohol, phenethyl alcohol, dehydroacetic acid, sorbic acid, etc.

Examples of antioxidants include a sulfite, ascorbic acid, α-tocopherol, etc.

In addition, the compound of the present invention can be used in combination with drugs other than the compound of the present invention.

The drugs that can be used in combination with the compound of the present invention (hereinafter sometimes simply referred to as concomitant drugs) include medicaments such as chemotherapeutic agents for treating cancer, hormonal therapeutic agents, immunotherapeutic agents, drugs for inhibiting the actions of cell growth factors and their receptors, etc. (hereinafter simply referred to as concomitant drugs).

The "chemotherapeutic agent" includes, for example, an alkylating agent, a metabolic antagonist, an anticancer antibiotic, a plant-derived anticancer agent, etc.

Examples of the "alkylating agent" include nitrogen mustard, nitrogen mustard-N-oxide hydrochloride, chlorambutyl, cyclophosphamide, ifosfamide, thiotepa, carboquone, improsulfan tosylate, busulfan, nimustine hydrochloride, mitobronitol, melphalan, dacarbazine, ranimustine, estramustine sodium phosphate, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, ribomustin, temozolomide, treosulphan, trophosphamide, zinostatin stimalamer, carboquone, adozelesin, cystemustine, bizelesin, etc.

Examples of the "metabolic antagonist" include mercaptopurine, 6-mercaptopurine riboside, thioinosine, methotrexate, enocitabine, cytarabine, cytarabine ocfosfate, ancitabine hydrochloride, 5-FU drugs (e.g., fluorouracil, tegafur, UFT, doxifluridine, carmofur, gallocitabine, emmitefur, etc.), aminopterin, leucovorin calcium, tabloid, butocinc, folinate calcium, levofolinate calcium, cladribine, emitefur, fludarabine, gemcitabine, hydroxycarbamide, pentostatin, piritrexim, idoxuridine, mitoguazone, thiazophrine, ambamustine, etc.

Examples of the "anticancer antibiotic" include actinomycin D, actinomycin C, mitomycin C, chromomycin A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarcomycin, carzinophilin, mitotane, zorubicin hydrochloride, mitoxantrone hydrochloride, idarubicin hydrochloride, etc.

Examples of the "plant-derived anticancer agent" include etoposide, etoposide phosphate, vinblastine sulfate, vincristine sulfate, vindesine sulfate, teniposide, paclitaxel, docetaxel, vinorelbine, etc.

Examples of the "hormonal therapeutic agent" include fosfestrol, diethylstylbestrol, chlorotrianisene, medroxyprogesterone acetate, megestrol acetate, chlormadinone acetate, cyproterone acetate, danazol, allylestrenol, gestrinone, mepartricin, raloxifene, ormeloxifene, levormeloxifene, anti-estrogens (e.g., tamoxifen citrate, toremifene citrate, etc.), pill dosage forms, mepitiostane, testrolactone, aminoglutethimide, LH-RH agonists (e.g., goserelin acetate, buserelin, leuprorelin, etc.), droloxifene, epitiostanol, ethinylestradiol sulfonate, aromatase inhibitors (e.g., fadrozole hydrochloride, anastrozole, retrozole, exemestane, vorozole, formestane, etc.), anti-androgens (e.g., flutamide, bicartamide, nilutamide, etc.), 5α-reductase inhibitors (e.g., finasteride, epristeride, etc.), adrenocorticohormone drugs (e.g., dexamethasone, prednisolone, betamethasone, triamcinolone, etc.), androgen synthesis inhibitors (e.g., abiraterone, etc.), retinoid and drugs that retard retinoid metabolism (e.g., liarozole, etc.), ER downregulator (e.g. fulvestrant (Faslodex (trademark), etc. ), etc., and among others, LH-RH agonists (e.g., goserelin acetate, buserelin, leuprorelin, etc.) are preferred.

Examples of the "immunotherapeutic agent (BRM)" include picibanil, krestin, sizofiran, lentinan, ubenimex, interferons, interleukins, macrophage colony-stimulating factor, granulocyte colony-stimulating factor, erythropoietin, lymphotoxin, BCG vaccine, Corynebacterium parvum, levamisole, polysaccharide K, procodazole, etc.

The "cell growth factor" in the "drugs for inhibiting the actions of cell growth factors and their receptors" can be any substance so long as it is a substance capable of stimulating the cell growth and, normally, peptides which have a molecular weight of 20,000 or less and bind to their receptors to exhibit the actions in a lower level can be used as the factor. Specific examples are (1) EGF (epidermal growth factor) or a substance having substantially the same activity as EGF [e.g., EGF, hereglin, etc.], (2) insulin or a substance having substantially the same activity as insulin [e.g., insulin, IGF (insulin-like growth factor)-1, IGF-2, etc.], (3) FGF (fibroblast growth factor) or a substance having substantially the same activity as FGF [e.g., acidic FGF, basic FGF, KGF (keratinocyte growth factor), FGF-10, etc.], (4) other cell growth factors [e.g., CSF (colony stimulating factor), EPO (erythropoietin), IL-2 (interleukin-2), NGF (nerve growth factor), PDGF (platelet-derived growth factor), TGFβ (transforming growth factor β), HGF (hepatocyte growth factor), VEGF (vascular endothelial growth factor), etc.] and the like.

The "receptor of the cell growth factor" can be any receptor as long as it is capable of binding to the cell growth factors described above, and specific examples are EGF receptor, hereglin receptor (HER2), insulin receptor, IGF receptor, FGF receptor-1 or FGF receptor-2, etc.

The "drug for inhibiting the actions of cell growth factors" includes HER2 antibody (trastuzumab (Herceptin (trademark)), etc.), imatinib mesylate, ZD1839 or EGFR antibody (cetuximab (Erbitux (trademark)), etc.), antibody against VEGF (e.g., bevacizumab (Avastin (trademark))), VEGFR antibody, VEGFR inhibitor, EGFR inhibitor (erlotinib (Tarceva (trademark)) and gefitinib (Iressa (trademark)), etc.).

In addition to the medicaments described above, there are also used L-asparginase, aceglatone, procarbazine hydrochloride, protoporphyrin-cobalt complex, mercury-hematoporphyrin sodium, topoisomerase I inhibitor (e.g., irinotecan, topotecan, etc.), topoisomerase II inhibitor (e.g., sobzoxan, etc.), differentiation-inducing agent (e.g., retinoid, vitamin D group, etc.), angiogenesis inhibitor (e.g., thalidomide, SU11248, etc.), α-blocker (e.g., tamsulosin hydrochloride, naftopidil, urapidil, alfuzosin, terazosin, prazosin, silodosin, etc.), serine-threonine kinase inhibitor, endothelin receptor antagonist (e.g., atrasentan, etc.), proteasome inhibitor (e.g., bortezomib, etc.), Hsp90 inhibitor (e.g., 17-AAG, etc.), spironolactone, minoxidil, 11α-hydroxyprogesterone, bone resorption inhibitor or bone metastasis suppressor (e.g., zoledronic acid, alendronic acid, pamidronic acid, ctidronic acid, ibandronic acid, clodronic acid), etc.

Use of the compound of the present invention in combination with the concomitant drug exhibits the following excellent effects.
(1) The dose can be reduced as compared to the dose when the compound of the present invention or the concomitant drug is administered alone.
(2) A drug concomitantly administered with the compound of the present invention can be chosen depending on the condition (mild, severe, etc.) of a patient.
(3) The concomitant drug, whose functional mechanism is different from that of the compound of the present invention, can be chosen so that a treatment period can be set longer.
(4) The concomitant drug, whose functional mechanism is different from that of the compound of the present invention, can be chosen so that sustained therapeutic effects can be achieved.
(5) Synergistic effects can be obtained by the use of the compound of the present invention in combination with the concomitant drug.

In addition, the compound of the present invention can reduce a testosterone level to an emasculate level immediately after medication. Thus, when the concomitant drug such as LH-RH agonist (e.g., goserelin acetate, buserelin, leuprorelin etc., preferably leuprorelin) is used in combination with the compound of the present invention, the testosterone level can be reduced to an emasculate level immediately after medication of the compound of the present invention. Further, since the combined use of the concomitant drug such as LH-RH agonist (e.g., goserelin acetate, buserelin, leuprorelin, etc.; preferably leuprorelin) and the compound of the present invention results in prolonged preservation of hormone-dependent period, it can advantageously be used.

Hereinafter, the use of the compound of the present invention in combination with the concomitant drug is referred to as "the concomitant preparation of the present invention."

When the concomitant preparation of the present invention is used, a dosing period of the compound of the present invention and the concomitant drug is not limited; the compound of the present invention or its pharmaceutical composition and the concomitant drug or its pharmaceutical composition may be administered to the subject to be administered either simultaneously or at certain time intervals. The dose of the concomitant drug may be modified according to the dose used clinically and may be appropriately chosen depending upon subject to be administered, route for administration, disease, combination, etc.

A mode for administration of the concomitant preparation of the present invention is not particularly limited, but it is sufficient that the compound of the present invention is used in combination with the concomitant drug at the time of administration. For such mode of administration, there are, for example, (1) administration of a single dosage form obtained by mixing the compound of the present invention and the concomitant drug together at the same time, (2) simultaneous administration of two dosage forms prepared separately from the compound of the present invention and the concomitant drug through the same route for administration, (3) administration of two dosage forms prepared separately from the compound of the present invention and the concomitant drug at certain time intervals through the same route for administration, (4) simultaneous administration of two dosage forms prepared separately from the compound of the present invention and the concomitant drug through different routes for administration, (5) administration of two dosage forms prepared separately from the compound of the present invention and the concomitant drug at certain time intervals (e.g., administration of the compound of the present invention and the concomitant drug in this order, or administration in a reversed order) through different routes for administration, etc.

The concomitant preparation of the present invention is low toxic and thus can be safely administered orally or parenterally (e.g., topically, rectally, intravenously, etc.) in the form of pharmaceutical preparations such as tablets (including dragees and film-coated tablets), powders, granules, capsules (including soft capsules), liquid dosage forms, injections, suppositories, sustained release dosage forms, etc., which are obtained by mixing the compound of the present invention or (and) the concomitant drug described above with pharmacologically acceptable carriers. Injectable dosage forms can be administered intravenously, intramuscularly or subcutaneously, into the organ, or directly at the focus.

Pharmacologically acceptable carriers, which may be used to manufacture the concomitant preparation of the present invention, include various organic or inorganic carrier substances conventionally used as materials for pharmaceutical preparations. These substances include, e.g., an excipient, a lubricant, a binder and a disintegrating agent in a solid dosage form, and a solvent, a dissolution aid, a suspending agent, an isotonizing agent, a buffer, a soothing agent, etc. in a liquid dosage form. In addition, conventional additives such as a preservative, an antioxidant, a colorant, a sweetener, an adsorbent, a wetting agent, etc. can be appropriately used in suitable amounts, if necessary.

Examples of excipients include lactose, saccharose, D-mannitol, starch, cornstarch, crystalline cellulose, light anhydrous silicic acid, etc.

Examples of lubricants include magnesium stearate, calcium stearate, talc, colloidal silica, etc.

Examples of binders include crystalline cellulose, saccharose, D-mannitol, dextrin, hydroxypropylcellulose, hydroxypropylmethylcellulose, polyvinylpyrrolidone, starch, sucrose, gelatin, methylcellulose, sodium carboxymethylcellulose, etc.

Examples of disintegrating agents include starch, carboxymethylcellulose, carboxymethylcellulose calcium, sodium carboxymethyl starch, L-hydroxypropylcellulose, etc.

Examples of solvents include water for injection, alcohol, propylene glycol, Macrogol, sesame oil, corn oil, olive oil, etc.

Examples of dissolution aids include polyethylene glycol, propylene glycol, D-mannitol, benzyl benzoate, ethanol, trisaminomethane, cholesterol, triethanolamine, sodium carbonate, sodium citrate, etc.

Examples of suspending agents include surfactants such as stearyltriethanolamine, sodium laurylsulfate, lauryl aminopropionate, lecithin, benzalkonium chloride, benzethonium chloride, glycerin monostearate, etc.; hydrophilic polymers such as polyvinyl alcohol, polyvinyl pyrrolidone, sodium carboxymethylcellulose, methylcellulose, hydroxymethylcellulose, hydroxyethylcellulose, hydroxypropylcellulose, etc.

Examples of isotonizing agents include glucose, D-sorbitol, sodium chloride, glycerin, D-mannitol, etc.

Examples of buffers include buffering solutions of a phosphate, acetate, carbonate, citrate, etc.

Examples of soothing agents include benzyl alcohol, etc.

Examples of preservatives include p-hydroxybenzoates, chlorobutanol, benzyl alcohol, phenethyl alcohol, dehydroacetic acid, sorbic acid, etc.

Examples of antioxidants include a sulfite, ascorbic acid, α-tocopherol, etc.

In the concomitant preparation of the present invention, a ratio of the compound of the present invention to the concomitant drug may be appropriately chosen depending upon subject to be administered, route for administration, disease, etc.

For example, the amount of the compound of the present invention contained in the concomitant preparation of the present invention varies depending on the dosage form of the preparation, but is usually about 0.01 to 100% by weight, preferably about 0.1 to 50% by weight, and more preferably about 0.5 to 20% by weight, based on the total weight of the preparation.

The amount of the concomitant drug contained in the concomitant preparation of the present invention varies depending on the dosage form of the preparation, but is usually about 0.01 to 100% by weight, preferably about 0.1 to 50% by weight, and more preferably about 0.5 to 20% by weight, based on the total weight of the preparation.

The amount of additives such as a carrier, etc. contained in the concomitant preparation of the present invention varies depending on the dosage form of the preparation, and is usually about 1 to 99.99% by weight, preferably about 10 to 90% by weight, based on the total weight of the preparation.

These amounts may be the same, also when the compound of the present invention and the concomitant drug are separately prepared.

These preparations can be manufactured by per se publicly known methods conventionally used in the step of manufacturing pharmaceutical preparations.

For example, an injectable dosage form can be prepared into an aqueous injection of the compound of the present invention or the concomitant drug together with a dispersing agent (e.g., Tween 80 (manufactured by Atlas Powder Company, USA), HCO 60 (manufactured by Nikko Chemicals Co., Ltd.), polyethylene glycol, carboxymethyl cellulose, sodium alginate, hydroxypropylmethyl cellulose, dextrin, etc.), a stabilizer (e.g., ascorbic acid, sodium pyrosulfite), a surfactant (e.g., polysorbate 80, macrogol, etc.), a solubilizing agent (e.g., glycerin, ethanol, etc.), a buffering agent (e.g., phosphoric acid or its alkali metal salt, citric acid or its alkali metal salt, etc.), an isotonizing agent (e.g., sodium chloride, potassium chloride, mannitol, sorbitol, glucose, etc.), a pH adjusting agent (e.g., hydrochloric acid, sodium hydroxide, etc.), a preservative (e.g., ethyl p-oxybenzoate, benzoic acid, methylparabene, propylparabene, benzyl alcohol, etc.), a solubilizer (e.g., concentrated glycerin, meglumine, etc.), a dissolution aid (e.g., propylene glycol, saccharose, etc.), a soothing agent (e.g., glucose, benzyl alcohol, etc.), and the like; or, by dissolving, suspending or emulsifying the compound of the present invention or the concomitant drug in a vegetable oil such as olive oil, sesame oil, cottonseed oil, corn oil, etc. and a dissolution aid such as propylene glycol or the like to dissolve, suspend or emulsify and prepare into an oily injection; which can be used as the injectable dosage form.

An oral dosage form can be produced in a conventional manner by adding to the compound of the present invention or the concomitant drug, for example, an excipient (e.g., lactose, saccharose, starch, etc.), a disintegrating agent (e.g., starch, calcium carbonate, etc.), a binder (e.g., starch, gum arabic, carboxymethyl cellulose, polyvinylpyrrolidone, hydroxypropyl cellulose, etc.), a lubricant (e.g., talc, magnesium stearate, polyethylene glycol 6000, etc.) and other additives, compressing the resulting mixture and, if necessary, coating the compressed product for the purpose of taste masking, enteric degradation or sustained release by techniques per se publicly known. Coating agents for this purpose include, for example, hydroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose acetate succinate, Eudragit (manufactured by Rohm Company, Germany, methacrylic acid-acrylic acid copolymer) and pigments (e.g., iron oxide, titanium dioxide). The oral dosage form may be either an immediate release dosage form or a sustained release dosage form.

For example, to make a suppository, the compound of the present invention or the concomitant drug is prepared into an oily or aqueous solid, semi-solid or liquid suppository composition by techniques per se publicly known. Oily bases used for the composition described above include glycerides of higher fatty acids [e.g., cacao butter, Witepsols (manufactured by Dynamite Nobel Company, Germany), etc.], moderate fatty acids [e.g., miglyols (manufactured by Dynamite Nobel Company, Germany), etc.], vegetable oils (e.g., sesame oil, soybean oil, cottonseed oil, etc.), and the like. Aqueous bases include, for example, polyethylene glycols and propylene glycol. Aqueous gel bases include, for example, natural rubbers, cellulose derivatives, vinyl polymers, acrylic polymers, etc.

The sustained release dosage form above includes sustained release microcapsules, and the like.

Sustained release microcapsules can be obtained by per se publicly known methods, and are preferably prepared in the form of, e.g., a sustained release dosage form by the method [2] shown below for administration.

Preferably, the compound of the present invention is prepared into a dosage form for oral administration such as a solid dosage form (e.g., powders, granules, tablets, capsules) or into a dosage form for rectal administration such as a suppository, etc. A dosage form for oral administration is particularly preferred.

The concomitant drug can be prepared into the dosage form described above, depending on kind of the drug.

Hereinafter, [1] an injectable preparation of the compound of the present invention or the concomitant drug and its production, [2] a sustained release or immediate release preparation of the compound of the present invention or the concomitant drug and its production and [3] a sublingual, buccal or rapid oral disintegrating preparations of the compound of the present invention or the concomitant drug and its production are specifically described below.

### [1] Injectable Preparation and its Production

An injectable preparation obtained by dissolving the compound of the present invention or the concomitant drug in water is preferred. The injectable preparation may contain a benzoate and/or a salicylate.

The injectable preparation is obtained by dissolving the compound of the present invention or the concomitant drug and optionally a benzoate and/or a salicylate in water.

Examples of the benzoate and/or salicylate described above include an alkali metal salt such as sodium and potassium salts, etc., an alkaline earth metal salt such as calcium and magnesium salts, etc., an ammonium salt, a meglumine salt, a salt of an organic acid such as trometamol, and the like.

The concentration of the compound of the present invention or the concomitant drug in the injectable preparation is 0.5 to 50 w/v %, preferably about 0.3 to 20 w/v %. The concentration of the benzoate and/or salicylate is 0.5 to 50 w/v %, preferably 3 to 20 w/v %.

Furthermore, additives generally used in an injectable preparation such as a stabilizer (ascorbic acid, sodium pyrosulfitc, etc.), a surfactant (polysorbate 80, macrogol, etc.), a solubilizing agent (glycerin, ethanol, etc.), a buffering agent (phosphoric acid and its alkali metal salts, citric acid and its alkali metal salts, etc.), an isotonizing agent (sodium chloride, potassium chloride, etc.), a dispersing agent (hydroxypropylmethyl cellulose, dextrin), a pH adjusting agent (hydrochloric acid, sodium hydroxide, etc.), a preservative (ethyl p-oxybenzoate, benzoic acid, etc.), a solubilizer (concentrated glycerin, meglumine, etc.), a dissolution aid (propylene glycol, saccharose, etc.), a soothing agent (glucose, benzyl alcohol, etc.) may be appropriately added to the preparation. Any of these additives is added in an amount generally used in an injectable preparation.

The injectable preparation is adjusted to pH of 2 to 12, preferably 2.5 to 8.0 by adding a pH adjusting agent.

The injectable preparation can be obtained by dissolving both the compound of the present invention or the concomitant drug and optionally a benzoate and/or salicylate, and, if necessary, the above additives in water. These ingredients may be dissolved in any order and the dissolution may be appropriately performed according to the same procedures for preparing a conventional injectable preparation.

An aqueous solution for the injectable preparation is preferably warmed, and used as the injectable preparation after filtration sterilization by filtration or autoclaved as in a conventional injectable preparation to provide for the injectable preparation.

An aqueous solution for the injectable preparation is preferably autoclaved, e.g., at 100 to 121°C for 5 to 30 minutes.

Moreover, the preparation may be in a solution form to which antibacterial activity is imparted to be usable as a multiple dosage form in divided dosing.

### [2] Sustained Release or Immediate Release Preparation and its Production

A preferred sustained release preparation comprises a core comprising the compound of the present invention or the concomitant drug, which is optionally coated with a water-insoluble material or a swelling polymer. For example, a sustained release preparation for oral administration of a once-daily dosage form is preferred.

Examples of the water-insoluble material used for the coating agent include cellulose ethers such as ethyl cellulose, butyl cellulose, etc., cellulose esters such as cellulose acetate, cellulose propionatc, etc., polyvinyl esters such as polyvinyl acetate, polyvinyl butyrate, etc., acrylic acid polymers such as an acrylic acid/methacrylic acid copolymer, a methyl methacrylate copolymer, an ethoxyethyl methacrylate/cinnamoethyl methacrylate/aminoalkyl methacrylate copolymer, a polyacrylic acid, a polymethacrylic acid, a methacrylic acid alkylamide copolymer, a poly(methyl methacrylate), a polymethacrylate, a polymethacrylamide, an aminoalkyl methacrylate copolymer, a poly(methacrylic anhydride), a glycidyl methacrylate copolymer, in particular, a series of Eudragits (Rohm & Pharma) such as Eudragit RS-100, RL-100, RS-30D, RL-30D, RL-PO and RS-PO (ethyl acrylate/methyl methacrylate/chlorotrimethyl methacrylate/ethyl ammonium copolymer) and Eudragit NE-30D (methyl methacrylate/ethyl acrylate copolymer), etc., hydrogenated oils such as hydrogenated castor oil (e.g., LUBRI WAX (Freund Industrial Co., Ltd.), etc.), waxes such as carnauba wax, a fatty acid glycerin ester, paraffin, etc., polyglycerin fatty acid esters, etc.

The swelling polymer is preferably a polymer having an acidic removable group and exhibiting pH-dependent swelling. It is preferred that a polymer has an acidic removable group and undergoes a less swelling at an acidic pH such as in the stomach but is swollen extensively at a neutral pH such as in the small and large intestines.

Examples of such a polymer having an acidic removable group and exhibiting pH-dependent swelling include a crosslinked polyacrylic acid polymer such as Carbomers 934P, 940, 941, 974P, 980, 1342, etc., polycarbophil and calcium polycarbophil (all manufactured by BF Goodrich Chemicals), Hivis Wakos 103, 104, 105 and 304 (all manufactured by Wako Pure Chemical Industries, Ltd.), etc.

The coating agent used in the sustained release preparation may further contain a hydrophilic material.

Examples of the hydrophilic material include a polysaccharide which may have a sulfate group, such as pullulan, dextrin, an alkali metal alginate, etc., a polysaccharide having a hydroxyalkyl group or a carboxyalkyl group such as hydroxypropyl cellulose, hydroxypropylmethyl cellulose, sodium carboxymethylcellulose, etc., methyl cellulose, polyvinyl pyrrolidone, polyvinyl alcohol, polyethylene glycol, etc.

The amount of the water-insoluble material contained in the coating agent of the sustained release preparation is about 30 to about 90% (w/w), preferably about 35 to about 80% (w/w), more preferably about 40 to about 75% (w/w), and the swelling polymer content is about 3 to about 30% (w/w), preferably about 3 to about 15% (w/w). The coating agent may further contain a hydrophilic material, and the amount of the hydrophilic material contained in the coating agent is about 50% (w/w) or less, preferably about 5 to about 40% (w/w), more preferably about 5 to about 35% (w/w). As used herein, the term % (w/w) above is used to mean % by weight based on the coating agent composition, which is the remainder of the coating agent solution after removing any solvent (e.g., water, a lower alcohol such as methanol, ethanol, etc.).

The sustained release preparation is manufactured by preparing a core containing a drug as illustratively shown below, followed by coating the resulting core with a coating agent solution obtained by heat-melting a water-insoluble material or a swelling polymer or by dissolving or dispersing such a material in a solvent.

### 1. Production of Drug-Containing Core

The shape of a core containing a drug to be coated with a coating agent (hereinafter sometimes simply referred to as a core) is not specifically limited but preferably prepared into a particulate shape such as granules, fine granules, or the like.

When the core is granules or fine granules, they have a mean particle size of preferably about 150 to about 2,000 µm, more preferably about 500 to about 1,400 µm.

The core can be prepared in a conventional manner. For example, a drug is blended with a suitable excipient, binder, disintegrating agent, lubricant, stabilizer, etc., which is then subjected to wet extrusion granulation, fluidized bed granulation, or the like.

The drug content in the core is about 0.5 to about 95% (w/w), preferably about 5.0 to about 80% (w/w), more preferably about 30 to about 70% (w/w).

Examples of the excipient contained in the core include a saccharide such as saccharose, lactose, mannitol, glucose, etc., starch, crystalline cellulose, calcium phosphate, cornstarch, etc. Among others, crystalline cellulose and cornstarch are preferred.

Examples of the binder used include polyvinyl alcohol, hydroxypropyl cellulose, polyethylene glycol, polyvinyl pyrrolidone, Pluronic F68, gum arabic, gelatin, starch, etc. Examples of the disintegrating agent include calcium carboxymethyl cellulose (ECG505), sodium croscarmellose (Ac-Di-Sol), crosslinked polyvinyl pyrrolidone (crospovidone), a low substituted hydroxypropyl cellulose (L-HPC), etc. Among them, hydroxypropyl cellulose, polyvinyl pyrrolidone and a low substituted hydroxypropyl cellulose are preferred. Examples of the lubricant and the anticoagulant include talc, magnesium stearate and its inorganic salts, and examples of the lubricant include polyethylene glycol, etc. Examples of the stabilizer include an acid such as tartaric acid, citric acid, succinic acid, fumaric acid, maleic acid, etc.

In addition to the procedures described above, the core can be prepared by using other techniques such as an tumbling granulation technique, a pan coating technique, a fluidized bed coating technique and a melt granulation technique, wherein a drug or a mixture of the drug with an excipient, a lubricant, etc. is portionwise added to inert carrier particles as seeds for the core, while spraying a binder dissolved in a suitable solvent such as water, a lower alcohol (e.g., methanol, ethanol, etc.) or the like. Examples of the inert carrier particles include those prepared from saccharose, lactose, starch, crystalline cellulose and waxes, and, preferably, these carriers have a mean particle size of about 100 µm to about 1,500 µm.

In order to separate the drug contained in the core from a coating agent, the surface of the core may be covered with a protective material. Examples of the protective material include the hydrophilic material described above and water-insoluble material. The preferred protective material is polyethylene glycol or a polysaccharide having a hydroxyalkyl group or a carboxyalkyl group, more preferably, hydroxypropylmethyl cellulose and hydroxypropyl cellulose. The protective material may contain, as a stabilizer, an acid such as tartaric acid, citric acid, succinic acid, fumaric acid, maleic acid, etc., and a lubricant such as talc. When the protective material is used, the amount thereof to be coated is about 1 to about 15% (w/w), preferably about 1 to about 10% (w/w), more preferably about 2 to about 8% (w/w) based on the core.

The protective material can be coated by a conventional coating method. Specifically, the core is spray-coated with the protective material by a fluidized bed coating technique, a pan coating technique, etc.

### II. Coating of Core with Coating Agent

The core obtained in I above is coated with a solution of the coating agent prepared by melt-heating the water-insoluble material and ph-dependent swelling polymer described above and a hydrophilic material or by dissolving or dispersing them in a solvent to obtain a sustained release preparation.

A coating method of the core with the coating agent solution includes, for example, spray-coating, etc.

The composition ratio of the water-insoluble material, swelling polymer and hydrophilic material in the coating agent solution can be appropriately chosen to be within the amounts of the respective ingredients contained in the coating.

The amount of the coating agent is about 1 to about 90% (w/w), preferably about 5 to about 50% (w/w), more preferably about 5 to about 35% (w/w) based on the core (excluding the amount of the protective material coating).

As the solvent for the coating agent solution, water and an organic solvent can be used singly or as a mixture thereof. When a mixture is used, the ratio of water and the organic solvent (water/organic solvent: a weight ratio) may vary with the range of 1 to 100%, and is preferably 1 to about 30%. The organic solvent is not particularly limited so far as it can dissolve the water-insoluble material, and examples of the solvent include a lower alcohol such as methyl alcohol, ethyl alcohol, isopropyl alcohol, n-butyl alcohol, etc., a lower alkanone such as acetone, etc,, acetonitrile, chloroform, methylene chloride, etc. Among others, a lower alcohol is preferred, with ethyl alcohol and isopropyl alcohol being more preferred. Water and a mixture of water and an organic solvent are used preferably as solvents for the coating agent solution. In this case, an acid such as tartaric acid, citric acid, succinic acid, fumaric acid, maleic acid, etc. may be added to the coating agent solution, if necessary, for the purpose of stabilizing the coating agent solution.

The coating through spray coating can be performed using a conventional coating method. Specifically, the core is sprayed with a coating agent solution by a fluidized bed coating technique, a pan coating technique, or the like. Upon coating, a lubricant such as talc, titanium oxide, magnesium stearate, calcium stearate, light silicic anhydride, etc., and a plasticizer such as glycerin fatty ester, hardened castor oil, triethyl citrate, cetyl alcohol, stearyl alcohol, etc. may also be added.

After coating with the coating agent, an antistatic agent such as talc may also be admixed, if necessary.

The immediate release preparation may be a liquid (solution, suspension, emulsion, etc.) or a solid (particles, pills, tablets, etc.). An oral preparation and a parenteral preparation such as an injectable preparation may be used, and an oral preparation is preferred.

The immediate release preparation may usually contain a carrier, additives and an excipient (hereinafter sometimes abbreviated as excipients) which are conventionally used in the pharmaceutical field, in addition to a drug which is an active ingredient. The pharmaceutical excipients are not specifically limited so long as they are excipients conventionally used in the pharmaceutical field. Examples of the excipient for an oral solid preparation include lactose, starch, cornstarch, crystalline cellulose (Avicel PH101, manufactured by Asahi Kasei Corporation, etc.), powdered sugar, granulated sugar, mannitol, light silicic anhydride, magnesium carbonate, calcium carbonate, L-cysteine, etc., preferably, cornstarch and mannitol. Any of these excipients may be employed alone or in combination with each other. The amounts of the excipients are, for example, about 4.5 to about 99.4 w/w %, preferably about 20 to about 98.5 w/w %, more preferably about 30 to about 97 w/w %, based on the total weight of the immediate release preparation.

The content of drug in the immediate release preparation may appropriately be selected from the range of about 0.5% to about 95%, preferably about 1% to about 60% to the whole amount of the immediate release preparation.

When the immediate release preparation is an oral solid preparation, the preparation contains a disintegrating agent in addition to the ingredients described above. Examples of the disintegrating agent include calcium carboxymethylcellulose (ECG505 manufactured by GOTOKU CHEMICAL Co., Ltd.), sodium croscarmellose (for example, Ac-Di-Sol manufactured by Asahi Kasei Corporation), crospovidone (for example, COLIDON CL manufactured by BASF), low-substituted hydroxypropyl cellulose (Shin-Etsu chemical Co., Ltd.), carboxymethyl starch (MATSUTANI CHEMICAL INDUSTRY Co., Ltd.), sodium carboxymethyl starch (EXORITAB manufactured by KIMURA SANGYO), partial α starch (PCS manufactured by Asahi Kasei Corporation), etc. For example, the disintegrating agent that disintegrates granules by water absorption or swelling upon contact with water, or forming a channel between the active ingredient comprising the core and an excipient can be used. Any of these disintegrating agents can be used alone or in combination with each other. The amount of the disintegrating agent used may be appropriately chosen depending upon the type and the amount of the drug used or a particular preparation design for the intended release performance. For example, the amount is about 0.05 to about 30 w/w %, preferably about 0.5 to about 15 w/w % based on the total weight of the immediate release preparation.

When the immediate release preparation is an oral solid preparation, the oral solid preparation may optionally contain additives conventionally used in a solid preparation, in addition to the ingredients described above. Examples of the additives include binders (for example, sucrose, gelatin, powdery gum arabic, methyl cellulose, hydroxypropyl cellulose, hydroxypropylmethyl cellulose, carboxymethylcellulose, polyvinyl pyrrolidone, pullulan, dextrin, etc.), lubricants (polyethylene glycol, magnesium stearate, talc, light silicic anhydride (for example, Aerosil (NIPPON AEROSIL)), surfactants (for example, anionic surfactants such as sodium alkylsulfate, nonionic surfactants such as polyoxyethylene fatty ester, polyoxyethylene sorbitan fatty ester, polyoxyethylene castor oil derivatives, etc.), colorants (for example, tar colorants, caramel, colcothar, titanium oxide, riboflavins), if necessary, corrigents (for example, sweeteners, flavors, etc.), adsorbents, preservatives, wetting agents, antistatic agents, etc. Furthermore, an organic acid such as tartaric acid, citric acid, succinic acid, fumaric acid or the like can also be added as a stabilizer.

As the binder above, hydroxypropyl cellulose, polyethylene glycol and polyvinyl pyrrolidone, etc. are preferably used.

The immediate release preparation can be prepared by mixing the ingredients described above and kneading the mixture, if necessary, and then molding according to a conventional technique for making pharmaceutical preparations. The mixing above can be carried out in a conventional manner, e.g., by mixing, kneading, etc. Specifically, where the immediate release preparation is in the form of particles, the preparation can be prepared by mixing ingredients with a vertical granulator, a multi-purpose kneader (HATA IRON WORKS CO., LTD), a fluidized bed granulator FD-5S (POWREX CORPORATION) or the like, and then granulating the resulting by wet extrusion granulation or fluidized bed granulation by a technique similar to that for preparing the core of the sustained release preparation described above.

The immediate release preparation and the sustained release preparation thus obtained can be formulated, as they are, or, together with appropriate pharmaceutical excipients, in pharmaceutical preparations separately in a conventional manner to prepare respective preparations for administering in combination with each other simultaneously or at certain time intervals. Alternatively, both preparations may be formulated in a single dosage form for oral administration (e.g., granules, fine granules, tablets, capsules) as they are, or, together with appropriate pharmaceutical excipients. Both preparations in the form of granules or fine granules may also be filled in a single capsule for oral administration.

### [3] Sublingual, Buccal or Rapid Oral Disintegrating Preparation and its Production

A sublingual, buccal or rapid oral disintegrating preparation may be in the form of a solid preparation such as a tablet, or may be in the form of an oral mucosal patch (film), or an oral disintegrating film.

The sublingual, buccal or rapid oral disintegrating preparation is preferably a preparation containing the compound of the present invention or the concomitant drug and an excipient. The preparation may also contain auxiliary agents such as a lubricant, an isotonizing agent, a hydrophilic carrier, a water-dispersible polymer, a stabilizer, etc. Further for the purpose of promoting the absorption and enhancing the bioavailability, the preparation may also contain β-cyclodextrin or β-cyclodextrin derivatives (e.g., hydroxypropyl-β-cyclodextrin, etc.), and the like.

Examples of the above excipient include lactose, saccharose, D-mannitol, starch, crystalline cellulose, light silicic anhydride, etc. Examples of the lubricant include magnesium stearate, calcium stearate, talc, colloidal silica, etc., preferably, magnesium stearate and colloidal silica. Examples of the isotonizing agent include sodium chloride, glucose, fructose, mannitol, sorbitol, lactose, saccharose, glycerin and urea, particularly preferably, mannitol. As the hydrophilic carrier, there are, for example, swelling hydrophilic carriers such as crystalline cellulose, ethyl cellulose, crosslinked polyvinyl pyrrolidone, light silicic anhydride, silicic acid, dicalcium phosphate, calcium carbonate, etc., preferably, crystalline cellulose (e.g., microcrystalline cellulose, etc.). As the water-dispersible polymer, there are, for example, a gum (e.g., tragacanth gum, acacia gum, guar gum), alginate (e.g., sodium alginate), cellulose derivatives (e.g., methyl cellulose, carboxymethylcellulose, hydroxymethyl cellulose, hydroxypropyl cellulose, hydroxypropylmethyl cellulose), gelatin, water-soluble starch, polyacrylic acid (e.g., carbomer), polymethacrylic acid, polyvinyl alcohol, polyethylene glycol, polyvinyl pyrrolidone, polycarbophil, ascorbate palmitate salt, etc., preferably, hydroxypropylmethyl cellulose, polyacrylic acid, alginate, gelatin, carboxymethylcellulose, polyvinyl pyrrolidone and polyethylene glycol. Hydroxypropylmethyl cellulose is particularly preferred. As the stabilizer, there are, for example, cysteine, thiosorbitol, tartatic acid, citric acid, sodium carbonate, ascrobic acid, glycine, sodium sulfite, etc., particularly preferably citric acid and ascorbic acid.

The sublingual, buccal or rapid oral disintegrating preparation can be prepared by blending the compound of the present invention or the concomitant drug and an excipient by a method per se known. Furthermore, if desired, the auxiliary agents described above, such as the lubricant, isotonizing agent, hydrophilic carrier, water-dispersible polymer, stabilizer, colorant, sweetener, preservative, etc. may also be blended therewith. After blending the ingredients described above simultaneously or at certain time intervals, the mixture is compressed into tablets to obtain the sublingual, buccal or oral quick disintegration tablet. In order to obtain a suitable hardness, a solvent such as water, an alcohol, etc. can be used to moisturize or wet the ingredients before or after tabletting, followed by drying.

In preparing the oral mucosal patch (film), the compound of the present invention or the concomitant drug and the water-dispersible polymer (preferably, hydroxypropyl cellulose, hydroxypropylmethyl cellulose), excipient, etc. described above are dissolved in a solvent such as water, etc. and then the resulting solution is cast into a film. In addition, additives such as a plasticizer, a stabilizer, an antioxidant, a preservative, a colorant, a buffering agent, a sweeteners, etc. may be added to the preparation. A glycol such as polyethylene glycol, propylene glycol, etc. may be added to impart an appropriate elasticity to a film, and a bioadhesive polymer (e.g., polycarbophile, carbopol) may also be added to enhance the adhesion of the film to the oral mucosal lining. The casting can be carried out by pouring a solution onto a non-adhesive surface, spreading the solution using a coater such as a doctor blade in a uniform thickness (preferably, approximately 10 to 1000 microns), and then drying the solution to form a film. The film thus formed is dried at room temperature or while warming, and then cut into pieces each having a desired surface area.

A preferred rapid oral disintegrating preparation is, for example, a rapid diffusion preparation in a solid network form, which comprises the compound of the present invention or the concomitant drug and a water-soluble or water-diffusible carrier inert to the compound of the present invention or the concomitant drug. The network is formed by sublimating a solvent from the solid composition comprising a solution of the compound of the present invention or the concomitant drug in a suitable solvent.

In addition to the compound of the present invention or the concomitant drug, the composition of the rapid oral disintegrating preparation may preferably contain a matrix-forming agent and secondary ingredients.

Examples of the matrix-forming agent include gelatins, dextrins and animal or vegetable proteins from soybean, wheat, psyllium seed, etc.; gummy materials such as gum arabic, guar gum, agar, xanthane gum, etc.; polysaccharides; alginates; carboxymethylcelluloses; carrageenans; dextrans; pectins; synthetic polymers such as polyvinyl pyrrolidone; materials derived from gelatin-gum arabic complexes, etc. The matrix-forming agent further includes saccharides such as mannitol, dextrose, lactose, galactose, trehalose, etc.; cyclic saccharides such as cyclodextrins, etc.; inorganic salts such as sodium phosphate, sodium chloride, aluminum silicate, etc.; amino acids having 2 to 12 carbon atoms such as glycine, L-alanine, L-aspartic acid, L-glutamic acid, L-hydroxyproline, L-isoleucine, L-leucine, L-phenylalanine, etc.

One or more matrix-forming agents can be incorporated into a solution or suspension before solidification. The matrix-forming agents may be present in addition to a surfactant, or may be present in the absence of a surfactant. The matrix-forming agents serve not only to form a matrix itself, but also assist to maintain diffusion of the compound of the present invention or the concomitant drug in the solution or suspension.

The composition may contain a secondary ingredient such as a preservative, an antioxidant, a surfactant, a thickening agent, a colorant, pH adjusting agent, a flavor, a sweetener, a taste masking agent, etc. As the suitable colorant, there are, for example, iron oxide red, black and yellow, FD & C dyes available from ERIS & EVERALD such as FD & C Blue No. 2 and FD & C Red No. 40, etc. Examples of the suitable flavor include mint, raspberry, licorice, orange, lemon, grape fruit, caramel, vanilla, cherry, grape flavor and a combination thereof. Examples of the suitable pH adjusting agent include citric acid, tartaric acid, phosphoric acid, hydrochloric acid and maleic acid. Examples of the suitable sweetener include aspartame, acesulfame K and thaumatine. Examples of the suitable taste masking agent include sodium bicarbonate, ion exchange resins, cyclodextrin inclusion compounds, adsorbents and microencapsulated apomorphine.

The preparation generally contains the compound of the present invention or the concomitant drug in an amount of about 0.1 to about 50% by weight, preferably about 0.1 to about 30% by weight and, preferably, the preparation (the sublingual tablet, buccal, etc. described above) allows 90% or more of the compound of the present invention or the concomitant drug to be dissolved (in water) within a time period of about 1 to about 60 minutes, preferably about 1 minute to about 15 minutes, more preferably about 2 minutes to about 5 minutes, or is a rapid oral disintegrating preparation which disintegrates within about 1 to about 60 seconds, preferably about 1 to about 30 seconds, more preferably about 1 to about 10 seconds, after being placed in the oral cavity.

The amount of the above excipient is about 10 to about 99% by weight, preferably about 30 to about 90% by weight based on the total weight of the preparation. The amount of β-cyclodextrin or β-cyclodextrin derivative is 0 to about 30% by weight based on the total weight of the preparation. The amount of the lubricant is about 0.01 to about 10% by weight, preferably about 1 to about 5% by weight based on the total weight of the preparation. The amount of the isotonizing agent is about 0.1 to about 90% by weight, preferably about 10 to about 70% by weight based on the total weight of the preparation. The amount of the hydrophilic carrier is about 0.1 to about 50% by weight, preferably about 10 to about 30% by weight based on the total weight of the preparation. The amount of the water-dispersible polymer is about 0.1 to about 30% by weight, preferably about 10 to about 25% by weight based on the total weight of the preparation. The amount of the stabilizer is about 0.1 to about 10% by weight, preferably about 1 to about 5% by weight based on the total weight of the preparation. If necessary, the preparation described above may further contain additives such as a colorant, a sweetener, a preservative, etc.

A dose of the concomitant preparations of the present invention varies depending upon kind of the compound of the present invention, age, body weight, conditions, dosage form, route for administration, dosing period, etc.

A dose of the compound of the present invention may vary depending upon subject to be administered, target organ, conditions, route of administration, etc., and in oral administration, the compound is generally administered to the patient (as 60 kg body weight) with cancer in a daily dose of about 0.01 to 100 mg, preferably about 0.1 to 50 mg and more preferably about 0.1 to 20 mg. In parenteral administration, a single dose of the compound may vary depending upon subject to be administered, target organ, conditions, route of administration, etc., and in the form of an injectable dosage form, it is advantageous to intravenously administer the compound to the patient (as 60 kg body weight) with cancer generally in a daily dose of about 0.001 to 30 mg, preferably about 0.01 to 20 mg, and more preferably about 0.01 to 10 mg. For other animal species, the corresponding dose as converted per 60 kg weight can be administered. Of course, the dose may vary depending on individual conditions as described above; in such a case, a dose less than the dose given above may be sufficient, or may be higher than the range above.

It is possible to set any range of a dose for the concomitant drug, so long as it causes no adverse side effects. A daily dose of the concomitant drug may vary depending on the severity of disease, the age, sex, body weight and susceptibility of the subject, dosing period and intervals, the properties, formulation, type and active ingredients of the pharmaceutical preparation, etc. and is not particularly limited. For example, in oral administration, the dose is about 0.001 to 2000 mg, preferably about 0.01 to 500 mg, and more preferably about 0.1 to 100 mg in terms of a drug, per kg body weight of a mammal; usually, this dose is administered by dividing 1 to 4 times per day.

When the pharmaceutical of the present invention is administered, it may be administered concomitantly. Alternatively, the concomitant drug is first administered and then the compound of the present invention is administered, or the compound of the present invention is first administered and then the concomitant drug is administered. When they are administered at certain time intervals, the intervals vary depending on the active ingredient to be administered, dosage form and route of administration; for example, when the concomitant drug is first administered, the compound of the present invention may be administered within 1 minute to 3 days, preferably 10 minutes to 1 day, more preferably 15 minutes to 1 hour after the administration of the concomitant drug. When the compound of the present invention is first administered, the concomitant drug may be administered within 1 minute to 1 day, preferably 10 minutes to 6 hours, more preferably 15 minutes to 1 hour after the administration of the compound of the present invention.

As a preferred dosing method, for example, about 0.001 to 200 mg/kg of the concomitant drug in the form of an oral dosage preparation is administered orally and, after about 15 minutes, about 0.005 to 0.5 mg/kg of the compound of the present invention in the form of a parenteral preparation is administered parenterally as a daily dose.

As the metastins, there are used, for example, human metastin described in WO 00/24890, mouse or rat metastin described in WO 01/75104, etc.

Specific examples of human metastin include a peptide comprising the N-terminal 47th-54th amino acid sequence in the amino acid sequence represented by SEQ ID NO: 1 and consisting of 8 to 54 amino acid residues, and the like.

The "peptide comprising the N-terminal 47th-54th amino acid sequence in the amino acid sequence represented by SEQ ID NO: 1 and consisting of 8 to 54 amino acid residues" may be any peptide, as far as it is a peptide comprising the N-terminal 47th-54th amino acid sequence in the amino acid sequence represented by SEQ ID NO: 1 and consisting of 8 to 54 amino acid residues, but means that these peptides have substantially the same physiological activity (e.g., a receptor binding activity, a signal transduction action, a blood glucose level elevating action, a pancreatic glucagon secretion promoting action, a urine formation promoting action, etc.). Specifically, there are used (i) a peptide having the amino acid sequence represented by SEQ ID NO: 1, (ii) a peptide comprising the N-terminal 47th-54th amino acid sequence at the C terminus in the amino acid sequence represented by SEQ ID NO: 1 and consisting of 8 to 54 amino acid residues, etc.

More specifically, human metastin used includes (i) a peptide consisting of the amino acid sequence represented by SEQ ID NO: 1 (human metastin 54 (1-54)), (ii) a peptide consisting of the N-terminal 40th-54th amino acid sequence in the amino acid sequence represented by SEQ ID NO: 1 (human metastin 15 (40-54); SEQ ID NO: 15), (iii) a peptide consisting of the N-terminal 45th-54th amino acid sequence in the amino acid sequence represented by SEQ ID NO: 1 (human metastin 10 (45-54); SEQ ID NO: 16), (iv) a peptide consisting of the N-terminal 46th-54th amino acid sequence in the amino acid sequence represented by SEQ ID NO: 1 (human metastin 9 (46-54); SEQ ID NO: 17), (v) a peptide consisting of the N-terminal 47th-54th amino acid sequence in the amino acid sequence represented by (human metastin 8 (47-54); SEQ ID NO: 18), etc.

As mouse metastin (A), there are used, for example, (i) a peptide comprising the N-terminal 134th-141st amino acid sequence in the amino acid sequence represented by SEQ ID NO: 3 and consisting of 8 to 52 amino acid residues. Specific examples of mouse metastin (A) used include (i) a peptide consisting of the N-terminal 90th-141 st amino acid sequence in the amino acid sequence represented by SEQ ID NO: 3, (ii) a peptide consisting of the N-terminal 132nd-141st amino acid sequence in the amino acid sequence represented by SEQ ID NO: 3, (iii) a peptide consisting of the N-terminal 127th-141st amino acid sequence in the amino acid sequence represented by SEQ ID NO: 3, and the like.

As mouse metastin (B), there are used, for example, a peptide comprising the N-terminal 138th-145th amino acid sequence in the amino acid sequence represented by SEQ ID NO: 5 and consisting of 8 to 52 amino acid residues. Specific examples of mouse metastin (B) used include a peptide consisting of the N-terminal 94th-145th amino acid sequence in the amino acid sequence represented by SEQ ID NO: 5, and the like.

As rat metastin, there are used, for example, a peptide comprising the N-terminal 112th-119th amino acid sequence in the amino acid sequence represented by SEQ ID NO: 7 and consisting of 8 to 52 amino acid residues. Specific examples of rat metastin used include (i) a peptide consisting of the N-terminal 68th-119th amino acid sequence in the amino acid sequence represented by SEQ ID NO: 7, (ii) a peptide consisting of the N-terminal 110th-119th amino acid sequence in the amino acid sequence represented by SEQ ID NO: 7, (iii) a peptide consisting of the N-terminal 105th-119th amino acid sequence in the amino acid sequence represented by SEQ ID NO: 7, and the like.

As used herein, the metastins are represented in accordance with the conventional way of describing peptides, that is, the N-terminus (amino end) at the left hand and the C-terminus (carboxyl end) at the right hand. In the peptide represented by SEQ ID NO: 1, the C-terminus may be in any form of a carboxyl group (-COOH), a carboxylate (-COO-), an amide (-CONH₂) and an ester (-COOR). Herein, examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl such as a phenyl-C₁₋₂ alkyl group such as benzyl, phenethyl, etc., an α-naphthyl-C₁₋₂ alkyl group such as α-naphthylmethyl, etc.; pivaloyloxymethyl group, which are widely used as an ester for oral use, and the like.

Furthermore, the metastins include peptides, wherein the amino at the N-terminal methionine residue is protected with a protecting group (e.g., a C₁₋₆ acyl group such as formyl, a C₂₋₆ alkanoyl group such as acetyl, etc.); those wherein the N-terminal region is cleaved in vivo and the glutamyl group thus formed is pyroglutaminated; those wherein a substituent (e.g., -OH, -SH, -COOH, amino, imidazole, indole, guanidino, etc.) on the side chain of an amino acid in the molecule is protected with a suitable protecting group (e.g., a C₁₋₆ acyl group such as formyl, a C₂₋₆ alkanoyl group such as acetyl, etc.), or conjugated peptides such as glycopeptides bound to sugar chains.

Salts of the metastin of the present invention used are salts with physiologically acceptable bases (e.g., alkali metal salts) or acids (e.g., organic acids or inorganic acids), especially physiologically acceptable acid addition salts are preferred. Examples of such salts include salts with inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

As the DNA encoding metastins, there are used, for example, a DNA encoding human metastin described in WO 00/24890, a DNA encoding mouse or rat metastin described in WO 01/75104, etc.

The DNAs encoding the metastins may be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA or mRNA fraction prepared from the cells and tissues described above.

The DNA encoding human metastin, mouse metastin precursor (A), mouse metastin precursor (B) or rat metastin precursor may be any DNA, so long as each is a DNA containing a nucleotide sequence represented by SEQ ID NO: 2, SEQ ID NO: 4, SEQ ID NO: 6 or SEQ ID NO: 8, or a DNA containing a nucleotide sequence hybridizable to the nucleotide sequence represented by any nucleotide sequence represented by SEQ ID NO: 2, SEQ ID NO: 4, SEQ ID NO: 6 or SEQ ID NO: 8 under highly stringent conditions and encoding the human metastin, mouse metastin (A), mouse metastin (B) or rat metastin described above.

Specific examples of the DNA hybridizable to the nucleotide sequence represented by any of SEQ ID NO: 2, SEQ ID NO: 4, SEQ ID NO: 6 or SEQ ID NO: 8 include a DNA containing a nucleotide sequence having at least about 70% homology, preferably at least about 80% homology, more preferably at least about 90% homology and the most preferably at least about 95% homology, to the nucleotide sequence represented by any of SEQ ID NO: 2, SEQ ID NO: 4, SEQ ID NO: 6 or SEQ ID NO: 8.

Homology in the nucleotide sequence can be measured under the following conditions (an expectation value =10; gaps are allowed; filtering = ON; match score = 1; mismatch score = -3) using the homology scoring algorithm NCBI BLAST (National Center for Biotechnology Information Basic Local Alignment Search Tool).

The hybridization can be carried out by per se publicly known methods or by modifications of these methods, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under highly stringent conditions.

The highly stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration of about 19 mM at a temperature of about 65°C are most preferred.

Specifically, as the DNA encoding the human metastin comprising the amino acid sequence represented by SEQ ID NO: 1, the DNA consisting of the nucleotide sequence represented by SEQ ID NO: 2 can be used. Accordingly, for the nucleotide sequence encoding the human metastin consisting of the various amino acid sequences described above, a nucleotide sequence corresponding to each of the partial amino acid sequences in the amino acid sequence represented by SEQ ID NO: 1 may be chosen from the nucleotide sequence represented by SEQ ID NO: 2.

As the DNA encoding the mouse metastin precursor (A) comprising the amino acid sequence represented by SEQ ID NO: 3, there can be employed a DNA consisting of the nucleotide sequence represented by SEQ ID NO: 4. Accordingly, for the nucleotide sequence encoding the mouse metastin precursor (A) consisting of the various amino acid sequences described above, a nucleotide sequence corresponding to each of the partial amino acid sequences in the amino acid sequence represented by SEQ ID NO: 3 may be chosen from the nucleotide sequence represented by SEQ ID NO: 4.

As the DNA encoding the mouse metastin precursor (B) comprising the amino acid sequence represented by SEQ ID NO: 5, there can be employed a DNA consisting of the nucleotide sequence represented by SEQ ID NO: 6. Accordingly, for the nucleotide sequence encoding the mouse metastin precursor (B) comprising of the various amino acid sequences described above, a nucleotide sequence corresponding to each of the partial amino acid sequences in the amino acid sequence represented by SEQ ID NO: 5 may be chosen from the nucleotide sequence represented by SEQ ID NO: 6.

As the DNA encoding the rat metastin comprising the amino acid sequence represented by SEQ ID NO: 7, there can be employed a DNA consisting of the nucleotide sequence represented by SEQ ID NO: 8. Accordingly, for the nucleotide sequence encoding the rat metastin comprising the various amino acid sequences described above, a nucleotide sequence corresponding to each of the partial amino acid sequences in the amino acid sequence represented by SEQ ID NO: 7 may be chosen from the nucleotide sequence represented by SEQ ID NO: 8.

More specifically, for the peptide comprising the amino acid sequence represented by SEQ ID NO: 1 (human metastin 54 (1-54)), a DNA comprising the nucleotide sequence represented by SEQ ID NO: 2, etc. is used.

As a DNA encoding the peptide consisting of the N-terminal 40th-54th amino acid sequence in the amino acid sequence represented by SEQ ID NO: 1 (human metastin 15 (40-54); SEQ ID NO: 15), a DNA comprising the nucleotide sequence represented by SEQ ID NO: 19, etc. is used.

As a DNA encoding the peptide consisting of the N-terminal 45th-54th amino acid sequence in the amino acid sequence represented by SEQ ID NO: 1 (human metastin 10 (45-54); represented by SEQ ID NO: 16), a DNA comprising the nucleotide sequence represented by SEQ ID NO: 20, etc. is used.

As a DNA encoding the peptide consisting of the N-terminal 46th-54th amino acid sequence in the amino acid sequence represented by SEQ ID NO: 1 (human metastin 9 (46-54); represented by SEQ ID NO: 17), a DNA comprising the nucleotide sequence represented by SEQ ID NO: 21, etc. is used.

As a DNA encoding the peptide consisting of the N-terminal 47th-54th amino acid sequence in the amino acid sequence represented by SEQ ID NO: 1 (human metastin 8 (47-54); represented by SEQ ID NO: 18), a DNA containing the nucleotide sequence represented by SEQ ID NO: 22, etc. is used.

As the metastin receptor, its partial peptides or salts thereof, there are used, for example, a human metastin receptor, its partial peptides or salts thereof described in WO 00/24890, a mouse or rat human metastin receptor, its partial peptides or salts thereof described in WO 01/75104, etc.

Specifically, the metastin receptor includes a protein comprising the same or substantially the same amino acid sequence as the amino acid sequence represented by SEQ ID NO: 9, SEQ ID NO: 11 or SEQ ID NO: 13, etc.

The amino acid sequence which has substantially the same amino acid sequence as the amino acid sequence represented by SEQ ID NO: 9, SEQ ID NO: 11 or SEQ ID NO: 13 includes, for example, an amino acid sequence having at least about 70% homology, preferably at least about 80% homology, more preferably at least about 90% homology, and most preferably at least about 95% homology, to the amino acid sequence represented by SEQ ID NO: 9, SEQ ID NO: 11 or SEQ ID NO: 13.

Homology of the amino acid sequences can be determined under the following conditions (an expectation value =10; gaps are allowed; matrix = BLOSUM62; filtering = OFF) using a homology scoring algorithm NCBI BLAST (National Center for Biotechnology Information Basic Local Alignment Search Tool).

As the protein comprising substantially the same amino acid sequence as the amino acid sequence represented by SEQ ID NO: 9, SEQ ID NO: 11 or SEQ ID NO: 13, preferred is a protein comprising substantially the same amino acid sequence as the amino acid sequence represented by SEQ ID NO: 9, SEQ ID NO: 11 or SEQ ID NO: 13 and having the activity of the same nature as that of a protein having the amino acid sequence represented by SEQ ID NO: 9, SEQ ID NO: 11 or SEQ ID NO: 13, etc.

The activity of substantially the same nature includes, for example, a ligand binding activity, a signal transduction activity, and the like. The "substantially the same nature" is used to mean that the nature of these activities is equivalent in terms of quality. Thus, the activities such as a ligand binding activity, a signal transduction activity, etc. are preferably equivalent (e.g., about 0.01 to 100 times, preferably about 0.5 to 10 times, more preferably 0.5 to 2 times), but differences in degree such as a level of these activities, quantitative factors such as a molecular weight of the protein may be present and allowable.

The activities such as a ligand binding activity, a signal transduction activity, etc. can be assayed by per se publicly known methods with modifications and may be determined according to methods for determining a ligand or screening methods described in, e.g., WO 00/24890 or WO 01/75104.

Examples of the metastin receptor used include proteins comprising (i) the amino acid sequence represented by SEQ ID NO: 9, SEQ ID NO: 11 or SEQ ID NO: 13, of which at least 1 or 2 (preferably about 1 to about 30, more preferably about 1 to about 10 and most preferably several (1 or 2)) amino acids are deleted; (ii) the amino acid sequence represented by SEQ ID NO: 9, SEQ ID NO: 11 or SEQ ID NO: 13, to which at least 1 or 2 (preferably about 1 to about 30, more preferably about 1 to about 10 and most preferably several (1 or 2)) amino acids are added; (iii) the amino acid sequence represented by SEQ ID NO: 9, SEQ ID NO: 11 or SEQ ID NO: 13, in which at least 1 or 2 (preferably about 1 to about 30, more preferably about 1 to about 10 and most preferably several (1 or 2)) amino acids are substituted by other amino acids; or (iv) a combination of these amino acid sequences; and the like.

As used herein, the metastin receptors are represented in accordance with the conventional way of describing peptides, that is, the N-terminus (amino end) at the left hand and the C-terminus (carboxyl end) at the right hand. In the metastin receptors including the metastin receptor represented by SEQ ID NO: 9, SEQ ID NO: 11 or SEQ ID NO: 13, the C-terminus may be in any form of a carboxyl group (-COOH), a carboxylate (-COO-), an amide (-CONH₂) and an ester (-COOR). Herein, examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl such as a phenyl-C₁₋₂ alkyl group such as benzyl, phenethyl, etc., an α-naphthyl-C₁₋₂ alkyl group such as α-naphthylmethyl, etc.; and pivaloyloxymethyl group, which are widely used as an ester for oral use, and the like.

Where the metastin receptors contain a carboxyl group (or a carboxylate) at a position other than the C-terminus, the carboxyl group may be amidated or esterified and such amides or esters are also included within the receptor protein of the present invention. In this case, the ester group used may be the same group as the C-terminal esters described above.

Furthermore, the metastin receptors include those wherein the amino group at the N-terminal methionine residue is protected with a protecting group (e.g., a C₁₋₆ acyl group such as formyl, a C₂₋₆ alkanoyl group such as acetyl, etc.); those wherein the N-terminal region is cleaved in vivo and the glutamyl group thus formed is pyroglutaminated; those wherein a substituent (e.g., -OH, -SH, amino, imidazole, indole, guanidino, etc.) on the side chain of an amino acid in the molecule is protected with a suitable protecting group (e.g., a C₁₋₆ acyl group such as formyl, a C₂₋₆ alkanoyl group such as acetyl, etc.), or conjugated proteins such as glycoproteins bound to sugar chains.

Specific examples of the metastin receptors include human metastin receptor consisting of the amino acid sequence represented by SEQ ID NO: 9, rat metastin receptor consisting of the amino acid sequence represented by SEQ ID NO: 11, mouse metastin receptor consisting of the amino acid sequence represented by SEQ ID NO: 13, etc.

The partial peptide of the metastin receptor (hereinafter sometimes simply referred to as the partial peptide) may be any peptide, so long as it is a partial peptide of the metastin receptor described above; there are used those such as protein molecules of the metastin receptor, which are the sites exposed outside the cell membrane, and having a ligand binding activity.

Specifically, the partial peptide of the metastin receptor consisting of the amino acid sequence represented by SEQ ID NO: 9, SEQ ID NO: 11 or SEQ ID NO: 13 is a peptide containing the parts analyzed to be extracellular domains (hydrophilic domains) in the hydrophobic plotting analysis. A peptide containing a hydrophobic domain in part can be used as well. In addition, the peptide may contain each domain separately or a plurality of domains together.

In the metastin receptor, preferred partial peptides are those having the number of amino acids of at least 20, preferably at least 50, and more preferably at least 100, in the amino acid sequence described above, which constitutes the metastin receptor.

The partial peptide may be a peptide having the amino acid sequence described above, of which at least 1 or 2 (preferably about 1 to about 10 and more preferably several (1 or 2)) amino acids are deleted; to which at least 1 or 2 (preferably about 1 to about 20, more preferably about 1 to about 10 and much more preferably several (1 or 2)) amino acids are added; or, in which at least 1 or 2 (preferably about 1 to about 10 and more preferably several (1 or 2)) amino acids are substituted by other amino acids.

In the partial peptide, the C terminus may be any form of a carboxyl group (-COOH), a carboxylate (-COO-), an amide (-CONH₂) and an ester (-COOR), as in the metastin receptor described above.

Furthermore, the partial peptides include peptides, wherein the amino group at the N-terminal methionine residue is protected with a protecting group; those wherein the N-terminal region is cleaved in vivo and Gln thus formed is pyroglutaminated; those wherein a substituent on the side chain of an amino acid in the molecule is protected with a suitable protecting group, or conjugated peptides such as glycopeptides bound to sugar chains, as in the metastin receptors described above.

Salts of the metastin receptor or the partial peptide used are salts with physiologically acceptable bases or acids, especially physiologically acceptable acid addition salts are preferred. Examples of the salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The DNA encoding the metastin receptor or its partial peptides used includes, for example, a DNA encoding the human metastin receptor or its partial peptides described in WO 00/24890, a DNA encoding the mouse or rat metastin receptor or its partial peptides described in WO 01/75104, etc.

The DNAs encoding the metastin receptor or its partial peptides may be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagcmid. The DNA may also be directly amplified by reverse transcriptase polymerase chain reaction (hereinafter abbreviated as RT-PCR) using the total RNA or mRNA fraction prepared from the cells and tissues described above.

Specifically, the DNA encoding human metastin receptor, mouse metastin receptor or rat metastin receptor may be any DNA, so long as it is a DNA comprising each nucleotide sequence represented by SEQ ID NO: 10, SEQ ID NO: 12 or SEQ ID NO: 14, or a DNA comprising a nucleotide sequence hybridizable to the nucleotide sequence represented by SEQ ID NO: 10, SEQ ID NO: 12 or SEQ ID NO: 14 under highly stringent conditions and encoding a receptor having the activity of substantially the same nature (e.g., a ligand binding activity, a signal transduction activity, etc.) as that of the human metastin receptor, mouse metastin receptor or rat metastin receptor consisting of the amino acid sequence represented by SEQ ID NO: 10, SEQ ID NO: 12 or SEQ ID NO: 14.

Examples of the DNA hybridizable to the nucleotide sequence represented by any of SEQ ID NO: 10, SEQ ID NO: 12 or SEQ ID NO: 14 include DNAs containing a nucleotide sequence having at least about 70% homology, preferably at least about 80% homology, more preferably at least about 90% homology and the most preferably at least about 95% homology, to the nucleotide sequence represented by any of SEQ ID NO: 10, SEQ ID NO: 12 or SEQ ID NO: 14.

Homology in the nucleotide sequence can be measured under the following conditions (an expectation value = 10; gaps are allowed; filtering = ON; match score = 1; mismatch score = -3) using the homology scoring algorithm NCBI BLAST (National Center for Biotechnology Information Basic Local Alignment Search Tool).

The hybridization can be carried out by per se publicly known methods or by modifications of these methods, for example, according to the method described in Molecular Cloning, 2nd (J. Sambrook et al., Cold Spring Harbor Lab. Press, 1989), etc. A commercially available library may also be used according to the instructions of the attached manufacturer's protocol. Preferably, the hybridization can be carried out under highly stringent conditions.

The highly stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration of about 19 mM at a temperature of about 65°C are most preferred.

More specifically, as the DNA encoding the human metastin receptor consisting of the amino acid sequence represented by SEQ ID NO: 9, the DNA consisting of the nucleotide sequence represented by SEQ ID NO: 10 is used.

As the DNA encoding the rat metastin receptor consisting of the amino acid sequence represented by SEQ ID NO: 11, the DNA consisting of the nucleotide sequence represented by SEQ ID NO: 12 is used.

As the DNA encoding the mouse metastin receptor consisting of the amino acid sequence represented by SEQ ID NO: 13, the DNA consisting of the nucleotide sequence represented by SEQ ID NO: 14 is used.

The metastin receptors, their partial peptides or salts thereof and the DNAs encoding the metastin receptors or their partial peptides can be obtained or produced by the methods described in WO 00/24890 or WO 01/75104.

All publications referred to in the specification, e.g., prior art documents, patent applications laid open to public inspection, patent publications and other patent references, are incorporated herein by reference in their entirety. This application also includes the disclosure of Japanese Patent Application No. 2008-114480, to which the benefit of priority is hereby claimed.

The present invention will be described in detail by referring to EXAMPLES, FORMULATION EXAMPLES and TEST EXAMPLES, but is not deemed to be limited thereto, and any modification may be made without departing from the scope of the present invention.

In the following EXAMPLES, the term "room temperature" normally means a temperature of about 10°C to about 35°C. In percentages, the yield is shown by mol/mol% and the solvent used in chromatography by vol%, and the remaining by wt%. In proton NMR spectra, data on OH, NH protons, etc. that are broad and unidentified are not shown.

The other abbreviations used in the specification mean as follows.

| Abbreviation | Description |
|---|---|
| 10Ψ,CSNH | : C-terminal-CONH₂ at 10-position is substituted with -CSNH₂. |
| 1Ψ2,CH₂NH | : The -CONH- bond between 1- and 2-positions is substituted with the -CH₂NH-bond. |
| 2Ψ3,CH₂NH | : The -CONH- bond between 2- and 3-positions is substituted with the-CH₂NH-bond. |
| 3Ψ4,CH₂NH | : The -CONH- bond between 3- and 4-positions is substituted with the -CH₂NH-bond. |
| 4Ψ5,CH₂NH | : The -CONH- bond between 4- and 5-positions is substituted with the -CH₂NH- bond. |
| 6Ψ7,CSNH | : The -CONH- bond between 6- and 7-positions is substituted with the -CSNH- bond. |
| 6Ψ7,NHCO | : The -CONH- bond between 6- and 7-positions is substituted with the-NHCO-bond. |
| 6Ψ7,CH₂NH | : The -CONH- bond between 6- and 7-positions is substituted with the -CH₂NH- bond. |
| 6Ψ7,CH₂O | : The -CONH- bond between 6- and 7-positions is substituted with the -CH₂O- bond. |
| 7Ψ8,CH₂NH | : The -CONH- bond between 7- and 8-positions is substituted with the -CH₂NH- bond. |
| 8Ψ9,CH₂NH | : The -CONH- bond between 8- and 9-positions is substituted with the-CH₂NH-bond. |
| 9Ψ10,CH₂NH: | The -CONH- bond between 9- and 10-positions is substituted with the -CH₂NH- bond. |
| Aad | : 2-aminoadipic acid |
| Abu | : 2-aminobutanic acid |
| Abz(2) | : 2-aminobenzoic acid |
| Abz(3) | : 3-aminobenzoic acid |
| Ac | : acetyl |
| AcONB | : N-acetoxy-5-norbornene-2,3-dicarboximide |
| Acp | : 6-aminocaproic acid |
| AcOEt | : ethyl acetate |
| AcOH | : acetic acid |
| Aib | : α-aminoisobutanoic acid |
| Ala(2-Qui) | :2-quinolylalanine |
| Ala(3-Bzt) | :3-benzothienylalanine |
| Ala(cBu) | : cyclobutylalanine |
| Ala(cPr) | : cyclopropylalanine |
| Ala(Pip) | : (4-piperidin-1-yl)alanine |
| Alb | : albizziin 2-amino-3-ureidopropionic acid |
| Ambz(4) | :4-aminomethylbenzoyl |
| Arg(Ac) | : Nω-acetylarginine |
| Arg(Boc₂,Me) | : Nω,ω'- bis-tert-butoxycarbonyl-Nω-methylarginine |
| Arg(Et) | :Nω-ethylarginine |
| Arg(Me) | : Nω-methylarginine |
| Arg(asyMe₂) orArg(Me₂)asym | : asymmetric-Nω,ω-dimethylarginine |
| Arg(symMe₂) or Arg(Me₂)sym | : symmetric-Nω,ω'-dimethylarginine |
| Arg(NO₂) | : Nω-nitroarginine |
| Arg(Pbf) | : Nω-2,2,4,6,7-pentamethyldihydrobenzofuransulfonylarginine |
| Arg(n-Pr) | : Nω-propylarginine |
| Arg(Tos) | : Nω-tosylarginine |
| Asp(NHMe) | : Nω-methylasparagine |
| Asp(NMe₂) | : Nω,ω-dimethylasparagine |
| Asp(NHPen) | : Nω-pentylasparagine |
| Asp(NHcPr) | : Nω-cyclopropylasparagine |
| Asp(NHBzl) | : Nω-benzylasparagine |
| AzaGly | : azaglycine |
| AzaPhe | : azaphenylalanine |
| Aze(2) | : azetidine-2-carboxylic acid |
| β-Ala | : β-alanine |
| Bn | : benzyl |
| Boc | : tert-butoxycarbonyl |
| Boc₂O | : di-tert-butyl dicarbonate |
| Br-Z | : 2-bromobenzyloxycarbonyl |
| Bu^{t} | : tert-butyl |
| Bzl | : benzyl |
| CDI | : 1,1'-carbonyldiimidazole |
| Cha | : cyclohexylalanine |
| CIP | : 2-chloro-1,3-dimethylimidazolium tetrafluoroborate |
| Cit | : citrulline |
| Clt resin | : 2-chlorotrytyl resin |
| Cl-Z | : 2-chlorobenzyloxycarbonyl |
| Dab | : 2,4-diaminobutanoic acid |
| Dap | : 2,3-diaminopropionic acid |
| Dap(Ac) | : Nβ-acetyl-β-diaminopropionic acid |
| Dap(For) | : Nβ-formyl-β-diaminopropionic acid |
| Dap(Gly) | : Nβ-glycyl-β-diaminopropionic acid |
| Dap(GnGly) | : Nβ-(N-guanidinoglycyl)-β-diaminopropionic acid |
| DCM | : dichloromethane |
| DEA | : diethylamine |
| DEAD | : diethyl azodicarboxylate |
| DIEA | : N,N-diisopropylethylamine |
| DIPCDI | : 1,3-diisopropylcarbodiimide |
| DMAP | : 4-dimethylaminopyridine |
| DMF | : N,N-dimethylformamide |
| EDC | : 1-ethyl-3-(3-dimethylaminopropyl)carbodiimide |
| EDT | : 1,2-ethanedithiol |
| Fmoc | : 9-fluorenylmethoxycarbonyl |
| For | : formyl |
| γ-Abu | : 4-aminobutanoic acid |
| γ-MeLeu, Leu(Me) | : γ-methylleucine |
| Glyψ[(E)CF=CH]Leu | : The -CONH- bond between Gly and Leu is substituted with (E)-fluoroalkene. |
| Glyψ[(E)CH=CH]Leu | The -CONH- bond between Gly and Leu is substituted with (E)-alkene. |
| Glyψ[(E)CMe=CH]Leu : | The -CONH- bond between Gly and Leu is substituted with (E)-methylalkene. |
| Glyψ(CH2CH2)Leu | : The -CONH- bond between Gly and Leu is substituted with -CH₂CH₂-bond. |
| Glyψ(CH2S)Leu | : The -CONH- bond between Gly and Leu is substituted with -CH₂S- bond. |
| Gn | : guanidino |
| GuAmb | : 4-guanidinomethylbenzoyl |
| Har | : homoarginine |
| Har(Me) | : Nω-methylhomoarginine |
| His(3Me) | : 3-methylhistidine π-methylhistidine |
| HOAt | : 1-hydroxy-7-azabenzotriazole |
| HOBt | : 1-hydroxybenzotriazole |
| HOOBt | : 3,4-dihydro-3-hydroxy-4-oxo-1,2,3-bcnzotriazinc |
| HONB | : N-hydroxy-5-norbornene-2,3-dicarboxamide |
| Hph | : homophenylalanine |
| Hyp | : trans-4-hydroxyproline |
| Hyp(Bzl) | :O-benzyl-trans-4-hydroxyproline |
| iBu | : isobutyl |
| IndPr | :3-(indol-3-yl)propionyl |
| Izc | : imidazolidine-2-carboxylic acid |
| Lys(Me₂) | : Nε,ε-dimethyllysine |
| MBHA | : p-methylbenzhydrylamine |
| MeOH | : methanol |
| Ms | : mesyl |
| Mtt | : 4-methyltrytyl |
| N((CH₂)₃Gn)Gly | : N-(3-guanidinopropyl)glycine |
| Nal(1) | : 1-naphthylalamne |
| Nal(2) | : 2-naphthylalanine |
| Nar | : norarginine |
| Nar(Me) | : Nω-methylnorarginine |
| Nle | : norleucine |
| NMeAla | : Nα-methylalanine |
| NMeArg | : Nα-methylarginine |
| NMeAsn | : Nα-methylasparagine |
| NMeLeu | : Nα-methylleucine |
| NMePhe | : Nα-methylphenylalanine |
| NMeSer | : Nα-methylserine |
| NMeTrp | : Nα-methyltryptophan |
| NMcTyr | : Nα-mcthyltyrosinc |
| Nva | : Norvaline |
| OBu^{t} | : tert-butoxy |
| Om | : ornithine |
| Orn(Mtt) | : Nδ-(4-methyltrytyl)ornithme |
| PAL | : 5-(4-(9-fluorenylmethoxycarbonyl)aminomethyl-3,5-dimethoxyphenoxy)valeric acid |
| Pbf | : 2,2,4,6,7-pentamethyldihydrobenzofuran-5-sulfonyl |
| pGlu | : pyroglutamic acid |
| Phc(2Cl) | : 2-chlorophcnylalaninc |
| Phe(2F) | : 2-fluorophenylalanine |
| Phe(3,4Cl₂) | :3,4-dichlorophenylalanine |
| Phe(3,4F₂) | :3,4-difluorophenylalanine |
| Phe(3CF₃) | : 3-trifluoromethylphenylalanine |
| Phe(3Cl) | : 3-chlorophenylalanine |
| Phe(3F) | : 3-fluorophenylalanine |
| Phe(4Cl) | : 4-chlorophenylalanine |
| Phe(4CN) | : 4-cyanophenylalanine |
| Phe(4F) | : 4-fluorophenylalanine |
| Phe(4Gn) | : 4-guanidinophenylalanine |
| Phe(4NH₂) | : 4-aminophenylalanine |
| Phe(4NO₂) | : 4-nitrophenylalanine |
| Phe(4CN) | : 4-cyanophenylalanine |
| Phe(F₅) | : pentafluorophenylalanine |
| Phe(2Me) | : 2-methylphenylalanine |
| Phe(3Me) | : 3- methylphenylalanine |
| Phe(4Me) | : 4- methylphenylalanine |
| Phey(CH₂CH₂)AzaGly : | The -CONH- bond between Phe and AzaGly is substituted with -CH₂CH₂- bond. |
| Phey(CH₂NH)AzaGly : | The -CONH- bond between Phe and AzaGly is substituted with -CH₂NH- bond. |
| Pheψ[(E)CH=CH]Gly | The -CONH- bond between Phe and Gly is substituted with (E)-alkene. |
| Pheψ(CH₂CH₂)Gly | : The -CONH- bond between Phe and Gly is substituted with -CH₂CH₂- bond. |
| Pheψ(CH₂S)Gly | : The -CONH- bond between Phe and Gly is substituted with -CH₂S- bond. |
| Pheψ((R)CH(OH)-(E)CH=)Gly : | The -CONH- bond between Phe and Gly is substituted with -CH(OH)-CH- bond, the -CH(OH)- position is in (R) configuration, and the bond between the carbon atom of -CH- position and the α-carbon atom of Gly is (E) alkene. |
| | |
| Pheψ((S)CH(OH)-(E)CH=)Gly : | The -CONH- bond between Phe and Gly is substituted with -CH(OH)-CH- bond, the -CH(OH)- position is in (S) configuration, and the bond between the carbon atom of -CH- position and the α-carbon atom of Gly is (E) alkene. |
| Pheψ((R)CH(OH)-CH₂)Gly | : The -CONH- bond between Phe and Gly is substituted with -CH(OH)-CH₂- bond, and the -CH(OH)- position is in (R) configuration. |
| Phey((S)CH(OH)-CH₂)Gly | : The -CONH- bond between Phe and Gly is substituted with -CH(OH)-CH₂- bond, and the -CH(OH)- position is in (S) configuration. |
| Pheψ(CH₂O)Gly | : The -CONH- bond between Phe and Gly is substituted with -CH₂O- bond. |
| Pheψ(COCH₂)Gly | : The -CONH- bond between Phe and Gly is substituted with -COCH₂- bond. |
| Pheψ(CSNH)Gly | : The -CONH- bond between Phe and Gly is substituted with -CSNH- bond. |
| Pheψ(CSNH)-NH₂ | : The C terminal phenylalanylamide is substituted with phenylalanylthioamide. |
| Pheψ(NHCO)Gly | : The -CONH- bond between Phe and Gly is substituted with -NHCO- bond. |
| Phe-ψ(CH₂NH)-oxoGly : | The -CONH- bond between Phe and Gly is substituted with -CH₂NH- bond, and hydrogen on the α-carbon of Gly is substituted with oxygen. |
| Pheψ(CH₂NHCOCONH)Leu | : The -CONH- bond between Phe and Leu is -CH₂ substituted with NHCOCONH- bond. |
| Phg | : phenylglycine |
| PhOH | : phenol |
| PhSMe | : thioanisole |
| Pip(2) | : pipecolinic acid |
| Pic(3) | : 3-piperidinecarboxylic acid |
| Pip | : pipecolinic acid |
| Pro | : proline |
| Pro(4F) | : trans-4-fluoroproline |
| Pro(4NH₂) | : cis-4-aminoproline |
| Pya(2) | : 2-pyridylalanine |
| Pya(3) | : 3-pyridylalanine |
| Pya(4) | : 4-pyridylalanine |
| PyAOP | : (7-azabenzotriazolc-1-yloxy)-tris(pyrrolidino)phosphonium hexafluorophosphate |
| PyBOP | : (benzotriazole-1-yloxy)-tris(pyrrolidino)phosphonium hexafluorophosphate |
| PyBrop | : bromo-tris(pyrrolidino)phosphonium hexafluorophosphate |
| Pzc(2) | : piperazine-2-carboxylic acid |
| Sar | : Nα-methylglycine |
| Ser(Ac) | :O-acetylserine |
| Ser(Me) | :O-methylserine |
| Thi | : 2-thienylalanine |
| Thz | : thioproline |
| Tic | : 1,2,3,4-tetrahydroisoquinoline-2-carboxylic acid |
| TIS | : triisopropylsilane |
| Tle | : tert-leucine |
| Tos | : tosyl |
| Trp(For) | : Nⁱⁿ-formyltryptophan |
| Trt | : trytyl |
| Tyr(Me) | : O-methyltyrosine |
| Tyr(PO₃H₂) | : O-phosphotyrosine |
| Tyrψ(CH₂NH)Asn | : The -CONH- bond between Tyr and Asn is substituted with the -CH₂NH- bond. |
| TFA | : trifluoroacetic acid |
| TFE | : trifluoroethanol |
| Z | : benzyloxycarbonyl |

In the specification and drawings, where the codes of nucleotides and amino acids are denoted by abbreviations, they are based on the abbreviations in accordance with the IUPAC-IUB Commission on Biochemical Nomenclature or the common codes in the art, examples of which are shown below. For amino acids that may have the optical isomer, L form is presented unless otherwise indicated.
- DNA: : deoxyribonucleic acid
- cDNA: : complementary deoxyribonucleic acid
- A: : adenine
- T: : thymine
- G: : guanine
- C: : cytosine
- Y: : thymine or cytosine
- N: : thymine, cytosine, adenine or guanine
- R: : adenine or guanine
- M: : cytosine or adenine
- W: : thymine or adenine
- S: : cytosine or guanine
- RNA: : ribonucleic acid
- mRNA: : messenger ribonucleic acid
- dATP: : deoxyadenosine triphosphate
- dTTP: : deoxythymidine triphosphate
- dGTP: : deoxyguanosine triphosphate
- dCTP: : deoxycytidine triphosphate
- ATP: : adenosine triphosphate
- EDTA: : ethylenediaminetetraacetic acid
- SDS: : sodium dodecyl sulfate
- TFA: : trifluoroacetic acid
- EIA: : enzyme immunoassay
- Gly or G: : glycine
- Ala or A: : alanine
- Val or V: : valine
- Leu or L: : leucine
- Ile or I: : isoleucine
- Ser or S: : serine
- Thr or T: : threonine
- Cys or C: : cysteine
- Met or M: : methionine
- Glu or E: : glutamic acid
- Asp or D: : aspartic acid
- Lys or K: : lysine
- Arg or R: : arginine
- His or H: : histidine
- Phe or F: : phenylalanine
- Tyr or Y: : tyrosine
- Trp or W: : tryptophan
- Pro or P: : proline
- Asn or N: : asparagine
- Gln or Q: : glutamine
- pGlu: : pyroglutamic acid

The sequence identification numbers in the sequence listing of the specification indicates the following sequence, respectively.

### [SEQ ID NO: 1]

This shows the amino acid sequence of human-derived metastin (metastin).

### [SEQ ID NO: 2]

This shows the nucleotide sequence of DNA encoding human metastin.

### [SEQ ID NO: 3]

This shows the amino acid sequence of mouse metastin precursor (A).

### [SEQ ID NO: 4]

This shows the nucleotide sequence of DNA encoding mouse metastin precursor (A), which is the nucleotide sequence contained in plasmid pCMV-mKiSS-1 harbored on transformant Escherichia coli DH10B/pCMV-mKiSS-1.

### [SEQ ID NO: 5]

This shows the amino acid sequence of mouse metastin precursor (B).

### [SEQ ID NO: 6]

This shows the nucleotide sequence of DNA encoding mouse metastin precursor (B), which is the nucleotide sequence contained in plasmid pCR2.1-mKiSS-1.4A harbored on transformant Escherichia coli DH5α/pCR2.1-mKiSS-1.4A.

### [SEQ ID NO: 7]

This shows the amino acid sequence of rat-derived metastin precursor.

### [SEQ ID NO: 8]

This shows the nucleotide sequence of DNA encoding rat metastin precursor.

### [SEQ ID NO: 9]

This shows the amino acid sequence of human OT7T175 (metastin receptor).

### [SEQ ID NO: 10]

This shows the nucleotide sequence of DNA encoding human OT7T175 (metastin receptor).

### [SEQ ID NO: 11]

This shows the amino acid sequence ofrat OT7T175 (metastin receptor).

### [SEQ ID NO: 12]

This shows the nucleotide sequence of DNA encoding rat OT7T175 (metastin receptor).

### [SEQ ID NO: 13]

This shows the amino acid sequence of mouse OT7T175 (metastin receptor).

### [SEQ ID NO: 14]

This shows the nucleotide sequence of DNA encoding mouse OT7T175 (metastin receptor).

### [SEQ ID NO: 15]

This shows the amino acid sequence of human metastin 15 (40-54).

### [SEQ ID NO: 16]

This shows the amino acid sequence of human metastin 10 (45-54) (MS10).

### [SEQ ID NO: 17]

This shows the amino acid sequence of human metastin 9 (46-54).

### [SEQ ID NO: 18]

This shows the amino acid sequence of human metastin 8 (47-54).

### [SEQ ID NO: 19]

This shows the nucleotide sequence of DNA encoding human metastin 15 (40-54).

### [SEQ ID NO: 20]

This shows the nucleotide sequence of DNA encoding human metastin 10 (45-54).

### [SEQ ID NO: 21]

This shows the nucleotide sequence of DNA encoding human metastin 9 (46-54).

### [SEQ ID NO: 22]

This shows the nucleotide sequence of DNA encoding human metastin 8 (47-54).

The transformant *Escherichia coli* DH10B/pCMV-mKiSS-1 has been on deposit since January 24, 2000 with International Patent Organisms Depository, National Institute of Advanced Industrial Science and Technology (the former Ministry of International Trade and Industry, Agency of Industrial Science and Technology, National Institute of Bioscience and Human Technology (NIBH)), located at Central 6, 1-1-1 Higashi, Tsukuba, Ibaraki (postal code 305-8566), Japan, under the Accession Number FERM BP-7003 and since December 16, 1999 with Institute for Fermentation (IFO), located at 2-17-85, Juso-Honmachi, Yodogawa-ku, Osaka-shi, Osaka, Japan, under the Accession Number IFO 16348.

The transformant *Escherichia coli* DH5α/pCR2.1-mKiSS-1.4A has been on deposit since March 6, 2000 with International Patent Organisms Depository, National Institute of Advanced Industrial Science and Technology (the former Ministry of International Trade and Industry, Agency of Industrial Science and Technology, National Institute of Bioscience and Human Technology (NIBH)), located at Central 6, 1-1-1 Higashi, Tsukuba, Ibaraki (postal code 305-8566), Japan, under the Accession Number FERM BP-7073 and since February 16, 2000 with Institute for Fermentation (IFO), located at 2-17-85 Juso-Honmachi, Yodogawa-ku, Osaka-shi, Osaka, Japan, under the Accession Number IFO 16360.

### REFERENCE EXAMPLE 1

### Production of N-methyl-N,N'-bis-Boc-1-guanylpyrazole

Under a nitrogen flow, 720 mg of 60% NaH in oil was dissolved in 20 mL of dry DMF and 20 mL of dry DMF solution of 5.59 g of N,N'-bis-Boc-1-guanylpyrazole commercially available was added to the solution at 0°C, followed by stirring for 10 minutes. After 1.68 mL of methyl iodide was added thereto, the mixture was stirred at room temperature for 24 hours. After the solvent was removed by distillation, the residue was dissolved in AcOEt and the solution was washed with 1N HCl aqueous solution, saturated NaHCO₃ aqueous solution and then saturated NaCl aqueous solution. After drying over Na₂SO₄, the solvent was concentrated and the concentrate was purified by flash column chromatography (ethyl acetate/n-hexane = 1/4) using silica gel 60 (200 mL) to give 5.35 g of N-methyl-N,N'-bis-Boc-1-guanylpyrazole (yield 91.6%).
¹H NMR (300 MHz, CDCl₃): δ 8.00 (br s, 1H), 7.69 (br s, 1H), 6.42 (dd, 1H, J=2.7, 1.5 Hz), 3.25 (s, 3H), 1.53 (s, 9H), 1.30 (s, 9H),
Elemental analysis as C₁₅H₂₄N₄O₄

| | | | |
|---|---|---|---|
| Calcd.: | C, 55.54; | H, 7.46; | N, 17.27 |
| Found: | C, 55.36; | H, 7.48; | N, 17.06 |

Rf1: 0.64, Rf2: 0.79
Developing solvent for TLC: Rf1 (ethyl acetate/n-hexane = 1/2), Rf2
(methanol/chloroform = 2/98)
Elution time on HPLC: 26.7 mins.
Elution conditions:
Column: Wakosil-II 5C18 HG (4.6 x 100 mm)
Eluants: elution in a linear density gradient using eluant A: 0.1% TFA/water and eluant B: 0.1 % TFA-containing acetonitrile, with A/B = 100/0 to 20/80, (40 mins.)
Flow rate: 1.0mL/min.

### REFERENCE EXAMPLE 2

### Production of N-Methyl-N,N'-bis-Z-1-guanylpyrazole

In an argon atmosphere, 40 mg of 60% NaH in oil was dissolved in 5 mL of dry DMF and 5 mL of dry DMF solution of 380 mg of N,N'-bis-Z-1-guanylpyrazole commercially available was added to the solution at 0°C, followed by stirring for 10 minutes. After 125 µL of methyl iodide was added thereto, the mixture was stirred at room temperature for 15 hours. After the solvent was distilled off, the residue was dissolved in AcOEt and the solution was washed with 1N HCl aqueous solution, saturated NaHCO₃ aqueous solution and then saturated NaCl aqueous solution. After drying over Na₂SO₄, the solvent was concentrated to give 393 mg of the crude product. From the crude product, 170 mg was purified by flash column chromatography (ethyl acetate/n-hexane = 1/4) using silica gel 60 (75 mL) to give 152 mg of N-methyl-N,N'-bis-Z-1-guanylpyrazole (yield 89.5%).
¹H NMR (300 MHz, CDCl₃): δ 7.97 (br s, 1H), 7.61 (d, 1H, J=1.0Hz), 7.37-7.32 (m, 4H), 7.29-7.26 (m, 4H), 7.16-7.13 (m, 2H), 6.36 (dd, 1H, J=2.8, 1.6 Hz), 5.18 (s, 2H), 5.04 (s, 2H), 3.22 (s, 3H)
Elemental analysis as C₂₁H₂₀N₄O₄

| | | | |
|---|---|---|---|
| Calcd.: | C, 64.28; | H, 5.14; | N, 14.28 |
| Found: | C, 64.09; | H, 5.24; | N, 14.43 |

Rf1: 0.50, Rf2: 0.86
Developing solvent for TLC: Rf1 (ethyl acetate/n-hexane = 1/2), Rf2
(methanol/chloroform = 2/98)
Elution time on HPLC: 28.9 mins.
Elution conditions
Column: Wakosil-II 5C18 HG (4.6 x 100 mm)
Eluants: elution in a linear density gradient using eluant A: 0.1% TFA/water and eluant B: 0.1 % TFA-containing acetonitrile, with A/B = 100/0 to 20/80, (40 mins.)
Flow rate: 1.0 mL/min.

### EXAMPLE 1

### Production of Fmoc-AzaGly-Leu-Arg(Boc₂, Me)-Trp(Boc)-Rink Amide MBHA resin

Commercially available Rink Amide MBHA resin (0.4 mmol/g) (5 g) was swollen in DMF and then treated with 50 ml of 20% piperidine/DMF solution for 20 minutes to remove the Fmoc group. The resin obtained was washed in DMF and treated with of 4.213 g (8 mmol) of Fmoc-Trp(Boc)-OH, 1.272 mL (8 mmol) of DIPCDI and 16 mL (8 mmol) of 0.5 M HOAt/DMF solution at room temperature for 90 minutes to introduce Trp(Boc) to give the Fmoc-Trp(Boc)-Rink Amide MBHA resin. Om(Mtt) was introduced in a similar fashion to give 2 mmol of the Fmoc-Om(Mtt)-Trp(Boc)- Rink Amide MBHA resin. The resin obtained was washed and swollen in DCM. Then 50 mL of TFA/TIS/DCM (1/5/94) was added thereto. The mixture was shaken for 10 minutes to remove the solution through filtration. This procedure was repeated until yellow color caused by free Mtt group in the TFA/TIS/DCM (1/5/94) solution disappeared when the solution was added, thereby removing the Mtt group.

The resulting Fmoc-Orn-Trp(Boc)-Rink Amide MBHA resin was neutralized with 5% DIEA/DCM solution. After washing with DCM, 25 mL of DCM-TFE (4:1) and 1.946 g (6 mmol) of N-methyl-N,N'-bis-Boc-1-guanylpyrazole obtained in REFERENCE EXAMPLE 1 were added to the resin. DIEA was added to the mixture to adjust pH of the resulting solution to 10. The solution was shaken for 15 hours to give 6.195 g of Fmoc-Arg(Boc₂,Me)-Trp(Boc)-Rink Amide MBHA resin. Fmoc-Leu was introduced into the obtained resin as in the same manner described above. The resin was divided into halves and the Fmoc group was removed from the thus obtained Fmoc-Leu-Arg(Boc_{2,}Me)-Trp(Boc)-Rink Amide MBHA resin (1 mmol) to give H-Leu-Arg(Boc₂,Me)-Trp(Boc)-RinkAmide MBHA resin (1 mmol).

Separately, 1.745 g (6 mmol) of Fmoc-NHNH₂ HCl was suspended in 20 mL of DMF-THF (4:1). Under ice cooling, 973 mg (6 mmol) of CDI and 2.09 mL (12 mmol) of DIEA were added to the suspension, followed by stirring at room temperature for an hour. The resulting reaction solution was added to the H-Leu-Arg(Boc₂,Me)-Trp(Boc)-Rink Amide MBHA resin described above, and the mixture was stirred at room temperature for 15 hours. After completion of the reaction, the resin was washed and dried to give 3.314 g of the Fmoc-AzaGly-Leu-Arg(Boc₂,Me)-Trp(Boc)-Rink Amide MBHA resin.

### EXAMPLE 2

### Production of des(1)-Ms-[D-Tyr2,Hyp3,Thr5,AzaGly7,Arg(Me)9,Trp10]MS10 (Compound No. 963)

### (Synthesis A):

Using 613 mg (0.2 mmol) of the Fmoc-AzaGly-Leu-Arg(Boc₂,Me)-Trp(Boc)-Rink amide MBHA resin as the starting material, Phe, Thr(Bu^{t}), Asn(Trt), Hyp(Bu^{t}) and D-Tyr(Bu^{t}) were introduced in this order by the manual solid phase synthesis (Fmoc/DIPCDI/HOAt). The resin was then washed in MeOH and dried under reduced pressure to give the dry resin, H-D-Tyr(Bu^{t})-Hyp(Bu^{t})-Asn(Trt)-Thr(Bu^{t})-Phe-AzaGly-Leu-Arg(Boc₂,Me)-Trp( Boc)-Rink amide MBHA resin (752 mg, 0.266 mmol/g). After 75.2 mg (0.02 mmol) of the resin obtained was swollen in DMF, the resin was treated in DMF with methanesulfonyl chloride (3.1 µL, 0.04 mmol) and DIEA (7.0 µL, 0.04 mmol) for 30 minutes, then with methanesulfonyl chloride (6.2 µL, 0.08 mmol) and DIEA (14.0 µL, 0.08 mmol) for 60 minutes and further with methanesulfonyl chloride (3.1 µL, 0.04 mmol) and DIEA (7.0 µL, 0.04 mmol) for 90 minutes in DMF. After it was confirmed that a Kaiser test was negative, the resin was washed in MeOH and dried under reduced pressure. To the whole amount of the resin obtained, 1.0 mL of TFA/PhSMe/m-cresol/H₂O/TIS/EDT (80/5/5/5/2.5/2.5) was added. The mixture was stirred for 90 minutes. Diethyl ether was added to the reaction solution, the resulting precipitate was centrifuged and the supernatant was removed. This procedure was repeated twice followed by washing. The residue was extracted with an aqueous acetic acid solution. The extract was filtered to remove the resin. Then, linear density gradient elution (60 minutes) was performed at a flow rate of 15 ml/min using eluant A: 0.1% TFA-water and eluant B: 0.1% TFA-containing acetonitrile with A/B: 74.5/25.5 to 64.5/35.5, on preparative HPLC using YMC Pack R&D-ODS-5-B S-5, 120A column (30 x 250 mm). The fractions containing the product were collected and lyophilized to give 3.0 mg of white powders.
Mass spectrum: (M+H)⁺ 1261.5 (Calcd. 1261.6)
Elution time on HPLC: 18.1 mins.
Elution conditions:
Column: SHISEIDO CAPCELL PAK C18 MGII (4.6 x 100 mm)
Linear density gradient elution using 0.1 % TFA-water as eluant A and 0.1 % TFA-containing acetonitrile as eluant B, with A/B = 95/5 to 45/55 (25 mins.)
Flow rate: 1.0 ml/min.

### EXAMPLE 3

### Production of

### des(1)-benzylureido-[D-Tyr2,Hyp3,Thr5,AzaGly7,Arg(Me)9,Trp 10]MS 10 (Compound No. 966)

### (Synthesis B):

The dried resin obtained in EXAMPLE 2, i.e., H-D-Tyr(Bu^{t})-Hyp(Bu^{t})-Asn(Trt)-Thr(Bu^{t})-Phe-AzaGly-Leu-Arg(Boc₂,Me)-Trp( Boc)-Rink amide MBHA resin (75.2 mg, 0.02 mmol) was swollen in DMF, which was then treated in THF with CDI (25.9 mg, 0.16 mmol) and DIEA (27.9 µL, 0.16 mmol) for 3 hours. The resin was washed in DMF and treated with benzylamine (17.5 µL, 0.16 mmol) in DMF for 6 hours. The resin was then washed successively with DMF and MeOH and dried under reduced pressure. To the whole amount of the resin, 1.0 mL of TFA/PhSMe/m-cresol/H₂O/TIS/EDT (80/5/5/5/2.5/2.5) was added and the mixture was stirred for 90 minutes. Diethyl ether was added to the reaction solution, the resulting precipitate was centrifuged and the supernatant was removed. This procedure was repeated twice followed by washing. The residue was extracted with an aqueous acetic acid solution. After the extract was filtered to remove the resin, linear density gradient elution (60 minutes) was performed at a flow rate of 15 ml/min using eluant A: 0.1% TFA-water and eluant B: 0.1% TFA-containing acetonitrile with A/B: 72.5/27.5 to 62.5/37.5, on preparative HPLC using YMC Pack R&D-ODS-5-B S-5, 120A column (30 x 250 mm). The fractions containing the product were collected and lyophilized to give 1.7 mg of white powders.
Mass spectrum: (M+H)⁺ 1316.5 (Calcd. 1316.7)
Elution time on HPLC: 20.4 mins.
Elution conditions:
Column: SHISEIDO CAPCELL PAK C18 MGII (4.6 x 100 mm)
Linear density gradient elution using 0.1% TFA-water as eluant A and 0.1 % TFA-containing acetonitrile as eluant B with A/B = 95/5 to 45/55 (25 mins.)
Flow rate: 1.0 ml/min.

### EXAMPLE 4

### Production of

### des(1)-ureido-[D-Tyr2,Hyp3,Thr5,AzaGly7,Arg(Me)9,Trp10]MS10 (Compound No. 967)

### (Synthesis C):

The dried resin obtained in EXAMPLE 2, i.e., H-D-Tyr(Bu^{t})-Hyp(Bu^{t})-Asn(Trt)-Thr(Bu^{t})-Phe-AzaGly-Leu-Arg(Boc₂,Me)-Trp( Boc)-Rink amide MBHA resin (75.2 mg, 0.02 mmol) was swollen in DMF, which was then treated with TMS-NCO (12.7 µL, 0.08 mmol) in DMF overnight. The resin was washed successively with DMF and MeOH and dried under reduced pressure. To the whole amount of the resin obtained, 1.0 mL of TFA/PhSMe/m-cresol/H₂O/TIS/EDT (80/5/5/5/2.5/2.5) was added and the mixture was stirred for 90 minutes. Diethyl ether was added to the reaction solution, the resulting precipitate was centrifuged and the supernatant was removed. This procedure was repeated twice followed by washing. The residue was extracted with an aqueous acetic acid solution. After the extract was filtered to remove the resin, linear density gradient elution (60 minutes) was performed at a flow rate of 15 ml/min using eluant A: 0.1 % TFA-water and eluant B: 0.1 % TFA-containing acetonitrile with A/B: 74.5/25.5 to 64.5/35.5, on preparative HPLC using YMC Pack R&D-ODS-5-B S-5, 120A column (30 x 250 mm). The fractions containing the product were collected and lyophilized to give 1.7 mg of white powders.
Mass spectrum: (M+H)⁺ 1226.6 (Calcd. 1226.6)
Elution time on HPLC: 17.4 mins.
Elution conditions:
Column: SHISEIDO CAPCELL PAK C18 MGII (4.6 x 100 mm)
Linear density gradient elution using 0.1 % TFA-water as eluant A and 0.1 % TFA-containing acetonitrile as eluant B, with A/B = 95/5 to 45/55 (25 mins.)
Flow rate: 1.0 ml/min.

### EXAMPLE 5

### (Synthesis D):

### Production of

### des(1)-Ac-[D-Tyr2,Hyp3,Thr5,Cha6,AzaGly7,Arg(Me)9,Trp10]MS 10 (Compound No. 969)

Using 53 mg (0.02 mmol) of the Fmoc-AzaGly-Leu-Arg(Boc₂,Me)-Trp(Boc)-Rink amide MBHA resin as the starting material, Cha, Thr(Bu^{t}), Asn(Trt), Hyp(Bu^{t}) and D-Tyr(Bu^{t}) were introduced in this order by the manual solid phase synthesis (Fmoc/DIPCDI/HOAt) to give the H-D-Tyr(Bu^{t})-Hyp(Bu^{t})-Asn(Trt)-Thr(Bu^{t})-Cha-AzaGly-Leu-Arg(Boc₂,Me)-Trp( Boc)-Rink amide MBHA resin. Subsequently, the resin was treated in DMF with 9.4 µL (0.1 mmol) of Ac₂O and 17.4 µl (0.1 mmol) of DIEA for 20 minutes to acetylate the N terminus. Thus, 79.3 mg of the Ac-D-Tyr(Bu^{t})-Hyp(Bu^{t})-Asn(Trt)-Thr(Bu^{t})-Cha-AzaGly-Leu-Arg(Boc₂,Me)-Trp (Boc)-Rink amide MBHA resin. To the whole amount of the resin obtained, 0.6 mL of TFA/PhSMe/m-cresol/H₂O/TIS/EDT (80/5/5/5/2.5/2.5) was added. The mixture was stirred for 90 minutes. Diethyl ether was added to the reaction solution, the resulting precipitate was centrifuged and the supernatant was removed. This procedure was repeated twice followed by washing. The residue was extracted with an aqueous acetic acid solution. The extract was filtered to remove the resin. Then, linear density gradient elution (60 minutes) was performed at a flow rate of 8 ml/min using eluant A: 0.1 % TFA-water and eluant B: 0.1% TFA-containing acetonitrile with A/B: 72/28 to 62/38, on preparative HPLC using Daisopak SP-100-5-ODS-P column (250 x 20 mm I.D). The fractions containing the product were collected and lyophilized to give 7.4 mg of white powders.
Mass spectrum: (M+H)⁺ 1231.9 (Calcd. 1231.7)
Elution time on HPLC: 11.4 min.
Elution conditions:
Column YMC-AM301 (4.6 x 100 mm)
Linear density gradient elution using 0.1 % TFA-water as eluant A and 0.1 % TFA-containing acetonitrile as eluant B, with A/B = 80/20 to 30/70 (25 mins.)
Flow rate: 1.0 ml/min.

### EXAMPLE 6

### (Synthesis E):

### Production of

### des(1)-amidino-[D-Tyr2,Hyp3,Thr5,AzaGly7,Arg(Me)9,Trp10]MS 10 (Compound No. 971)

The dried resin obtained in EXAMPLE 2, namely, H-D-Tyr(Bu^{t})-Hyp(Bu^{t})-Asn(Trt)-Thr(Bu^{t})-Phe-AzaGly-Leu-Arg(Boc₂,Me)-Trp( Boc)-Rink amide MBHA resin (75.2 mg, 0.02 mmol) was swollen in DMF, which was then treated in DMF with N,N'-bis-Boc-1-guanylpyrazole (62.1 mg, 0.2 mmol) and DIEA (34.8 µL, 0.2 mmol) for 3 days. After washing subsequently with DMF and MeOH, the resin was dried under reduced pressure. To the whole amount of the resin obtained, 1.0 mL of TFA/PhSMe/m-cresol/H₂O/TIS/EDT (80/5/5/5/2.5/2.5) was added. The mixture was stirred for 180 minutes. Diethyl ether was added to the reaction solution, the resulting precipitate was centrifuged and the supernatant was removed. This procedure was repeated twice followed by washing. The residue was extracted with an aqueous acetic acid solution. The extract was filtered to remove the resin. Then, linear density gradient elution (60 minutes) was performed at a flow rate of 15 ml/min using eluant A: 0.1% TFA-water and eluant B: 0.1% TFA-containing acetonitrile withA/B: 75.5/24.5 to 64.5/35.5, on preparative HPLC using YMC Pack R&D-ODS-5-B S-5, 120A column (30 x 250 mm). The fractions containing the product were collected and lyophilized to give 1.9 mg of white powders.
Mass spectrum: (M+H)⁺ 1225.0 (Calcd. 1225.6)
Elution time on HPLC: 16.4 mins.
Elution conditions:
Column SHISEIDO CAPCELL PAK C18 MGII (4.6 x 100 mm)
Linear density gradient elution using 0.1 % TFA-water as eluant A and 0.1 % TFA-containing acetonitrile as eluant B, with A/B = 95/5 to 45/55 (25 mins.)
Flow rate: 1.0 ml/min.

### EXAMPLE 7

### (Synthesis F):

### Production of

### des(1)-Ac-[D-Tyr2,Hyp3,Alb4,Thr5,D-Cha6,Gly7ψ((E)CH=CH)Leu8,Arg(Me)9, Trp10]MS10 (Compound No. 975)

HCl.H-Ser-OMe, 3.5 g (22.6 mmol), was suspended in CHCl₃ (40 mL). Under ice cooling, DIPEA (8.7 mL, 49.7 mmol) and Pbf-Cl (5.86 g, 20.3 mmol) were sequentially added to the suspension. The mixture was stirred overnight while gradually elevating to room temperature. After saturated citric acid aqueous solution was added to stop the reaction, CHCl₃ was distilled off under reduced pressure and the whole was extracted with AcOEt. The organic layer was washed sequentially with saturated citric acid aqueous solution, saturated sodium bicarbonate aqueous solution and saturated sodium chloride aqueous solution, and dried over anhydrous MgSO₄. After concentration under reduced pressure, flash chromatography was performed using AcOEt : n-hexane = 1:1 to give Pbf-Ser-OMe (6.94 g, 82.7%). In an argon atmosphere under ice cooling, 923 µL (2.03 mmol) of a toluene solution of 2.2 M diethyl azodicarboxylate was added to a solution of 500 mg (1.35 mmol) of Pbf-Ser-OMe, 522 mg (2.03 mmol) of PPh₃ in THF (20 mL). The mixture was stirred overnight at room temperature and THF was then distilled off under reduced pressure. Flash chromatography was performed using AcOEt:n-hexane = 1:1 to give 481 mg of aziridine methyl ester (quantitative).

In an argon atmosphere, 429 µL (0.644 mmol) of a toluene solution of 1.5 M DIBAL-H was dropwise added to a solution of aziridine methyl ester (198 mg, 0.56 mmol) in toluene (10 mL) at -78°C. After stirring for 20 minutes at the same temperature, 0.1 N HCl aqueous solution was added to the mixture to stop the reaction. The whole was extracted with diethyl ether and the organic layer was washed sequentially with 0.1 N HCl aqueous solution and saturated sodium chloride aqueous solution. After drying over anhydrous MgSO₄, the reaction mixture was concentrated under reduced pressure to give the aldehyde as an oil. On the other hand, 146 µL (0.84 mmol) of DIEA and 197 µL (0.84 mmol) of (EtO)₂P(O)CH₂CO₂ But were sequentially added under ice cooling to a suspension of 35.6 mg (0.84 mmol) of anhydrous LiCl in acetonitrile (2 mL). The mixture was stirred at the same temperature for 20 minutes. Subsequently, a solution of the aldehyde obtained above in acetonitrile (4 mL) was dropwise added to the mixture, followed by stirring at 0°C for 3 hours. After the whole was extracted with AcOEt, the organic layer was washed sequentially with saturated citric acid aqueous solution, saturated sodium bicarbonate aqueous solution and saturated sodium chloride aqueous solution, and then dried over anhydrous MgSO₄. The mixture was concentrated under reduced pressure and the concentrate was recrystallized from diethyl ether/n-hexane to give 86.5 mg of the objective aziridine enoate. The mother liquor was concentrated under reduced pressure and subjected to flash chromatography using AcOEt : n-hexane = 1:19 to give additional 82.4 mg of the objective aziridine enoate (total yield: 71.5%).

In an argon atmosphere, 1.59 mL (3.17 mmol) of a 2.0 M THF solution of i-BuMgCl was dropwise added at -78°C to an anhydrous THF (6 mL) solution of 284 mg (3.17 mmol) of CuCN and 269 mg (6.34 mmol) of anhydrous LiCl. The temperature was elevated to 0°C and the mixture was stirred for 10 minutes. The mixture was again cooled to -78°C, and a solution of 402 µL (3.17 mmol) of BF₃· Et₂O and 334 mg (0.792 mmol) of aziridine enoate in anhydrous THF (6 mL) were dropwise added in this order. The mixture was stirred for 20 minutes at the same temperature. After quenching with saturated ammonium chloride aqueous solution : 28% ammonia aqueous solution (1:1), the mixture was stirred at room temperature until the reaction solution turned blue. The whole was extracted with diethyl ether. The organic layer was washed with water and dried over anhydrous MgSO₄. After concentration under reduced pressure, the residue was dissolved in 95% TFA aqueous solution (10 mL) and the solution was stirred at room temperature for 3 hours. TFA was removed by distillation under reduced pressure and the residue was azeotropically distilled twice with toluene. Recrystallization from diethyl ether/n-hexane gave 254 mg of Pbf-Glyψ[(E)-CH=CH]Leu-OH as white powders (75.7%).

After 180 mg (0.425 mmol) of Pbf-Glyψ[(E)-CH=CH]Leu-OH and 352 µL (3 mmol) ofthioanisole were dissolved in TFA (2.65 mL), the solution was stirred at room temperature for 24 hours. TFA was distilled off under reduced pressure and the resulting residue was dissolved in CH₃CN:H₂O (2:1, 9 mL). Under ice cooling, Et₃N was added until the solution became basic and 168 mg (0.446 mmol) of Fmoc-OSu was further added thereto. While elevating to room temperature, the mixture was stirred for 4 hours and the whole was extracted with AcOEt. The organic layer was washed sequentially with 0.1 N HCl aqueous solution and saturated sodium chloride aqueous solution, and dried over anhydrous MgSO₄. Following concentration under reduced pressure, flash chromatography using AcOEt : n-hexane = 3.5 : 6.5 was performed to give 145 mg (89%) of Fmoc-Glyψ[(E)-CH=CH]Leu-OH. After Fmoc-Arg(Boc₂,Mc)-Trp(Boc)-Rink Amide MBHA resin (0.347 mmol/g, 115 mg, 0.04 mmol) was swollen in DMF, the mixture was treated with 20% piperidine/DMF solution to cleave the Fmoc group. Subsequently, the resin was treated in DMF with 47.2 mg (0.12 mmol) of Fmoc-Glyψ[(E)-CH=CH]Leu-OH, 20.9 µL (0.12 mmol) of DIPEA, 240 µL (0.12 mmol) of a 0.5 M DMF solution of HOAt and 62.6 mg (0.12 mmol) of PyAOP at room temperature for 12 hours. After the resin was washed with DMF, D-Cha, Thr(Bu^{t}), Alb, Hyp(Bu^{t}) and D-Tyr(Bu^{t}) were introduced in this order by the manual solid phase synthesis (Fmoc/DIPCDI/HOAt) to give the H-D-Tyr(Bu^{t})-Hyp(Bu^{t})-Asn(Trt)-Thr(Bu^{t})-D-Cha-Glyψ[(E)CH=CH]Leu-Arg(Bo c₂,Me)-Trp(Boc)-Rink Amide MBHA resin. The resulting resin was swollen in DMF with Ac₂O (14.6 µL, 0.16 mmol) and DIEA (27.9 µL, 0.16 mmol) in DMF for 30 minutes, washed sequentially with DMF and MeOH, and dried under reduced pressure. To the whole amount of the resin obtained, 2 mL of TFA/PhSMe/m-cresol/H₂O/TIS/EDT (80/5/5/5/2.5/2.5) was added. The mixture was stirred for 180 minutes. Diethyl ether was added to the reaction solution, the resulting precipitate was centrifuged and the supernatant was removed. This procedure was repeated twice followed by washing. The residue was extracted with an aqueous acetic acid solution. The extract was filtered to remove the resin. Then, linear density gradient elution (60 minutes) was performed at a flow rate of 15 ml/min using eluant A: 0.1 % TFA-water and eluant B: 0.1 % TFA-containing acetonitrile with A/B: 71.5/28.5 to 61.5/38.5, on preparative HPLC using YMC Pack R&D-ODS-5-B S-5, 120A column (30 x 250 mm). The fractions containing the product were collected and lyophilized to give 4.5 mg of white powders.
Mass spectrum: (M+H)¹ 1228.5 (Calcd. 1228.7)
Elution time on HPLC: 20.5 mins.
Elution conditions:
Column: SHISEIDO CAPCELL PAK C18 MGII (4.6 x 100 mm)
Linear density gradient elution using 0.1 % TFA-water as eluant A and 0.1 % TFA-containing acetonitrile as eluant B, with A/B = 95/5 to 45/55 (25 mins.)
Flow rate: 1.0 ml/min.

### EXAMPLE 8

### (Synthesis G):

### Production of

### des(1)-Ac-[D-Tyr2,Hyp3,Alb4,Thr5,Cha6,Gly7ψ((E)CH=CH)D-Leu8,Arg(Me)9, Trp10]MS10 (Compound No. 976)

HCl.H-Ser-OMe, 4.0 g (25.7 mmol), was suspended in acetonitrile (40 mL). Under ice cooling, DIEA (8.95 mL, 51.4 mmol) and a solution of Pbf-Cl (90%, 7.41 g, 23.1 mmol) in acetonitrile (30 mL) were sequentially added to the suspension. The mixture was stirred overnight while gradually elevating to room temperature. Acetonitrile was distilled off under reduced pressure and the whole was extracted with AcOEt. The organic layer was washed sequentially with saturated citric acid aqueous solution, saturated sodium bicarbonate aqueous solution and saturated sodium chloride aqueous solution, and then dried over anhydrous MgSO₄. After concentration under reduced pressure, flash chromatography was performed using AcOEt : n-hexane = 1:1 to give Pbf-Ser-OMe (8.0 g, 83.8%). Under ice cooling, Ms-Cl (2.24 mL, 29.0 mmol) was dropwise added to a solution of Pbf-D-Ser-OMe (8.3 g, 22.3 mmol), DMAP (137 mg, 1.12 mmol) and triethylamine (4.66 mL, 33.5 mmol) in acetonitrile (40 mL). The mixture was stirred at the same temperature for 15 minutes. After saturated citric acid aqueous solution was added to stop the reaction, acetonitrile was distilled off under reduced pressure. The whole was then extracted with AcOEt. The organic layer was washed sequentially with saturated citric acid aqueous solution, saturated sodium bicarbonate aqueous solution and saturated sodium chloride aqueous solution, and dried over anhydrous MgSO₄. After concentration under reduced pressure, the residue was dissolved in DMF (50 mL) and Cs₂CO₃ (14.5 g, 44.6 mmol) was added to the solution. The mixture was stirred at room temperature for 2 hours. After the whole was extracted with AcOEt, the organic layer was washed sequentially with saturated sodium chloride aqueous solution and dried over anhydrous MgSO₄. After concentration under reduced pressure, flash chromatography was performed using AcOEt : n-hexane = 1:4. Recrystallization of the resulting powders from AcOEt/n-hexane gave aziridine methyl ester (3.5 g, 44.4%).

In an argon atmosphere, a toluene solution (1.5 M, 9.22 mL, 13.8 mmol) of DIBAL-H was dropwise added to a toluene (90 mL) solution of aziridine methyl ester (3.26 g, 9.22 mmol) at -78°C. The mixture was stirred at the same temperature for 10 minutes. After saturated citric acid aqueous solution was added to stop the reaction, the whole was extracted with AcOEt. The organic layer was washed sequentially with saturated citric acid aqueous solution, saturated sodium bicarbonate aqueous solution and saturated sodium chloride aqueous solution, and dried over anhydrous MgSO₄. The mixture was concentrated under reduced pressure to give the aldehyde as an oil. On the other hand, DIEA (2.40 mL, 13.8 mmol) and (EtO)₂P(O)CH₂CO₂ Bu^{t} (3.24 mL, 13.8 mmol) were sequentially added to an acetonitrile (45 mL) suspension of anhydrous LiCl (585 mg, 13.8 mmol) under ice cooling. The mixture was stirred at the same temperature for 20 minutes, and an acetonitrile (60 mL) of the aldehyde obtained above was dropwise added thereto. While elevating to room temperature, the mixture was stirred overnight. By adding water to the mixture, the precipitate was taken out by filtration, washed with water and n-hexane and dried under reduced pressure to give aziridine enoate (2.2 g, 56.6%).

In an argon atmosphere, a THF solution (2.0 M, 4.36 mL, 8.72 mmol) of i-BuMgCl was dropwise added at -78°C to an anhydrous THF (25 mL) solution of CuI (1.66 g, 8.72 mmol) and anhydrous LiCl (738 mg, 17.4 mmol). The temperature was then elevated to 0°C and the mixture was stirred for 10 minutes. The mixture was again cooled to -78°C, and BF₃· Et₂O (1.10 mL, 8.72 mmol) and an anhydrous THF (30 mL) solution of aziridine enoate (2.1 g, 4.98 mmol) were dropwise added thereto sequentially. The mixture was then stirred at the same temperature for 15 minutes. After quenching with saturated ammonium chloride aqueous solution: 28% ammonia aqueous solution (1:1), the mixture was stirred at room temperature until the reaction solution turned blue. The whole was extracted with diethyl ether. The organic layer was washed with water and dried over anhydrous MgSO₄. After concentration under reduced pressure, the residue was dissolved in 95% TFA aqueous solution (50 mL). The solution was stirred at room temperature for 3 hours. TFA was removed by distillation under reduced pressure and the residue was azeotropically distilled twice with toluene. Crystallization from AcOEt/n-hexane gave white powders containing the product, Pbf-Gly-ψ[(E)CH=CH]-D-Leu-OH (weight 2 g, about 90% purity on NMR).

After Z-Om(Boc)-Trp-NH₂(21 g, 38.1 mmol) was dissolved in MeOH (100 mL), AcOEt (300 mL) was further added to the solution for dilution. Under cooling on an ice bath, 4 N HCl/AcOEt (110 mL) was dropwise added to the mixture. While gradually elevating to room temperature, the mixture was stirred overnight. The solvent was distilled off under reduced pressure. The residue was azeotropically distilled twice with toluene and diethyl ether was added thereto. The precipitate formed was taken out by filtration. After washing with AcOEt, the precipitate was dried under reduced pressure to give 21 g (quantitative) of Z-Orn(HCl)-Trp-NH₂ as brown powders.

Under cooling on an ice bath, DIEA (7.0 mL, 40.2 mmol) and N-methyl-N,N'-bis-Boc-1-guanylpyrazole (7.17 g, 22.1 mmol) were added sequentially to a solution of Z-Orn(HCl)-Trp-NH₂ (9.8 g, 20.1 mmol) in acetonitrile/H₂O (72 mL/8 mL), and the mixture was stirred overnight. After the solvent was distilled off under reduced pressure, the whole was extracted with AcOEt. The organic layer was washed sequentially with saturated citric acid aqueous solution, saturated sodium bicarbonate aqueous solution and saturated sodium chloride aqueous solution, and dried over anhydrous MgSO₄. After concentration under reduced pressure, silica gel chromatography was performed using as eluants AcOEt : n-hexane = 3:1, then AcOEt = 100% and finally AcOEt : MeOH = 30:1 to give Z-Arg(Boc₂,Me)-Trp-NH₂ (8.8 g, 61.8%).

To a solution of Z-Arg(Boc₂,Me)-Trp-NH₂ (1.23 g, 1.74 mmol) in MeOH (10 mL) 100 mg of 10% Pd/C was added, and the mixture was stirred at room temperature for 4 hours in a hydrogen atmosphere. The Pd catalyst was removed by filtration through celite and the filtrate was concentrated under reduced pressure. The residue obtained was combined with Pbf-Gly-ψ[(E)CH=CH]-D-Leu-OH (90%, 700 mg, 1.65 mmol), and the mixture was dissolved in DMF (10 mL). Under cooling on an ice bath, DMT-MM (85% in H₂O, 536 mg, 1.65 mmol) was added to the mixture, which was then stirred for 24 hours while gradually elevating to room temperature. After the whole was extracted with AcOEt, the organic layer was washed with H₂O and dried over anhydrous MgSO₄. After drying under reduced pressure, Pbf-Gly-ψ[(E)CH=CH]-D-Leu- Arg(Boc₂,Me)-Trp-NH₂ (1.76 g) obtained was used in the following step without further purification.

Under cooling on an ice bath, Pbf-Gly-ψ[(E)CH=CH]-D-Leu-Arg(BOC₂,Me)-Trp-NH₂ (1.7 g, 1.74 mmol) was added to a solution ofthioanisole (1.54 mL, 13.1 mmol), m-cresol (910 µL, 8.7 mmol) and methanesulfonic acid (565 µL, 8.70 mmol) in TFA (70 mL). After elevating to room temperature, the mixture was stirred for 24 hours. Subsequently, the reaction solution was dropwise added to ice-chilled diethyl ether while vigorously stirring. The mixture was then allowed to stand for 2 hours. The precipitate formed was taken out by filtration, washed with diethyl ether and dissolved in McOH. The solution was concentrated under reduced pressure. The residue was dissolved in 50% AcOH aqueous solution (6.5 mL). The solution was passed through a membrane filter with a pore diameter of 0.45 µm and then purified separately twice on preparative HPLC using SHISEIDO CAPCELL PAK MGII column (50 x 250 mm). After applying eluant A: 0.1 % TFA-water and eluant B: 0.1% TFA-containing acetonitrile at a flow rate of 45 mL/min and maintaining A/B: 87/13 for 20 minutes, linear density gradient elution (60 minutes) to A/B: 87/13 to 64/36 was performed. The fractions containing the objective product only were collected and concentrated to give 386 mg of deprotected H-Gly-ψ[(E)CH=CH]-D-Leu- Arg(Me)-Trp-NH₂. Under ice cooling, NMM (135 µL, 1.23 mmol) and DMT-MM (85% in H₂O, 200 mg, 0.614 mmol) were sequentially added to a solution of the deprotected product (386 mg, 0.511 mmol) and Ac-D-Tyr-Hyp-Alb-Thr-Cha-OH (442 mg, 0.614 mmol) in DMF (8 mL). While gradually elevating to room temperature, the mixture was stirred for 16 hours. DMF was distilled off under reduced pressure and the residue was dissolved in 50% acetic acid aqueous solution (25 mL). The solution was passed through a membrane filter with a pore diameter of 0.45 µm and then purified separately three times on preparative HPLC using SHISEIDO CAPCELL PAK MGII column (50 x 250 mm). After applying eluant A: 0.1 % TFA-water and eluant B: 0.1% TFA-containing acetonitrile at a flow rate of 45 mL/min and maintaining A/B: 90/10 for 10 minutes, linear density gradient elution (10 minutes) to A/B: 90/10 to 75/25 and linear density gradient elution (60 minutes) to A/B: 75/25 to 55/45 were performed. The fractions containing the objective product only were collected, concentrated and lyophilized to give 216 mg of while powders.
Mass spectrum: (M+H)⁺ 1228.5 (Calcd. 1228.7)
Elution time on HPLC: 19.7 mins.
Elution conditions:
Column: SHISEIDO CAPCELL PAK C18 MGII (4.6 x 100 mm)
Linear density gradient elution using 0.1% TFA-water as eluant A and 0.1 % TFA-containing acetonitrile as eluant B, with A/B = 95/5 to 45/55 (25 mins.)
Flow rate: 1.0 ml/min.

### EXAMPLE 9

### (Synthesis H):

### Production of

### des(1)-Ac-[D-Tyr2,Hyp3,Alb4,Thr5,Cha6,Gly7ψ((E)CMe=CH)Leu8,Arg(Me)9,T rp10]MS10 (Compound No. 986)

Under ice cooling, a solution of isobutyl malonate (9.30 mL, 50 mmol) in diethyl ether (10 mL) was dropwise added to a suspension of LiAlH₄ (5.69 g, 150 mmol) in diethyl ether (225 mL). The mixture was stirred for 5 hours while elevating to room temperature. H₂O (6 mL), 2M NaOH aqueous solution (6 mL) and H₂O (18 mL) were dropwise added sequentially to the reaction solution at -78°C carefully to stop the reaction. The resulting precipitate was distilled off by filtration through a glass filter. The filtrate was washed with saturated sodium chloride aqueous solution, dried over anhydrous MgSO₄ and concentrated under reduced pressure to give the diol (5.54 g, 83.8%) as an oil.

In an nitrogen atmosphere, a n-hexane solution (1.59 M, 27.7 mL, 44.0 mmol) of n-BuLi was dropwise added to an anhydrous THF solution (120 mL) of the diol (5.54 g, 41.9 mmol) at -78°C. While elevating to room temperature, the mixture was stirred for an hour, a solution of TBDMS-Cl (6.67 g, 44.0 mmol) in anhydrous THF (30 mL) dropwise added to the mixture at -78°C. After stirring at room temperature overnight, saturated ammonium chloride aqueous solution was added to the mixture under ice cooling to stop the reaction. The whole was extracted with AcOEt. The organic layer was then washed with saturated sodium chloride aqueous solution, dried over anhydrous MgSO₄ and concentrated under reduced pressure to give TBDMS alcohol (10.5 g, quantitative) as an oil.

Pyridine-SO₃ complex (3.23 g, 20.3 mmol) was added to a solution of the TBDMS alcohol (2 g, 8.11 mmol) and DIEA (5.64 mL, 32.4 mmol) in DMSO (45 mL). After the mixture was stirred at room temperature for 3 hours, saturated sodium bicarbonate aqueous solution was added to the mixture under ice cooling to stop the reaction. The whole was extracted with AcOEt. The organic layer was washed sequentially with saturated sodium bicarbonate aqueous solution and sodium chloride aqueous solution, dried over anhydrous MgSO₄. After concentration under reduced pressure, the resulting residue was purified by flash chromatography using AcOEt : n-hexane = 1:100 to give the TBDMS aldehyde as an oil (1.11 g,56.0%).

The TBDMS aldehyde (365 mg, 1.49 mmol) and Ph₃P=C(Me)CO₂Et (1.08 g, 2.98 mmol) were dissolved in toluene (10 mL), and the mixture was reacted under reflux for 5 hours. The reaction solution was purified by flash chromatography using AcOEt : n-hcxanc = 1:50 to give the (E)-enoate (444 mg, 90.7%) as an oil.

In a nitrogen atmosphere, BF₃.Et₂O (189 µL, 1.49 mmol) was dropwise added at -78°C to a solution of the (E)-enoate (408 mg, 1.24 mmol) in toluene (15 mL). After stirring at the same temperature for 15 minutes, a toluene solution (2.48 mL, 3.72 mmol) of DIBAL-H was dropwise added to the mixture at -78°C. The mixture was stirred at -78°C for 45 minutes and then at room temperature for further 45 minutes. Saturated citric acid aqueous solution was added to the mixture at -78°C to stop the reaction. The whole was extracted with AcOEt. The organic layer was then washed sequentially with saturated citric acid aqueous solution, saturated sodium bicarbonate aqueous solution and saturated sodium chloride aqueous solution and dried over anhydrous MgSO₄. After concentration under reduced pressure, the residue was purified by flash chromatography using AcOEt : n-hexane = 1:20 to give the alcohol (223 mg, 62.8%) as an oil.

Under ice cooling, a toluene solution (2.2 M, 623 µL, 1.38 mmol) of DEAD was dropwise added to an anhydrous THF solution of the alcohol (198 mg, 0.691 mmol), N-Boc-o-nitrobenzenesulfonamide (417 mg, 1.38 mmol) and PPh₃ (362 mg, 1.38 mmol). While elevating to room temperature, the mixture was stirred overnight. The reaction solution was purified by flash chromatography using AcOEt : n-hexane = 1:10 to give the sulfonamide (350 mg, 88.7%) as an oil.

Under ice cooling, Jones reagent (200 ml) was dropwise added to a solution of the sulfonamide (98 mg, 0.172 mmol) in acetone (3 mL). The mixture was stirred at the same temperature for 2 hours, and 2-propanol (3 mL) was added thereto. The mixture was stirred for 30 minutes. The whole was extracted with AcOEt. The organic layer was then washed with saturated sodium chloride aqueous solution and dried over anhydrous MgSO₄. After concentration under reduced pressure, the resulting carboxylic acid (82.3 mg, quantitative, oil) was used in the following step as it was. To a solution of Z-Arg(Boc₂,Me)-Trp-NH₂ (122 mg, 0.172 mmol) in MeOH (2 mL) 20 mg of 10% Pd/C was added, and the mixture was stirred at room temperature for 3 hours in a hydrogen atmosphere. The Pd catalyst was removed by filtration through celite and the filtrate was concentrated under reduced pressure. The residue obtained was combined with the carboxylic acid (80 mg, 0.170 mmol) and the mixture was dissolved in DMF (3 mL). Under cooling on an ice bath, DMT-MM (85% in H₂O, 53.6 mg, 0.165 mmol) was added to the mixture, which was then stirred for 3 days while gradually elevating to room temperature. The whole was extracted with AcOEt, and the organic layer was washed with H₂O and dried over anhydrous MgSO₄. After concentration under reduced pressure, the resulting tetrapeptide derivative (191 mg, quantitative, oil) was used in the following step as it was.

Under ice cooling, LiOH.H₂O (71.3 mg, 1.70 mmol) and HSCH₂CO₂H (59.0 µL, 0.85 mmol) were added sequentially to a solution of the tetrapeptide derivative (191 mg, ca.0.170 mmol) in DMF (3 mL). After stirring at room temperature for 2 hours, the whole was extracted with AcOEt. The organic layer was then washed sequentially with saturated sodium bicarbonate aqueous solution and sodium chloride aqueous solution, and dried over anhydrous MgSO₄. After concentration under reduced pressure, the resulting Boc-Gly-ψ[(E)CMe=CH]-L/D-Leu-Arg(Boc₂,Me)-Trp-NH₂ (137 mg, 95.7%, oil) was used in the following step as it was.

Boc-Gly-ψ[(E)CMe=CH]-L/D-Leu-Arg(Boc₂,Me)-Trp-NH₂ (137 mg, 0.163 mmol) was dissolved in 3 mL of TFA/PhSMe/m-cresol/H₂O/T1S/EDT (80/5/5/5/2.5/2.5), and the solution was stirred at room temperature for 60 minutes. Diethyl ether was added to the reaction solution, the resulting precipitate was centrifuged and the supernatant was removed. This procedure was repeated twice followed by washing. Thus, the deprotected product (115 mg, 0.149 mmol) was obtained. NMM (39.4 µL, 0.358 mmol) and DMT-MM (85% in H₂O, 58.2 mg, 0.179 mmol) were sequentially added to a solution of the whole amount of the deprotected product and Ac-D-Tyr-Hyp-Alb-Thr-Cha-OH (129 mg, 0.179 mmol) in DMF (3 mL). While gradually elevating to room temperature, the mixture was stirred overnight. Diethyl ether was added to the reaction solution, the resulting precipitate was centrifuged and the supernatant was removed. This procedure was repeated twice followed by washing. The resulting precipitate was extracted with 50% acetic acid aqueous solution (3 mL). After solid substances were removed by filtration, linear density gradient elution (60 minutes) was performed at a flow rate of 15 ml/min using eluant A: 0.1% TFA-water and eluant B: 0.1% TFA-containing acetonitrile with A/B: 71.5/28.5 to 61.5/38.5 on preparative HPLC using YMC Pack R&D-ODS-5-B S-5, 120A column (30 x 250 mm). The fractions containing the product were collected and lyophilized to give 20.0 mg of white powders.
Mass spectrum: (M+H)⁺ 1242.3 (Calcd. 1242.7)
Elution time on HPLC: 20.7mins.
Elution conditions:
Column: SHISEIDO CAPCELL PAK C18 MGII (4.6 x 100 mm)
Linear density gradient elution using 0.1% TFA-water as eluant A and 0.1 % TFA-containing acetonitrile as eluant B, with A/B = 95/5 to 45/55 (25 mins.)
Flow rate: 1.0 ml/min.

### EXAMPLE 10

### (Synthesis I):

### Production of

### des(1)-Ac-[D-Tyr2,Hyp3,Thr5,Phe6ψ(CSNH)Gly7,Arg(Me)9,Trp10]MS10 (Compound No. 1002)

Fmoc-Arg(Boc₂,Me)-Trp(Boc)-Rink amide-MBHA resin (0.347 mmol/g, 86.5 mg, 0.03 mmol) was swollen in DMF, and then treated with 20% DMF solution of piperidine to remove the Fmoc group. Fmoc-Leu-OH (42.4 mg, 0.12 mmol), HOAt (0.5 M in DMF, 240 µL, 0.12 mmol) and DIPCDI (19.1 µL, 0.12 mmol) were added to the resin, and the mixture was shaken at room temperature for 2 hours. The resin was washed with DMF, and then treated with 20% DMF solution of piperidine to remove the Fmoc group. Fmoc-Phe-ψ(CSNH)-Gly-OH (69.1 mg, 0.15 mmol), HOAt (0.5 M in DMF, 300 µL, 0.15 mmol), DIPEA (26.1 µL, 0.15 mmol) and PyAOP (78.2 mg, 0.15 mmol) were sequentially added to the resin, followed by shaken at room temperature overnight. After washing with DMF, a Kaiser test was positive. Therefore, the mixture was treated with Fmoc-Phe-ψ(CSNH)-Gly-OH (14.0 mg, 0.03 mmol), HOAt (0.5 M in DMF, 60 µL, 0.03 mmol), DIPEA (5.22 µL, 0.03 mmol) and PyAOP (15.6 mg, 0.03 mmol) in DMF at room temperature for 4 hours. Next, the resin was treated with decanoic anhydride (44.2 µL, 0.12 mmol) and DIPEA (20.9 µL, 0.12 mmol) in DMF at room temperature for 30 minutes for capping of the unreacted amino groups. The resin was washed with DMF and then treated with 20% DMF solution of piperidine to remove the Fmoc group. Ac-D-Tyr-Hyp-Asn-Thr-OH (82.7 mg, 0.15 mmol), HOAt (0.5 M in DMF, 300 µL, 0.15 mmol), DIPEA (26.1 µL, 0.15 mmol) and PyAOP (78.2 mg, 0.15 mmol) were sequentially added, and the mixture was shaken at room temperature overnight. The resin was washed sequentially with DMF, MeOH and diethyl ether, followed by drying in vacuum. To the dried resin (103 mg), 1 mL of TFA:m-cresol:thioanisole:H₂O:EDT:TIS (80:5:5:5:2.5:2.5) was added, and the mixture was stirred at room temperature for 2 hours. Diethyl ether was added to the reaction solution, the resulting precipitate was centrifuged and the supernatant was removed. This procedure was repeated twice followed by washing. The residue was extracted with an aqueous acetic acid solution. The extract was filtered to remove the resin. Then, linear density gradient elution (60 minutes) was performed at a flow rate of 15 ml/min using eluant A: 0.1 % TFA-water and eluant B: 0.1 % TFA-containing acetonitrile with A/B: 70/30 to 60/40, on preparative HPLC using YMC Pack R&D-ODS-5-B S-5, 120A column (30 x 250 mm). The fractions containing the product were collected and lyophilized to give 3.7 mg of the product as white powders.
Mass spectrum: (M+H)⁺ 1240.6 (Calcd. 1240.6)
Elution time on HPLC: 12.0 mins.
Elution conditions:
Column: SHISEIDO CAPCELL PAK C18 MGII (4.6 x 100 mm)
Linear density gradient elution using 0.1 % TFA-water as eluant A and 0.1 % TFA-containing acetonitrile as eluant B, with A/B = 80/20 to 30/70 (25 mins.)
Flow rate: 1.0 mL/min

### EXAMPLE 11

### (Synthesis J):

### Production of

### des(1)-Ac-[D-Tyr2,Hyp3,Thr5,Phe6ψ(NHCO)Gly7,Arg(Me)9,Trp10]MS 10 (Compound No. 1003)

Under ice cooling, Z-Phe-NH₂ (1.0 g, 3.35 mmol) and pyridine (1.08 mL, 13.4 mmol) were sequentially added to a solution of [bis(trifluoroacetoxy)iodo]benzene (2.16 g, 5.03 mmol) in CH₃CN/H₂O (20 mL/5 mL). While gradually elevating to room temperature, the mixture was stirred overnight. The whole was extracted with AcOEt, and the extract was subjected to liquid-liquid separation using 1 N HCl aqueous solution. The aqueous layer was recovered and concentrated under reduced pressure. The residue was suspended in DMF (15 mL). Under ice cooling, mono-*tert*-butyl malonate (744 µL, 5.03 mmol), HOBt (1.36 g, 10.1 mmol), DIEA (4.66 mL, 26.8 mmol) and PyBOP (2.62 g, 5.03 mmol) were added to the suspension. The mixture was stirred overnight while elevating to room temperature. The whole was extracted with AcOEt. The organic layer was washed sequentially with saturated citric acid aqueous solution, saturated sodium bicarbonate aqueous solution and saturated sodium chloride aqueous solution, and dried over anhydrous MgSO₄. After concentration under reduced pressure, the resulting residue was purified by silica gel chromatography using n-hexane : AcOEt = 3:1 as an eluant to give Z-Phe-ψ(NHCO)-Gly-OBu^{t} as white powders (124.3 mg, yield 9.0%).

To a solution of Z-Arg(Boc₂,Me)-Trp-NH₂ (300 mg, 0.424 mmol) in MeOH (3 mL) 10% Pd/C (20 mg) was added, and the mixture was stirred at room temperature for 4 hours in a hydrogen atmosphere. The Pd catalyst was filtered off and the filtrate was concentrated under reduced pressure to give H-Arg(Boc₂,Me)-Trp-NH₂. On the other hand, Z-Leu-OH DCHA salt (180 mg, 0.203 mmol) was extracted with AcOEt, and the organic layer was sequentially washed with 1 N HCl aqueous solution and H₂O. After drying over anhydrous MgSO₄, the organic layer was concentrated under reduced pressure to give Z-Leu-OH. H-Arg(Boc₂,Me)-Trp-NH₂ described above was combined with Z-Leu-OH. The resulting mixture was dissolved in DMF (5 mL). Under ice cooling, DMT-MM (85% in H₂O, 124 mg, 0.403 mmol) was added to the solution. The mixture was stirred overnight while gradually elevating to room temperature. The whole was extracted withAcOEt. The organic layer was washed sequentially with saturated citric acid aqueous solution, saturated sodium bicarbonate aqueous solution and saturated sodium chloride aqueous solution, and dried over anhydrous MgSO₄. After concentration under reduced pressure, the resulting residue was dissolved in a small quantity of AcOEt, and n-hexane was added to the solution. The precipitate formed was taken out by filtration, and Z-Leu-Arg(Boc₂,Me)-Trp-NH₂ obtained as white powders was used in the following step as it was (250 mg, yield 71.8%).

10% Pd/C (40 mg) was added to a solution of Z-Leu-Arg(Boc₂,Me)-Trp-NH₂ (117 mg, 0.142 mmol) in MeOH (2 mL). In a hydrogen atmosphere, the mixture was stirred at room temperature for 3 hours. The Pd catalyst was filtered off, and the filtrate was concentrated under reduced pressure to give H-Leu-Arg(Boc₂,Me)-Trp-NH₂. On the other hand, Z-Phe-ψ(NHCO)-Gly-OBu^{t} was dissolved in TFA (3 mL). The solution was stirred for 30 minutes under ice cooling. After TFA was distilled off under reduced pressure, the residue was azeotropically distilled twice with toluene to give Z-Phe-ψ(NHCO)-Gly-OH. H-Leu-Arg(Boc₂,Me)-Trp-NH₂ described above and Z-Phe-ψ(NHCO)-Gly-OH were combined, and the mixture was dissolved in DMF (4 mL). Under ice cooling, DMT-MM (85% in H₂O, 46.1 mg, 0.142 mmol) was added to the solution. While gradually elevating to room temperature, the mixture was stirred overnight. The whole was extracted withAcOEt, and the organic layer was then washed sequentially with saturated citric acid aqueous solution, saturated sodium bicarbonate aqueous solution and saturated sodium chloride aqueous solution, and dried over anhydrous MgSO₄. After concentration under reduced pressure, the resulting residue (Z-Phe-ψ(NHCO)-Gly-Leu-Arg(Boc₂,Me)-Trp-NH₂) was used in the following step as it was (97.1 mg, yield 66.6%).

To a solution of Z-Phe-ψ(NHCO)-Gly-Leu-Arg(Boc₂,Me)-Trp-NH₂ (97.1 mg, 0.0946 mmol) in MeOH (2 mL) 10% Pd/C (30 mg) was added, and the mixture was stirred at room temperature for 1.5 hours in a hydrogen atmosphere. After the Pd catalyst was filtered off, the filtrate was concentrated under reduced pressure to give H-Phe-ψ(NHCO)-Gly-Leu-Arg(Boc₂,Me)-Trp-NH₂. The resulting crude H-Phe-ψ(NHCO)-Gly-Leu-Arg(Boc₂,Me)-Trp-NH₂ and Ac-D-Tyr-Hyp-Asn-Thr-OH (46.9 mg, 0.0851 mmol) were combined. The mixture was dissolved in DMF (2 mL). Under ice cooling, DMT-MM (85% in H₂O, 24.6 mg, 0.0757 mmol) was added to the solution, and the solution was stirred overnight while gradually elevating to room temperature. The whole was extracted with AcOEt, and the organic layer was washed with H₂O. After drying over anhydrous MgSO₄, the organic layer was concentrated under reduced pressure. The residue obtained was dissolved in 3 mL of TFA:m-cresolahioanisole:H₂O:EDT:TIS (80:5:5:5:2.5:2.5), and the solution was stirred at room temperature for 3 hours. Diethyl ether was added to the reaction solution, the resulting precipitate was centrifuged and the supernatant was removed. This procedure was repeated twice followed by washing. The residue was extracted with an aqueous acetic acid solution. Then, linear density gradient elution (60 minutes) was performed at a flow rate of 15 ml/min using eluant A: 0.1 % TFA-water and eluant B: 0.1% TFA-containing acetonitrile with A/B: 71.5/28.5 to 61.5/38.5, on preparative HPLC using YMC Pack R&D-ODS-5-B S-5, 120A column (30 x 250 mm). The fractions containing the product were collected and lyophilized to give 6.6 mg of the product as white powders.
Mass spectrum: (M+H)⁺ 1225.0 (Calcd. 1224.6)
Elution time on HPLC: 9.85 mins.
Elution conditions:
Column: SHISEIDO CAPCELL PAK C18 MGII (4.6 x 100 mm)
Linear density gradient elution using 0.1% TFA-water as eluant A and 0.1 % TFA-containing acetonitrile as eluant B, with A/B = 80/20 to 30/70 (25 mins.)
Flow rate: 1.0 mL/min.

### EXAMPLE 12

### (Synthesis K):

### Production of

### des(1)-Ac-[D-Tyr2,Hyp3,Thr5,Cha6ψ(CSNH)Gly7,Arg(Me)9,Trp10]MS 10 (Compound No. 1004)

Under ice cooling, DIEA (1.04 mL, 5.96 mmol) and EDC.HCl (571 mg, 2.98 mmol) were sequentially added to a suspension of Fmoc-Cha-OH (1.11g, 2.83 mmol), H-Gly-OBu^{t} · HCl (500 mg, 2.98 mmol) and HOBt (403 mg, 2.98 mmol) in DMF (10 mL). The mixture was stirred overnight while gradually elevating to room temperature. The whole was extracted with AcOEt. The organic layer was washed sequentially with saturated citric acid aqueous solution, saturated sodium bicarbonate aqueous solution and saturated sodium chloride aqueous solution, and dried over anhydrous MgSO₄. After concentration under reduced pressure, the resulting residue was used in the following step as it was (1.14 g, yield 79.5%).

Lawesson's reagent (866 mg, 2.14 mmol) was added to a toluene (10 mL) solution of Fmoc-Cha-Gly-OBu^{t} (433 mg, 0.854 mmol). The mixture was stirred for 2 hours on an oil bath, which temperature was set at 90°C. After insoluble matters were removed by filtration, the filtrate was concentrated under reduced pressure. The resulting residue was extracted with AcOEt. The organic layer was washed sequentially with saturated sodium bicarbonate aqueous solution and saturated sodium chloride aqueous solution, and dried over anhydrous MgSO₄. After concentration under reduced pressure, the resulting residue was purified by silica gel chromatography using n-hexane : AcOEt (5:1) as an eluant to give Fmoc-Cha-ψ(CSNH)-Gly-OBu^{t} as white powders (238 mg, yield 53.3%).

Fmoc-Cha-ψ(CSNH)-Gly-OBu^{t} (117 mg, 0.223 mmol) was dissolved in 95% TFA aq. (3 mL), followed by stirring at room temperature for an hour. After the solvent was distilled off under reduced pressure, the residue was azeotropically distilled twice with toluene to give Fmoc-Cha-ψ(CSNH)-Gly-OH. On the other hand, Fmoc-Arg(Boc₂,Me)-Trp(Boc) -Rink amide-MBHA resin (0.347 mmol/g, 144 mg, 0.05 mmol) was swollen in DMF, which was then treated with 20% DMF solution of piperidine to remove the Fmoc group. Fmoc-Leu-OH (70.7 mg, 0.2 mmol), HOAt (0.5 M in DMF, 400 µL, 0.2 mmol) and DIPCDI (31.8 µL, 0.2 mmol) were added to the resin, followed by shaking at room temperature for 2 hours. The resin was washed with DMF and treated with 20% DMF solution of piperidine to remove the Fmoc group. Fmoc-Cha-ψ(CSNH)-Gly-OH (0.223 mmol) described above, HOAt (0.5 M in DMF, 446 ψL, 0.223 mmol) and DIPCDI (35.4 µL, 0.223 mmol) were sequentially added to the resin. The mixture was shaken at room temperature for 3 hours. The resin was washed with DMF and treated with decanoic anhydride (73.6 µL, 0.2 mmol) and DIPEA (34.8 µL, 0.2 mmol) in DMF at room temperature for 30 minutes for capping of the unreacted amino groups. The resin was washed with DMF and then treated with 20% DMF solution of piperidine to remove the Fmoc group. Ac-D-Tyr-Hyp-Asn-Thr-OH (82.7 mg, 0.15 mmol), HOAt (0.5 M in DMF, 300 µL, 0.15 mmol), DIPEA (26.1 µL, 0.15 mmol) and PyAOP (78.2 mg, 0.15 mmol) were sequentially added, followed by shaking at room temperature overnight. The resin was washed sequentially with DMF, MeOH and diethyl ether, and then dried in vacuum. To the dried resin (144 mg) 2.5 mL of TFA:m-cresol:thioanisole:H₂O:EDT:TIS (80:5:5:5:2.5:2.5) was added, and the mixture was stirred at room temperature for 3 hours. Diethyl ether was added to the reaction solution, the resulting precipitate was centrifuged and the supernatant was removed. This procedure was repeated twice followed by washing. The residue was extracted with an aqueous acetic acid solution. The extract was filtered to remove the resin. Then, linear density gradient elution (60 minutes) was performed at a flow rate of 15 ml/min using eluant A: 0.1 % TFA-water and eluant B: 0.1 % TFA-containing acetonitrile with A/B: 68/32 to 58/42, on preparative HPLC using YMC Pack R&D-ODS-5-B S-5, 120A column (30 x 250 mm). The fractions containing the product were collected and lyophilized to give 6.1 mg of the product as white powders.
Mass spectrum: (M+H)⁺ 1246.6 (Calcd. 1246.6)
Elution time on HPLC: 13.4 mins.
Elution conditions:
Column: SHISEIDO CAPCELL PAK C18 MGII (4.6 x 100 mm)
Linear density gradient elution using 0.1 % TFA-water as eluant A and 0.1% TFA-containing acetonitrile as eluant B, with eluants A/B = 80/20 to 30/70 (25 mins.)
Flow rate: 1.0 mL/min.

### EXAMPLE 13

### (Synthesis L):

### Production of

### des(1)-Ac-[D-Tyr2,Hyp3,Thr5,Cha6ψ(CSNH)Gly7,Ala(cPr)8,Arg(Me)9,Trp10]MS 10 (Compound No. 1005)

Fmoc-Cha-ψ(CSNH)-Gly-OBu^{t} (121 mg, 0.231 mmol) was dissolved in 95% TFA aq. (3 mL), and the mixture was stirred at room temperature for 2 hours. After the solvent was distilled off under reduced pressure, the residue was azeotropically distilled twice with toluene to give Fmoc-Cha-ψ(CSNH)-Gly-OH. On the other hand, Fmoc-Arg(Boc₂,Me)-Trp(Boc)-Rink amide-MBHA resin (0.347 mmol/g, 144 mg, 0.05 mmol) was swollen in DMF, which was then treated with 20% DMF solution of piperidine to remove the Fmoc group. Fmoc-Ala(cPr)-OH (70.3 mg, 0.2 mmol), HOAt (0.5 M in DMF, 400 µL, 0.2 mmol) and DIPCDI(31.8 µL, 0.2 mmol) were added to the resin, followed by shaking at room temperature for 6 hours. The resin was washed with DMF and treated with 20% DMF solution ofpiperidine to remove the Fmoc group. Fmoc-Cha-ψ(CSNH)-Gly-OH (0.231 mmol) described above, HOAt (0.5 M in DMF, 400 µL, 0.2 mmol), DIPEA (34.8 µL, 0.2 mmol) and PyAOP (104 mg, 0.2 mmol) were sequentially added to the resin. The mixture was shaken at room temperature overnight. The resin was washed with DMF and then treated with decanoic anhydride (73.6 µL, 0.2 mmol) and DIEA (34.8 µL, 0.2 mmol) in DMF at room temperature for 30 minutes for capping of the unreacted amino groups. The resin was washed with DMF and then treated with 20% DMF solution ofpiperidine to remove the Fmoc group. Ac-D-Tyr-Hyp-Asn-Thr-OH (110 mg, 0.2 mmol), HOAt (0.5 M in DMF, 400 µL, 0.2 mmol), DIEA (34.8 µL, 0.2 mmol) and PyAOP (104 mg, 0.2 mmol) were sequentially added, followed by shaking at room temperature overnight. The resin was washed sequentially with DMF, MeOH and diethyl ether, and then dried in vacuum. To the dried resin (136 mg), 2 mL of TFA:m-cresol:thioanisole:H₂O:EDT:TIS (80:5:5:5:2.5:2.5) was added and the mixture was stirred at room temperature for 4 hours. Diethyl ether was added to the reaction solution, the resulting precipitate was centrifuged and the supernatant was removed. This procedure was repeated twice followed by washing. The residue was extracted with an aqueous acetic acid solution. The extract was filtered to remove the resin. Then, linear density gradient elution (60 minutes) was performed at a flow rate of 15 ml/min using eluant A: 0.1% TFA-water and eluant B: 0.1% TFA-containing acetonitrile with A/B: 68/32 to 58/42, on preparative HPLC using YMC Pack R&D-ODS-5-B S-5, 120A column (30 x 250 mm). The fractions containing the product were collected and lyophilized to give 6.2 mg of the product as white powders.
Mass spectrum: (M+H)⁺ 1244.8 (Calcd. 1244.6)
Elution time on HPLC: 12.6 mins.
Elution conditions:
Column: SHISEIDO CAPCELL PAK C18 MGII (4.6 x 100 mm)
Linear density gradient elution using 0.1 % TFA-water as eluant A and 0.1 % TFA-containing acetonitrile as eluant B, with A/B = 80/20 to 30/70 (25 mins.)
Flow rate: 1.0 mL/min.

### EXAMPLE 14

### (Synthesis M):

### Production of

### des(1,7)-Ac-[D-Tyr2,Hyp3,Thr5,Phe6ψ(CH₂NHCOCONH)LeuB,Arg(Me)9,Trp10] MS10 (Compound No. 1006)

Under ice cooling, a toluene solution (2.2 M, 1.35 mL, 2.98 mmol) of DEAD was dropwise added to a solution of Boc-Phe-ol (500 mg, 1.99 mmol), phthalimide (438 mg, 2.98 mmol) and PPh₃ (782 mg, 2.98 mmol) in anhydrous THF (10 mL). After the mixture was stirred at room temperature for 3 hours, the solvent was distilled off under reduced pressure. The residue was purified by silica gel chromatography using n-hexane:AcOEt (5:1) as an eluent. Crystallization of the resulting white powders from n-hexane/diethyl ether/AcOEt gave the phthalimide as white powders (352 mg, yield 46.4%). The phthalimide (300 mg, 0.789 mmol) obtained was dissolved in EtOH, and NH₂NH₂.H₂O (57.2 µL, 1.18 mmol) was added to the solution. The mixture was then stirred for 5 hours under reflux. As the reaction proceeded poorly, NH₂NH₂.H₂O (57.2 µL, 1.18 mmol) was further added and the mixture was stirred for further 3 hours under reflux. After concentration under reduced pressure, the resulting residue was suspended in CH₃CN (20 mL). Under ice cooling, triethylamine (439 µL, 3.16 mmol) and ClCOCO₂Et (219 µL, 1.97 mmol) were dropwise added sequentially to the suspension. While gradually elevating to room temperature, the mixture was stirred overnight. Under ice cooling, saturated sodium bicarbonate aqueous solution was added to stop the reaction and the whole was extracted with AcOEt. The organic layer was washed sequentially with H₂O, saturated citric acid aqueous solution, saturated sodium bicarbonate aqueous solution and saturated sodium chloride aqueous solution, and dried over anhydrous MgSO₄. After concentration under reduced pressure, the resulting residue was purified by silica gel chromatography using n-hexane : AcOEt (1:1) as an eluant to give Boc-Phe-ψ(CH₂NH)-oxoGly-OEt as white powders (82.6 mg, yield 29.9%).

Boc-Phe-ψ(CH₂NH)-oxoGly-OEt (35.0 mg, 0.1 mmol) was dissolved in EtOH/THF (800 µL/200 µL), and 2 M NaOH aq. (150 µL, 0.3 mmol) was added to the solution. After stirring at room temperature for 30 minutes, the whole was extracted with AcOEt. The organic layer was washed sequentially with 0.5 N HCl aqueous solution and H₂O and then dried over anhydrous MgSO₄. The residue (Boc-Phe-ψ(CH₂NH)-oxoGly-OH) obtained by concentration under reduced pressure was used in the following step as it was (32.0 mg, yield 99.3%).

10% Pd/C (30 mg) was added to a solution of Z-Leu-Arg(Boc₂,Me)-Trp-NH₂ (82.1 mg, 0.1 mmol) in MeOH (1.5 mL), and the mixture was stirred at room temperature for 3 hours in a hydrogen atmosphere. After the Pd catalyst was filtered off, the filtrate was concentrated under reduced pressure to give H-Leu-Arg(Boc₂,Me)-Trp-NH₂. H-Leu-Arg(Boc₂,Me)-Trp-NH₂ thus obtained was combined with Boc-Phe-ψ(CH₂NH)-oxoGly-OH (32 mg, 0.0993 mmol). The mixture was dissolved in DMF (2.5 mL). Under ice cooling, DMT-MM (85% in H₂O, 29.2 mg, 0.09 mmol) was added to the solution, and the mixture was stirred overnight while gradually elevating to room temperature. The whole was extracted with AcOEt and the organic layer was washed with H₂O. After drying over anhydrous MgSO₄, the extract was concentrated under reduced pressure. The resulting residue (Boc-Phe-ψ(CH₂NH)-oxoGly-Leu-Arg(Boc₂,Me)-Trp-NH₂) was used in the following step as it was (93.3 mg, quantitative).

Under ice cooling, Boc-Phe-ψ(CH₂NH)-oxoGly-Leu-Arg(Boc₂,Me)-Trp-NH₂ (93.3 mg, ca. 0.094 mmol) was dissolved in 2 mL of TFA:m-cresolahioanisole:H₂O:EDT:TIS (80:5:5:5:2.5:2.5). The solution was stirred at the same temperature for 45 minutes. Diethyl ether was added to the reaction solution, the resulting precipitate was centrifuged and the supernatant was removed. This procedure was repeated twice followed by washing. Since analysis on HPLC was found to be insufficient for deprotection of the Boc group, the precipitate was re-dissolved in 2 mL of TFA:m-cresol:thioanisole:H₂O:EDT:TIS (80:5:5:5:2.5:2.5), and the solution was stirred at room temperature for 1.5 hours. Diethyl ether was added to the reaction solution, the resulting precipitate was centrifuged and the supernatant was removed. This procedure was repeated twice followed by washing. The resulting precipitated was dissolved in DMF (2 mL). Under ice cooling, Ac-D-Tyr-Hyp-Asn-Thr-OH (51.8 mg, 0.094 mmol), NMM (25.8 µL, 0.235 mmol) and DMT-MM (85% in H₂O, 29.0 mg, 0.0893 mmol) were sequentially added to the solution. While gradually elevating to room temperature, the mixture was stirred overnight. Diethyl ether was added to the reaction solution and the mixture was centrifuged to remove the supernatant. The precipitate obtained was extracted with an aqueous acetic acid solution. Linear density gradient elution (60 minutes) was performed at a flow rate of 15 ml/min using eluant A: 0.1% TFA-water and eluant B: 0.1% TFA-containing acetonitrile with A/B: 70/30 to 60/40, on preparative HPLC using YMC Pack R&D-ODS-5-B S-5, 120A column (30 x 250 mm). The fractions containing the product were collected and lyophilized to give 13.5 mg of the product as white powders.
Mass spectrum: (M+H)⁺ 1224.5 (Calcd. 1224.6)
Elution time on HPLC: 11.4 mins.
Elution conditions:
Column: SHISEIDO CAPCELL PAK C18 MGII (4.6 x 100 mm)
Linear density gradient elution using 0.1 % TFA-water as eluant A and 0.1% TFA-containing acetonitrile as eluant B, with eluants A/B = 80/20 to 30/70 (25 mins.)
Flow rate: 1.0 mL/min.

The compounds synthesized in EXAMPLES 1 to 14 and the compounds synthesized by the procedures similar to EXAMPLES 1 to 14 are shown in TABLE 1 below, in terms of their structures, physicochemical properties, etc.

The description "Synthesis" in the table indicates that:
the compounds described in EXAMPLES 1 to 14 were synthesized by Synthesis A through M described at the column "Synthesis" of the respective compounds or
the compounds described in EXAMPLES 1 to 14 can be synthesized by Synthesis A through M described at the column "Synthesis" of the respective compounds; and,
the compounds not described in EXAMPLES 1 to 14 were synthesized by a modification of the synthesis described in "Synthesis" for the respective compounds.

The description "HPLC Mode" in the table indicate that:
the compounds described in EXAMPLES 1 to 14 can be eluted under the conditions by a, b, c or d described at the column "HPLC Mode" for the respective compounds; and,
the compounds not described in EXAMPLES 1 to 14 were eluted under the conditions by a, b, c or d described at the column "HPLC Mode" for the respective compounds.

| Compound No. | Structure | M+H⁺ (obs) | M+H⁺ (cal.) | HPLC (min.) | HPLC Mode | Synthesis |
|---|---|---|---|---|---|---|
| 963 | des(1)-Ms-[D-Tyr2Hyp3.Thr5AzaGly7.Arg(Me)9, Trp10]MS10 | 1261.5 | 1261.6 | 18.1 | d | A |
| 964 | des(1)-4-Fluorobenzoyl-[D-tyr2.Hyp3,Thr5,AzaGly7,Arg(Me)9.Trp10]MS10 | 1305.7 | 1305.6 | 20.5 | d | D |
| 965 | des(1)-Decanoyl-[D-Tyr2,+typ3.Thr6.AzaGly7,Arg(Me)9.Trp10]MS10 | 1337.5 | 1337.7 | 25.0 | d | D |
| 966 | des(1)-Benzylureido-[D-Tyr2.Hyp3.Thr5.AzaGly%Arg(Me)9.Trp10]MS10 | 1316.5 | 1316.7 | 20.4 | d | B |
| 967 | des(1)-Ureido-[D-Tyr2.Hyp3,Thr5,AzaGly7,Arg(Me)9.Trp10]MS10 | 12266 | 1226.6 | 17.4 | d | C |
| 969 | des(1)-Ac-[D-Tyr2.Hyp3,Thr5,Cha5,AzaGly7,Arg(Me)9.Trp10]MS10 | 1231.9 | 1231.7 | 11.4 | a | D |
| 970 | des(1)-Ac-[D-Tyr2,Pro(4F)3,Thr5,Cha6.AzaGly%Arg(Me)9.Trp10]MS10 | 1233.8 | 1233.7 | 12.6 | a | D |
| 971 | des(1)-Amidino-[D-Tyr2,Hyp3.Thr5.AzaGly7,Arg(Me)9,Trp10] | 1225.0 | 1225.6 | 16.4 | d | E |
| 972 | des(1)-Dimethylaminoethylureido-[D-Tyr2,Hyp3.Thr5.Cha6.AzaGly7,Arg(Me)9. Trp10]MS10 | 1297.2 | 1297.7 | 16.1 | d | B |
| 973 | des(1)-iso-Butylureido-[D-Tyr2,Hyp3.Thr5.Cha6.AzaGly7,Arg(Me)9. Trp10]MS10 | 1282.3 | 12827 | 19.8 | d | B |
| 975 | des(1)-Ac-[D-Tyr2,Hyp3Alb4.Thr5.D-Cha6,Gly7 ψ ((E)CH=CH)Leu8.Arg(Me)9. Trp10]MS10 | 1228.5 | 1228.7 | 20.5 | d | F |
| 976 | des(1)-Ac-[D-Tyr2,Hyp3,Alb4,Thr5,Cha6,Gly7 ψ ((E)CH=CH)D-Leu8.Arg(Me)9,Trp10]MS10 | 1228.5 | 1228.7 | 19.7 | d | G |
| 877 | des(1-2)-[Hyp3.Thr5,AzaGly3.Arg(Me)9,Trp10]MS10 | 1020.5 | 1020.5 | 14.2 | b | D |
| 986 | des(1)-Ac-[D-Tyr2.Hyp3,Alb4.Thr5.Cha6,GlyT ψ (E)CMe=CH)Leu8,Arg(Me)9.Trp10]MS10 | 1242.3 | 1242.7 | 20.7 | d | H |
| 1002 | des(1) -Ac-[D-Tyr2,Hyp3.Thr5.Phe6 ψ (CSNH)Gly7.Arg(Me)9. Trp10]MS10 | 1240.8 | 1240.6 | 12.0 | c | I |
| 1003 | des(1)-Ac-[D-Tyr2.Hyp3. Thr5.Phe6 ψ (NHCO)Gly7 Arg(Me)9. Trp10]MS10 | 1225.0 | 1224.6 | 9.9 | c | J |
| 1004 | des(1)-Ac-[D-Tyr2,Hyp3,Thr5,Cha6ψ(SCNH)Gly%.Arg(Me)9,Trp10]MS10 | 1246.6 | 1246.6 | 13.4 | c | K |
| 1005 | des(1)-Ac-[D-Tyr2,Hyp3.Thr5.Cha6 ψ (CSNH)Gly7,Ala(cPr)a,Arg(Me)9.Trp10]MS10 | 1244.8 | 1244.6 | 12.6 | c | L |
| 1006 | des(1.7)-Ac-[D-Tyr2.Hyp3,Thr5,,Phe6 ψ (CH2NHCOCONH)Leu8,Arg(Me)9. Trp10]MS10] | 1224.5 | 1224.6 | 11.4 | c | M |
| | a:20-70% AcCN/25min. fkow1 ml/min, YMC ODS AM-301 (4.6 x 100mm) | | | | | |
| | b_{:}0-50% AcCN/25min, flow 1 ml/min Wakesil-II 5C18 HG (46 x 100mm) | | | | | |
| | c:20-70% AcCN/25min flow 1 ml/min SHISEIDO CAPCELL PAK C18 MGH (4.6 x 100mm) | | | | | |
| | d:5 - 55% AcCN/25min flow 1 ml/min SHISEIDO CAPCELL PAK C18 MGH (4.6 x 100mm) | | | | | |

### TEST EXAMPLE 1

### Measurement of Agonist Activity by Assaying Changes in Intracellular Calcium Ion Level

### 1) Preparation of Human Metastin and Rat Metastin Stable Expression Cell Lines

Human metastin and rat metastin stable expression cell lines were acquired by transfecting the expression plasmid for animal cells into CHO/dhfr⁻ cells using CellPhect Transfection Kit (manufactured by GE Healthcare). First, 240 µL of Buffer A (attached to CellPhect Transfection Kit) was added to 9.6 µg of plasmid DNA dissolved in 240 µL of distilled water, followed by stirring. After the mixture was settled for 10 minutes, 480 µL of Buffer B (attached to CellPhect Transfection Kit) was added to the mixture, which was vigorously stirred to form liposomes containing the DNA. Then, 4 x 10⁵ CHO/dhfr⁻ cells (obtained from ATCC) were inoculated on a 60 mm Petri dish. After culturing the cells in Ham's F-12 medium (manufactured by Nissui Seiyaku Co., Ltd.) supplemented with 10% fetal calf serum (manufactured by BIO WHITTAKER, Inc.) at 37°C for 2 days in 5% carbon dioxide gas, 480 mL of the liposomes were dropwise added to the cells on the Petri dish. After culturing the cells in a CO₂ incubator (5% CO₂, 37°C) for 6 hours, the cells were washed twice with serum-free Ham's F-12 medium and 3 mL of 15% glycerol was added to the cells on the Petri dish. The mixture was treated for 2 minutes. After washing twice with serum-free Ham's F-12 medium, the cells were again incubated in Ham's F-12 medium supplemented with 10% fetal calf serum in a CO₂ incubator (5% CO₂, 37°C) for 15 hours. The cells were dispersed by trypsin treatment to recover from the Petri dish. The recovered cells were inoculated on a 6-wcll plate in 1.25 x 10⁴ cells each/well. Incubation was initiated in a CO₂ incubator (5% CO₂, 37°C) in Dulbecco's modified Eagle medium (DMEM, manufactured by Nissui Seiyaku) containing 10% dialyzed fetal calf serum (manufactured by JRH BIOSCIENCES, Inc.). The plasmid-transfected CHO transformants grew in the medium but the non-transfected cells gradually died. Accordingly, the medium was exchanged on Days 1 and 2 after the initiation of incubation to remove the dead cells. Approximately 20 colonies of the CHO transformants that kept growing on Days 8 to 10 after the incubation were isolated. Cells showing high reactivity with the ligand peptide metastin (hereinafter merely referred to as h175 KB 19 and h175 KB29 strains) were selected from the cells in these colonies and used in the following experiment.

### 2) Cell Plating

Human metastin expression CHO cell line (h175 KB 19 strain is described in a separate section) and rat metastin expression CHO cell line (h175 KB29 strain is described in a separate section) were plated in a 96-well plate (type 3904, manufactured by Coming) at 3 x 10⁴ cells/well, followed by incubation in a CO₂ incubator (5% CO₂, 37°C) for 24 hours. For the medium, MEMα medium (nucleic acid-free, manufactured by Nikken Bio Medical Laboratory) supplemented with dialyzed 10% fetal calf serum (MultiSer manufactured by Thermo) was used.

### 3) Loading with Fluo-4 NW in Cells

As an assay buffer, 1 x Hanks' balanced salt solution (HBSS, manufactured by GIBCO) supplemented with 0.1% BSA, 20 mM HEPES (pH 7.4, manufactured by GIBCO) and 1 mM Probenecid (manufactured by Molecular Probes) was prepared. The medium in the wells of the cell-seeded plate was removed and 100 µL each of the assay buffer (kept warm at 37°C) was added to each well. After 10 mL of the assay buffer (the volume for assaying two plates) was charged in one Fluo-4NW dye mix (Component A, Fluo-4 NW calcium assay kit (starter pack), manufactured by Molecular Probes) bottle, the mixture was gently stirred to prepare the Fluo-4 NW loading solution. This loading solution was charged in each well by 50 µl each and reacted in a CO₂ incubator (5% CO₂, 37°C) for 30 minutes for loading with Fluo-4 NW into the cells. Subsequently, the cells were settled at room temperature (25°C) for 15 minutes and then used for assay.

### 4) Measurement of Agonist Activity

To monitor the agonist activity of a test compound, the test compound diluted in the assay buffer described above was dispensed into a 96-well plate (type 3363, manufactured by Coming) by 80 µl each/well to prepare the compound plate. Fluo-4 NW-loaded cell plate and compound plate were set on a Fluorometric Imaging Plate Reader (FLIPR, manufactured by Molecular Devices) and 50 µl each was dispensed in each well through an automated pipettor in the FLIPR. The agonist response when stimulated by the compound was monitored via CCD camera in the FLIPR in terms of changes in intracellular calcium ion levels (changes in fluorescence of Fluo-4 NW).

Human Metastin (45-54)* specific agonist activity refers to the value obtained by subtracting the fluorescence change in the control group without any additive from the fluorescence change induced by Metastin (45-54). The specific agonist activity of a test compound refers to the value obtained by subtracting the fluorescence change observed in the control group in the absence of any test compound from the fluorescence change observed when added with the test compound. The compound level showing the 50% agonist response activity (EC₅₀ value) was calculated from the dose-response curve. When the maximum response of human Metastin (45-54) specific agonist activity was made 100%, the EC₅₀ was calculated on a test compound showing 70% or more of the maximum activity as compared to the maximum response.

*Peptide used and synthesized from the 45th to 54th in the amino acid sequence of human metastin [human Metastin (45-54)] is the synthetic product from Peptide Institute, Inc.

The agonist activity of each test compound (expressed by the specific activity of EC₅₀ of a test compound based on EC₅₀ of Metastin (45-54)) is shown in TABLE 2. The data reveal that the compounds of the present invention have excellent agonist activities on the metastin receptors.

**TABLE 2**

| Compound No. | Specific Activity |
|---|---|
| 963 | 0.9 |
| 965 | 4.1 |
| 966 | 1.9 |
| 967 | 0.6 |
| 969 | 0.3 |
| 970 | 0.6 |
| 971 | 0.4 |
| 972 | 2.2 |
| 973 | 0.4 |
| 975 | 2.3 |
| 976 | 1.0 |
| 977 | 1.7 |
| 986 | 0.3 |

### TEST EXAMPLE 2

### Evaluation of Blood Testosterone Level Reducing Effect of Metastin Peptide Derivatives Using Mature Male Rats

In the metastin peptide derivatives listed in TABLE 1, the blood testosterone level reducing effects were evaluated for the compounds except for compound Nos. 964, 975, 976 and 977.

A metastin peptide derivative (hereinafter referred to as peptide) was dissolved in 50% DMSO aqueous solution (DMSO: Sigma-Aldrich, distilled water for injection: Otsuka Pharmaceutical) to prepare a peptide solution with the concentration of 0.1, 0.03 or 0.01 mM. This peptide solution was filled in five ALZET osmotic pumps (Model 2001, 0.2 mL in volume, release rate: 0.001 mL/hr, DURECT Corporation). The ALZET pump filled with the peptide solution was implanted subcutaneously in 5 CD (SD) IGS male rats of 9 weeks old after birth (Charles River Japan, Inc.) on the back under ethereal anesthesia by one pump/animal. For negative control, 50% DMSO aqueous solution was filled in 5 ALZET osmotic pumps, which were similarly implanted in 5 male CD (SD) IGS rats (Charles River Japan, Inc.), respectively. These rats implanted with the pumps were fed for 6 days under normal feeding conditions. After weighing, the animal was decapitated to collect blood. After 0.03 mL/mL blood of aprotinin (Trasylol, Bayer) solution containing 0.1 g/mL EDTA.2Na was added to blood, the plasma was separated and recovered by centrifugation at 1,800 x g for 30 minutes. From the plasma obtained, 0.05 mL was applied to radioimmunoassay (DPC Total Testosterone Kit, Diagnostic Products Corporation) to measure the plasma testosterone level in each rat. The peptides are listed in TABLE 3, when the number of rats showing the testosterone level below the measurement limit (0.04 ng/mL of plasma level) in radioimmunoassay was 3 or more in the 5 rats receiving the peptides.

**TABLE 3**

| **Compound No.** |
|---|
| 963 |
| 966 |
| 967 |
| 969 |
| 970 |
| 972 |
| 973 |
| 986 |
| 1002 |
| 1004 |
| 1005 |

### INDUSTRIAL APPLICABILITY

The present invention provides stable metastin derivatives having excellent biological activities (a cancer metastasis suppressing activity, a cancer growth suppressing activity, a gonadotropic hormone secretion stimulating activity, a sex hormone secretion stimulating activity, a gonadotropic hormone secretion suppressing activity, a sex hormone secretion suppressing activity, etc.).

## Claims

1. A metastin derivative represented by formula:
XX0-XX2-XX3-XX4-XX5-XX6-AzaGly-XX8-XX9-XX 10-NH₂
wherein:
XX0 represents hydrogen, acetyl, mesyl, 4-fluorobenzoyl, decanoyl, benzylureido, ureido, amidino, dimethylaminoethylureido or isobutylureido;
XX2 represents Tyr, D-Tyr, D-Ala, D-Leu, D-Phe, D-Lys, D-Trp or a chemical bond;
XX3 represents:
i) an amino acid selected from Ala, Arg, Asn, Asp, Cys, Gln, Glu, Gly, His, Ile, Leu, Lys, Met, Phe, Ser, Thr, Trp, Tyr and Val, wherein the α-amino group may optionally be methylated;
ii) a cyclic amino acid selected from Pro, Aze(2), Aze(3), Pic(2), Pic(3), Hyp, Thz, Abz(2), Abz(3), Pzc(2), Pro(4NH₂), Hyp(Bzl), cisHyp, Pro(4F) and lzc;
iii) an amino acid selected from D-Pya(4), DL-Ala(Pip), Om, Aib and Tyr(PO₃H₂); or,
iv) a chemical bond;
XX4 represents Asn, 2-amino-3-ureidopropionic acid, Nβ-formyl-β-diaminopropionic acid, Nβ-acetyl-β-diaminopropionic acid, Nω-pentylasparagine, Nω-cyclopropylasparagine, Nω-benzylasparagine, 2,4-diaminobutanoic acid, 2,3-diaminopropionic acid, His, Gln, Gly, Arg, Cit, Nva, D-Asn or a chemical bond;
XX5 represents Ser, Thr, Val, NMeSer, Gly, Ala, Hyp, D-Ala, D-Thr, D-Pro or a chemical bond;
XX6 represents Phe, Tyr, Trp, Tyr(Me), Thi, Nal(2), Cha, Pya(4), threo-Ser(3Phenyl), erythro-Ser(3Phenyl) or an optionally substituted phenylalanine;
AzaGly represents azaglycine;
XX8 represents Leu, Nva, Val or Ala(cPr);
XX9 represents an optionally substituted arginine, an optionally substituted lysine or an optionally substituted ornithine; and,
XX10 represents 2-naphthylalanine, 2-thienylalanine, tyrosine, an optionally substituted phenylalanine or an optionally substituted tryptophan; or a salt thereof.

2. The metastin derivative or a salt thereof according to claim 1, wherein:
XX0 represents hydrogen, acetyl, mesyl, 4-fluorobenzoyl, decanoyl, benzylureido, ureido, amidino, dimethylaminoethylureido or isobutylureido;
XX2 represents D-Tyr;
XX3 represents Glu, Hyp or Pro(4F);
XX4 represents Asn or 2-amino-3-ureidopropionic acid;
XX5 represents Thr;
XX6 represents Phe or Cha;
AzaGly represents azaglycine;
XX8 represents Leu or Ala(cPr);
XX9 represents an arginine optionally substituted with methyl; and,
XX10 represents tryptophan.

3. Ms-D-Tyr-Hyp-Asn-Thr-Phe-AzaGly-Leu-Arg(Me)-Trp-NH₂, 4-fluorobenzoyl-D-Tyr-Hyp-Asn-Thr-Phe-AzaGly-Leu-Arg(Me)-Trp-NH₂, decanoyl-D-Tyr-Hyp-Asn-Thr-Phe-AzaGly-Leu-Arg(Me)-Trp-NH₂, benzylureido-D-Tyr-Hyp-Asn-Thr-Phe-AzaGly-Leu-Arg(Me)-Trp-NH₂, ureido-D-Tyr-Hyp-Asn-Thr-Phe-AzaGly-Leu-Arg(Me)-Trp-NH₂, Ac-D-Tyr-Hyp-Asn-Thr-Cha-AzaGly-Leu-Arg(Me)-Trp-NH₂, Ac-D-Tyr-Pro(4F)-Asn-Thr-Cha-AzaGly-Leu-Arg(Me)-Trp-NH₂, amidino-D-Tyr-Hyp-Asn-Thr-Phe-AzaGly-Leu-Arg(Me)-Trp-NH₂, dimethylaminoethylureido-D-Tyr-Hyp-Asn-Thr-Phe-AzaGly-Leu-Arg(Me) -Trp-NH₂, iso-butylurcido-D-Tyr-Hyp-Asn-Thr-Phc-AzaGly-Lcu-Arg(Mc)-Trp-NH₂, Hyp-Asn-Thr-Phe-AzaGly-Leu-Arg(Me)-Trp-NH₂, or a salt thereof.

4. A metastin derivative represented by formula:
XX0-XX2-XX3-XX4-XX5-T-XX9-XX 10-NH₂
wherein:
XX0 represents formyl, a C₁₋₂₀ alkanoyl, mesyl, an optionally substituted ureido, amidino, cyclopropanecarbonyl, 6-(acetyl-D-arginylamino)caproyl, 6-((R)-2,3-diaminopropionylamino)caproyl, 6-(D-norleucylamino)caproyl, 4-(D-arginylamino)butyryl, 3-(4-hydroxyphenyl)propionyl, glycyl, tyrosyl, acetylglycyl, acetyltyrosyl, D-tyrosyl, acetyl-D-tyrosyl, pyroglutamyl, 3-(pyridin-3-yl)propionyl, adipoyl, glycoloyl or 6-aminocaproyl;
XX2 represents Tyr, D-Tyr, D-Ala, D-Leu, D-Phe, D-Lys, D-Trp or a chemical bond;
XX3 represents D-Asp, D-Dap, D-Ser, D-Gln, D-His, D-NMeAla, D-NMePhe, Aze(2), Pic(2), Pic(3), Hyp, Thz, NMeAla, Gly, Aib, Abz(2), Abz(3), Sar, Leu, Lys, Glu, β-alanine, Pzc(2), Om, His(3Me), Tyr(PO₃H₂), Pro(4NH₂), Hyp(Bzl), Trp, Pro, 4-pyridylalanine, Tic, D-Trp, D-Ala, D-Leu, D-Phe, D-Lys, D-Glu, D-2-pyridylalanine, D-3-pyridylalanine, D-4-pyridylalanine, Aad, Pro(4F) or a chemical bond;
XX4 represents Asn, 2-amino-3-ureidopropionic acid, Nβ-formyldiaminopropionic acid, Nβ-acetyl-diaminopropionic acid, Nω-pentylasparagine, Nω-cyclopropylasparagine, Nω-benzylasparagine, 2,4-diaminobutanoic acid, His, Gln, Cit or D-Asn;
XX5 represents Ser, Thr, Val, NMeSer, Gly, Ala, Hyp, D-Ala, Dap or D-Thr;
T represents formula II: 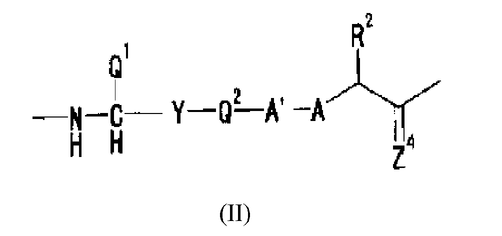
Z⁴ represents hydrogen atom, O or S;
R² represents (1) hydrogen atom or (2) a cyclic or linear C₁₋₁₀ alkyl group, (3) a C₁₋₁₀ alkyl group consisting of a cyclic alkyl group and a linear alkyl group, or (4) a C₁₋₈ alkyl group which may optionally be substituted with a group selected from the group consisting of an optionally substituted carbamoyl group, an optionally substituted hydroxy group and an optionally substituted aromatic cyclic group;
Q¹ represents a C₁₋₄ alkyl group, which may optionally be substituted with a substituent selected from the group consisting of (1) an optionally substituted C₆₋₁₂ aromatic hydrocarbon group, (2) an optionally substituted 5- to 14-membered aromatic heterocyclic group consisting of 1 to 7 carbon atoms and a hetero atom selected from the group consisting of nitrogen atom, oxygen atom and sulfur atom, (3) an optionally substituted C₈₋₁₄ aromatic condensed cyclic group, (4) an optionally substituted 5- to 14-membered aromatic condensed heterocyclic group consisting of 3 to 11 carbon atoms and a hetero atom selected from the group consisting of nitrogen atom, oxygen atom and sulfur atom, (5) an optionally substituted non-aromatic cyclic hydrocarbon group having not greater than 7 carbon atoms and (6) an optionally substituted non-aromatic heterocyclic group having not greater than 7 carbon atoms;
A represents:
(1) a nitrogen atom substituted with hydrogen atom or a C₁₋₃ alkyl group;
(2) a carbon atom substituted with hydrogen atom or a C₁₋₃ alkyl group;
(3) O; or,
(4) S;
A' represents:
(1) a carbon atom which may optionally be substituted with hydrogen atom, O, S, a halogen atom, an optionally halogenated C₁₋₃ alkyl group, carbamoyl or hydroxy;
(2) a nitrogen atom which may optionally be substituted with hydrogen atom or an optionally halogenated C₁₋₃ alkyl group;
(3) O; or,
(4) S;
Q² represents:
(1) CH₂, CO, CS or C=CH₂, which may optionally be substituted with a C₀₋₄ alkyl group optionally substituted with a substituent selected from the group consisting of carbamoyl, hydroxy, a C₁₋₃ alkoxy group, a halogen atom and amino;
(2) NH which may optionally be substituted with a C₁₋₄ alkyl group optionally substituted with a substituent selected from the group consisting of carbamoyl and hydroxy; or,
(3) O;
Y represents:
(1) a group represented by formula: -CONH-, -CSNH-, -CH₂NH-, -NHCO-, -CH₂O-, -COCH₂-, -CH₂S-, -CSCH₂-, -CH₂SO-, -CH₂SO₂-, -COO-, -CSO-, -CH₂CH₂- or -CH=CH-, which may optionally be substituted with a substituent selected from the group consisting of a C₁₋₆ alkyl group, hydroxy and a halogen atom;
(2) an optionally substituted C₆₋₇ aromatic hydrocarbon group;
(3) an optionally substituted 5- to 7-membered aromatic heterocyclic group consisting of 1 to 5 carbon atoms and a hetero atom selected from the group consisting of nitrogen atom, oxygen atom and sulfur atom;
(4) an optionally substituted non-aromatic cyclic hydrocarbon group having not greater than 5 carbon atoms;
(5) an optionally substituted non-aromatic heterocyclic group having not greater than 5 carbon atoms;
provided that when Y is (2), (3), (4) or (5), Q² may be a chemical bond; the bonds between Y-Q², Q²-A' and A'-A each independently represents a single bond or a double bond;
XX9 represents Arg, Om, Arg(Me) or Arg(asymMe₂); and,
XX10 represents Phe, Trp, 2-naphthylalanine, 2-thienylalanine, tyrosine or 4-fluorophenylalanine;
or a salt thereof.

5. A metastin derivative represented by formula:
XX0-XX2-XX3-XX4-XX5-T-XX9-XX 10-NH₂
wherein:
XX0 represents C₁₋₂₀ alkanoyl;
XX2 represents D-Tyr;
XX3 represents Hyp, Glu or Pro(4F);
XX4 represents Asn or 2-amino-3-ureidopropionic acid;
XX5 represents Thr;
T represents formula II: 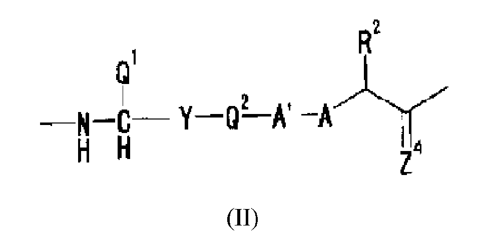
Z⁴ represents O;
R² represents (1) a cyclic or linear C₁₋₁₀ alkyl group, or (2) a C₁₋₁₀ alkyl group consisting of a cyclic alkyl group and a linear alkyl group;
Q¹ represents a C₁₋₄ alkyl group, which may optionally be substituted with a substituent selected from the group consisting of (1) an optionally substituted C₆₋₁₂ aromatic hydrocarbon group and (2) an optionally substituted non-aromatic cyclic hydrocarbon group having not greater than 7 carbon atoms;
A represents:
(1) a nitrogen atom substituted with hydrogen atom or a C₁₋₃ alkyl group;
(2) a carbon atom substituted with hydrogen atom or a C₁₋₃ alkyl group;
(3) O; or,
(4) S;
A' represents:
(1) a carbon atom which may optionally be substituted with hydrogen atom, O, S, a halogen atom, an optionally halogenated C₁₋₃ alkyl group, carbamoyl or hydroxy;
Q² represents:
(1) CH₂, CO, CS or C=CH₂, which may optionally be substituted with a C₀₋₄ alkyl group optionally substituted with a substituent selected from the group consisting of carbamoyl, hydroxy, a C₁₋₃ alkoxy group, a halogen atom and amino;
Y represents:
(1) a group represented by formula: -CONH-, -CSNH-, -CH₂NH- or -NHCO-, which may optionally be substituted with a substituent selected from the group consisting of a C₁₋₆ alkyl group, hydroxy and a halogen atom;
provided that the bonds between Y-Q², Q²-A' and A'-A each independently represents a single bond or a double bond;
XX9 represents Arg or Arg(Mc); and,
XX10 represents Trp;
or a salt thereof.

6. The metastin derivative or a salt thereof according to claim 5, wherein:
XX0 represents a C₁₋₆ alkanoyl (preferably acetyl);
XX2 represents D-Tyr;
XX3 represents Hyp, Glu or Pro(4F);
XX4 represents Asn or 2-amino-3-ureidopropionic acid;
XX5 represents Thr;
T represents formula II: 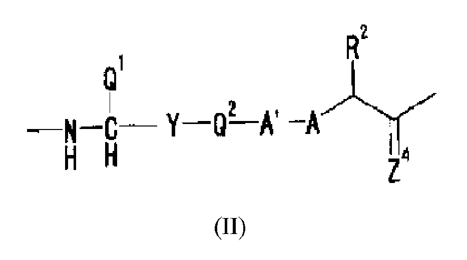
Z⁴ represents O;
R² represents propyl, isopropyl, isobutyl or cyclopropylmethyl;
Q¹ represents benzyl or cyclohexylmethyl;
A represents, -NH-, -CH- or S;
A' represents, -CH-, -CH₂- or -CO-;
Q² represents CH₂, CH(CH₃), CH(CH₂O), C(CH₃)₂, C=CH₂, CH(CH₂CH₃), CH(CH₂NH₂), CH(CH₂OCH₃) or CH(CH(CH₃)₂);
Y represents a group represented by formula: -CONH-, -CSNH-, -NHCO- or -CH₂NH-;
provided that the bonds between Y-Q², Q²-A' and A'-A each independently represents a single bond or a double bond;
XX9 represents Arg or Arg(Me); and,
XX10 represents Trp.

7. A metastin derivative, which is selected from:
Ac-D-Tyr-Hyp-Asn-Thr-D-Cha-Gly-ψ[(E)-CH=CH]-Leu-Arg(Me)-Trp-N H₂,
Ac-D-Tyr-Hyp-Asn-Thr-Cha-Gly-ψ[(E)-CH=CH]-D-Leu-Arg(Me)-Trp-N H₂,
Ac-D-Tyr-Hyp-Asn-Thr-Cha-Gly-ψ[(E)-CMe=CH]-Leu-Arg(Me)-Trp-NH 2,
Ac-D-Tyr-Hyp-Asn-Thr-Cha-Gly-ψ[(E)-CF=CH]-Leu-Arg(Me)-Trp-NH₂,
Ac-D-Tyr-Hyp-Asn-Thr-Phe-ψ(CSNH)-Gly-Leu-Arg(Me)-Trp-NH₂,
Ac-D-Tyr-Hyp-Asn-Thr-Phe-ψ(NHCO)-Gly-Leu-Arg(Me)-Trp-NH₂,
Ac-D-Tyr-Hyp-Asn-Thr-Phe-ψ(CH₂NH)-AzaGly-Leu-Arg(Me)-Trp-NH₂,
Ac-D-Tyr-Hyp-Asn-Thr-Phe-Gly-ψ(CSNH)-Leu-Arg(Me)-Trp-NH₂,
Ac-D-Tyr-Hyp-Asn-Thr-Cha-ψ(CSNH)-Gly-Leu-Arg(Me)-Trp-NH₂ and
Ac-D-Tyr-Hyp-Asn-Thr-Cha-ψ(CSNH)-Gly-Ala(cPr)- Arg(Me)-Trp-NH₂, or a salt thereof.

8. A prodrug of the metastin derivative or a salt thereof according to claims 1 through 7.

9. A pharmaceutical comprising the metastin derivative or a salt thereof according to claims 1 through 7, or a prodrug thereof.

10. The pharmaceutical according to claim 9, which is a cancer metastasis-inhibiting agent or a cancer proliferation-inhibiting agent.

11. The pharmaceutical according to claim 9, which is an agent for preventing or treating cancer.

12. The pharmaceutical according to claim 9, which is an agent for controlling placental function.

13. The pharmaceutical according to claim 9, which is an agent for preventing or treating choriocarcinoma, hydatid mole, invasive mole, miscarriage, fetal hypoplasia, abnormal glucose metabolism, abnormal lipid metabolism or induction of delivery

14. The pharmaceutical according to claim 9, which is an agent for improving gonadal function.

15. The pharmaceutical according to claim 9, which is an agent for preventing or treating hormone-dependent cancer, infertility, endometriosis, early puberty or myoma of the uterus.

16. The pharmaceutical according to claim 9, which is an agent for inducing or stimulating ovulation.

17. The pharmaceutical according to claim 9, which is an agent for promoting gonadotropic hormone secretion or promoting sex hormone secretion.

18. The pharmaceutical according to claim 9, which is an agent for preventing or treating Alzheimer's disease, moderate cognitive impairment or autism.

19. The pharmaceutical according to claim 15, which is a down-regulating agent for gonadotropic hormone or sex hormone.

20. The pharmaceutical according to claim 15, which is a down-regulating agent for human OT7T175 (metastin receptor) protein consisting of the amino acid sequence represented by SEQ ID NO: 9.

21. A method of preventing or treating cancer, which comprises administering to a mammal an effective dose of the metastin derivative or a salt thereof according to claims 1 through 7 or the prodrug according to claim 8.

22. Use of the metastin derivative or a salt thereof according to claims 1 through 7 or the prodrug according to claim 8 to manufacture an agent for preventing or treating cancer.


SEQUENCE LISTING

<110> Takeda Pharmaceutical Company Limited

<120> Metastin Derivatives And Its Use

<130> PCT09-0024

<150> JP 2008-114480
<151> 2008-04-24

<160> 22

<210> 1
<211> 54
<212> PRT
<213> Homo sapiens

<400> 1
Gly Thr Ser Leu Ser Pro Pro Pro Glu Ser Ser Gly Ser Arg Gln Gln
1               5                   10                  15
Pro Gly Leu Ser Ala Pro His Ser Arg Gln Ile Pro Ala Pro Gln Gly
            20                  25                  30
Ala Val Leu Val Gln Arg Glu Lys Asp Leu Pro Asn Tyr Asn Trp Asn
        35                  40                  45
Ser Phe Gly Leu Arg Phe
    50

<210> 2
<211> 162
<212> DNA
<213> Homo sapiens

<400> 2
ggtacttctc tgtctccgcc gccggaatct tctggttctc gtcagcagcc gggtctgtct     60
gctccgcact ctcgtcagat cccggctccg cagggtgctg ttctggttca gcgtgaaaaa    120
gacctgccga actacaactg gaactctttc ggtctgcgtt tc                       162

<210> 3
<211> 152
<212> PRT
<213> Mus musculus

<400> 3

Met Tyr Leu Arg Phe Gly Val Asp Val Cys Ser Leu Ser Pro Trp Lys
                5                   10                  15
Glu Thr Val Asp Leu Pro Leu Pro Pro Arg Met Ile Ser Met Ala Ser
            20                  25                  30
Trp Gln Leu Leu Leu Leu Leu Cys Val Ala Thr Tyr Gly Glu Pro Leu
        35                  40                  45
Ala Lys Val Ala Pro Gly Ser Thr Gly Gln Gln Ser Gly Pro Gln Glu
    50                  55                  60
Leu Val Asn Ala Trp Glu Lys Glu Ser Arg Tyr Ala Glu Ser Lys Pro
65                  70                  75                  80
Gly Ser Ala Gly Leu Arg Ala Arg Arg Ser Ser Pro Cys Pro Pro Val
                85                  90                  95
Glu Gly Pro Ala Gly Arg Gln Arg Pro Leu Cys Ala Ser Arg Ser Arg
            100                 105                 110
Leu Ile Pro Ala Pro Arg Gly Ala Val Leu Val Gln Arg Glu Lys Asp
        115                 120                 125
Leu Ser Thr Tyr Asn Trp Asn Ser Phe Gly Leu Arg Tyr Gly Arg Arg
    130                 135                 140
Gln Ala Ala Arg Ala Ala Arg Gly
145                 150

<210> 4
<211> 456
<212> DNA
<213> Mus musculus

<400> 4
atgtatctga gatttggcgt tgatgtctgc agcctgagtc cctggaagga gactgtagac   60
ctgccccttc ctcccagaat gatctcaatg gcttcttggc agctgctgct tctcctctgt  120
gtcgccacct atggggagcc gctggcaaaa gtgaagcctg gatccacagg ccagcagtcc  180
ggaccccagg aactcgttaa tgcctgggaa aaggaatcgc ggtatgcaga gagcaagcct  240
gggtctgcag ggctgcgcgc tcgtaggtcg tcgccatgcc cgccggttga gggccccgcg  300
gggcgccagc ggcccctgtg tgcctcccgc agtcgcctga tccctgcgcc ccgcggagcg  360
gtgctggtgc agcgggagaa ggacctgtcc acctacaact ggaactcctt cggcctgcgc  420
tacggcagga ggcaggcggc gcgggcagca cggggc                            456

<210> 5
<211> 156
<212> PRT
<213> Mus musculus

<400> 5
Met Tyr Leu Arg Phe Gly Val Asp Val Cys Ser Leu Ser Pro Trp Lys
                5                   10                  15

Glu Thr Val Asp Leu Pro Leu Pro Pro Arg Met Ile Ser Met Ala Ser
            20                  25                  30
Trp Gln Leu Leu Leu Leu Leu Cys Val Ala Thr Tyr Gly Glu Pro Leu
        35                  40                  45
Ala Lys Val Ala Pro Leu Val Lys Pro Gly Ser Thr Gly Gln Gln Ser
    50                  55                  60
Gly Pro Gln Glu Leu Val Asn Ala Trp Glu Lys Glu Ser Arg Tyr Ala
65                  70                  75                  80
Glu Ser Lys Pro Gly Ser Ala Gly Leu Arg Ala Arg Arg Ser Ser Pro
                85                  90                  95
Cys Pro Pro Val Glu Gly Pro Ala Gly Arg Gln Arg Pro Leu Cys Ala
            100                 105                 110
Ser Arg Ser Arg Leu Ile Pro Ala Pro Arg Gly Ala Val Leu Val Gln
        115                 120                 125
Arg Glu Lys Asp Leu Ser Thr Tyr Asn Trp Asn Ser Phe Gly Leu Arg
    130                 135                 140
Tyr Gly Arg Arg Gln Ala Ala Arg Ala Ala Arg Gly
145                 150                 155

<210> 6
<211> 468
<212> DNA
<213> Mus musculus

<400> 6
atgtatctga gatttggcgt tgatgtctgc agcctgagtc cctggaagga gactgtagac 60
ctgccccttc ctcccagaat gatctcaatg gcttcttggc agctgctgct tctcctctgt 120
gtcgccacct atggggagcc gctggcaaaa gtggcacctt tggtgaagcc tggatccaca 180
ggccagcagt ccggacccca ggaactcgtt aatgcctggg aaaaggaatc gcggtatgca 240
gagagcaagc ctgggtctgc agggctgcgc gctcgtaggt cgtcgccatg cccgccggtt 300
gagggccccg cggggcgcca gcggcccctg tgtgcctccc gcagtcgcct gatccctgcg 360
ccccgcggag cggtgctggt gcagcgggag aaggacctgt ccacctacaa ctggaactcc 420
ttcggcctgc gctacggcag gaggcaggcg gcgcgggcag cacggggc 468

<210> 7
<211> 130
<212> PRT
<213> Rattus sp.

<400> 7
Met Thr Ser Leu Ala Ser Trp Gln Leu Leu Leu Leu Leu Cys Val Ala
                5                   10                  15
Ser Phe Gly Glu Pro Leu Ala Lys Met Ala Pro Val Val Asn Pro Glu
            20                  25                  30

Pro Thr Gly Gln Gln Ser Gly Pro Gln Glu Leu Val Asn Ala Trp Gln
        35                  40                  45
Lys Gly Pro Arg Tyr Ala Glu Ser Lys Pro Gly Ala Ala Gly Leu Arg
    50                  55                  60
Ala Arg Arg Thr Ser Pro Cys Pro Pro Val Glu Asn Pro Thr Gly His
65                  70                  75                  80
Gln Arg Pro Pro Cys Ala Thr Arg Ser Arg Leu Ile Pro Ala Pro Arg
                85                  90                  95
Gly Ser Val Leu Val Gln Arg Glu Lys Asp Met Ser Ala Tyr Asn Trp
            100                 105                 110
Asn Ser Phe Gly Leu Arg Tyr Gly Arg Arg Gln Val Ala Arg Ala Ala
        115                 120                 125
Arg Gly
    130

<210> 8
<211> 390
<212> DNA
<213> Rattus sp.

<400> 8
atgacctcgc tggcttcttg gcagctgctg cttctcctct gtgtggcctc ttttggggag 60
ccactggcaa aaatggcacc tgtggtgaac cctgaaccca caggccaaca gtccggaccc 120
caggaactcg ttaatgcctg gcaaaagggc ccgcggtatg cagagagcaa gcctggggct 180
gcaggactgc gcgctcgccg aacatcgcca tgcccgccgg tggagaaccc cacggggcac 240
cagcggcccc cgtgtgccac ccgcagtcgc ctgatccctg cgccccgcgg atcggtgctg 300
gtgcagcgcg agaaggacat gtcagcctac aactggaact cctttggcct gcgctacggc 360
aggaggcagg tggcgcgggc ggcacggggc 390

<210> 9
<211> 398
<212> PRT
<213> Homo sapiens

<400> 9
Met His Thr Val Ala Thr Ser Gly Pro Asn Ala Ser Trp Gly Ala Pro
                5                   10                  15
Ala Asn Ala Ser Gly Cys Pro Gly Cys Gly Ala Asn Ala Ser Asp Gly
            20                  25                  30
Pro Val Pro Ser Pro Arg Ala Val Asp Ala Trp Leu Val Pro Leu Phe
        35                  40                  45
Phe Ala Ala Leu Met Leu Leu Gly Leu Val Gly Asn Ser Leu Val Ile
    50                  55                  60
Tyr Val Ile Cys Arg His Lys Pro Met Arg Thr Val Thr Asn Phe Tyr

65                  70                  75                  80
Ile Ala Asn Leu Ala Ala Thr Asp Val Thr Phe Leu Leu Cys Cys Val
                85                  90                  95
Pro Phe Thr Ala Leu Leu Tyr Pro Leu Pro Gly Trp Val Leu Gly Asp
            100                 105                 110
Phe Met Cys Lys Phe Val Asn Tyr Ile Gln Gln Val Ser Val Gln Ala
        115                 120                 125
Thr Cys Ala Thr Leu Thr Ala Met Ser Val Asp Arg Trp Tyr Val Thr
    130                 135                 140
Val Phe Pro Leu Arg Ala Leu His Arg Arg Thr Pro Arg Leu Ala Leu
145                 150                 155                 160
Ala Val Ser Leu Ser Ile Trp Val Gly Ser Ala Ala Val Ser Ala Pro
                165                 170                 175
Val Leu Ala Leu His Arg Leu Ser Pro Gly Pro Arg Ala Tyr Cys Ser
            180                 185                 190
Glu Ala Phe Pro Ser Arg Ala Leu Glu Arg Ala Phe Ala Leu Tyr Asn
        195                 200                 205
Leu Leu Ala Leu Tyr Leu Leu Pro Leu Leu Ala Thr Cys Ala Cys Tyr
    210                 215                 220
Ala Ala Met Leu Arg His Leu Gly Arg Val Ala Val Arg Pro Ala Pro
225                 230                 235                 240
Ala Asp Ser Ala Leu Gln Gly Gln Val Leu Ala Glu Arg Ala Gly Ala
                245                 250                 255
Val Arg Ala Lys Val Ser Arg Leu Val Ala Ala Val Val Leu Leu Phe
            260                 265                 270
Ala Ala Cys Trp Gly Pro Ile Gln Leu Phe Leu Val Leu Gln Ala Leu
        275                 280                 285
Gly Pro Ala Gly Ser Trp His Pro Arg Ser Tyr Ala Ala Tyr Ala Leu
    290                 295                 300
Lys Thr Trp Ala His Cys Met Ser Tyr Ser Asn Ser Ala Leu Asn Pro
305                 310                 315                 320
Leu Leu Tyr Ala Phe Leu Gly Ser His Phe Arg Gln Ala Phe Arg Arg
                325                 330                 335
Val Cys Pro Cys Ala Pro Arg Arg Pro Arg Arg Pro Arg Arg Pro Gly
            340                 345                 350
Pro Ser Asp Pro Ala Ala Pro His Ala Glu Leu His Arg Leu Gly Ser
        355                 360                 365
His Pro Ala Pro Ala Arg Ala Gln Lys Pro Gly Ser Ser Gly Leu Ala
    370                 375                 380
Ala Arg Gly Leu Cys Val Leu Gly Glu Asp Asn Ala Pro Leu
385                 390                 395

<210> 10
<211> 1194

<212> DNA
<213> Homo sapiens

<400> 10
atgcacaccg tggctacgtc cggacccaac gcgtcctggg gggcaccggc caacgcctcc      60
ggctgcccgg gctgtggcgc caacgcctcg gacggcccag tcccttcgcc gcgggccgtg     120
gacgcctggc tcgtgccgct cttcttcgcg gcgctgatgc tgctgggcct ggtggggaac     180
tcgctggtca tctacgtcat ctgccgccac aagccgatgc ggaccgtgac caacttctac     240
atcgccaacc tggcggccac ggacgtgacc ttcctcctgt gctgcgtccc cttcacggcc     300
ctgctgtacc cgctgcccgg ctgggtgctg ggcgacttca tgtgcaagtt cgtcaactac     360
atccagcagg tctcggtgca ggccacgtgt gccactctga ccgccatgag tgtggaccgc     420
tggtacgtga cggtgttccc gttgcgcgcc ctgcaccgcc gcacgccccg cctggcgctg     480
gctgtcagcc tcagcatctg ggtaggctct gcggcggtgt ctgcgccggt gctcgccctg     540
caccgcctgt cacccgggcc gcgcgcctac tgcagtgagg ccttccccag ccgcgccctg     600
gagcgcgcct tcgcactgta caacctgctg gcgctgtacc tgctgccgct gctcgccacc     660
tgcgcctgct atgcggccat gctgcgccac ctgggccggg tcgccgtgcg ccccgcgccc     720
gccgatagcg ccctgcaggg gcaggtgctg gcagagcgcg caggcgccgt gcgggccaag     780
gtctcgcggc tggtggcggc cgtggtcctg ctcttcgccg cctgctgggg ccccatccag     840
ctgttcctgg tgctgcaggc gctgggcccc gcgggctcct ggcacccacg cagctacgcc     900
gcctacgcgc ttaagacctg ggctcactgc atgtcctaca gcaactccgc gctgaacccg     960
ctgctctacg ccttcctggg ctcgcacttc cgacaggcct tccgccgcgt ctgcccctgc    1020
gcgccgcgcc gcccccgccg cccccgccgg cccggaccct cggaccccgc agccccacac    1080
gcggagctgc accgcctggg gtcccacccg gcccccgcca gggcgcagaa gccagggagc    1140
agtgggctgg ccgcgcgcgg gctgtgcgtc ctgggggagg acaacgcccc tctc          1194

<210> 11
<211> 396
<212> PRT
<213> Rattus sp.

<400> 11
Met Ala Ala Glu Ala Thr Leu Gly Pro Asn Val Ser Trp Trp Ala Pro
                5                   10                  15
Ser Asn Ala Ser Gly Cys Pro Gly Cys Gly Val Asn Ala Ser Asp Gly
            20                  25                  30
Pro Gly Ser Ala Pro Arg Pro Leu Asp Ala Trp Leu Val Pro Leu Phe
        35                  40                  45
Phe Ala Ala Leu Met Leu Leu Gly Leu Val Gly Asn Ser Leu Val Ile
    50                  55                  60
Phe Val Ile Cys Arg His Lys His Met Gln Thr Val Thr Asn Phe Tyr
65                  70                  75                  80
Ile Ala Asn Leu Ala Ala Thr Asp Val Thr Phe Leu Leu Cys Cys Val
                85                  90                  95
Pro Phe Thr Ala Leu Leu Tyr Pro Leu Pro Thr Trp Val Leu Gly Asp

100                 105                 110
Phe Met Cys Lys Phe Val Asn Tyr Ile Gln Gln Val Ser Val Gln Ala
        115                 120                 125
Thr Cys Ala Thr Leu Thr Ala Met Ser Val Asp Arg Trp Tyr Val Thr
    130                 135                 140
Val Phe Pro Leu Arg Ala Leu His Arg Arg Thr Pro Arg Leu Ala Leu
145                 150                 155                 160
Thr Val Ser Leu Ser Ile Trp Val Gly Ser Ala Ala Val Ser Ala Pro
                165                 170                 175
Val Leu Ala Leu His Arg Leu Ser Pro Gly Pro His Thr Tyr Cys Ser
            180                 185                 190
Glu Ala Phe Pro Ser Arg Ala Leu Glu Arg Ala Phe Ala Leu Tyr Asn
        195                 200                 205
Leu Leu Ala Leu Tyr Leu Leu Pro Leu Leu Ala Thr Cys Ala Cys Tyr
    210                 215                 220
Gly Ala Met Leu Arg His Leu Gly Arg Ala Ala Val Arg Pro Ala Pro
225                 230                 235                 240
Thr Asp Gly Ala Leu Gln Gly Gln Leu Leu Ala Gln Arg Ala Gly Ala
                245                 250                 255
Val Arg Thr Lys Val Ser Arg Leu Val Ala Ala Val Val Leu Leu Phe
            260                 265                 270
Ala Ala Cys Trp Gly Pro Ile Gln Leu Phe Leu Val Leu Gln Ala Leu
        275                 280                 285
Gly Pro Ser Gly Ala Trp His Pro Arg Ser Tyr Ala Ala Tyr Ala Leu
    290                 295                 300
Lys Ile Trp Ala His Cys Met Ser Tyr Ser Asn Ser Ala Leu Asn Pro
305                 310                 315                 320
Leu Leu Tyr Ala Phe Leu Gly Ser His Phe Arg Gln Ala Phe Cys Arg
                325                 330                 335
Val Cys Pro Cys Gly Pro Gln Arg Gln Arg Arg Pro His Ala Ser Ala
            340                 345                 350
His Ser Asp Arg Ala Ala Pro His Ser Val Pro His Ser Arg Ala Ala
        355                 360                 365
His Pro Val Arg Val Arg Thr Pro Glu Pro Gly Asn Pro Val Val Arg
    370                 375                 380
Ser Pro Ser Val Gln Asp Glu His Thr Ala Pro Leu
385                 390                 395

<210> 12
<211> 1188
<212> DNA
<213> Rattus sp.

<400> 12
   atggccgcag aggcgacgtt gggtccgaac gtgagctggt gggctccgtc caacgcttcg 60
   ggatgcccgg gctgcggtgt caatgcctcg gatggcccag gctccgcgcc aaggcccctg 120
   gatgcctggc tggtgcccct gtttttcgct gccctaatgt tgctggggct agtcgggaac 180
   tcactggtca tcttcgttat ctgccgccac aagcacatgc agaccgtcac caatttctac 240
   atcgctaacc tggcggccac agatgtcact ttccttctgt gctgcgtacc cttcaccgcg 300
   ctcctctatc cgctgcccac ctgggtgctg ggagacttca tgtgcaaatt cgtcaactac 360
   atccagcagg tctcggtgca agccacatgt gccactttga cagccatgag tgtggaccgc 420
   tggtacgtga ctgtgttccc gctgcgtgca cttcaccgcc gcactccgcg cctggccctg 480
   actgtcagcc ttagcatctg ggtgggttcc gcagctgttt ccgccccggt gctggctctg 540
   caccgcctgt cgcccgggcc tcacacctac tgcagtgagg cgtttcccag ccgtgccctg 600
   gagcgcgctt tcgcgctcta caacctgctg gccctatacc tgctgccgct gctcgccacc 660
   tgcgcctgct acggtgccat gctgcgccac ctgggccgcg ccgctgtacg ccccgcaccc 720
   actgatggcg ccctgcaggg gcagctgcta gcacagcgcg ctggagcagt gcgcaccaag 780
   gtctcccggc tggtggccgc tgtcgtcctg ctcttcgccg cctgctgggg cccgatccag 840
   ctgttcctgg tgcttcaagc cctgggcccc tcgggggcct ggcaccctcg aagctatgcc 900
   gcctacgcgc tcaagatctg ggctcactgc atgtcctaca gcaattctgc gctcaacccg 960
   ctgctctatg ccttcctggg ttcccacttc agacaggcct tctgccgcgt gtgcccctgc 1020
   ggcccgcaac gccagcgtcg gccccacgcg tcagcgcact cggaccgagc cgcaccccat 1080
   agtgtgccgc acagccgggc tgcgcaccct gtccgggtca ggacccccga gcctgggaac 1140
   cctgtggtgc gctcgccctc tgttcaggat gaacacactg ccccactc 1188

<210> 13
<211> 396
<212> PRT
<213> Mus musculus

<400> 13
Met Ala Thr Glu Ala Thr Leu Ala Pro Asn Val Thr Trp Trp Ala Pro
1               5                   10                  15
Ser Asn Ala Ser Gly Cys Pro Gly Cys Gly Val Asn Ala Ser Asp Asp
            20                  25                  30
Pro Gly Ser Ala Pro Arg Pro Leu Asp Ala Trp Leu Val Pro Leu Phe
        35                  40                  45
Phe Ala Thr Leu Met Leu Leu Gly Leu Val Gly Asn Ser Leu Val Ile
    50                  55                  60
Tyr Val Ile Cys Arg His Lys His Met Gln Thr Val Thr Asn Phe Tyr
65                  70                  75                  80
Ile Ala Asn Leu Ala Ala Thr Asp Val Thr Phe Leu Leu Cys Cys Val
                85                  90                  95
Pro Phe Thr Ala Leu Leu Tyr Pro Leu Pro Ala Trp Val Leu Gly Asp
            100                 105                 110
Phe Met Cys Lys Phe Val Asn Tyr Ile Gln Gln Val Ser Val Gln Ala
        115                 120                 125
Thr Cys Ala Thr Leu Thr Ala Met Ser Val Asp Arg Trp Tyr Val Thr

130                 135                 140
Val Phe Pro Leu Arg Ala Leu His Arg Arg Thr Pro Arg Leu Ala Leu
145                 150                 155                 160
Ala Val Ser Leu Ser Ile Trp Val Gly Ser Ala Ala Val Ser Ala Pro
                165                 170                 175
Val Leu Ala Leu His Arg Leu Ser Pro Gly Pro Arg Thr Tyr Cys Ser
            180                 185                 190
Glu Ala Phe Pro Ser Arg Ala Leu Glu Arg Ala Phe Ala Leu Tyr Asn
        195                 200                 205
Leu Leu Ala Leu Tyr Leu Leu Pro Leu Leu Ala Thr Cys Ala Cys Tyr
    210                 215                 220
Gly Ala Met Leu Arg His Leu Gly Arg Ala Ala Val Arg Pro Ala Pro
225                 230                 235                 240
Thr Asp Gly Ala Leu Gln Gly Gln Leu Leu Ala Gln Arg Ala Gly Ala
                245                 250                 255
Val Arg Thr Lys Val Ser Arg Leu Val Ala Ala Val Val Leu Leu Phe
            260                 265                 270
Ala Ala Cys Trp Gly Pro Ile Gln Leu Phe Leu Val Leu Gln Ala Leu
        275                 280                 285
Gly Pro Ser Gly Ala Trp His Pro Arg Ser Tyr Ala Ala Tyr Ala Val
    290                 295                 300
Lys Ile Trp Ala His Cys Met Ser Tyr Ser Asn Ser Ala Leu Asn Pro
305                 310                 315                 320
Leu Leu Tyr Ala Phe Leu Gly Ser His Phe Arg Gln Ala Phe Cys Arg
                325                 330                 335
Val Cys Pro Cys Cys Arg Gln Arg Gln Arg Arg Pro His Thr Ser Ala
            340                 345                 350
His Ser Asp Arg Ala Ala Thr His Thr Val Pro His Ser Arg Ala Ala
        355                 360                 365
His Pro Val Arg Ile Arg Ser Pro Glu Pro Gly Asn Pro Val Val Arg
    370                 375                 380
Ser Pro Cys Ala Gln Ser Glu Arg Thr Ala Ser Leu
385                 390                 395

<210> 14
<211> 1188
<212> DNA
<213> Mus musculus

<400> 14
atggccaccg aggcgacatt ggctcccaat gtgacctggt gggctccgtc caacgcttca    60
ggatgcccag gctgcggtgt caacgcctcg gatgacccag gctctgcgcc aaggcccctg   120
gatgcctggc tggttcccct gtttttcgct acactcatgt tgcttgggct ggtcggaaac   180
tcattggtca tctacgttat ctgccgccac aagcacatgc agacagttac caacttctac   240

atcgctaacc tggctgccac agacgtcact ttcctactgt gctgcgtgcc cttcaccgca 300
ctcctctacc cgctgcccgc ctgggtgctg ggagacttca tgtgcaaatt cgtcaactac 360
atccagcagg tctcggtgca agccacatgt gccactctga cggccatgag tgtggaccgc 420
tggtatgtga ctgtgttccc gctgcgtgca cttcaccgcc gcactccgcg cctggccctg 480
gctgtcagcc tcagcatctg ggtggggtca gcagctgtgt ccgccccggt gctggccctg 540
caccgcctgt cgccagggcc tcgcacctac tgcagcgagg cgtttcccag ccgcgccctg 600
gagcgcgcct tcgcgctcta caacctgctg gctctatatc tgctgccgct gctcgccacc 660
tgcgcctgct acggcgccat gctgcgccac ctgggccgtg cggctgtacg ccccgcaccc 720
actgacggcg ccctgcaggg acagctgcta gcacagcgcg ccggagcagt gcgcaccaag 780
gtctcccggc tggtggccgc tgtcgtcctg ctcttcgccg cctgctgggg cccgatccag 840
ctgttcctgg tgcttcaagc cctgggcccc tcgggggcct ggcaccctcg aagctatgcc 900
gcctacgcgg tcaagatctg ggctcactgc atgtcctaca gcaactcggc gctcaatccg 960
ctgctctatg ccttcctggg ttcacacttc agacaggcct tctgccgcgt gtgcccctgc 1020
tgccggcaac gccagcgccg gccccacacg tcagcgcact cggaccgagc tgcaactcac 1080
actgtgccgc acagccgtgc tgcgcaccct gtgcggatca ggagcccgga gcctgggaac 1140
cctgtggtgc gctcgccctg cgctcagagt gaacgcactg cctcactc 1188

<210> 15
<211> 15
<212> PRT
<213> Artificial

<220>
<223> the C-terminus of the polypeptide is amide (-CONH2) form

<400> 15
Lys Asp Leu Pro Asn Tyr Asn Trp Asn Ser Phe Gly Leu Arg Phe
1               5                   10                  15

<210> 16
<211> 10
<212> PRT
<213> Artificial

<220>
<223> the C-terminus of the polypeptide is amide (-CONH2) form

<400> 16
Tyr Asn Trp Asn Ser Phe Gly Leu Arg Phe
1               5                   10

<210> 17
<211> 9
<212> PRT

<213> Artificial

<220>
<223> the C-terminus of the polypeptide is amide (-CONH2) form

<400> 17
Asn Trp Asn Ser Phe Gly Leu Arg Phe
1               5                   9

<210> 18
<211> 8
<212> PRT
<213> Artificial

<220>
<223> the C-terminus of the polypeptide is amide (-CONH2) form

<400> 18
Trp Asn Ser Phe Gly Leu Arg Phe
1               5           8

<210> 19
<211> 45
<212> DNA
<213> Homo sapiens

<400> 19
aaggacctgc cgaactacaa ctggaactcc ttcggcctgc gcttc                45

<210> 20
<211> 30
<212> DNA
<213> Homo sapiens

<400> 20
tacaactgga actccttcgg cctgcgcttc                                 30

<210> 21
<211> 27
<212> DNA
<213> Homo sapiens

<400> 21
aactggaact ccttcggcct gcgcttc                                    27

<210> 22
<211> 24
<212> DNA
<213> Homo sapiens

<400> 22
tggaactcct tcggcctgcg cttc                                                24